# EUROPEAN PATENT APPLICATION

(11) **EP 3 875 590 A1**
(43) Date of publication of application: **08.09.2021**
(21) Application number: 18938531.3
(22) Date of filing: 30.10.2018
(51) Int. Cl.: C12N 15/60, C12N 9/88, C12P 7/18, C12P 7/42, C12P 7/50, C12P 11/00, C12P 13/00

(54) **METHOD FOR PRODUCING ORGANIC COMPOUND AND CORYNEFORM BACTERIUM**

(71) Applicant: Green Earth Institute Co., Ltd., Tokyo 113-0033 (JP)
(72) Inventor: YAMAMOTO, Keisuke, Chiba 292-0818 (JP); TSUCHISAKA, Atsunari, Chiba 292-0818 (JP); NAKANE, Shuhei, Chiba 292-0818 (JP); NAKAYASHIKI, Toru, Chiba 292-0818 (JP)
(74) Representative: Becker, Eberhard
(86) International application number: PCT/JP2018/040415
(87) International publication number: WO 2020/090016

(57) **Abstract**

A method for producing an α-keto acid includes a step of culturing a microorganism containing a gene encoding an enzyme that catalyzes an aldol reaction between pyruvic acid and a carbonyl compound in the presence of a pyruvic acid-supplying compound selected from pyruvic acid and saccharides, and a carbonyl compound selected from an aldehyde and a ketone to produce an α-keto acid.

SEQUENCE LISTING

<110>  Green Earth Institute Co., Ltd.

<120>  Method for producing organic compounds and coryneform bacteria

<130>  18F0400PCT

<160>  147

<170>  PatentIn version 3.5

<210>  1
<211>  789
<212>  DNA
<213>  Escherichia coli

<400>  1
atggaaaaca gttttaaagc ggcgctgaaa gcaggccgtc cgcagattgg attatggctg      60

gggctgagca gcagctacag cgcggagtta ctggccggag caggattcga ctggttgttg     120

atcgacggtg agcacgcacc gaacaacgta caaaccgtgc tcacccagct acaggcgatt     180

gcgccctatc ccagccagcc ggtagtacgt ccgtcgtgga acgatccggt gcaaatcaaa     240

caactgctgg acgtcggcac acaaacctta ctggtgccga tggtacaaaa cgccgacgaa     300

gcccgtgaag cggtacgcgc cacccgttat ccccccgccg gtattcgcgg tgtgggcagt     360

gcgctggctc gcgcctcgcg ctggaatcgc attcctgatt acctgcaaaa agccaacgat     420

caaatgtgcg tgctggtgca gatcgaaacg cgtgaggcaa tgaagaactt accgcagatt     480

ctggacgtgg aaggcgtcga cggcgtgttt atcggcccgg cggatctgag cgccgatatg     540

ggttatgccg gtaatccgca gcacccggaa gtacaggccg ccattgagca ggcgatcgtg     600

cagatccgcg aagcgggcaa agcgccgggg atcctgatcg ccaatgagct actggcaaaa     660

cgctatctgg aactgggcgc gctgtttgtc gccgtcggcg ttgacaccac cctgctcgcc     720

cgcgccgccg aagcgctggc agcacggttt ggcgcgcagg ctacagcgat taagcccggc     780

gtgtattaa                                                             789


<210>  2
<211>  262
<212>  PRT
<213>  Escherichia coli

<400>  2

Met Glu Asn Ser Phe Lys Ala Ala Leu Lys Ala Gly Arg Pro Gln Ile
1               5                   10                  15


Gly Leu Trp Leu Gly Leu Ser Ser Ser Tyr Ser Ala Glu Leu Leu Ala
            20                  25                  30


Gly Ala Gly Phe Asp Trp Leu Leu Ile Asp Gly Glu His Ala Pro Asn

35                  40                  45

Asn Val Gln Thr Val Leu Thr Gln Leu Gln Ala Ile Ala Pro Tyr Pro
    50                  55                  60

Ser Gln Pro Val Val Arg Pro Ser Trp Asn Asp Pro Val Gln Ile Lys
65                  70                  75                  80

Gln Leu Leu Asp Val Gly Thr Gln Thr Leu Leu Val Pro Met Val Gln
                85                  90                  95

Asn Ala Asp Glu Ala Arg Glu Ala Val Arg Ala Thr Arg Tyr Pro Pro
            100                 105                 110

Ala Gly Ile Arg Gly Val Gly Ser Ala Leu Ala Arg Ala Ser Arg Trp
        115                 120                 125

Asn Arg Ile Pro Asp Tyr Leu Gln Lys Ala Asn Asp Gln Met Cys Val
    130                 135                 140

Leu Val Gln Ile Glu Thr Arg Glu Ala Met Lys Asn Leu Pro Gln Ile
145                 150                 155                 160

Leu Asp Val Glu Gly Val Asp Gly Val Phe Ile Gly Pro Ala Asp Leu
                165                 170                 175

Ser Ala Asp Met Gly Tyr Ala Gly Asn Pro Gln His Pro Glu Val Gln
            180                 185                 190

Ala Ala Ile Glu Gln Ala Ile Val Gln Ile Arg Glu Ala Gly Lys Ala
        195                 200                 205

Pro Gly Ile Leu Ile Ala Asn Glu Leu Leu Ala Lys Arg Tyr Leu Glu
    210                 215                 220

Leu Gly Ala Leu Phe Val Ala Val Gly Val Asp Thr Thr Leu Leu Ala
225                 230                 235                 240

Arg Ala Ala Glu Ala Leu Ala Ala Arg Phe Gly Ala Gln Ala Thr Ala
                245                 250                 255

Ile Lys Pro Gly Val Tyr
            260

<210>  3
<211>  804
<212>  DNA
<213>  Escherichia coli

<400>  3
atgaacgcat tattaagcaa tccctttaaa gaacgtttac gcaagggcga agtgcaaatt      60

ggtctgtggt taagctcaac gactgcctat atggcagaaa ttgccgccac ttctggttat     120

gactggttgc tgattgacgg ggagcacgcg ccaaacacca ttcaggatct ttatcatcag     180

ctacaggcgg tagcgcccta tgccagccaa cccgtgatcc gtccggtgga aggcagtaaa     240

ccgctgatta aacaagtcct ggatattggc gcgcaaactc tactgatccc gatggtcgat     300

actgccgaac aggcacgtca ggtggtgtct gccacgcgct atcctcccta cggtgagcgt     360

ggtgtcgggg ccagtgtggc acggctgcgg cgctggggac gcattgagaa ttacatggcg     420

caagttaacg attcgctttg tctgttggtg caggtggaaa gtaaaacggc actggataac     480

ctggacgaaa tcctcgacgt cgaagggatt gatggcgtgt ttattggacc tgcggatctt     540

tctgcgtcgt tgggctaccc ggataacgcc gggcacccgg aagtgcagcg aattattgaa     600

accagtattc ggcggatccg tgctgcgggt aaagcggctg gttttctggc tgtggctcct     660

gatatggcgc agcaatgcct ggcgtgggga gcgaactttg tcgctgttgg cgttgacacg     720

atgctctaca gcgatgccct ggatcaacga ctggcgatgt ttaaatcagg caaaaatggg     780

ccacgcataa aaggtagtta ttaa                                            804

<210>  4
<211>  267
<212>  PRT
<213>  Escherichia coli

<400>  4

Met Asn Ala Leu Leu Ser Asn Pro Phe Lys Glu Arg Leu Arg Lys Gly
1               5                   10                  15

Glu Val Gln Ile Gly Leu Trp Leu Ser Ser Thr Thr Ala Tyr Met Ala
            20                  25                  30

Glu Ile Ala Ala Thr Ser Gly Tyr Asp Trp Leu Leu Ile Asp Gly Glu
        35                  40                  45

His Ala Pro Asn Thr Ile Gln Asp Leu Tyr His Gln Leu Gln Ala Val
    50                  55                  60

Ala Pro Tyr Ala Ser Gln Pro Val Ile Arg Pro Val Glu Gly Ser Lys
65                  70                  75                  80

Pro Leu Ile Lys Gln Val Leu Asp Ile Gly Ala Gln Thr Leu Leu Ile
                85                  90                  95

Pro Met Val Asp Thr Ala Glu Gln Ala Arg Gln Val Val Ser Ala Thr

            100                 105                 110

Arg Tyr Pro Pro Tyr Gly Glu Arg Gly Val Gly Ala Ser Val Ala Arg
        115                 120                 125

Ala Ala Arg Trp Gly Arg Ile Glu Asn Tyr Met Ala Gln Val Asn Asp
    130                 135                 140

Ser Leu Cys Leu Leu Val Gln Val Glu Ser Lys Thr Ala Leu Asp Asn
145                 150                 155                 160

Leu Asp Glu Ile Leu Asp Val Glu Gly Ile Asp Gly Val Phe Ile Gly
                165                 170                 175

Pro Ala Asp Leu Ser Ala Ser Leu Gly Tyr Pro Asp Asn Ala Gly His
            180                 185                 190

Pro Glu Val Gln Arg Ile Ile Glu Thr Ser Ile Arg Arg Ile Arg Ala
        195                 200                 205

Ala Gly Lys Ala Ala Gly Phe Leu Ala Val Ala Pro Asp Met Ala Gln
    210                 215                 220

Gln Cys Leu Ala Trp Gly Ala Asn Phe Val Ala Val Gly Val Asp Thr
225                 230                 235                 240

Met Leu Tyr Ser Asp Ala Leu Asp Gln Arg Leu Ala Met Phe Lys Ser
                245                 250                 255

Gly Lys Asn Gly Pro Arg Ile Lys Gly Ser Tyr
            260                 265

<210> 5
<211> 894
<212> DNA
<213> Escherichia coli

<400> 5
atggcaacga atttacgtgg cgtaatggct gcactcctga ctccttttga ccaacaacaa 60

gcactggata aagcgagtct gcgtcgcctg gttcagttca atattcagca gggcatcgac 120

ggtttatacg tgggtggttc gaccggcgag gcctttgtac aaagcctttc cgagcgtgaa 180

caggtactgg aaatcgtcgc cgaagaggcg aaaggtaaga ttaaactcat cgcccacgtc 240

ggttgcgtca gcaccgccga aagccaacaa cttgcggcat cggctaaacg ttatggcttc 300

gatgccgtct ccgccgtcac gccgttctac tatcctttca gctttgaaga acactgcgat 360

cactatcggg caattattga ttcggcggat ggtttgccga tggtggtgta caacattcca 420

gccctgagtg gggtaaaact gaccctggat cagatcaaca cacttgttac attgcctggc 480

gtaggtgcgc tgaaacagac ctctggcgat ctctatcaga tggagcagat ccgtcgtgaa 540

catcctgatc ttgtgctcta taacggttac gacgaaatct tcgcctctgg tctgctggcg 600

ggcgctgatg gtggtatcgg cagtacctac aacatcatgg gctggcgcta tcaggggatc 660

gttaaggcgc tgaaagaagg cgatatccag accgcgcaga aactgcaaac tgaatgcaat 720

aaagtcattg atttactgat caaaacgggc gtattccgcg gcctgaaaac tgtcctccat 780

tatatggatg tcgtttctgt gccgctgtgc cgcaaaccgt ttggaccggt agatgaaaaa 840

tatctgccag aactgaaggc gctggcccag cagttgatgc aagagcgcgg gtaa 894

<210> 6
<211> 297
<212> PRT
<213> Escherichia coli

<400> 6

Met Ala Thr Asn Leu Arg Gly Val Met Ala Ala Leu Leu Thr Pro Phe
1               5                   10                  15

Asp Gln Gln Gln Ala Leu Asp Lys Ala Ser Leu Arg Arg Leu Val Gln
            20                  25                  30

Phe Asn Ile Gln Gln Gly Ile Asp Gly Leu Tyr Val Gly Gly Ser Thr
        35                  40                  45

Gly Glu Ala Phe Val Gln Ser Leu Ser Glu Arg Glu Gln Val Leu Glu
    50                  55                  60

Ile Val Ala Glu Glu Ala Lys Gly Lys Ile Lys Leu Ile Ala His Val
65                  70                  75                  80

Gly Cys Val Ser Thr Ala Glu Ser Gln Gln Leu Ala Ala Ser Ala Lys
                85                  90                  95

Arg Tyr Gly Phe Asp Ala Val Ser Ala Val Thr Pro Phe Tyr Tyr Pro
            100                 105                 110

Phe Ser Phe Glu Glu His Cys Asp His Tyr Arg Ala Ile Ile Asp Ser
        115                 120                 125

Ala Asp Gly Leu Pro Met Val Val Tyr Asn Ile Pro Ala Leu Ser Gly
    130                 135                 140

Val Lys Leu Thr Leu Asp Gln Ile Asn Thr Leu Val Thr Leu Pro Gly
145                 150                 155                 160

Val Gly Ala Leu Lys Gln Thr Ser Gly Asp Leu Tyr Gln Met Glu Gln
                165                 170                 175

Ile Arg Arg Glu His Pro Asp Leu Val Leu Tyr Asn Gly Tyr Asp Glu
            180                 185                 190

Ile Phe Ala Ser Gly Leu Leu Ala Gly Ala Asp Gly Gly Ile Gly Ser
        195                 200                 205

Thr Tyr Asn Ile Met Gly Trp Arg Tyr Gln Gly Ile Val Lys Ala Leu
    210                 215                 220

Lys Glu Gly Asp Ile Gln Thr Ala Gln Lys Leu Gln Thr Glu Cys Asn
225                 230                 235                 240

Lys Val Ile Asp Leu Leu Ile Lys Thr Gly Val Phe Arg Gly Leu Lys
                245                 250                 255

Thr Val Leu His Tyr Met Asp Val Val Ser Val Pro Leu Cys Arg Lys
            260                 265                 270

Pro Phe Gly Pro Val Asp Glu Lys Tyr Leu Pro Glu Leu Lys Ala Leu
        275                 280                 285

Ala Gln Gln Leu Met Gln Glu Arg Gly
    290                 295

<210>  7
<211>  642
<212>  DNA
<213>  Escherichia coli

<400>  7
atgaaaaact ggaaaacaag tgcagaatca atcctgacca ccggcccggt tgtaccggtt      60

atcgtggtaa aaaaactgga acacgcggtg ccgatggcaa aagcgttggt tgctggtggg     120

gtgcgcgttc tggaagtgac tctgcgtacc gagtgtgcag ttgacgctat ccgtgctatc     180

gccaaagaag tgcctgaagc gattgtgggt gccggtacgg tgctgaatcc acagcagctg     240

gcagaagtca ctgaagcggg tgcacagttc gcaattagcc cgggtctgac cgagccgctg     300

ctgaaagctg ctaccgaagg gactattcct ctgattccgg ggatcagcac tgtttccgaa     360

ctgatgctgg gtatggacta cggtttgaaa gagttcaaat tcttcccggc tgaagctaac     420

ggcggcgtga aagccctgca ggcgatcgcg ggtccgttct cccaggtccg tttctgcccg     480

acgggtggta tttctccggc taactaccgt gactacctgg cgctgaaaag cgtgctgtgc     540

atcggtggtt cctggctggt tccggcagat gcgctggaag cgggcgatta cgaccgcatt     600

actaagctgg cgcgtgaagc tgtagaaggc gctaagctgt aa                    642

<210>  8
<211>  213
<212>  PRT
<213>  Escherichia coli

<400>  8

Met Lys Asn Trp Lys Thr Ser Ala Glu Ser Ile Leu Thr Thr Gly Pro
1               5                   10                  15

Val Val Pro Val Ile Val Val Lys Lys Leu Glu His Ala Val Pro Met
            20                  25                  30

Ala Lys Ala Leu Val Ala Gly Gly Val Arg Val Leu Glu Val Thr Leu
        35                  40                  45

Arg Thr Glu Cys Ala Val Asp Ala Ile Arg Ala Ile Ala Lys Glu Val
    50                  55                  60

Pro Glu Ala Ile Val Gly Ala Gly Thr Val Leu Asn Pro Gln Gln Leu
65                  70                  75                  80

Ala Glu Val Thr Glu Ala Gly Ala Gln Phe Ala Ile Ser Pro Gly Leu
                85                  90                  95

Thr Glu Pro Leu Leu Lys Ala Ala Thr Glu Gly Thr Ile Pro Leu Ile
            100                 105                 110

Pro Gly Ile Ser Thr Val Ser Glu Leu Met Leu Gly Met Asp Tyr Gly
        115                 120                 125

Leu Lys Glu Phe Lys Phe Phe Pro Ala Glu Ala Asn Gly Gly Val Lys
    130                 135                 140

Ala Leu Gln Ala Ile Ala Gly Pro Phe Ser Gln Val Arg Phe Cys Pro
145                 150                 155                 160

Thr Gly Gly Ile Ser Pro Ala Asn Tyr Arg Asp Tyr Leu Ala Leu Lys
                165                 170                 175

Ser Val Leu Cys Ile Gly Gly Ser Trp Leu Val Pro Ala Asp Ala Leu
            180                 185                 190

Glu Ala Gly Asp Tyr Asp Arg Ile Thr Lys Leu Ala Arg Glu Ala Val
        195                 200                 205

Glu Gly Ala Lys Leu

    210

<210> 9
<211> 906
<212> DNA
<213> Escherichia coli

<400> 9
atgaaaaaat tcagcggcat tattccaccg gtatccagca cgtttcatcg tgacggaacc 60

cttgataaaa aggcaatgcg cgaagttgcc gacttcctga ttaataaagg ggtcgacggg 120

ctgttttatc tgggtaccgg tggtgaattt agccaaatga atacagccca gcgcatggca 180

ctcgccgaag aagctgtaac cattgtcgac gggcgagtgc cggtattgat tggcgtcggt 240

tccccttcca ctgacgaagc ggtcaaactg gcgcagcatg cgcaagccta cggcgctgat 300

ggtatcgtcg ccatcaaccc ctactactgg aaagtcgcac cacgaaatct tgacgactat 360

taccagcaga tcgcccgtag cgtcacccta ccggtgatcc tgtacaactt tccgaatctg 420

acgggtcagg acttaacccc ggaaaccgtg acgcgtctgg ctctgcaaaa cgagaatatc 480

gttggcatca aagacaccat cgacagcgtt ggtcacttgc gtacgatgat caacacagtt 540

aagtcggtac gcccgtcgtt ttcggtattc tgcggttacg atgatcattt gctgaatacg 600

atgctgctga gcggcgacgg tgcgataacc gccagcgcta actttgctcc ggaactctcc 660

gtcggcatct accgcgcctg gcgtgaaggc gatctggcga ccgctgcgac gctgaataaa 720

aaactactac aactgcccgc tatttacgcc ctcgaaacac cgtttgtctc actgatcaaa 780

tacagcatgc agtgtgtcgg gctgcctgta gagacatatt gcttaccacc gattcttgaa 840

gcatctgaag aagcaaaaga taaagtccac gtgctgctta ccgcgcaggg cattttacca 900

gtctaa 906

<210> 10
<211> 301
<212> PRT
<213> Escherichia coli

<400> 10

Met Lys Lys Phe Ser Gly Ile Ile Pro Pro Val Ser Ser Thr Phe His
1               5                   10                  15

Arg Asp Gly Thr Leu Asp Lys Lys Ala Met Arg Glu Val Ala Asp Phe
            20                  25                  30

Leu Ile Asn Lys Gly Val Asp Gly Leu Phe Tyr Leu Gly Thr Gly Gly
        35                  40                  45

Glu Phe Ser Gln Met Asn Thr Ala Gln Arg Met Ala Leu Ala Glu Glu
    50                  55                  60

Ala Val Thr Ile Val Asp Gly Arg Val Pro Val Leu Ile Gly Val Gly
65                  70                  75                  80

Ser Pro Ser Thr Asp Glu Ala Val Lys Leu Ala Gln His Ala Gln Ala
                85                  90                  95

Tyr Gly Ala Asp Gly Ile Val Ala Ile Asn Pro Tyr Tyr Trp Lys Val
            100                 105                 110

Ala Pro Arg Asn Leu Asp Asp Tyr Tyr Gln Gln Ile Ala Arg Ser Val
        115                 120                 125

Thr Leu Pro Val Ile Leu Tyr Asn Phe Pro Asp Leu Thr Gly Gln Asp
    130                 135                 140

Leu Thr Pro Glu Thr Val Thr Arg Leu Ala Leu Gln Asn Glu Asn Ile
145                 150                 155                 160

Val Gly Ile Lys Asp Thr Ile Asp Ser Val Gly His Leu Arg Thr Met
                165                 170                 175

Ile Asn Thr Val Lys Ser Val Arg Pro Ser Phe Ser Val Phe Cys Gly
            180                 185                 190

Tyr Asp Asp His Leu Leu Asn Thr Met Leu Leu Gly Gly Asp Gly Ala
        195                 200                 205

Ile Thr Ala Ser Ala Asn Phe Ala Pro Glu Leu Ser Val Gly Ile Tyr
    210                 215                 220

Arg Ala Trp Arg Glu Gly Asp Leu Ala Thr Ala Ala Thr Leu Asn Lys
225                 230                 235                 240

Lys Leu Leu Gln Leu Pro Ala Ile Tyr Ala Leu Glu Thr Pro Phe Val
                245                 250                 255

Ser Leu Ile Lys Tyr Ser Met Gln Cys Val Gly Leu Pro Val Glu Thr
            260                 265                 270

Tyr Cys Leu Pro Pro Ile Leu Glu Ala Ser Glu Glu Ala Lys Asp Lys
        275                 280                 285

Val His Val Leu Leu Thr Ala Gln Gly Ile Leu Pro Val
    290                 295                 300

<210>  11

<211>  618
<212>  DNA
<213>  Escherichia coli

<400>  11
atgcagtggc aaactaaact cccgctgatc gccattttgc gcggtattac gcccgacgag      60

gcgctggcgc atgttggcgc ggtgattgac gccgggttcg acgcggttga aatcccgctg     120

aattccccac aatgggagca aagcattccc gccatcgttg atgcgtacgg cgacaaggcg     180

ttgattggcg caggtacggt actgaaacct gaacaggtcg atgcgctcgc caggatgggc     240

tgtcagctca tcgttacgcc caatatccat agtgaagtga tccgccgtgc ggtgggctac     300

ggcatgaccg tctgccccgg ctgcgcgacg gcgaccgaag cctttaccgc gctcgaagcg     360

ggcgcgcagg cgctgaaaat atttccgtca tcggcttttg gtccgcaata catcaaagcg     420

ttaaaagcgg tattgccatc ggacatcgca gtctttgccg ttggcggcgt gacgccagaa     480

aacctggcgc agtggataga cgcaggttgt gcaggggcgg gcttaggcag cgatctctat     540

cgcgccgggc aatccgtaga gcgcaccgcg cagcaggcag cagcatttgt taaggcgtat     600

cgagaggcag tgcaataa                                                   618


<210>  12
<211>  205
<212>  PRT
<213>  Escherichia coli

<400>  12

Met Gln Trp Gln Thr Lys Leu Pro Leu Ile Ala Ile Leu Arg Gly Ile
1               5                   10                  15


Thr Pro Asp Glu Ala Leu Ala His Val Gly Ala Val Ile Asp Ala Gly
            20                  25                  30


Phe Asp Ala Val Glu Ile Pro Leu Asn Ser Pro Gln Trp Glu Gln Ser
        35                  40                  45


Ile Pro Ala Ile Val Asp Ala Tyr Gly Asp Lys Ala Leu Ile Gly Ala
    50                  55                  60


Gly Thr Val Leu Lys Pro Glu Gln Val Asp Ala Leu Ala Arg Met Gly
65                  70                  75                  80


Cys Gln Leu Ile Val Thr Pro Asn Ile His Ser Glu Val Ile Arg Arg
                85                  90                  95


Ala Val Gly Tyr Gly Met Thr Val Cys Pro Gly Cys Ala Thr Ala Thr
            100                 105                 110

Glu Ala Phe Thr Ala Leu Glu Ala Gly Ala Gln Ala Leu Lys Ile Phe
        115                 120                 125

Pro Ser Ser Ala Phe Gly Pro Gln Tyr Ile Lys Ala Leu Lys Ala Val
    130                 135                 140

Leu Pro Ser Asp Ile Ala Val Phe Ala Val Gly Gly Val Thr Pro Glu
145                 150                 155                 160

Asn Leu Ala Gln Trp Ile Asp Ala Gly Cys Ala Gly Ala Gly Leu Gly
                165                 170                 175

Ser Asp Leu Tyr Arg Ala Gly Gln Ser Val Glu Arg Thr Ala Gln Gln
            180                 185                 190

Ala Ala Ala Phe Val Lys Ala Tyr Arg Glu Ala Val Gln
        195                 200                 205

<210>  13
<211>  1014
<212>  DNA
<213>  Escherichia coli

<400>  13
atgaacggta aaaaacttta tatctcggac gtcacattgc gtgacggtat gcacgccatt      60

cgtcatcagt attcgctgga aaacgttcgc cagattgcca aagcactgga cgatgcccgc     120

gtggattcga ttgaagtggc ccacggcgac ggtttgcaag gttccagctt taactatggt     180

ttcggcgcac atagcgacct tgaatggatt gaagcggcgg cggatgtggt gaagcacgcc     240

aaaatcgcga cgttgttgct gccaggaatc ggcactattc acgatctgaa aaatgcctgg     300

caggctggcg cgcgggtggt tcgtgtggca acgcactgta ccgaagctga tgtttccgcc     360

cagcatattc agtatgcccg cgagctcgga atggacaccg ttggttttct gatgatgagc     420

catatgacca cgccggagaa tctcgccaag caggcaaagc tgatggaagg ctacggtgcg     480

acctgtattt atgtggtgga ttctggcggt gcgatgaaca tgagcgatat ccgtgaccgt     540

ttccgcgccc tgaaagcaga gctgaaacca gaaacgcaaa ctggcatgca cgctcaccat     600

aacctgagtc ttggcgtggc gaactctatc gcggcggtgg aagagggctg cgaccgaatc     660

gacgccagcc tcgcgggaat gggcgcgggc gcaggtaacg caccgctgga agtgtttatt     720

gccgccgcgg ataaactggg ctggcagcat gggaccgatc tctatgcgtt aatggatgcc     780

gccgacgacc tggtgcgtcc gttgcaggat cgaccggtac gagtcgatcg cgaaacgctg     840

gcgctgggat acgctggtgt ttactcgagc ttcctgcgtc actgtgaaac ggcggcggcg     900

cgttatggct taagtgcggt ggatattctc gttgagctgg gcaaacgccg gatggttggc     960

ggccaggagg atatgatcgt tgacgtggcg ctggatctgc gcaacaacaa ataa          1014

<210>  14
<211>  337
<212>  PRT
<213>  Escherichia coli

<400>  14

Met Asn Gly Lys Lys Leu Tyr Ile Ser Asp Val Thr Leu Arg Asp Gly
1               5                   10                  15

Met His Ala Ile Arg His Gln Tyr Ser Leu Glu Asn Val Arg Gln Ile
            20                  25                  30

Ala Lys Ala Leu Asp Asp Ala Arg Val Asp Ser Ile Glu Val Ala His
        35                  40                  45

Gly Asp Gly Leu Gln Gly Ser Ser Phe Asn Tyr Gly Phe Gly Ala His
    50                  55                  60

Ser Asp Leu Glu Trp Ile Glu Ala Ala Ala Asp Val Val Lys His Ala
65                  70                  75                  80

Lys Ile Ala Thr Leu Leu Leu Pro Gly Ile Gly Thr Ile His Asp Leu
                85                  90                  95

Lys Asn Ala Trp Gln Ala Gly Ala Arg Val Val Arg Val Ala Thr His
            100                 105                 110

Cys Thr Glu Ala Asp Val Ser Ala Gln His Ile Gln Tyr Ala Arg Glu
        115                 120                 125

Leu Gly Met Asp Thr Val Gly Phe Leu Met Met Ser His Met Thr Thr
    130                 135                 140

Pro Glu Asn Leu Ala Lys Gln Ala Lys Leu Met Glu Gly Tyr Gly Ala
145                 150                 155                 160

Thr Cys Ile Tyr Val Val Asp Ser Gly Gly Ala Met Asn Met Ser Asp
                165                 170                 175

Ile Arg Asp Arg Phe Arg Ala Leu Lys Ala Glu Leu Lys Pro Glu Thr
            180                 185                 190

Gln Thr Gly Met His Ala His His Asn Leu Ser Leu Gly Val Ala Asn
        195                 200                 205

Ser Ile Ala Ala Val Glu Glu Gly Cys Asp Arg Ile Asp Ala Ser Leu
    210                 215                 220

Ala Gly Met Gly Ala Gly Ala Gly Asn Ala Pro Leu Glu Val Phe Ile
225                 230                 235                 240

Ala Ala Ala Asp Lys Leu Gly Trp Gln His Gly Thr Asp Leu Tyr Ala
                245                 250                 255

Leu Met Asp Ala Ala Asp Asp Leu Val Arg Pro Leu Gln Asp Arg Pro
            260                 265                 270

Val Arg Val Asp Arg Glu Thr Leu Ala Leu Gly Tyr Ala Gly Val Tyr
        275                 280                 285

Ser Ser Phe Leu Arg His Cys Glu Thr Ala Ala Ala Arg Tyr Gly Leu
    290                 295                 300

Ser Ala Val Asp Ile Leu Val Glu Leu Gly Lys Arg Arg Met Val Gly
305                 310                 315                 320

Gly Gln Glu Asp Met Ile Val Asp Val Ala Leu Asp Leu Arg Asn Asn
                325                 330                 335

Lys

<210>  15
<211>  771
<212>  DNA
<213>  Escherichia coli

<400>  15
atgaataacg atgttttccc gaataaattc aaagccgcac tggctgcgaa acaggtacaa      60

attggttgct ggtcagcact ctctaacccg attagcactg aagttcttgg tttggctggg     120

tttgactggc tggtgctgga tggcgaacat gcgccaaacg atatctccac gtttattccg     180

cagttaatgg ccttgaaagg cagcgccagc gcgccagtag tgcgagtgcc gaccaacgag     240

ccggtaatta ttaagcgtct tctggatatc ggtttctata acttcctgat tccttttgta     300

gaaacaaaag aggaagcaga gctggcggtg gcatcaaccc gttacccacc ggaaggcatt     360

cgcggcgtct ccgtttctca ccgcgccaat atgtttggca ccgtggcgga ttatttcgct     420

cagtcgaaca agaacatcac tattctggtc cagatagaaa gtcagcaggg cgtagataac     480

gtcgatgcca ttgccgctac cgaaggcgta gacggcatct tcgtcggccc cagcgatctg     540

gccgcggcat taggccatct cggcaatgca tcacacccgg atgtacaaaa agcaattcag     600

cacattttta accgtgccag cgcgcacggc aaacccagcg gtatcctcgc gccggtcgaa     660

gccgatgcgc gtcgttatct ggaatggggc gcgacgtttg tggctgtcgg cagcgatctc     720

ggcgtcttcc gctctgccac tcagaaactg gctgatacct ttaaaaaata a 771

<210> 16
<211> 256
<212> PRT
<213> Escherichia coli

<400> 16

<210>  17
<211>  717
<212>  DNA
<213>  Pseudomonas putida

<400>  17
atgagtacgc tcgtgggaaa aaccggcgtt gtccttcgca acatcgagcg agcgggcgcg      60

ggcctggtgg acgaactggc ggcgtacggt gttgccacca tccacgaggc acaagggcgc      120

aaagggttgc tggcgccctc gatatcggcg attcagcagg gcacttctgt cagcggctcg      180

gccgtcactg tgctggtggc gcccggggat aactggatgt tccacgttgc tgttgaacaa      240

tgccgtccta gcgatgtgct ggtagtggca cccagctctc cctgtaccga cggctacttt      300

ggtgacctgc tcgcaacctc gctcaaggcg catggtgttc gtgcgctggt gatcgatgcc      360

ggggtgcgcg atacccgtac ccttcggcaa atgggcttcc ccgtatggtc acgggcggtc      420

agcgcgcaag gcaccatcaa ggaaacactt gggtcggtca atctaccgat catttgcggc      480

gggcagctgg tcaaccccgg tgacgtggtc gtggcggatg atgacggtgt ggttattgtg      540

cgtcgcgatg aggtgacgca ggtgctggag gcgacccgtc ggcgtgctga tctggaagag      600

cagaaggcgt tgcgcctggc cagcggcgaa ctcggtctgg acatctacaa catgcgtccc      660

cgcttggccg agaaggggtt acggtacatc gacagcctgg atgaactggg agagtaa        717

<210>  18
<211>  238
<212>  PRT
<213>  Pseudomonas putida

<400>  18

Met Ser Thr Leu Val Gly Lys Thr Gly Val Val Leu Arg Asn Ile Glu
1               5                   10                  15

Arg Ala Gly Ala Gly Leu Val Asp Glu Leu Ala Ala Tyr Gly Val Ala
            20                  25                  30

Thr Ile His Glu Ala Gln Gly Arg Lys Gly Leu Leu Ala Pro Ser Ile
        35                  40                  45

Ser Ala Ile Gln Gln Gly Thr Ser Val Ser Gly Ser Ala Val Thr Val

50                  55                  60

Leu Val Ala Pro Gly Asp Asn Trp Met Phe His Val Ala Val Glu Gln
65                  70                  75                  80

Cys Arg Pro Ser Asp Val Leu Val Val Ala Pro Ser Ser Pro Cys Thr
                85                  90                  95

Asp Gly Tyr Phe Gly Asp Leu Leu Ala Thr Ser Leu Lys Ala His Gly
            100                 105                 110

Val Arg Ala Leu Val Ile Asp Ala Gly Val Arg Asp Thr Arg Thr Leu
        115                 120                 125

Arg Gln Met Gly Phe Pro Val Trp Ser Arg Ala Val Ser Ala Gln Gly
    130                 135                 140

Thr Ile Lys Glu Thr Leu Gly Ser Val Asn Leu Pro Ile Ile Cys Gly
145                 150                 155                 160

Gly Gln Leu Val Asn Pro Gly Asp Val Val Val Ala Asp Asp Asp Gly
                165                 170                 175

Val Val Ile Val Arg Arg Asp Glu Val Thr Gln Val Leu Glu Ala Thr
            180                 185                 190

Arg Arg Arg Ala Asp Leu Glu Glu Gln Lys Ala Leu Arg Leu Ala Ser
        195                 200                 205

Gly Glu Leu Gly Leu Asp Ile Tyr Asn Met Arg Pro Arg Leu Ala Glu
    210                 215                 220

Lys Gly Leu Arg Tyr Ile Asp Ser Leu Asp Glu Leu Gly Glu
225                 230                 235

<210>  19
<211>  999
<212>  DNA
<213>  Nocardioides sp.

<400>  19
atgacgtcac ccgccgtgac atccgccgac attacagggc tcgttggcat cgtgccgacg      60

ccctcgaagc cgggctcgga agcgccggac gctgtagaca ccgtcgacct cgacgagacc     120

gcgcgcatgg tcgaactcat cgtcgcttcc ggtgttgacg tgctgctgac caatggcacc     180

ttcggcgaag tcgctactct cacttatgaa gaactactgg cgttcaacga cacggtcatc     240

cgtacggtcg ctaatcgcat acccgtcttc tgcggggcct cgacgctgaa cacccgcgac     300

acgatcgccc gcagcctcgc cctaatgggg ctcggtgcga acggtctctt cgtcgggcga 360

ccaatgtggc tacctctcga cgacgagcag ctggtgtcct actacgcggc cgtgtgcgat 420

gccgttccgg ctgccgctgt cgtcgtctac gacaacactg gagtgttcaa gggcaaaatc 480

tcctccgccg cgtacgcggc gctggccgag atcccgcaga tcgtggcatc gaagcatctg 540

ggcgtactct cgggcagcga tgcctatgcg agcgacctgg ccgctgtaaa aggccgcttt 600

cctctgttgc ccaccgcgga caattggctc ccctcactcg aggcattccc cggcgaagtg 660

ccggcggctt ggagcggtga tgtggcctgc ggccccgaac ccgtgatggc gctccgccgc 720

gcgatcgcgg agggactctg ggacgacgcc cgtgccgtgc atgaagacat cgcatgggcc 780

acggagcccc tgttcccggg cggcgatata agtaagttca tgccatacag catccagatc 840

gaccgggccg agttcgaggc cgccggttac atcgtgcccg gccccagccg gcatccctac 900

ggcaccgccc cagccgccta cctcgaaggg ggcgccgagg tcggccgccg ttgggcgggc 960

attcgccaga agtacgtcgc aactctcgcc gaaccataa 999

<210> 20
<211> 332
<212> PRT
<213> Nocardioides sp.

<400> 20

<210>  21
<211>  1041
<212>  DNA
<213>  Burkholderia xenovorans

<400>  21
atgaagctag aaggcaaaaa agtcaccgtc cacgacatga cgctgcgcga cggcatgcac        60

cccaagcgcc accagatgac gctggagcaa atgaaatcca tcgcctgcgg cctggatgcc       120

gcgggcatcc cgctgatcga agtcacccac ggcgacggcc tgggcggctc ctccgtcaac 180

tacggctttc cggcgcacag cgacgaggaa tacctgggcg ccgtgattcc gttgatgaag 240

caggccaagg tcagtgccct gctcttgccc ggcatcggca ccgtcgaaca cctgaagatg 300

gccaaggacc tgggcgtgaa caccatccgc gtggccaccc actgcaccga agccgatgtg 360

tcggagcagc acatcaccca atcgcgcaag ctgggtctgg acaccgtggg ctttttgatg 420

atggcgcaca tggccagccc agaaaagctg gtcagccagg cactcttgat gcaaggctac 480

ggcgccaact gcatctacgt caccgactcg gccggctaca tgctgcctga cgacgtgaaa 540

gcgcgcctga gtgccgtgcg tgccgcgctc aaacccgaaa ccgagttggg ctttcacggc 600

catcacaacc tggccatggg cgtcgccaac tcgatcgccg cgatcgaggc cggggccacc 660

cgcatcgatg ccgctgccgc tggcctgggt gccggcgccg gcaacacgcc gatggaggtg 720

ttcatcgccg tatgcgcgcg catggggatc gaaaccggag tcgatgtgtt caagatccag 780

gacgtggccg aagacctggt ggtgccgatc atggaccatg tcatccgcat cgaccgcgac 840

tcgctcacgc tgggctatgc cggggtctat tccagctttc tgctgtttgc caaacgcgcc 900

agtgcgaagt acggcgtgcc ggcgcgcgac atcctggtcg agctgggacg gcgcggcatg 960

gtcggcggcc aggaagacat gattgaagac accgccatga ccatggcacg tgagcgcggg 1020

ttgaccttga ccgccgcata a 1041

<210> 22
<211> 346
<212> PRT
<213> Burkholderia xenovorans

<400> 22

Met Lys Leu Glu Gly Lys Lys Val Thr Val His Asp Met Thr Leu Arg
1               5                   10                  15

Asp Gly Met His Pro Lys Arg His Gln Met Thr Leu Glu Gln Met Lys
            20                  25                  30

Ser Ile Ala Cys Gly Leu Asp Ala Ala Gly Ile Pro Leu Ile Glu Val
        35                  40                  45

Thr His Gly Asp Gly Leu Gly Gly Ser Ser Val Asn Tyr Gly Phe Pro
    50                  55                  60

Ala His Ser Asp Glu Glu Tyr Leu Gly Ala Val Ile Pro Leu Met Lys
65                  70                  75                  80

Gln Ala Lys Val Ser Ala Leu Leu Leu Pro Gly Ile Gly Thr Val Glu
                85                  90                  95

His Leu Lys Met Ala Lys Asp Leu Gly Val Asn Thr Ile Arg Val Ala
        100                 105                 110

Thr His Cys Thr Glu Ala Asp Val Ser Glu Gln His Ile Thr Gln Ser
    115                 120                 125

Arg Lys Leu Gly Leu Asp Thr Val Gly Phe Leu Met Met Ala His Met
    130                 135                 140

Ala Ser Pro Glu Lys Leu Val Ser Gln Ala Leu Leu Met Gln Gly Tyr
145                 150                 155                 160

Gly Ala Asn Cys Ile Tyr Val Thr Asp Ser Ala Gly Tyr Met Leu Pro
                165                 170                 175

Asp Asp Val Lys Ala Arg Leu Ser Ala Val Arg Ala Ala Leu Lys Pro
            180                 185                 190

Glu Thr Glu Leu Gly Phe His Gly His His Asn Leu Ala Met Gly Val
        195                 200                 205

Ala Asn Ser Ile Ala Ala Ile Glu Ala Gly Ala Thr Arg Ile Asp Ala
    210                 215                 220

Ala Ala Ala Gly Leu Gly Ala Gly Ala Gly Asn Thr Pro Met Glu Val
225                 230                 235                 240

Phe Ile Ala Val Cys Ala Arg Met Gly Ile Glu Thr Gly Val Asp Val
                245                 250                 255

Phe Lys Ile Gln Asp Val Ala Glu Asp Leu Val Val Pro Ile Met Asp
            260                 265                 270

His Val Ile Arg Ile Asp Arg Asp Ser Leu Thr Leu Gly Tyr Ala Gly
        275                 280                 285

Val Tyr Ser Ser Phe Leu Leu Phe Ala Lys Arg Ala Ser Ala Lys Tyr
    290                 295                 300

Gly Val Pro Ala Arg Asp Ile Leu Val Glu Leu Gly Arg Arg Gly Met
305                 310                 315                 320

Val Gly Gly Gln Glu Asp Met Ile Glu Asp Thr Ala Met Thr Met Ala
                325                 330                 335

Arg Glu Arg Gly Leu Thr Leu Thr Ala Ala
            340                 345

<210>  23
<211>  1587
<212>  DNA
<213>  Pseudomonas putida

<400>  23
atggcttcgg tacacggcac cacatacgaa ctcttgcgac gtcaaggcat cgatacggtc      60

ttcggcaatc ctggctcgaa cgagctcccg tttttgaagg actttccaga ggactttcga     120

tacatcctgg ctttgcagga agcgtgtgtg gtgggcattg cagacggcta tgcgcaagcc     180

agtcggaagc cggctttcat taacctgcat tctgctgctg gtaccggcaa tgctatgggt     240

gcactcagta acgcctggaa ctcacattcc ccgctgatcg tcactgccgg ccagcagacc     300

agggcgatga ttggcgttga agctctgctg accaacgtcg atgccgccaa cctgccacga     360

ccacttgtca aatggagcta cgagcccgca agcgcagcag aagtccctca tgcgatgagc     420

agggctatcc atatggcaag catggcgcca caaggccctg tctatctttc ggtgccatat     480

gacgattggg ataaggatgc tgatcctcag tcccaccacc tttttgatcg ccatgtcagt     540

tcatcagtac gcctgaacga ccaggatctc gatattctgg tgaaagctct caacagcgca     600

tccaacccgg cgatcgtcct gggcccggac gtcgacgcag caaatgcgaa cgcagactgc     660

gtcatgttgg ccgaacgcct caaagctccg gtttgggttg cgccatccgc tccacgctgc     720

ccattcccta cccgtcatcc ttgcttccgt ggattgatgc cagctggcat cgcagcgatt     780

tctcagctgc tcgaaggtca cgatgtggtt ttggtaatcg gcgctccagt gttccgttac     840

caccaatacg acccaggtca atatctcaaa cctggcacgc gattgatttc ggtgacctgc     900

gacccgctcg aagctgcacg cgcgccaatg ggcgatgcga tcgtggcaga cattggtgcg     960

atggctagcg ctcttgccaa cttggttgaa gagagcagcc gccagctccc aactgcagct    1020

ccggaacccg cgaaggttga ccaagacgct ggccgacttc acccagagac agtgttcgac    1080

acactgaacg acatggcccc ggagaatgcg atttacctga acgagtcgac ttcaacgacc    1140

gcccaaatgt ggcagcgcct gaacatgcgc aaccctggta gctactactt ctgtgcagct    1200

ggcggactgg gcttcgccct gcctgcagca attggcgttc aactcgcaga acccgagcga    1260

caagtcatcg ccgtcattgg cgacggatcg gcgaactaca gcattagtgc gttgtggact    1320

gcagctcagt acaacatccc cactatcttc gtgatcatga acaacggcac ctacggtgcg    1380

ttgcgatggt ttgccggcgt tctcgaagca gaaaacgttc ctgggctgga tgtgccaggg    1440

atcgacttcc gcgcactcgc caagggctat ggtgtccaag cgctgaaagc cgacaacctt    1500

gagcagctca agggttcgct acaagaagcg ctttctgcca aaggcccggt acttatcgaa    1560

gtaagcaccg taagcccggt gaagtga                                        1587

<210>  24
<211>  528
<212>  PRT
<213>  Pseudomonas putida

<400>  24

<210>  25
<211>  1164
<212>  DNA
<213>  Klebsiella pneumoniae

<400>  25
   atgagctatc gtatgttcga ttatctggtg ccaaacgtta acttttttgg ccccaacgcc      60
   atttccgtag tcggcgaacg ctgccagctg ctggggggga aaaaagccct gctggtcacc     120
   gacaaaggcc tgcgggcaat taaagatggc gcggtggaca aaaccctgca ttatctgcgg     180
   gaggccggga tcgaggtggc gatctttgac ggcgtcgagc cgaacccgaa agacaccaac     240
   gtgcgcgacg gcctcgctgt gtttcgccgc gaacagtgcg acatcatcgt caccgtgggc     300
   ggcggcagcc cgcacgattg cggcaaaggc atcggcatcg ccgccaccca tgagggcgat     360
   ctgtaccagt atgccggaat cgagaccctg accaacccgc tgccgcctat cgtcgcggtc     420
   aataccaccg ccggcaccgc cagcgaggtc acccgccact gcgtcctgac caacaccgaa     480
   accaaagtga agtttgtgat cgtcagctgg cgcaacctgc cgtcggtctc tatcaacgat     540
   ccactgctga tgatcggtaa accggccgcc ctgaccgcgg cgaccgggat ggatgccctg     600
   acccacgccg tagaggccta tatctccaaa gacgctaacc cggtgacgga cgccgccgcc     660
   atgcaggcga tccgcctcat cgcccgcaac ctgcgccagg ccgtggccct cggcagcaat     720
   ctgcaggcgc gggaaaacat ggcctatgcc tctctgctgg ccgggatggc tttcaataac     780
   gccaacctcg gctacgtgca cgccatggca caccagctgg gcggcctgta cgacatgccg     840
   cacggcgtgg ccaacgctgt cctgctgccg catgtggcgc gctacaacct gatcgccaac     900
   ccggagaaat tcgccgatat tgctgaactg atgggcgaaa atatcaccgg actgtccact     960
   ctcgacgcgg cggaaaaagc catcgccgct atcacgcgtc tgtcgatgga tatcggtatt    1020
   ccgcagcatc tgcgcgatct gggagtaaaa gaggccgact tcccctacat ggcggagatg    1080
   gctctgaaag acggcaatgc gttctcgaac ccgcgtaaag gcaacgagca ggagattgcc    1140
   gcgatttccc gccaggcatt ctaa                                            1164

<210>  26
<211>  387
<212>  PRT

<213>  Klebsiella pneumoniae

<400>  26

Met Ser Tyr Arg Met Phe Asp Tyr Leu Val Pro Asn Val Asn Phe Phe
1               5                   10                  15

Gly Pro Asn Ala Ile Ser Val Val Gly Glu Arg Cys Gln Leu Leu Gly
            20                  25                  30

Gly Lys Lys Ala Leu Leu Val Thr Asp Lys Gly Leu Arg Ala Ile Lys
        35                  40                  45

Asp Gly Ala Val Asp Lys Thr Leu His Tyr Leu Arg Glu Ala Gly Ile
    50                  55                  60

Glu Val Ala Ile Phe Asp Gly Val Glu Pro Asn Pro Lys Asp Thr Asn
65                  70                  75                  80

Val Arg Asp Gly Leu Ala Val Phe Arg Arg Glu Gln Cys Asp Ile Ile
                85                  90                  95

Val Thr Val Gly Gly Gly Ser Pro His Asp Cys Gly Lys Gly Ile Gly
            100                 105                 110

Ile Ala Ala Thr His Glu Gly Asp Leu Tyr Gln Tyr Ala Gly Ile Glu
        115                 120                 125

Thr Leu Thr Asn Pro Leu Pro Pro Ile Val Ala Val Asn Thr Thr Ala
    130                 135                 140

Gly Thr Ala Ser Glu Val Thr Arg His Cys Val Leu Thr Asn Thr Glu
145                 150                 155                 160

Thr Lys Val Lys Phe Val Ile Val Ser Trp Arg Asn Leu Pro Ser Val
                165                 170                 175

Ser Ile Asn Asp Pro Leu Leu Met Ile Gly Lys Pro Ala Ala Leu Thr
            180                 185                 190

Ala Ala Thr Gly Met Asp Ala Leu Thr His Ala Val Glu Ala Tyr Ile
        195                 200                 205

Ser Lys Asp Ala Asn Pro Val Thr Asp Ala Ala Ala Met Gln Ala Ile
    210                 215                 220

Arg Leu Ile Ala Arg Asn Leu Arg Gln Ala Val Ala Leu Gly Ser Asn
225                 230                 235                 240

Leu Gln Ala Arg Glu Asn Met Ala Tyr Ala Ser Leu Leu Ala Gly Met
                245                 250                 255

Ala Phe Asn Asn Ala Asn Leu Gly Tyr Val His Ala Met Ala His Gln
            260                 265                 270

Leu Gly Gly Leu Tyr Asp Met Pro His Gly Val Ala Asn Ala Val Leu
        275                 280                 285

Leu Pro His Val Ala Arg Tyr Asn Leu Ile Ala Asn Pro Glu Lys Phe
    290                 295                 300

Ala Asp Ile Ala Glu Leu Met Gly Glu Asn Ile Thr Gly Leu Ser Thr
305                 310                 315                 320

Leu Asp Ala Ala Glu Lys Ala Ile Ala Ala Ile Thr Arg Leu Ser Met
                325                 330                 335

Asp Ile Gly Ile Pro Gln His Leu Arg Asp Leu Gly Val Lys Glu Ala
            340                 345                 350

Asp Phe Pro Tyr Met Ala Glu Met Ala Leu Lys Asp Gly Asn Ala Phe
        355                 360                 365

Ser Asn Pro Arg Lys Gly Asn Glu Gln Glu Ile Ala Ala Ile Phe Arg
    370                 375                 380

Gln Ala Phe
385

<210>  27
<211>  978
<212>  DNA
<213>  Lactococcus lactis

<400>  27
atggctgata aacaacgtaa gaaagttatc cttgttggtg acggtgctgt aggttcatca      60

tacgcttttg cccttgttaa ccaaggaatt gcacaagaat taggtattgt tgaccttttt     120

aaagaaaaaa ctcaagggga tgcagaagac ctttctcatg ccttggcatt tacatcacct     180

aaaaagattt actctgcaga ctactctgat gcaagcgacg ctgacctcgt tgtcttgact     240

tctggtgctc catgcaaacc aggtgaaact cgtcttgacc ttgttgaaaa aaatcttcgt     300

attactaaag atgttgtaac taaaattgtt gcttcaggat tcaaaggaat cttcctcgtt     360

gctgctaacc cagttgacat cttgacatac gcaacttgga aattctctgg tttccctaaa     420

aaccgtgttg taggttcagg tacttcactt gatactgcac gttttcgtca agcattggct     480

gaaaaagttg acgttgatgc tcgttcaatc cacgcataca tcatgggtga acatggtgac 540

tcagaatttg ctgtttggtc acacgctaac gttgctggtg ttaaattgga acaatggttc 600

caagaaaatg actaccttaa cgaagcagaa atcgttgaat tgtttgaatc tgtacgtgat 660

gcagcttact caatcatcgc taaaaaaggt gcaacattct acggtgtggc tgtagccctt 720

gctcgtatta ctaaagcaat tcttgatgat gaacatgcag tacttcctgt atcagtattc 780

caagatggac aatatggcgt aagcgactgc taccttggtc aaccagctgt agttggtgct 840

gaaggtgttg ttaacccaat tcacattcca ttgaacgatg ctgaaatgca aaaaatggaa 900

gcttctggag ctcaattgaa agctatcatc gatgaggctt ttgctaaaga agaatttgct 960

tctgcagtta aaaactaa 978

<210> 28
<211> 325
<212> PRT
<213> Lactococcus lactis

<400> 28

Met Ala Asp Lys Gln Arg Lys Lys Val Ile Leu Val Gly Asp Gly Ala
1               5                   10                  15

Val Gly Ser Ser Tyr Ala Phe Ala Leu Val Asn Gln Gly Ile Ala Gln
            20                  25                  30

Glu Leu Gly Ile Val Asp Leu Phe Lys Glu Lys Thr Gln Gly Asp Ala
        35                  40                  45

Glu Asp Leu Ser His Ala Leu Ala Phe Thr Ser Pro Lys Lys Ile Tyr
    50                  55                  60

Ser Ala Asp Tyr Ser Asp Ala Ser Asp Ala Asp Leu Val Val Leu Thr
65                  70                  75                  80

Ser Gly Ala Pro Cys Lys Pro Gly Glu Thr Arg Leu Asp Leu Val Glu
                85                  90                  95

Lys Asn Leu Arg Ile Thr Lys Asp Val Val Thr Lys Ile Val Ala Ser
            100                 105                 110

Gly Phe Lys Gly Ile Phe Leu Val Ala Ala Asn Pro Val Asp Ile Leu
        115                 120                 125

Thr Tyr Ala Thr Trp Lys Phe Ser Gly Phe Pro Lys Asn Arg Val Val
    130                 135                 140

Gly Ser Gly Thr Ser Leu Asp Thr Ala Arg Phe Arg Gln Ala Leu Ala

145                 150                 155                 160

Glu Lys Val Asp Val Asp Ala Arg Ser Ile His Ala Tyr Ile Met Gly
                165                 170                 175

Glu His Gly Asp Ser Glu Phe Ala Val Trp Ser His Ala Asn Val Ala
            180                 185                 190

Gly Val Lys Leu Glu Gln Trp Phe Gln Glu Asn Asp Tyr Leu Asn Glu
        195                 200                 205

Ala Glu Ile Val Glu Leu Phe Glu Ser Val Arg Asp Ala Ala Tyr Ser
    210                 215                 220

Ile Ile Ala Lys Lys Gly Ala Thr Phe Tyr Gly Val Ala Val Ala Leu
225                 230                 235                 240

Ala Arg Ile Thr Lys Ala Ile Leu Asp Asp Glu His Ala Val Leu Pro
                245                 250                 255

Val Ser Val Phe Gln Asp Gly Gln Tyr Gly Val Ser Asp Cys Tyr Leu
            260                 265                 270

Gly Gln Pro Ala Val Val Gly Ala Glu Gly Val Val Asn Pro Ile His
        275                 280                 285

Ile Pro Leu Asn Asp Ala Glu Met Gln Lys Met Glu Ala Ser Gly Ala
    290                 295                 300

Gln Leu Lys Ala Ile Ile Asp Glu Ala Phe Ala Lys Glu Glu Phe Ala
305                 310                 315                 320

Ser Ala Val Lys Asn
                325

<210>  29
<211>  1095
<212>  DNA
<213>  Lysinibacillus sphaericus

<400>  29
atggaaatct tcaagtatat ggaaaagtat gattatgaac aattggtatt ttgccaagac      60

gaagcatctg ggttaaaagc gattatcgct atccatgaca caacacttgg accagcatta     120

ggtggtgctc gtatgtggac ctacgcgaca gaagaaaatg cgattgagga tgcattaaga     180

ttagcacgcg ggatgacata taaaaatgca gctgctggtt taaaccttgg cggtggaaaa     240

acggtcatta ttggggaccc atttaaagat aaaaacgaag aaatgttccg tgctttaggt     300

cgtttcattc aaggattaaa cggtcgctat attaccgctg aagatgttgg tacaaccgta 360

acagatatgg atttaatcca tgaggaaaca aattacgtta caggtatatc gccagcgttt 420

ggttcatcgg gtaatccttc accagtaact gcttatggcg tttatcgtgg catgaaagca 480

gcggcgaaag aagcatttgg tacggatatg ctagaaggtc gtactatatc ggtacaaggg 540

ctaggaaacg tagcttacaa gctttgcgag tatttacata atgaaggtgc aaaacttgta 600

gtaacagata ttaaccaagc ggctattgat cgtgttgtca atgatttggg cgctacagca 660

gttgcacctg atgaaatcta ttcacaagaa gtcgatattt tctcaccgtg tgcacttggc 720

gcaattttaa atgacgaaac gattccgcaa ttaaaagcaa aagttattgc tggttctgct 780

aataaccaac tacaagattc acgacatgga gattatttac acgagctagg cattgtttat 840

gcacctgact atgtcattaa tgcaggtggt gtaataaatg tcgcggacga attatatggc 900

tataatcgtg aacgagcgtt gaaacgtgta gatggtattt acgatagtat tgaaaaaatc 960

tttgaaattt ccaaacgtga tagtattcca acatatgttg cggcaaatcg tttggcagaa 1020

gaacgtattg ctcgtgtagc gaaatcgcgt agtcagttct taaaaaatga aaaaaatatt 1080

ttgaacggcc gttaa 1095

<210> 30
<211> 364
<212> PRT
<213> Lysinibacillus sphaericus

<400> 30

Met Glu Ile Phe Lys Tyr Met Glu Lys Tyr Asp Tyr Glu Gln Leu Val
1               5                   10                  15

Phe Cys Gln Asp Glu Ala Ser Gly Leu Lys Ala Ile Ile Ala Ile His
            20                  25                  30

Asp Thr Thr Leu Gly Pro Ala Leu Gly Gly Ala Arg Met Trp Thr Tyr
        35                  40                  45

Ala Thr Glu Glu Asn Ala Ile Glu Asp Ala Leu Arg Leu Ala Arg Gly
    50                  55                  60

Met Thr Tyr Lys Asn Ala Ala Ala Gly Leu Asn Leu Gly Gly Gly Lys
65                  70                  75                  80

Thr Val Ile Ile Gly Asp Pro Phe Lys Asp Lys Asn Glu Glu Met Phe
                85                  90                  95

Arg Ala Leu Gly Arg Phe Ile Gln Gly Leu Asn Gly Arg Tyr Ile Thr
            100                 105                 110

Ala Glu Asp Val Gly Thr Thr Val Thr Asp Met Asp Leu Ile His Glu
        115                 120                 125

Glu Thr Asn Tyr Val Thr Gly Ile Ser Pro Ala Phe Gly Ser Ser Gly
    130                 135                 140

Asn Pro Ser Pro Val Thr Ala Tyr Gly Val Tyr Arg Gly Met Lys Ala
145                 150                 155                 160

Ala Ala Lys Glu Ala Phe Gly Thr Asp Met Leu Glu Gly Arg Thr Ile
                165                 170                 175

Ser Val Gln Gly Leu Gly Asn Val Ala Tyr Lys Leu Cys Glu Tyr Leu
            180                 185                 190

His Asn Glu Gly Ala Lys Leu Val Val Thr Asp Ile Asn Gln Ala Ala
        195                 200                 205

Ile Asp Arg Val Val Asn Asp Phe Gly Ala Thr Ala Val Ala Pro Asp
    210                 215                 220

Glu Ile Tyr Ser Gln Glu Val Asp Ile Phe Ser Pro Cys Ala Leu Gly
225                 230                 235                 240

Ala Ile Leu Asn Asp Glu Thr Ile Pro Gln Leu Lys Ala Lys Val Ile
                245                 250                 255

Ala Gly Ser Ala Asn Asn Gln Leu Gln Asp Ser Arg His Gly Asp Tyr
            260                 265                 270

Leu His Glu Leu Gly Ile Val Tyr Ala Pro Asp Tyr Val Ile Asn Ala
        275                 280                 285

Gly Gly Val Ile Asn Val Ala Asp Glu Leu Tyr Gly Tyr Asn Arg Glu
    290                 295                 300

Arg Ala Leu Lys Arg Val Asp Gly Ile Tyr Asp Ser Ile Glu Lys Ile
305                 310                 315                 320

Phe Glu Ile Ser Lys Arg Asp Ser Ile Pro Thr Tyr Val Ala Ala Asn
                325                 330                 335

Arg Leu Ala Glu Glu Arg Ile Ala Arg Val Ala Lys Ser Arg Ser Gln
            340                 345                 350

Phe Leu Lys Asn Glu Lys Asn Ile Leu Asn Gly Arg
        355                 360

<210> 31
<211> 1143
<212> DNA
<213> Bacillus badius

<400> 31
atgagcttag tagaaaaaac atccatcata aaagatttca ctctttttga aaaaatgtct 60

gaacatgaac aagttgtttt ttgcaacgat ccggcgacag gactaagggc cattatcgct 120

attcatgaca ccacactcgg acctgcgctc ggcggctgcc gcatgcagcc ttataacagt 180

gtggaagaag cattggaaga tgctcttcgc ctttccaaag gaatgactta caaatgcgcg 240

gcgtccgatg tcgactttgg cggcggaaaa gcagtcatta tcggtgatcc gcagaaagat 300

aaatctccag aactgttccg cgcgtttggc caatttgttg attcgcttgg cggccgtttc 360

tatacaggta ctgatatggg aacgaatatg gaagatttca ttcacgccat gaaagaaaca 420

aactgcattg ttggggtgcc ggaagcttac ggcggcggcg gagattcctc tattccaact 480

gccatgggtg tcctgtacgg cattaaagca accaacaaaa tgttgtttgg caaggacgat 540

cttggcggcg tcacttatgc cattcaagga cttggcaaag taggctacaa agtagcggaa 600

gggctgctcg aagaaggtgc tcatttattt gtaacggata ttaacgagca aagcttggag 660

gctatccagg aaaaagcaaa aacaacatcc ggttctgtca cggtagtagc gagcgatgaa 720

atttattccc aggaagccga tgtgttcgtt ccgtgtgcat ttggcggcgt tgttaatgat 780

gaaacgatga agcagttcaa ggtgaaagca atcgccggtt cagccaacaa tcagctgctt 840

acggaggatc acggcagaca gcttgcagac aaaggcattc tgtatgctcc ggattatatt 900

gttaactctg gcggtctgat ccaagtagcc gacgaattgt atgaggtgaa caaagaacgc 960

gtgcttgcga agacgaagca tatttacgac gcaattcttg aagtgtacca gcaagcggaa 1020

ttagatcaaa tcaccacaat ggaagcagcc aacagaatgt gtgagcaaag aatggcggca 1080

agaggccgac gcaacagctt ctttacttct tctgttaagc caaaatggga tattcgcaac 1140

taa 1143

<210> 32
<211> 380
<212> PRT
<213> Bacillus badius

<400> 32

Met Ser Leu Val Glu Lys Thr Ser Ile Ile Lys Asp Phe Thr Leu Phe
1               5                   10                  15

Glu Lys Met Ser Glu His Glu Gln Val Val Phe Cys Asn Asp Pro Ala
            20                  25                  30

Thr Gly Leu Arg Ala Ile Ile Ala Ile His Asp Thr Thr Leu Gly Pro
        35                  40                  45

Ala Leu Gly Gly Cys Arg Met Gln Pro Tyr Asn Ser Val Glu Glu Ala
    50                  55                  60

Leu Glu Asp Ala Leu Arg Leu Ser Lys Gly Met Thr Tyr Lys Cys Ala
65                  70                  75                  80

Ala Ser Asp Val Asp Phe Gly Gly Gly Lys Ala Val Ile Ile Gly Asp
                85                  90                  95

Pro Gln Lys Asp Lys Ser Pro Glu Leu Phe Arg Ala Phe Gly Gln Phe
            100                 105                 110

Val Asp Ser Leu Gly Gly Arg Phe Tyr Thr Gly Thr Asp Met Gly Thr
        115                 120                 125

Asn Met Glu Asp Phe Ile His Ala Met Lys Glu Thr Asn Cys Ile Val
    130                 135                 140

Gly Val Pro Glu Ala Tyr Gly Gly Gly Gly Asp Ser Ser Ile Pro Thr
145                 150                 155                 160

Ala Met Gly Val Leu Tyr Gly Ile Lys Ala Thr Asn Lys Met Leu Phe
                165                 170                 175

Gly Lys Asp Asp Leu Gly Gly Val Thr Tyr Ala Ile Gln Gly Leu Gly
            180                 185                 190

Lys Val Gly Tyr Lys Val Ala Glu Gly Leu Leu Glu Glu Gly Ala His
        195                 200                 205

Leu Phe Val Thr Asp Ile Asn Glu Gln Ser Leu Glu Ala Ile Gln Glu
    210                 215                 220

Lys Ala Lys Thr Thr Ser Gly Ser Val Thr Val Val Ala Ser Asp Glu
225                 230                 235                 240

Ile Tyr Ser Gln Glu Ala Asp Val Phe Val Pro Cys Ala Phe Gly Gly
                245                 250                 255

Val Val Asn Asp Glu Thr Met Lys Gln Phe Lys Val Lys Ala Ile Ala
            260                 265                 270

Gly Ser Ala Asn Asn Gln Leu Leu Thr Glu Asp His Gly Arg Gln Leu
        275                 280                 285

Ala Asp Lys Gly Ile Leu Tyr Ala Pro Asp Tyr Ile Val Asn Ser Gly
    290                 295                 300

Gly Leu Ile Gln Val Ala Asp Glu Leu Tyr Glu Val Asn Lys Glu Arg
305                 310                 315                 320

Val Leu Ala Lys Thr Lys His Ile Tyr Asp Ala Ile Leu Glu Val Tyr
                325                 330                 335

Gln Gln Ala Glu Leu Asp Gln Ile Thr Thr Met Glu Ala Ala Asn Arg
            340                 345                 350

Met Cys Glu Gln Arg Met Ala Ala Arg Gly Arg Arg Asn Ser Phe Phe
        355                 360                 365

Thr Ser Ser Val Lys Pro Lys Trp Asp Ile Arg Asn
    370                 375                 380

<210>  33
<211>  1116
<212>  DNA
<213>  Streptomyces fradiae

<400>  33
atgaccgacg cgtcccaccc caccgccgcc gacgacctcg gcgccctctc caccctcttc      60

cgctccgagc agggcggcca cgagcgggtc ctgctctgcc aggaccgcga gagcggcctc     120

aaggccgtca tcgccctcca ctcgaccgcc ctgggcccgg ccctcggcgg cacccgcttc     180

catgcctacg cctccgacga ggaggccgtg ctcgacgccc tgaacctcgc ccgcggcatg     240

tcgtacaaga acgccctcgc cgggctgccg cacggcggcg gcaaggccgt gatcatcggc     300

agccccgccc cggtctccga gggcgggctg aagagcgagg cgctgctccg cgcgtacggc     360

cgcttcgtcg cctccctgga cggccgttac gtcacggcct gtgacgtcgg cacgtatgtc     420

gccgacatgg acgtcgtcgc ccgcgagtgc cggtggacca ccggccgctc cccggagaac     480

ggcggcgccg gcgactcctc cgtcctcacc gccttcggcg tcttccaggg catgcgggcc     540

agcgctcagg cgctgtgggg cgagcccacc ctgcgcggcc ggaccgtcgg cgtggccggg     600

gtcggcaagg tcggccacca cctggtggac cacctggtgg aggacggtgc ccgggtcgtg     660

gtcaccgatg tgcggccgga gagcgtggag cgggtgcgcg cccggcaccc gcgggtggtg     720

gccgtgcccg acacggagtc cctgatccgc gcggacctgg acgtctacgc cccctgcgcc     780

ctcggcggtg ccctgaacga cgacacggtg cccgcgctga ccgcgcgcgt cgtctgcggc     840

gcggccaaca accagctcgc ccacccgggc gtcgaaaagg acctcgccgg ccgcggcatc     900

ctgtacgcgc ccgactacgt cgtcaacgcc ggtggcgtga tccaggtcgc cgacgagctg     960

ctcggttttg atttcgaccg ggcgaaggcc aaggcggcgc agatcttcga cacgacgctg 1020

gccatattcg accgcgccag gaccgacggc gtcccgcccg ccgtcgccgc cgaccggatc 1080

gccgagcagc gcatggcgga ggcccgcacc gtctaa 1116

<210> 34
<211> 371
<212> PRT
<213> Streptomyces fradiae

<400> 34
   Met Thr Asp Ala Ser His Pro Thr Ala Ala Asp Asp Leu Gly Ala Leu
   1 5 10 15
   Ser Thr Leu Phe Arg Ser Glu Gln Gly Gly His Glu Arg Val Leu Leu
   20 25 30
   Cys Gln Asp Arg Glu Ser Gly Leu Lys Ala Val Ile Ala Leu His Ser
   35 40 45
   Thr Ala Leu Gly Pro Ala Leu Gly Gly Thr Arg Phe His Ala Tyr Ala
   50 55 60
   Ser Asp Glu Glu Ala Val Leu Asp Ala Leu Asn Leu Ala Arg Gly Met
   65 70 75 80
   Ser Tyr Lys Asn Ala Leu Ala Gly Leu Pro His Gly Gly Gly Lys Ala
   85 90 95
   Val Ile Ile Gly Ser Pro Ala Pro Val Ser Glu Gly Gly Leu Lys Ser
   100 105 110
   Glu Ala Leu Leu Arg Ala Tyr Gly Arg Phe Val Ala Ser Leu Asp Gly
   115 120 125
   Arg Tyr Val Thr Ala Cys Asp Val Gly Thr Tyr Val Ala Asp Met Asp
   130 135 140
   Val Val Ala Arg Glu Cys Arg Trp Thr Thr Gly Arg Ser Pro Glu Asn
   145 150 155 160
   Gly Gly Ala Gly Asp Ser Ser Val Leu Thr Ala Phe Gly Val Phe Gln
   165 170 175
   Gly Met Arg Ala Ser Ala Gln Ala Leu Trp Gly Glu Pro Thr Leu Arg
   180 185 190
   Gly Arg Thr Val Gly Val Ala Gly Val Gly Lys Val Gly His His Leu
           195                 200                 205
   Val Asp His Leu Val Glu Asp Gly Ala Arg Val Val Val Thr Asp Val
       210                 215                 220
   Arg Pro Glu Ser Val Glu Arg Val Arg Ala Arg His Pro Arg Val Val
   225                 230                 235                 240
   Ala Val Pro Asp Thr Glu Ser Leu Ile Arg Ala Asp Leu Asp Val Tyr
                   245                 250                 255
   Ala Pro Cys Ala Leu Gly Gly Ala Leu Asn Asp Asp Thr Val Pro Ala
               260                 265                 270
   Leu Thr Ala Arg Val Val Cys Gly Ala Ala Asn Asn Gln Leu Ala His
           275                 280                 285
   Pro Gly Val Glu Lys Asp Leu Ala Gly Arg Gly Ile Leu Tyr Ala Pro
       290                 295                 300
   Asp Tyr Val Val Asn Ala Gly Gly Val Ile Gln Val Ala Asp Glu Leu
   305                 310                 315                 320
   Leu Gly Phe Asp Phe Asp Arg Ala Lys Ala Lys Ala Ala Gln Ile Phe
                   325                 330                 335
   Asp Thr Thr Leu Ala Ile Phe Asp Arg Ala Arg Thr Asp Gly Val Pro
               340                 345                 350
   Pro Ala Val Ala Ala Asp Arg Ile Ala Glu Gln Arg Met Ala Glu Ala
           355                 360                 365
   Arg Thr Val
       370

<210> 35
<211> 1491
<212> DNA
<213> Arabidopsis thaliana

<400> 35
atggtgcgcc agctgtccat caaggcacgc cgtaactgct ccaacatcgg cgtggcacag 60

atcgtggcag caaagtggtc caacaaccca tcctccgcac tgccatccgc agcagcagca 120

gccgcaacct cctccgcatc cgcagtgtcc tccgcagcat ccgcagctgc cgcatcctcc 180

gctgcagcag caccagtggc agcagcccca ccagtggtgc tgaagtccgt ggatgaagag 240

gtggtggtgg cagaagaagg catccgcgaa aagatcggct ccgtgcagct gaccgattcc 300

aagcactcct tcttgtcctc cgatggctcc ctgaccgtgc acgcaggcga acgcttgggt 360

cgcggtatcg tgaccgatgc aatcaccacc ccagtggtga acacctccgc atactttttc 420

aagaaaaccg cagaactgat cgacttcaaa gaaaagcgct ccgtgtcctt cgaatacggt 480

cgctacggca acccaaccac cgtggtgctg gaagataaga tctccgcact ggaaggcgca 540

gaatccaccc tggtgatggc atccggcatg tgcgcatcca ccgtgatgct gctggccctg 600

gtgccagcag gcggtcacat cgtgaccacc accgattgct accgcaagac ccgtatcttc 660

atggaaaact ttctgccaaa gctgggcatc accgtgaccg tgatcgatcc agcagatatc 720

gcaggcctgg aagcagcagt gaacgaattc aaggtgtccc tgttcttcac cgaatcccca 780

accaacccat tcctgcgctg cgtggatatc gaactggtgt ccaagatctg ccacaagcgt 840

ggcaccctgg tgtgcatcga tggcaccttc gcaaccccac tgaaccagaa ggcactggca 900

cttggcgcag atctggtggt gcactccgca accaagtaca tcggtggcca caacgatgtg 960

ctggcaggct gcatctgcgg ctccctgaag ctggtgtccg aaatccgcaa cctgcaccac 1020

gtgctgggtg gcaccctgaa cccaaacgca gcatacctga tcatccgtgg catgaaaacc 1080

ctgcacttgc gcgtgcagca gcagaactcc accgcattcc gcatggcaga aatcctggaa 1140

gcacacccaa aggtgtccca cgtgtactac ccaggcctgc catcccaccc agaacacgaa 1200

ctggcaaagc gccagatgac cggcttcggt ggcgtggtgt ccttcgagat cgatggcgat 1260

atcgaaacca ccatcaagtt cgtggattcc ctgaagatcc catacattgc accatccttc 1320

ggtggctgcg aatccatcgt ggatcagcca gcaatcatgt cctactggga tctcccacaa 1380

gaagaacgcc tgaagtacgg catcaaggat aacctggtgc gcttctcctt cggcgtggaa 1440

gatttcgaag atgtgaaggc agatatcctg caggcactcg aagcaatcta a 1491

<210> 36
<211> 496
<212> PRT
<213> Arabidopsis thaliana

<400> 36

Met Val Arg Gln Leu Ser Ile Lys Ala Arg Arg Asn Cys Ser Asn Ile
1               5                   10                  15

Gly Val Ala Gln Ile Val Ala Ala Lys Trp Ser Asn Asn Pro Ser Ser
            20                  25                  30

Ala Leu Pro Ser Ala Ala Ala Ala Ala Ala Thr Ser Ser Ala Ser Ala
        35                  40                  45

Val Ser Ser Ala Ala Ser Ala Ala Ala Ala Ser Ser Ala Ala Ala Ala
    50                  55                  60

Pro Val Ala Ala Ala Pro Pro Val Val Leu Lys Ser Val Asp Glu Glu
65                  70                  75                  80

Val Val Val Ala Glu Glu Gly Ile Arg Glu Lys Ile Gly Ser Val Gln
                85                  90                  95

Leu Thr Asp Ser Lys His Ser Phe Leu Ser Ser Asp Gly Ser Leu Thr
            100                 105                 110

Val His Ala Gly Glu Arg Leu Gly Arg Gly Ile Val Thr Asp Ala Ile
        115                 120                 125

Thr Thr Pro Val Val Asn Thr Ser Ala Tyr Phe Phe Lys Lys Thr Ala
    130                 135                 140

Glu Leu Ile Asp Phe Lys Glu Lys Arg Ser Val Ser Phe Glu Tyr Gly
145                 150                 155                 160

Arg Tyr Gly Asn Pro Thr Thr Val Val Leu Glu Asp Lys Ile Ser Ala
                165                 170                 175

Leu Glu Gly Ala Glu Ser Thr Leu Val Met Ala Ser Gly Met Cys Ala
            180                 185                 190

Ser Thr Val Met Leu Leu Ala Leu Val Pro Ala Gly Gly His Ile Val
        195                 200                 205

Thr Thr Thr Asp Cys Tyr Arg Lys Thr Arg Ile Phe Met Glu Asn Phe
    210                 215                 220

Leu Pro Lys Leu Gly Ile Thr Val Thr Val Ile Asp Pro Ala Asp Ile
225                 230                 235                 240

Ala Gly Leu Glu Ala Ala Val Asn Glu Phe Lys Val Ser Leu Phe Phe
                245                 250                 255

Thr Glu Ser Pro Thr Asn Pro Phe Leu Arg Cys Val Asp Ile Glu Leu
            260                 265                 270

Val Ser Lys Ile Cys His Lys Arg Gly Thr Leu Val Cys Ile Asp Gly
        275                 280                 285

Thr Phe Ala Thr Pro Leu Asn Gln Lys Ala Leu Ala Leu Gly Ala Asp
    290                 295                 300

Leu Val Val His Ser Ala Thr Lys Tyr Ile Gly Gly His Asn Asp Val

305                 310                 315                 320

Leu Ala Gly Cys Ile Cys Gly Ser Leu Lys Leu Val Ser Glu Ile Arg
                325                 330                 335

Asn Leu His His Val Leu Gly Gly Thr Leu Asn Pro Asn Ala Ala Tyr
            340                 345                 350

Leu Ile Ile Arg Gly Met Lys Thr Leu His Leu Arg Val Gln Gln Gln
        355                 360                 365

Asn Ser Thr Ala Phe Arg Met Ala Glu Ile Leu Glu Ala His Pro Lys
    370                 375                 380

Val Ser His Val Tyr Tyr Pro Gly Leu Pro Ser His Pro Glu His Glu
385                 390                 395                 400

Leu Ala Lys Arg Gln Met Thr Gly Phe Gly Gly Val Val Ser Phe Glu
                405                 410                 415

Ile Asp Gly Asp Ile Glu Thr Thr Ile Lys Phe Val Asp Ser Leu Lys
            420                 425                 430

Ile Pro Tyr Ile Ala Pro Ser Phe Gly Gly Cys Glu Ser Ile Val Asp
        435                 440                 445

Gln Pro Ala Ile Met Ser Tyr Trp Asp Leu Pro Gln Glu Glu Arg Leu
    450                 455                 460

Lys Tyr Gly Ile Lys Asp Asn Leu Val Arg Phe Ser Phe Gly Val Glu
465                 470                 475                 480

Asp Phe Glu Asp Val Lys Ala Asp Ile Leu Gln Ala Leu Glu Ala Ile
                485                 490                 495

<210>  37
<211>  30
<212>  DNA
<213>  Artificial Sequence

<220>
<223>  Forward primer for sacB gene

<400>  37
ggggaagctt gacgtccaca tatacctgcc                                        30

<210>  38
<211>  24
<212>  DNA
<213>  Artificial Sequence

<220>
<223>  Reverse primer for sacB gene

<400>  38
attcggatcc gtatccacct ttac                                              24


<210>  39
<211>  26
<212>  DNA
<213>  Artificial Sequence

<220>
<223>  Forward primer for deleting KpnI recognition site on sacB gene

<400>  39
tgaacagata ccatttgccg ttcatt                                            26


<210>  40
<211>  26
<212>  DNA
<213>  Artificial Sequence

<220>
<223>  Reverse primer for deleting KpnI recognition site on sacB gene

<400>  40
aatggtatct gttcactgac tcccgc                                            26


<210>  41
<211>  35
<212>  DNA
<213>  Artificial Sequence

<220>
<223>  Up-Forward primer for ppc gene deletion

<400>  41
ccatgattac gaattcggga aactttttta agaaa                                  35


<210>  42
<211>  20
<212>  DNA
<213>  Artificial Sequence

<220>
<223>  Up-Reverse primer for ppc gene deletion

<400>  42
taactacttt aaacactctt                                                   20


<210>  43
<211>  35
<212>  DNA
<213>  Artificial Sequence

<220>
<223>  Down-Forward primer for ppc gene deletion

<400>  43
tgtttaaagt agttatccag ccggctgggt agtac                                35


<210>  44
<211>  35
<212>  DNA
<213>  Artificial Sequence

<220>
<223>  Down-Reverse primer for ppc gene deletion

<400>  44
taccgagctc gaattgaagt attcaagggg atttc                                35


<210>  45
<211>  35
<212>  DNA
<213>  Artificial Sequence

<220>
<223>  Up-Forward primer for ldhA gene deletion

<400>  45
gacggccagt gaatttttca tacgaccacg ggcta                                35


<210>  46
<211>  20
<212>  DNA
<213>  Artificial Sequence

<220>
<223>  Up-Reverse primer for ldhA gene deletion

<400>  46
gacaatcttg ttaccgacgg                                                 20


<210>  47
<211>  35
<212>  DNA
<213>  Artificial Sequence

<220>
<223>  Down-Forward primer for ldhA gene deletion

<400>  47
ggtaacaaga ttgtccattc cgcaaatacc ctgcg                                35


<210>  48
<211>  35
<212>  DNA
<213>  Artificial Sequence

<220>
<223>  Down-Reverse primer for ldhA gene deletion

<400>  48
taccgagctc gaattgggac gttgatgacg ctgcc                                35

<210>  49
<211>  35
<212>  DNA
<213>  Artificial Sequence

<220>
<223>  Up-Forward primer for pyc gene deletion

<400>  49
gacggccagt gaattgttga tgactgttgg ttcca                                35


<210>  50
<211>  26
<212>  DNA
<213>  Artificial Sequence

<220>
<223>  Up-Reverse primer for pyc gene deletion

<400>  50
ctcgagtaga gtaattattc ctttca                                          26


<210>  51
<211>  35
<212>  DNA
<213>  Artificial Sequence

<220>
<223>  Down-Forward primer for pyc gene deletion

<400>  51
attactctac tcgagacctt tctgtaaaaa gcccc                                35


<210>  52
<211>  35
<212>  DNA
<213>  Artificial Sequence

<220>
<223>  Down-Reverse primer for pyc gene deletion

<400>  52
taccgagctc gaattcacga tccgccgggc agcct                                35


<210>  53
<211>  33
<212>  DNA
<213>  Artificial Sequence

<220>
<223>  Up-Forward primer for poxB gene deletion

<400>  53
gacggccagt gaaaacgtta atgaggaaaa ccg                                  33


<210>  54

<211>  20
<212>  DNA
<213>  Artificial Sequence

<220>
<223>  Up-Reverse primer for poxB gene deletion

<400>  54
aattaattgt tctgcgtagc                                                    20


<210>  55
<211>  41
<212>  DNA
<213>  Artificial Sequence

<220>
<223>  Down-Forward primer for poxB gene deletion

<400>  55
gcagaacaat taattctcga gtcgaacata aggaatattc c                            41


<210>  56
<211>  35
<212>  DNA
<213>  Artificial Sequence

<220>
<223>  Down-Reverse primer for poxB gene deletion

<400>  56
taccgagctc gaattttcca ggtacggaaa gtgcc                                   35


<210>  57
<211>  35
<212>  DNA
<213>  Artificial Sequence

<220>
<223>  Up-Forward primer for alaA gene deletion

<400>  57
gacggccagt gaatttttca ccgaagtgac accta                                   35


<210>  58
<211>  26
<212>  DNA
<213>  Artificial Sequence

<220>
<223>  Up-Reverse primer for alaA gene deletion

<400>  58
ctcgagccgc tcaatgttgc cacttt                                             26


<210>  59
<211>  35
<212>  DNA
<213>  Artificial Sequence

<220>
<223>  Down-Forward primer for alaA gene deletion

<400>  59
attgagcggc tcgagtagtt gttaggattc accac                                35


<210>  60
<211>  35
<212>  DNA
<213>  Artificial Sequence

<220>
<223>  Down-Reverse primer for alaA gene deletion

<400>  60
taccgagctc gaattgttcc ctggaaattg tttgc                                35


<210>  61
<211>  35
<212>  DNA
<213>  Artificial Sequence

<220>
<223>  Up-Forward primer for ilvB gene deletion

<400>  61
gacggccagt gaattaccgc acgcgaccag ttatt                                35


<210>  62
<211>  26
<212>  DNA
<213>  Artificial Sequence

<220>
<223>  Up-Reverse primer for ilvB gene deletion

<400>  62
gctagcgact ttctggctcc tttact                                          26


<210>  63
<211>  35
<212>  DNA
<213>  Artificial Sequence

<220>
<223>  Down-Forward primer for ilvB gene deletion

<400>  63
cagaaagtcg ctagcggaga gacccaagat ggcta                                35


<210>  64
<211>  35
<212>  DNA
<213>  Artificial Sequence

<220>
<223>  Down-Reverse primer for ilvB gene deletion

<400>  64
taccgagctc gaattctcga tgtcgttggt gaaga                                35

<210>  65
<211>  35
<212>  DNA
<213>  Artificial Sequence

<220>
<223>  Up-Forward primer for cg0931 gene deletion

<400>  65
gacggccagt gaattagcag ggacgaaagt cggga                                35

<210>  66
<211>  26
<212>  DNA
<213>  Artificial Sequence

<220>
<223>  Up-Reverse primer for cg0931 gene deletion

<400>  66
ctcgaggcag ctactatatt tgatcc                                          26

<210>  67
<211>  35
<212>  DNA
<213>  Artificial Sequence

<220>
<223>  Down-Forward primer for cg0931 gene deletion

<400>  67
agtagctgcc tcgagtttga acaggttgtt ggggg                                35

<210>  68
<211>  35
<212>  DNA
<213>  Artificial Sequence

<220>
<223>  Down-Reverse primer for cg0931 gene deletion

<400>  68
taccgagctc gaattatcga cggcaaaacg cccaa                                35

<210>  69
<211>  35
<212>  DNA
<213>  Artificial Sequence

<220>
<223>  Up-Forward primer for adhE gene deletion

<400>  69
gtgaattcga gctcgcagaa gcagatcttg caatc                                35

<210>  70
<211>  32
<212>  DNA
<213>  Artificial Sequence

<220>
<223>  Up-Reverse primer for adhE gene deletion

<400>  70
cttcctcgag aattccaggc actacagtgc tc                                    32


<210>  71
<211>  32
<212>  DNA
<213>  Artificial Sequence

<220>
<223>  Down-Forward primer for adhE gene deletion

<400>  71
gaattctcga ggaagaggct ttcaacacca tg                                    32


<210>  72
<211>  35
<212>  DNA
<213>  Artificial Sequence

<220>
<223>  Down-Reverse primer for adhE gene deletion

<400>  72
aggtggatac ggatcggaac aaagtgtctc cagag                                 35


<210>  73
<211>  37
<212>  DNA
<213>  Artificial Sequence

<220>
<223>  Up-Forward primer for adhE gene deletion

<400>  73
gtgaattcga gctcgattca ggatttgctt cgcgacg                               37


<210>  74
<211>  37
<212>  DNA
<213>  Artificial Sequence

<220>
<223>  Down-Reverse primer for adhE gene deletion

<400>  74
aggtggatac ggatcacagt gtccggatca tctgctg                               37


<210>  75

<211>  35
<212>  DNA
<213>  Artificial Sequence

<220>
<223>  Up-Forward primer for ald gene deletion

<400>  75
gtgaattcga gctcgccgaa acctcaaaga atccc                                35


<210>  76
<211>  32
<212>  DNA
<213>  Artificial Sequence

<220>
<223>  Up-Reverse primer for ald gene deletion

<400>  76
ggtactcgag tcctggattt gcgtagacag tc                                   32


<210>  77
<211>  32
<212>  DNA
<213>  Artificial Sequence

<220>
<223>  Down-Forward primer for ald gene deletion

<400>  77
caggactcga gtaccagcag accaagaacc tg                                   32


<210>  78
<211>  35
<212>  DNA
<213>  Artificial Sequence

<220>
<223>  Down-Reverse primer for ald gene deletion

<400>  78
aggtggatac ggatcgatct ccagaggttt caagc                                35


<210>  79
<211>  38
<212>  DNA
<213>  Artificial Sequence

<220>
<223>  Up-Forward primer for ald gene deletion

<400>  79
gtgaattcga gctcgaacgc cagatggtcg tactttgc                             38


<210>  80
<211>  38
<212>  DNA
<213>  Artificial Sequence

<220>
<223>  Down-Reverse primer for ald gene deletion

<400>  80
aggtggatac ggatcttcaa caccgctgat ttcctacg                              38


<210>  81
<211>  39
<212>  DNA
<213>  Artificial Sequence

<220>
<223>  Forward primer for KnR gene

<400>  81
cggtaccgtc gacgatatcg aggtctgcct cgtgaagaa                             39


<210>  82
<211>  35
<212>  DNA
<213>  Artificial Sequence

<220>
<223>  Reverse primer for KnR gene

<400>  82
gcctttttac ggttcgattt attcaacaaa gccgc                                  35


<210>  83
<211>  35
<212>  DNA
<213>  Artificial Sequence

<220>
<223>  Forward primer for pUCori

<400>  83
gaaccgtaaa aaggccgcgt tgctggcgtt tttcc                                 35


<210>  84
<211>  35
<212>  DNA
<213>  Artificial Sequence

<220>
<223>  Reverse primer for pUCori

<400>  84
gcggccgcgt agaaaagatc aaaggatctt cttga                                 35


<210>  85
<211>  35
<212>  DNA
<213>  Artificial Sequence

<220>
<223>  Forward primer for pCG1ori

<400>  85
tttctacgcg gccgcccatg gtcgtcacag agctg                                35

<210>  86
<211>  40
<212>  DNA
<213>  Artificial Sequence

<220>
<223>  Reverse primer for pCG1ori

<400>  86
tcgtcgacgg taccggatcc ttgggagcag tccttgtgcg                           40

<210>  87
<211>  35
<212>  DNA
<213>  Artificial Sequence

<220>
<223>  Forward primer for PgapA

<400>  87
ctgctcccaa ggatcgaaga aatttagatg attga                                35

<210>  88
<211>  39
<212>  DNA
<213>  Artificial Sequence

<220>
<223>  Reverse primer for PgapA

<400>  88
ccgtcgacga tatcatatgg tgtctcctct aaagattgt                            39

<210>  89
<211>  39
<212>  DNA
<213>  Artificial Sequence

<220>
<223>  Forward primer for TrrnB

<400>  89
tgatatcgtc gacggatcct gcctggcggc agtagcgcg                            39

<210>  90
<211>  35
<212>  DNA
<213>  Artificial Sequence

<220>
<223>  Reverse primer for TrrnB

<400>  90
gaggcagacc tcgataaaaa ggccatccgt cagga                                35

<210>  91
<211>  35
<212>  DNA
<213>  Artificial Sequence

<220>
<223>  Forward primer for Ec_hpaI

<400>  91
cgcgcggcag ccatatggaa aacagtttta aagcg                                35


<210>  92
<211>  37
<212>  DNA
<213>  Artificial Sequence

<220>
<223>  Reverse primer for Ec_hpaI

<400>  92
ggatcctcga gcatattaat acacgccggg cttaatc                              37


<210>  93
<211>  36
<212>  DNA
<213>  Artificial Sequence

<220>
<223>  Forward primer for Ec_hpaI

<400>  93
agaggagaca ccatatgggc agcagccatc atcatc                               36


<210>  94
<211>  38
<212>  DNA
<213>  Artificial Sequence

<220>
<223>  Reverse primer for Ec_hpaI

<400>  94
ccgccaggca ggatccttaa tacacgccgg gcttaatc                             38


<210>  95
<211>  37
<212>  DNA
<213>  Artificial Sequence

<220>
<223>  Forward primer for PgapA-hpaI-TrrnB

<400>  95
gcctggaatt ctcgagaagg actgctccca aggatcg                              37


<210>  96

<211>  34
<212>  DNA
<213>  Artificial Sequence

<220>
<223>  Reverse primer for PgapA-hpaI-TrrnB

<400>  96
aagcctcttc ctcgatcttc acgaggcaga cctc                                    34


<210>  97
<211>  37
<212>  DNA
<213>  Artificial Sequence

<220>
<223>  Forward primer for PgapA-hpaI-TrrnB

<400>  97
aaatccagga ctcgagaagg actgctccca aggatcg                                 37


<210>  98
<211>  34
<212>  DNA
<213>  Artificial Sequence

<220>
<223>  Reverse primer for PgapA-hpaI-TrrnB

<400>  98
tctgctggta ctcgatcttc acgaggcaga cctc                                    34


<210>  99
<211>  34
<212>  DNA
<213>  Artificial Sequence

<220>
<223>  Forward primer for Ec_rhmA

<400>  99
agaggagaca ccatatgaac gcattattaa gcaa                                    34


<210>  100
<211>  41
<212>  DNA
<213>  Artificial Sequence

<220>
<223>  Reverse primer for Ec_rhmA

<400>  100
ccgccaggca ggatcagatc ttaataacta ccttttatgc g                            41


<210>  101
<211>  34
<212>  DNA
<213>  Artificial Sequence

<220>
<223>  Forward primer for Ec_nanA

<400>  101
agaggagaca ccatatggca acgaatttac gtgg                                    34


<210>  102
<211>  38
<212>  DNA
<213>  Artificial Sequence

<220>
<223>  Reverse primer for Ec_nanA

<400>  102
ccgccaggca ggatccttac ccgcgctctt gcatcaac                                38


<210>  103
<211>  35
<212>  DNA
<213>  Artificial Sequence

<220>
<223>  Forward primer for pCASE1ori

<400>  103
tttctacgcg gccgccactg gaagggttct tcagg                                   35


<210>  104
<211>  40
<212>  DNA
<213>  Artificial Sequence

<220>
<223>  Reverse primer for pCASE1ori

<400>  104
tcgtcgacgg taccggatcc ctgacttggt tacgatggac                              40


<210>  105
<211>  35
<212>  DNA
<213>  Artificial Sequence

<220>
<223>  Forward primer for PldhA

<400>  105
accaagtcag ggatcgcgga actagctctg caatg                                   35


<210>  106
<211>  39
<212>  DNA
<213>  Artificial Sequence

<220>
<223>  Reverse primer for PldhA

<400>  106
ccgtcgacga tatcatatgc gatcccactt cctgatttc                              39


<210>  107
<211>  39
<212>  DNA
<213>  Artificial Sequence

<220>
<223>  Forward primer for SpR gene

<400>  107
cggtaccgtc gacgatatct ctagaccagc caggacaga                              39


<210>  108
<211>  35
<212>  DNA
<213>  Artificial Sequence

<220>
<223>  Reverse primer for SpR gene

<400>  108
gcctttttac ggttcctcga gggttatttg ccgac                                   35


<210>  109
<211>  35
<212>  DNA
<213>  Artificial Sequence

<220>
<223>  Reverse primer for TrrnB

<400>  109
ggctggtcta gagataaaaa ggccatccgt cagga                                  35


<210>  110
<211>  34
<212>  DNA
<213>  Artificial Sequence

<220>
<223>  Forward primer for Pp_mdlC

<400>  110
cgcgcggcag ccatatggct tcggtacacg gcac                                   34


<210>  111
<211>  37
<212>  DNA
<213>  Artificial Sequence

<220>
<223>  Reverse primer for Pp_mdlC

<400>  111
ggatcctcga gcatatcact tcaccgggct tacggtg                                37

<210>  112
<211>  34
<212>  DNA
<213>  Artificial Sequence

<220>
<223>  Forward primer for Pp_mdlC

<400>  112
agaggagaca ccatatggct tcggtacacg gcac                                    34


<210>  113
<211>  36
<212>  DNA
<213>  Artificial Sequence

<220>
<223>  Reverse primer for Pp_mdlC

<400>  113
gtcgacgata tcatatcact tcaccgggct tacggt                                  36


<210>  114
<211>  39
<212>  DNA
<213>  Artificial Sequence

<220>
<223>  Forward primer for Kp_dhaT

<400>  114
cgcgcggcag ccatatgagc tatcgtatgt tcgattatc                               39


<210>  115
<211>  38
<212>  DNA
<213>  Artificial Sequence

<220>
<223>  Reverse primer for Kp_dhaT

<400>  115
ggatcctcga gcatattaga atgcctggcg gaaaatcg                                38


<210>  116
<211>  38
<212>  DNA
<213>  Artificial Sequence

<220>
<223>  Forward primer for Ll_ldhA

<400>  116
cgcgcggcag ccatatggct gataaacaac gtaagaaa                                38


<210>  117

<211>  38
<212>  DNA
<213>  Artificial Sequence

<220>
<223>  Reverse primer for Ll_ldhA

<400>  117
ggatcctcga gcatattagt ttttaactgc agaagcaa                              38


<210>  118
<211>  27
<212>  DNA
<213>  Artificial Sequence

<220>
<223>  Forward primer for Ll_ldhA

<400>  118
gctccatgca aaccaggtga aactcgt                                          27


<210>  119
<211>  27
<212>  DNA
<213>  Artificial Sequence

<220>
<223>  Reverse primer for Ll_ldhA

<400>  119
tggtttgcat ggagcaccag aagtcaa                                          27


<210>  120
<211>  38
<212>  DNA
<213>  Artificial Sequence

<220>
<223>  Forward primer for Ll_ldhA

<400>  120
aagtgggatc gcatatggct gataaacaac gtaagaaa                              38


<210>  121
<211>  39
<212>  DNA
<213>  Artificial Sequence

<220>
<223>  Reverse primer for Ll_ldhA

<400>  121
ccgccaggca ggatccttag tttttaactg cagaagcaa                             39


<210>  122
<211>  35
<212>  DNA
<213>  Artificial Sequence

<220>
<223>  Forward primer for Ls_leudh

<400>  122
cgcgcggcag ccatatggaa atcttcaagt atatg                                35


<210>  123
<211>  40
<212>  DNA
<213>  Artificial Sequence

<220>
<223>  Reverse primer for Ls_leudh

<400>  123
ggatcctcga gcatattaac ggccgttcaa aatatttttt                           40


<210>  124
<211>  36
<212>  DNA
<213>  Artificial Sequence

<220>
<223>  Forward primer for Ls_leudh

<400>  124
aagtgggatc gcatatggaa atcttcaagt atatgg                               36


<210>  125
<211>  40
<212>  DNA
<213>  Artificial Sequence

<220>
<223>  Reverse primer for Ls_leudh

<400>  125
ccgccaggca ggatccttaa cggccgttca aaatattttt                           40


<210>  126
<211>  37
<212>  DNA
<213>  Artificial Sequence

<220>
<223>  Forward primer for Sf_valdh

<400>  126
cgcgcggcag ccatatgacc gacgcgtccc accccac                              37


<210>  127
<211>  40
<212>  DNA
<213>  Artificial Sequence

<220>
<223>  Reverse primer for Sf_valdh

<400>  127
ggatcctcga gcatattaga cggtgcgggc ctccgccatg                              40


<210>  128
<211>  34
<212>  DNA
<213>  Artificial Sequence

<220>
<223>  Forward primer for Bb_phedh

<400>  128
cgcgcggcag ccatatgagc ttagtagaaa aaac                                    34


<210>  129
<211>  38
<212>  DNA
<213>  Artificial Sequence

<220>
<223>  Reverse primer for Bb_phedh

<400>  129
ggatcctcga gcatattagt tgcgaatatc ccattttg                                38


<210>  130
<211>  31
<212>  DNA
<213>  Artificial Sequence

<220>
<223>  Forward primer for AtCGS

<400>  130
aagtgggatc gcatatggtg cgccagctgt c                                       31


<210>  131
<211>  39
<212>  DNA
<213>  Artificial Sequence

<220>
<223>  Reverse primer for AtCGS

<400>  131
gtcgacgata tcatattaga ttgcttcgag tgcctgcag                               39


<210>  132
<211>  34
<212>  DNA
<213>  Artificial Sequence

<220>
<223>  Forward primer for Ec_eda

<400>  132
agaggagaca ccatatgaaa aactggaaaa caag                                    34

<210>  133
<211>  36
<212>  DNA
<213>  Artificial Sequence

<220>
<223>  Reverse primer for Ec_eda

<400>  133
ccgccaggca ggatccttac agcttagcgc cttcta                                36


<210>  134
<211>  34
<212>  DNA
<213>  Artificial Sequence

<220>
<223>  Forward primer for Ec_yjhH

<400>  134
agaggagaca ccatatgaaa aaattcagcg gcat                                  34


<210>  135
<211>  38
<212>  DNA
<213>  Artificial Sequence

<220>
<223>  Reverse primer for Ec_yjhH

<400>  135
ccgccaggca ggatccttag actggtaaaa tgccctgc                              38


<210>  136
<211>  34
<212>  DNA
<213>  Artificial Sequence

<220>
<223>  Forward primer for Ec_dgoA

<400>  136
agaggagaca ccatatgcag tggcaaacta aact                                  34


<210>  137
<211>  38
<212>  DNA
<213>  Artificial Sequence

<220>
<223>  Reverse primer for Ec_dgoA

<400>  137
ccgccaggca ggatccttat tgcactgcct ctcgatac                              38


<210>  138

<211>  34
<212>  DNA
<213>  Artificial Sequence

<220>
<223>  Forward primer for Ec_mhpFE

<400>  138
agaggagaca ccatatgagt aagcgtaaag tcgc                                34


<210>  139
<211>  36
<212>  DNA
<213>  Artificial Sequence

<220>
<223>  Reverse primer for Ec_mhpFE

<400>  139
ccgccaggca ggatccttat ttgttgttgc gcagat                              36


<210>  140
<211>  34
<212>  DNA
<213>  Artificial Sequence

<220>
<223>  Forward primer for Ec_garL

<400>  140
agaggagaca ccatatgaat aacgatgttt tccc                                34


<210>  141
<211>  36
<212>  DNA
<213>  Artificial Sequence

<220>
<223>  Reverse primer for Ec_garL

<400>  141
ccgccaggca ggatccttat tttttaaagg tatcag                              36


<210>  142
<211>  34
<212>  DNA
<213>  Artificial Sequence

<220>
<223>  Forward primer for Pp_galC

<400>  142
agaggagaca ccatatgagt acgctcgtgg gaaa                                34


<210>  143
<211>  37
<212>  DNA
<213>  Artificial Sequence

<220>
<223>  Reverse primer for Pp_galC

<400>  143
ccgccaggca ggatccttac tctcccagtt catccag                                37


<210>  144
<211>  34
<212>  DNA
<213>  Artificial Sequence

<220>
<223>  Forward primer for Ns_phdJ

<400>  144
agaggagaca ccatatgacg tcacccgccg tgac                                   34


<210>  145
<211>  37
<212>  DNA
<213>  Artificial Sequence

<220>
<223>  Reverse primer for Ns_phdJ

<400>  145
ccgccaggca ggatccttat ggttcggcga gagttgc                                37


<210>  146
<211>  34
<212>  DNA
<213>  Artificial Sequence

<220>
<223>  Forward primer for Bx_bphJI

<400>  146
agaggagaca ccatatgacg aaaaaaatca aatg                                   34


<210>  147
<211>  38
<212>  DNA
<213>  Artificial Sequence

<220>
<223>  Reverse primer for Bx_bphJI

<400>  147
ccgccaggca ggatccttat gcggcggtca aggtcaac                               38

## Description

### [Technical Field]

The present invention relates to a method for manufacturing organic compounds.

### [Background Art]

Currently, instead of petroleum resources which are considered the cause of global warming, it is strongly desired to produce chemical products using biological resources, which are renewable resources, as raw materials. For this reason, for the purpose of producing chemical products from biological materials such as saccharides, research has been actively conducted to produce chemical products used for industrial raw materials, fuel, feedstocks, food additives, and the like using microorganisms and their transformants. For example, it has been reported that organic compounds such as alcohols or amino acids can be manufactured from saccharides using a transformant of yeast or Escherichia coli (refer to US Patent No. 9926577 and US Patent No. 8647847).

### [Summary of Invention]

### [Technical Problem]

An objective of the present invention is to provide a novel method for producing an organic compound.

Production of chemical products using microorganisms is generally limited to microbial metabolites as production targets. In addition, even in the case of microbial metabolites, there are many microbial metabolites that are difficult to produce due to lack of energy in the microbial metabolic system and imbalance in the oxidation-reduction balance. One effective approach for solving such problems is to create a transformant. However, the inventors of the present invention consider that when attempting to solve the problems only by this method, it may take a lot of time to develop bacterial cells capable of producing a target compound. Meanwhile, they consider that, in the future, it is necessary to produce various compounds other than microbial metabolites from biological materials. Accordingly, the inventors of the present invention have examined development of a new process in which production of useful substances is not simply depend on creation of a transformant of microorganisms.

### [Solution to Problem]

As a result of intensive studies conducted by the inventors of the present invention, the inventors have found a production process in which a key intermediate for synthesizing useful compounds such as amino acids can be obtained by culturing a microorganism having a specific gene in the presence of a carbonyl compound, and therefore have completed the present invention.

According to a first aspect of the present invention, a method for producing an α-keto acid is provided, the method including:
a step of culturing a microorganism containing a gene encoding an enzyme that catalyzes an aldol reaction between pyruvic acid and a carbonyl compound in the presence of a pyruvic acid-supplying compound selected from pyruvic acid and saccharides, and a carbonyl compound represented by General Formula (1) to produce an α-keto acid represented by General Formula (2),

   General Formula (1): R₁₀₁C(O)R₁₀₂
where R₁₀₁ and R₁₀₂ are each independently hydrogen or an alkyl group having 1 to 5 carbon atoms, and

   General Formula (2): R₁₀₁C(R₁₀₂)(OH)CH₂COCOOH,
where R₁₀₁ and R₁₀₂ in General Formula (2) are respectively the same groups as R₁₀₁ and R₁₀₂ in General Formula (1).

According to a second aspect of the present invention, a method for producing a 1,3-diol is provided, the method including:
a step of culturing a microorganism containing the following genes in the presence of a pyruvic acid-supplying compound selected from pyruvic acid and saccharides, and a carbonyl compound represented by General Formula (3) to produce a 1,3-diol represented by General Formula (4),
   (a) first gene encoding an enzyme that catalyzes an aldol reaction between pyruvic acid and a carbonyl compound,
   (b) second gene encoding an enzyme that catalyzes a decarboxylation reaction of an α-keto acid, and
   (c) third gene encoding an enzyme that catalyzes a reduction reaction of an aldehyde,

      General Formula (3): R₁₀₃C(O)R₁₀₄,
where R₁₀₃ and R₁₀₄ are each independently hydrogen or an alkyl group having 1 to 5 carbon atoms, provided that in a case where R₁₀₃ is hydrogen, R₁₀₄ is not hydrogen, and

   General Formula (4): R₁₀₃C(R₁₀₄)(OH)CH₂CH₂OH,
where R₁₀₃ and R₁₀₄ in General Formula (4) are respectively the same groups as R₁₀₃ and R₁₀₄ in General Formula (3).

According to a third aspect of the present invention, a method for producing 1,3-butanediol is provided, the method including:
a step of culturing a microorganism containing the following genes in the presence of a pyruvic acid-supplying compound selected from pyruvic acid and saccharides to produce 1,3-butanediol,
(a) first gene encoding an enzyme that catalyzes an aldol reaction between pyruvic acid and a carbonyl compound,
(b) second gene encoding an enzyme that catalyzes a decarboxylation reaction of an α-keto acid, and
(c) third gene encoding an enzyme that catalyzes a reduction reaction of an aldehyde.

According to a fourth aspect of the present invention, a method for producing a 2,4-dihydroxycarboxylic acid is provided, the method including:
a step of culturing a microorganism containing the following genes in the presence of a pyruvic acid-supplying compound selected from pyruvic acid and saccharides, and a carbonyl compound represented by General Formula (1) to produce a 2,4-dihydroxycarboxylic acid represented by General Formula (5),
   (a) first gene encoding an enzyme that catalyzes an aldol reaction between pyruvic acid and a carbonyl compound, and
   (d) fourth gene encoding an enzyme that catalyzes a reduction reaction of an α-keto acid,

      General Formula (1): R₁₀₁C(O)R₁₀₂,
where R₁₀₁ and R₁₀₂ are each independently hydrogen or an alkyl group having 1 to 5 carbon atoms, and

   General Formula (5): R₁₀₁C(R₁₀₂)(OH)CH₂CH(OH)COOH,
where R₁₀₁ and R₁₀₂ in General Formula (5) are respectively the same groups as R₁₀₁ and R₁₀₂ in General Formula (1).

According to a fifth aspect of the present invention, a method for producing a 2-amino-4-hydroxycarboxylic acid is provided, the method including:
a step of reacting an α-keto acid represented by General Formula (2), a nitrogen source, and a reducing agent in the presence of glutamate/phenylalanine/leucine/valine dehydrogenase to produce a 2-amino-4-hydroxycarboxylic acid represented by General Formula (6),

   General Formula (2): R₁₀₁C(R₁₀₂)(OH)CH₂COCOOH,
where R₁₀₁ and R₁₀₂ are each independently hydrogen or an alkyl group having 1 to 5 carbon atoms, and

   General Formula (6): R₁₀₁C(R₁₀₂)(OH)CH₂CH(NH₂)COOH,
where R₁₀₁ and R₁₀₂ in General Formula (6) are respectively the same groups as R₁₀₁ and R₁₀₂ in General Formula (2).

According to a sixth aspect of the present invention, a method for producing an α-keto acid is provided, the method including:
a step of reacting a 2-amino-4-hydroxycarboxylic acid represented by General Formula (6) and an oxidizing agent in the presence of glutamate/phenylalanine/leucine/valine dehydrogenase to produce an α-keto acid represented by General Formula (2),

   General Formula (6): R₁₀₁C(R₁₀₂)(OH)CH₂CH(NH₂)COOH,
where R₁₀₁ and R₁₀₂ are each independently hydrogen or an alkyl group having 1 to 5 carbon atoms, and

   General Formula (2): R₁₀₁C(R₁₀₂)(OH)CH₂COCOOH,
where R₁₀₁ and R₁₀₂ in General Formula (2) are respectively the same groups as R₁₀₁ and R₁₀₂ in General Formula (6).

According to a seventh aspect of the present invention, a method for producing a 2-amino-4-hydroxycarboxylic acid is provided, the method including:
a step of culturing a microorganism containing the following genes in the presence of a pyruvic acid-supplying compound selected from pyruvic acid and saccharides, and a carbonyl compound represented by General Formula (1) to produce a 2-amino-4-hydroxycarboxylic acid represented by General Formula (6),
   (a) first gene encoding an enzyme that catalyzes an aldol reaction between pyruvic acid and a carbonyl compound, and
   (e) fifth gene encoding an enzyme that catalyzes a reductive amination reaction of an α-keto acid,

      General Formula (1): R₁₀₁C(O)R₁₀₂,
where R₁₀₁ and R₁₀₂ are each independently hydrogen or an alkyl group having 1 to 5 carbon atoms, and

   General Formula (6): R₁₀₁C(R₁₀₂)(OH)CH₂CH(NH₂)COOH,
where R₁₀₁ and R₁₀₂ in General Formula (6) are respectively the same groups as R₁₀₁ and R₁₀₂ in General Formula (1).

According to an eighth aspect of the present invention, a method for producing at least one of homoserine, threonine, and 2-aminobutyric acid is provided, the method including:
a step of culturing a microorganism containing the following genes in the presence of a pyruvic acid-supplying compound selected from pyruvic acid and saccharides, and formaldehyde to produce at least one of homoserine, threonine, and 2-aminobutyric acid,
(a) first gene encoding an enzyme that catalyzes an aldol reaction between pyruvic acid and a carbonyl compound, and
(e) fifth gene encoding an enzyme that catalyzes a reductive amination reaction of an α-keto acid.

According to a ninth aspect of the present invention, a method for producing a 2-amino-4-methylthiocarboxylic acid is provided, the method including:
a step of culturing a microorganism containing the following genes in the presence of a pyruvic acid-supplying compound selected from pyruvic acid and saccharides, methyl mercaptan, and a carbonyl compound represented by General Formula (1) to produce a 2-amino-4-methylthiocarboxylic acid represented by General Formula (7),
   (a) first gene encoding an enzyme that catalyzes an aldol reaction between pyruvic acid and a carbonyl compound,
   (e) fifth gene encoding an enzyme that catalyzes a reductive amination reaction of an α-keto acid, and
   (f) sixth gene encoding an enzyme that catalyzes a reaction of substituting a phosphoric acid group contained in 2-amino-4-phosphonooxycarboxylic acid with a methylmercapto group contained in methyl mercaptan,

      General Formula (1): R₁₀₁C(O)R₁₀₂,
where R₁₀₁ and R₁₀₂ are each independently hydrogen or an alkyl group having 1 to 5 carbon atoms, and

   General Formula (7): R₁₀₁C(R₁₀₂)(SCH₃)CH₂CH(NH₂)COOH,
where R₁₀₁ and R₁₀₂ in General Formula (7) are respectively the same groups as R₁₀₁ and R₁₀₂ in General Formula (1).

According to a tenth aspect of the present invention, a coryneform bacterium is provided, the coryneform bacterium including:
a first gene selected from the group consisting of the following genes,
(1-1) gene encoding a protein that consists of an amino acid sequence set forth in SEQ ID NO: 2 and catalyzes an aldol reaction between pyruvic acid and a carbonyl compound,
(1-2) gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound,
(1-3) gene encoding a protein that consists of an amino acid sequence set forth in SEQ ID NO: 4 and catalyzes an aldol reaction between pyruvic acid and a carbonyl compound,
(1-4) gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound,
(1-5) gene encoding a protein that consists of an amino acid sequence set forth in SEQ ID NO: 6 and catalyzes an aldol reaction between pyruvic acid and a carbonyl compound, and
(1-6) gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound.

According to the present invention, a method for producing an organic compound, and a coryneform bacterium that can be used in this production method are provided.

### [Brief Description of Drawings]

FIG. 1 is a schematic diagram showing a process in which a microorganism produces 4-hydroxy-2-oxobutyric acid.
FIG. 2 is a schematic diagram showing a process in which a microorganism produces 1,3-butanediol.
FIG. 3 is a schematic diagram showing a process in which a microorganism produces 2,4-dihydroxybutyric acid.
FIG. 4 is a schematic diagram showing a process in which a microorganism produces homoserine.
FIG. 5 is a schematic diagram showing a process in which a microorganism produces methionine.
FIG. 6 is a graph showing results of gas chromatography-mass spectrometry.
FIG. 7 is a graph showing results of gas chromatography-mass spectrometry.
FIG. 8 is a graph showing results of gas chromatography-mass spectrometry.

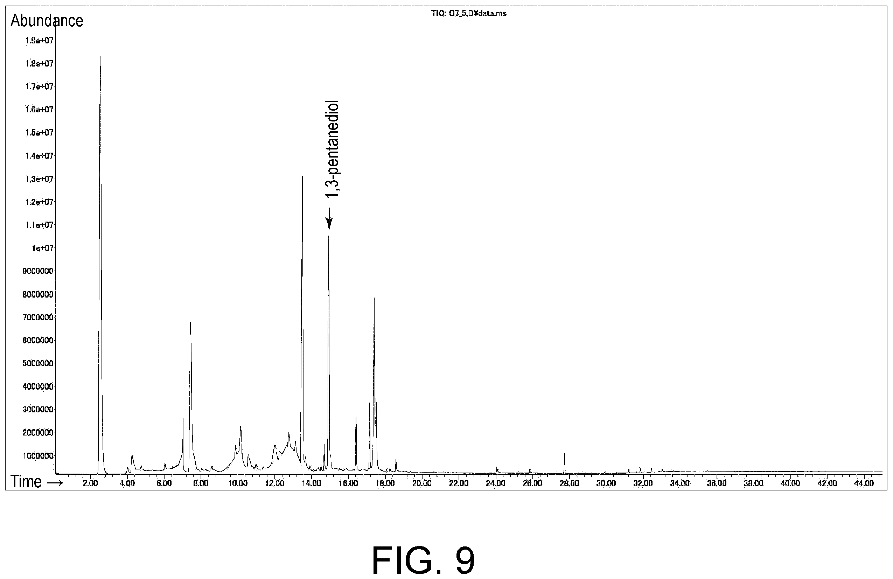

FIG. 9 is a graph showing results of gas chromatography-mass spectrometry.

### [Description of Embodiments]

Hereinafter, the present invention will be described in detail, but the following description is intended to explain the present invention in detail and is not intended to limit the present invention.

### (1.) Method for producing α-keto acid

A method for producing an α-keto acid includes a step of culturing a microorganism containing a gene encoding an enzyme that catalyzes an aldol reaction between pyruvic acid and a carbonyl compound in the presence of a pyruvic acid-supplying compound selected from pyruvic acid and saccharides, and a carbonyl compound represented by General Formula (1) to produce an α-keto acid represented by General Formula (2).

General Formula (1): R₁₀₁C(O)R₁₀₂,

where R₁₀₁ and R₁₀₂ are each independently hydrogen or an alkyl group having 1 to 5 carbon atoms,

General Formula (2): R₁₀₁C(R₁₀₂)(OH)CH₂COCOOH,

where R₁₀₁ and R₁₀₂ in General Formula (2) are respectively the same groups as R₁₀₁ and R₁₀₂ in General Formula (1).

In the following description, the above-mentioned microorganism containing a gene (hereinafter, referred to as a first gene) encoding an enzyme that catalyzes an aldol reaction between pyruvic acid and a carbonyl compound is also referred to as an "a-keto acid-producing microorganism." The α-keto acid-producing microorganism may be a microorganism that naturally has the first gene, or may be a microorganism obtained by transforming a host with the first gene. Hereinafter, a case in which the α-keto acid-producing microorganism is a transformant will be described as an example.

### (1.1) Transformant

### (1.1.1) Host

Any host can be used as a host. As the host, for example, it is possible to use yeasts and bacteria, more specifically, it is possible to use aerobic bacteria, and even more specifically, it is possible to use coryneform bacteria, *Escherichia coli,* or *Vibrio natriegens.* The host is preferably coryneform bacteria.

Coryneform bacteria are a group of microorganisms defined in Bargeys Manual of Determinative Bacteriology, Vol. 8, 599 (1974), and are not particularly limited as long as they grow in general aerobic conditions. Specific examples thereof include bacteria belonging to the genus *Corynebacterium,* bacteria belonging to the genus *Brevibacterium,* bacteria belonging to the genus *Arthrobacter,* bacteria belonging to the genus *Mycobacterium,* bacteria belonging to the genus *Micrococcus,* and the like. Among the coryneform bacteria, bacteria belonging to the genus *Corynebacterium* are preferable.

Examples of bacteria belonging to the genus *Corynebacterium* include *Corynebacterium glutamicum, Corynebacterium* v, *Corynebacterium ammoniagenes, Corynebacterium halotolerance, Corynebacterium alkanolyticum,* and the like. Among them, *Corynebacterium glutamicum* is preferable in terms of high productivity of α-keto acids. Preferable bacterial strains thereof include the strains ATCC 13032, ATCC 13869, ATCC 13058, ATCC 13059, ATCC 13060, ATCC 13232, ATCC 13286, ATCC 13287, ATCC 13655, ATCC 13745, ATCC 13746, ATCC 13761, ATCC 14020, ATCC 31831, MJ-233 (FERM BP-1497), MJ-233AB-41 (FERM BP-1498), and the like. Among them, the strains ATCC 13032 and ATCC 13869 are preferable.

According to molecular biology classification, bacterial names of coryneform bacteria such as *Brevibacterium flavum, Brevibacterium lactofermentum, Brevibacterium divaricatum,* and *Corynebacterium lilium* are unified by being classified as *Corynebacterium glutamicum* (Liebl, W. et al., Transfer of Brevibacterium divaricatum DSM 20297T, "Brevibacterium flavum" DSM 20411, "Brevibacterium lactofermentum" DSM 20412 and DSM 1412, and Corynebacterium glutamicum and their distinction by rRNA gene restriction patterns. Int J Syst Bacteriol. 41: 255-260. (1991), Kazuo Komagata et al., Classification of Coryneform Bacteria, Fermentation and Industry, 45: 944-963 (1987)).

The strain ATCC 13869 of *Brevibacterium lactofermentum,* the strains MJ-233 (FERM BP-1497) and MJ-233AB-41 of *Brevibacterium flavum* (FERM BP-1498), and the like in the old classification are also suitable *Corynebacterium glutamicum.*

Examples of the genus *Brevibacterium* include *Brevibacterium ammoniagenes* (for example, the strain ATCC 6872) and the like.

Examples of the genus *Arthrobacter* include *Arthrobacter globiformis* (for example, the strains ATCC 8010, ATCC 4336, ATCC 21056, ATCC 31250, ATCC 31738, and ATCC 35698), and the like.

Examples of the genus *Mycobacterium* include *Mycobacterium bovis* (for example, the strains ATCC 19210 and ATCC 27289), and the like.

Examples of the genus *Micrococcus* include *Micrococcus freudenreichii* (for example, No. 239 strain (FERM P-13221)), *Micrococcus leuteus* (for example, No. 240 strain (FERM P-13222)), *Micrococcus ureae* (for example, the strain IAM1010), *Micrococcus roseus* (for example, the strain IF03964), and the like.

Next, the first, second, and third genes to be introduced into a host will be described.

### (1.1.2) First gene

The first gene encodes an "enzyme that catalyzes an aldol reaction between pyruvic acid and a carbonyl compound". The "enzyme that catalyzes an aldol reaction between pyruvic acid and a carbonyl compound" refers to an enzyme that has an activity of 10 mU/mg protein or higher when being measured according to the measurement method mentioned hereinafter and using an enzymatic reaction solution containing a purified protein, pyruvic acid (an initial substrate concentration 1 mM) and a carbonyl compound (an initial substrate concentration 1 mM). The carbonyl compounds in the "enzyme that catalyzes an aldol reaction between pyruvic acid and a carbonyl compound" are, for example, formaldehyde, acetaldehyde, propionaldehyde, butyraldehyde, isobutyraldehyde, valeraldehyde or acetone. The butyraldehyde is, in specific, n-butyraldehyde or isobutyraldehyde. The valeraldehyde is, in specific, n-valeraldehyde. The "enzyme that catalyzes an aldol reaction between pyruvic acid and a carbonyl compound" is, for example, an aldolase.

The first gene may be a gene encoding an aldolase to be exemplified below.
(a1) 2-Dehydro-3-deoxy-phosphogluconate aldolase (EC number: 4.1.2.14),
(a2) 2-Dehydro-3-deoxy-6-phosphogluconate aldolase (EC number: 4.1.2.55),
(a3) 4-Hydroxy-2-oxoglutarate aldolase (EC number: 4.1.3.16),
(a4) (4S)-4-Hydroxy-2-oxoglutarate aldolase (EC number; 4.1.3.42),
(a5) 2-Dehydro-3-deoxy-D-pentonate aldolase (EC number: 4.1.2.28),
(a6) 2-Dehydro-3-deoxy-D-gluconate aldolase (EC number: 4.1.2.51),
(a7) 3-Deoxy-D-manno-octulosonate aldolase (EC number: 4.1.2.23),
(a8) 4-Hydroxyl-2-oxovalerate aldolase (EC number: 4.1.3.39),
(a9) 4-(2-Carboxyphenyl)-2-oxobut-3-enoate aldolase (EC number: 4.1.2.34),
(a10) 4-Hydroxy-2-oxoheptanedioate aldolase (EC number: 4.1.2.52),
(a11) 2-Dehydro-3-deoxyglucarate aldolase (EC number: 4.1.2.20),
(a12) 2-Keto-3-deoxy-L-rhamnonate aldolase (EC number: 4.1.2.53),
(a13) 4-Hydroxyl-4-methyl-2-oxoglutarate aldolase (EC number: 4.1.3.17), and
(a14) 4-Hydroxy-2-oxohexanoate aldolase (EC number: 4.1.3.43).

Examples of aldolases included in (a1), (a2), (a3), and (a4) include an eda protein. The eda protein is preferably derived from *Escherichia coli.*

Examples of aldolases included in (a2) include a dgoA protein. The dgoA protein is preferably derived from *Escherichia coli.*

Examples of aldolases included in (a5) include a yjhH protein. The yjhH protein is preferably derived from *Escherichia coli.*

Examples of aldolases included in (a6) include a yagE protein. The yagE protein is preferably derived from *Escherichia coli.*

Examples of aldolases included in (a7) include a nanA protein. The nanA protein is preferably derived from *Escherichia coli.*

Examples of aldolases included in (a8) include an mhpE protein. The mhpE protein is preferably derived from *Escherichia coli.* When the first gene is an mhpE gene, the mhpE gene may be introduced into a host in a state of being linked to an mhpF gene.

Examples of aldolases included in (a8) and (a10) include an hpal protein. The hpal protein is preferably derived from *Escherichia coli.*

Examples of aldolases included in (a8) and (a14) include a bphl protein. The bphl protein is preferably derived from *Burkholderia xenovorans.* When the first gene is a bphl gene, the bphl gene may be introduced into a host in a state of being linked to a bphJ gene.

Examples of aldolases included in (a9) include a phdJ protein. The phdJ protein is preferably derived from bacteria belonging to *Nocardioides* sp.

Examples of aldolases included in (a11) include a garL protein. The garL protein is preferably derived from *Escherichia coli.*

Examples of aldolases included in (a12) include a rhmA protein. The rhmA protein is preferably derived from *Escherichia coli.*

Examples of aldolases included in (a13) include a galC protein. The galC protein is preferably derived from *Pseudomonas putida.*

The first gene may encode class I aldolases or class II aldolases.

Examples of class I aldolases include an eda protein, a yjhH protein, a yagE protein, a dgoA protein, a nanA protein, a mphE protein, and a phdJ protein.

Examples of class II aldolases include a hpal protein, a garL protein, a rhmA protein, a galC protein, and bphl protein.

Preferably, the first gene is selected from the following group consisting of:
(1-1) a gene encoding a protein (that is, a hpal protein derived from *Escherichia coli*) that consists of an amino acid sequence set forth in SEQ ID NO: 2 and catalyzes an aldol reaction between pyruvic acid and a carbonyl compound;
(1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
(1-3) a gene encoding a protein (that is, a rhmA protein derived from *Escherichia coli*) that consists of an amino acid sequence set forth in SEQ ID NO: 4 and catalyzes an aldol reaction between pyruvic acid and a carbonyl compound;
(1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
(1-5) a gene encoding a protein (that is, a nanA protein derived from *Escherichia coli*) that consists of an amino acid sequence set forth in SEQ ID NO: 6 and catalyzes an aldol reaction between pyruvic acid and a carbonyl compound;
(1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
(1-7) a gene encoding a protein (that is, an eda protein derived from *Escherichia coli*) that consists of an amino acid sequence set forth in SEQ ID NO: 8 and catalyzes an aldol reaction between pyruvic acid and a carbonyl compound;
(1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound;
(1-9) a gene encoding a protein (that is, a yjhH protein derived from *Escherichia coli*) that consists of an amino acid sequence set forth in SEQ ID NO: 10 and catalyzes an aldol reaction between pyruvic acid and a carbonyl compound;
(1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound;
(1-11) a gene encoding a protein (that is, a dgoA protein derived from *Escherichia coli*) that consists of an amino acid sequence set forth in SEQ ID NO: 12 and catalyzes an aldol reaction between pyruvic acid and a carbonyl compound;
(1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
(1-13) a gene encoding a protein (that is, an mhpE protein derived from *Escherichia coli*) that consists of an amino acid sequence set forth in SEQ ID NO: 14 and catalyzes an aldol reaction between pyruvic acid and a carbonyl compound;
(1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
(1-15) a gene encoding a protein (that is, a garL protein derived from *Escherichia coli*) that consists of an amino acid sequence set forth in SEQ ID NO: 16 and catalyzes an aldol reaction between pyruvic acid and a carbonyl compound;
(1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
(1-17) a gene encoding a protein (that is, a galC protein derived from *Pseudomonas putida*) that consists of an amino acid sequence set forth in SEQ ID NO: 18 and catalyzes an aldol reaction between pyruvic acid and a carbonyl compound;
(1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
(1-19) a gene encoding a protein (that is, a phdJ protein derived from bacteria belonging to *Nocardioides* sp.) that consists of an amino acid sequence set forth in SEQ ID NO: 20 and catalyzes an aldol reaction between pyruvic acid and a carbonyl compound;
(1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
(1-21) a gene encoding a protein (that is, a bphl protein derived from *Burkholderia xenovorans*) that consists of an amino acid sequence set forth in SEQ ID NO: 22 and catalyzes an aldol reaction between pyruvic acid and a carbonyl compound;
(1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound;

In the present specification, regarding "one or a plurality of amino acids" in the expression, "one or a plurality of amino acids is deleted, substituted, or added," the number thereof varies depending on positions of amino acid residues in a three-dimensional structure of a protein or types of amino acid residue, but it is preferably 1 to 60, is more preferably 1 to 30, is even more preferably 1 to 15, is still more preferably 1 to 10, and is yet more preferably 1 to 5.

The above-described deletion, substitution, or addition of one or a plurality of amino acids is, for example, a conservative mutation that enables a protein to maintain its normal function. A typical conservative mutation is a conservative substitution. A conservative substitution is a mutation in which a substitution occurs between Phe, Trp, and Tyr when a substitution site is an aromatic amino acid, in which a substitution occurs between Leu, Ile, and Val when a substitution site is a hydrophobic amino acid, in which a substitution occurs between Gln and Asn when a substitution site is a polar amino acid, in which a substitution occurs between Lys, Arg, and His when a substitution site is a basic amino acid, in which a substitution occurs between Asp and Glu when a substitution site is an acidic amino acid, and in which a substitution occurs between Ser and Thr when a substitution site is an amino acid having a hydroxyl group. Specific examples of substitutions regarded as conservative substitutions include a substitution of Ala to Ser or Thr; a substitution of Arg to Gln, His, or Lys; a substitution of Asn to Glu, Gln, Lys, His, or Asp; a substitution of Asp to Asn, Glu, or Gln; a substitution of Cys to Ser or Ala; a substitution of Gln to Asn, Glu, Lys, His, Asp, or Arg; a substitution of Glu to Gly, Asn, Gln, Lys, or Asp; a substitution of Gly to Pro; a substitution of His to Asn, Lys, Gln, Arg, or Tyr; a substitution of Ile to Leu, Met, Val, or Phe; a substitution of Leu to Ile, Met, Val, or Phe; a substitution of Lys to Asn, Glu, Gln, His, or Arg; a substitution of Met to Ile, Leu, Val, or Phe; a substitution of Phe to Trp, Tyr, Met, Ile, or Leu; a substitution of Ser to Thr or Ala; a substitution of Thr to Ser or Ala; a substitution of Trp to Phe or Tyr; a substitution of Tyr to His, Phe, or Trp; and a substitution of Val to Met, Ile, or Leu. In addition, deletions, substitutions, or additions of amino acids include deletions, substitutions, or additions caused by naturally occurring mutations (mutants or variants) which are based on individual differences or species differences in bacteria from which a gene is derived.

A "gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound" may be a "gene encoding a protein that consists of an amino acid sequence showing a homology of 80% or more, preferably 90% or more, more preferably 95% or more, even more preferably 97% or more, still more preferably 98% or more, and yet more preferably 99% or more to an amino acid sequence set forth in SEQ ID NO: 2, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound" may be a "gene encoding a protein that consists of an amino acid sequence showing a homology of 80% or more, preferably 90% or more, more preferably 95% or more, even more preferably 97% or more, still more preferably 98% or more, and yet more preferably 99% or more to an amino acid sequence set forth in SEQ ID NO: 4, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 6, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 8, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 10, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 12, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 14, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 16, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 18, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 20, which is a protein having an activity of catalyzing an aldol reaction between pyruvic acid and a carbonyl compound."

A "gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound" may be a "gene encoding a protein that consists of an amino acid sequence showing a homology of 80% or more, preferably 95% or more, more preferably 97% or more, even more preferably 98% or more, and still more preferably 99% or more to an amino acid sequence set forth in SEQ ID NO: 22, which is a protein having an activity of catalyzing an aldol reaction between pyruvic acid and a carbonyl compound."

A UniProtKB accession number of a hpal protein derived from *Escherichia coli* is, for example, Q47098; a UniProtKB accession number of a rhmA protein derived from *Escherichia coli* is, for example, P76469; and a UniProtKB accession number of a nanA protein derived from *Escherichia coli* is, for example, P0A6L4, a UniProtKB accession number of an eda protein derived from *Escherichia coli* is, for example, P0A955; a UniProtKB accession number of a yjhH protein derived from *Escherichia coli* is, for example, P39359; a UniProtKB accession number of a dgoA protein derived from *Escherichia coli* is, for example, Q6BF16; a UniProtKB accession number of an mhpE protein derived from *Escherichia coli* is, for example, P51020; a UniProtKB accession number of a garL protein derived from *Escherichia coli* is, for example, P23522; a UniProtKB accession number of a galC protein derived from *Pseudomonas putida* is, for example, Q88JX9; a UniProtKB accession number of a phdJ protein derived from bacteria belonging to *Nocardioides* sp. is, for example, Q79EM8; a UniProtKB accession number of a bphl protein derived from *Burkholderia xenovorans* is, for example, P51015.

More preferably, the first gene is
a hpal gene which is derived from *Escherichia coli* and consists of a base sequence set forth in SEQ ID NO: 1,
a rhmA gene which is derived from *Escherichia coli* and consists of a base sequence set forth in SEQ ID NO: 3,
a nanA gene which is derived from *Escherichia coli* and consists of a base sequence set forth in SEQ ID NO: 5.
an eda gene which is derived from *Escherichia coli* and consists of a base sequence set forth in SEQ ID NO: 7,
a yjhH gene which is derived from *Escherichia coli* and consists of a base sequence set forth in SEQ ID NO: 9,
a dgoA gene which is derived from *Escherichia coli* and consists of a base sequence set forth in SEQ ID NO: 11,
an mhpE gene which is derived from *Escherichia coli* and consists of a base sequence set forth in SEQ ID NO: 13,
a garL gene which is derived from *Escherichia coli* and consists of a base sequence set forth in SEQ ID NO: 15,
a galC gene which is derived from *Pseudomonas putida* and consists of a base sequence set forth in SEQ ID NO: 17,
a phdJ gene which is derived from bacteria belonging to *Nocardioides* sp. and consists of a base sequence set forth in SEQ ID NO: 19, or
a bphl gene which is derived from *Burkholderia xenovorans* and consists of a base sequence set forth in SEQ ID NO: 21.

Base sequences set forth in SEQ ID NO: 1, 3, 5, 7, 9, 11, 13, 15, 17, 19 and 21 and amino acid sequences set forth in SEQ ID NO: 2, 4, 6, 8, 10, 14, 16, 18, 20 and 22 are as follows.

**[Table 1]**

| |
|---|
| (SEQ ID NO: 1) Ec_hpaI gene |
| atgGAAAACAGTTTTAAAGCGGCGCTGAAAGCAGGCCGTCCGCAGATTGGATTATGGCTG GGGCTGAGCAGCAGCTACAGCGCGGAGTTACTGGCCGGAGCAGGATTCGACTGGTTGTTG ATCGACGGTGAGCACGCACCGAACAACGTACAAACCGTGCTCACCCAGCTACAGGCGATT GCGCCCTATCCCAGCCAGCCGGTAGTACGTCCGTCGTGGAACGATCCGGTGCAAATCAAA CAACTGCTGGACGTCGGCACACAAACCTTACTGGTGCCGATGGTACAAAACGCCGACGAA GCCCGTGAAGCGGTACGCGCCACCCGTTATCCCCCCGCCGGTATTCGCGGTGTGGGCAGT GCGCTGGCTCGCGCCTCGCGCTGGAATCGCATTCCTGATTACCTGCAAAAAGCCAACGAT CAAATGTGCGTGCTGGTGCAGATCGAAACGCGTGAGGCAATGAAGAACTTACCGCAGATT CTGGACGTGGAAGGCGTCGACGGCGTGTTTATCGGCCCGGCGGATCTGAGCGCCGATATG GGTTATGCCGGTAATCCGCAGCACCCGGAAGTACAGGCCGCCATTGAGCAGGCGATCGTG CAGATCCGCGAAGCGGGCAAAGCGCCGGGGATCCTGATCGCCAATGAGCTACTGGCAAAA CGCTATCTGGAACTGGGCGCGCTGTTTGTCGCCGTCGGCGTTGACACCACCCTGCTCGCC CGCGCCGCCGAAGCGCTGGCAGCACGGTTTGGCGCGCAGGCTACAGCGATTAAGCCCGGC GTGTATtaa |

**[Table 2]**

| |
|---|
| (SEQ ID NO: 2) Ec_hpaI protein |
| MENSFKAALKAGRPQIGLWLGLSSSYSAELLAGAGFDWLLIDGEHAPNNVQTVLTQLQAI APYPSQPVVRPSWNDPVQIKQLLDVGTQTLLVPMVQNADEAREAVRATRYPPAGIRGVGS ALARASRWNRIPDYLQKANDQMCVLVQIETREAMKNLPQILDVEGVDGVFIGPADLSADM GYAGNPQHPEVQAAIEQAIVQIREAGKAPGILIANELLAKRYLELGALFVAVGVDTTLLA RAAEALAARFGAQATAIKPGVY |

**[Table 3]**

| |
|---|
| (SEQ ID NO: 3) Ec_rhmA gene |
| atgAACGCATTATTAAGCAATCCCTTTAAAGAACGTTTACGCAAGGGCGAAGTGCAAATT GGTCTGTGGTTAAGCTCAACGACTGCCTATATGGCAGAAATTGCCGCCACTTCTGGTTAT GACTGGTTGCTGATTGACGGGGAGCACGCGCCAAACACCATTCAGGATCTTTATCATCAG CTACAGGCGGTAGCGCCCTATGCCAGCCAACCCGTGATCCGTCCGGTGGAAGGCAGTAAA CCGCTGATTAAACAAGTCCTGGATATTGGCGCAAACTCTACTGATCCCGATGGTCGAT ACTGCCGAACAGGCACGTCAGGTGGTGTCTGCCACGCGCTATCCTCCCTACGGTGAGCGT GGTGTCGGGGCCAGTGTGGCACGGGCTGCGCGCTGGGGACGCATTGAGAATTACATGGCG CAAGTTAACGATTCGCTTTGTCTGTTGGTGCAGGTGGAAAGTAAAACGGCACTGGATAAC CTGGACGAAATCCTCGACGTCGAAGGGATTGATGGCGTGTTTATTGGACCTGCGGATCTT TCTGCGTCGTTGGGCTACCCGGATAACGCCGGGCACCCGGAAGTGCAGCGAATTATTGAA ACCAGTATTCGGCGGATCCGTGCTGCGGGTAAAGCGGCTGGTTTCTGGCTGTGGCTCCT GATATGGCGCAGCAATGCCTGGCGTGGGGAGCGAACTTTGTCGCTGTTGGCGTTGACACG ATGCTCTACAGCGATGCCCTGGATCAACGACTGGCGATGTTTAAATCAGGCAAAAATGGG CCACGCATAAAAGGTAGTTATtaa |

**[Table 4]**

| |
|---|
| (SEQ ID NO: 4) Ec_rhmA protein |
| MNALLSNPFKERLRKGEVQIGLWLSSTTAYMAEIAATSGYDWLLIDGEHAPNTIQDLYHQ LQAVAPYASQPVIRPVEGSKPLIKQVLDIGAQTLLIPMVDTAEQARQVVSATRYPPYGER GVGASVARAARWGRIENYMAQVNDSLCLLVQVESKTALDNLDEILDVEGIDGVFIGPADL SASLGYPDNAGHPEVQRIIETSIRRIRAAGKAAGFLAVAPDMAQQCLAWGANFVAVGVDT MLYSDALDQRLAMFKSGKNGPRIKGSY |

**[Table 5]**

| |
|---|
| (SEQ ID NO: 5) Ec_nanA gene |
| atgGCAACGAATTTACGTGGCGTAATGGCTGCACTCCTGACTCCTTTTGACCAACAACAA GCACTGGATAAAGCGAGTCTGCGTCGCCTGGTTCAGTTCAATATTCAGCAGGGCATCGAC GGTTTATACGTGGGTGGTTCGACCGGCGAGGCCTTTGTACAAAGCCTTTCCGAGCGTGAA CAGGTACTGGAAATCGTCGCCGAAGAGGCGAAAGGTAAGATTAAACTCATCGCCCACGTC GGTTGCGTCAGCACCGCCGAAAGCCAACAACTTGCGGCATCGGCTAAACGTTATGGCTTC GATGCCGTCTCCGCCGTCACGCCGTTCTACTATCCTTTCAGCTTTGAAGAACACTGCGAT CACTATCGGGCAATTATTGATTCGGCGGATGGTTTGCCGATGGTGGTGTACAACATTCCA GCCCTGAGTGGGGTAAAACTGACCCTGGATCAGATCAACACACTTGTTACATTGCCTGGC GTAGGTGCGCTGAAACAGACCTCTGGCGATCTCTATCAGATGGAGCAGATCCGTCGTGAA CATCCTGATCTTGTGCTCTATAACGGTTACGACGAAATCTTCGCCTCTGGTCTGCTGGCG |
| GGCGCTGATGGTGGTATCGGCAGTACCTACAACATCATGGGCTGGCGCTATCAGGGGATC GTTAAGGCGCTGAAAGAAGGCGATATCCAGACCGCGCAGAAACTGCAAACTGAATGCAAT AAAGTCATTGATTTACTGATCAAAACGGGCGTATTCCGCGGCCTGAAAACTGTCCTCCAT TATATGGATGTCGTTTCTGTGCCGCTGTGCCGCAAACCGTTTGGACCGGTAGATGAAAAA TATCTGCCAGAACTGAAGGCGCTGGCCCAGCAGTTGATGCAAGAGCGCGGGtaa |

**[Table 6]**

| |
|---|
| (SEQ ID NO: 6) Ec_nanA protein |
| MATNLRGVMAALLTPFDQQQALDKASLRRLVQFNIQQGIDGLYVGGSTGEAFVQSLSERE QVLEIVAEEAKGKIKLIAHVGCVSTAESQQLAASAKRYGFDAVSAVTPFYYPFSFEEHCD HYRAIIDSADGLPMVVYNIPALSGVKLTLDQINTLVTLPGVGALKQTSGDLYQMEQIRRE HPDLVLYNGYDEIFASGLLAGADGGIGSTYNIMGWRYQGIVKALKEGDIQTAQKLQTECN KVIDLLIKTGVFRGLKTVLHYMDVVSVPLCRKPFGPVDEKYLPELKALAQQLMQERG |

**[Table 7]**

| |
|---|
| (SEQ ID NO: 7) Ec_eda gene |
| atgAAAAACTGGAAAACAAGTGCAGAATCAATCCTGACCACCGGCCCGGTTGTACCGGTT ATCGTGGTAAAAAAACTGGAACACGCGGTGCCGATGGCAAAAGCGTTGGTTGCTGGTGGG GTGCGCGTTCTGGAAGTGACTCTGCGTACCGAGTGTGCAGTTGACGCTATCCGTGCTATC GCCAAAGAAGTGCCTGAAGCGATTGTGGGTGCCGGTACGGTGCTGAATCCACAGCAGCTG GCAGAAGTCACTGAAGCGGGTGCACAGTTCGCAATTAGCCCGGGTCTGACCGAGCCGCTG CTGAAAGCTGCTACCGAAGGGACTATTCCTCTGATTCCGGGGATCAGCACTGTTTCCGAA CTGATGCTGGGTATGGACTACGGTTTGAAAGAGTTCAAATTCTTCCCGGCTGAAGCTAAC GGCGGCGTGAAAGCCCTGCAGGCGATCGCGGGTCCGTTCTCCCAGGTCCGTTTCTGCCCG ACGGGTGGTATTTCTCCGGCTAACTACCGTGACTACCTGGCGCTGAAAAGCGTGCTGTGC ATCGGTGGTTCCTGGCTGGTTCCGGCAGATGCGCTGGAAGCGGGCGATTACGACCGCATT ACTAAGCTGGCGCGTGAAGCTGTAGAAGGCGCTAAGCTGtaa |

**[Table 8]**

| |
|---|
| (SEQ ID NO: 8) Ec_eda protein |
| MKNWKTSAESILTTGPVVPVIVVKKLEHAVPMAKALVAGGVRVLEVTLRTECAVDAIRAI AKEVPEAIVGAGTVLNPQQLAEVTEAGAQFAISPGLTEPLLKAATEGTIPLIPGISTVSE LMLGMDYGLKEFKFFPAEANGGVKALQAIAGPFSQVRFCPTGGISPANYRDYLALKSVLC IGGSWLVPADALEAGDYDRITKLAREAVEGAKL |

**[Table 9]**

| |
|---|
| (SEQ ID NO: 9) Ec_yjhH gene |
| atgAAAAAATTCAGCGGCATTATTCCACCGGTATCCAGCACGTTTCATCGTGACGGAACC CTTGATAAAAAGGCAATGCGCGAAGTTGCCGACTTCCTGATTAATAAAGGGGTCGACGGG CTGTTTTATCTGGGTACCGGTGGTGAATTTAGCCAAATGAATACAGCCCAGCGCATGGCA CTCGCCGAAGAAGCTGTAACCATTGTCGACGGGCGAGTGCCGGTATTGATTGGCGTCGGT TCCCCTTCCACTGACGAAGCGGTCAAACTGGCGCAGCATGCGCAAGCCTACGGCGCTGAT GGTATCGTCGCCATCAACCCCTACTACTGGAAAGTCGCACCACGAAATCTTGACGACTAT TACCAGCAGATCGCCCGTAGCGTCACCCTACCGGTGATCCTGTACAACTTTCCGaATCTG ACGGGTCAGGACTTAACCCCGGAAACCGTGACGCGTCTGGCTCTGCAAAACGAGAATATC GTTGGCATCAAAGACACCATCGACAGCGTTGGTCACTTGCGTACGATGATCAACACAGTT AAGTCGGTACGCCCGTCGTTTTCGGTATTCTGCGGTTACGATGATCATTTGCTGAATACG ATGCTGCTGaGCGGCGACGGTGCGATAACCGCCAGCGCTAACTTTGCTCCGGAACTCTCC GTCGGCATCTACCGCGCCTGGCGTGAAGGCGATCTGGCGACCGCTGCGACGCTGAATAAA AAACTACTACAACTGCCCGCTATTTACGCCCTCGAAACACCGTTTGTCTCACTGATCAAA TACAGCATGCAGTGTGTCGGGCTGCCTGTAGAGACATATTGCTTACCACCGATTCTTGAA GCATCTGAAGAAGCAAAAGATAAAGTCCACGTGCTGCTTACCGCGCAGGGCATTTTACCA GTCtaa |

**[Table 10]**

| |
|---|
| (SEQ ID NO: 10) Ec_yjhH protein |
| MKKFSGIIPPVSSTFHRDGTLDKKAMREVADFLINKGVDGLFYLGTGGEFSQMNTAQRMA LAEEAVTIVDGRVPVLIGVGSPSTDEAVKLAQHAQAYGADGIVAINPYYWKVAPRNLDDY YQQIARSVTLPVILYNFPDLTGQDLTPETVTRLALQNENIVGIKDTIDSVGHLRTMINTV KSVRPSFSVFCGYDDHLLNTMLLGGDGAITASANFAPELSVGIYRAWREGDLATAATLNK KLLQLPAIYALETPFVSLIKYSMQCVGLPVETYCLPPILEASEEAKDKVHVLLTAQGILP V |

**[Table 11]**

| |
|---|
| (SEQ ID NO: 11) Ec_dgoA gene |
| atgCAGTGGCAAACTAAACTCCCGCTGATCGCCATTTTGCGCGGTATTACGCCCGACGAG GCGCTGGCGCATGTTGGCGCGGTGATTGACGCCGGGTTCGACGCGGTTGAAATCCCGCTG AATTCCCCACAATGGGAGCAAAGCATTCCCGCCATCGTTGATGCGTACGGCGACAAGGCG TTGATTGGCGCAGGTACGGTACTGAAACCTGAACAGGTCGATGCGCTCGCCAGGATGGGC TGTCAGCTCATCGTTACGCCCAATATCCATAGTGAAGTGATCCGCCGTGCGGTGGGCTAC GGCATGACCGTCTGCCCCGGCTGCGCGACGGCGACCGAAGCCTTTACCGCGCTCGAAGCG GGCGCGCAGGCGCTGAAAATATTTCCGTCATCGGCTTTTGGTCCGCAATACATCAAAGCG |
| TTAAAAGCGGTATTGCCATCGGACATCGCAGTCTTTGCCGTTGGCGGCGTGACGCCAGAA AACCTGGCGCAGTGGATAGACGCAGGTTGTGCAGGGGCGGGCTTAGGCAGCGATCTCTAT CGCGCCGGGCAATCCGTAGAGCGCACCGCGCAGCAGGCAGCAGCATTTGTTAAGGCGTAT CGAGAGGCAGTGCAAtaa |

**[Table 12]**

| |
|---|
| (SEQ ID NO: 12) Ec_dgoA protein |
| MQWQTKLPLIAILRGITPDEALAHVGAVIDAGFDAVEIPLNSPQWEQSIPAIVDAYGDKA LIGAGTVLKPEQVDALARMGCQLIVTPNIHSEVIRRAVGYGMTVCPGCATATEAFTALEA GAQALKIFPSSAFGPQYIKALKAVLPSDIAVFAVGGVTPENLAQWIDAGCAGAGLGSDLY RAGQSVERTAQQAAAFVKAYREAVQ |

**[Table 13]**

| |
|---|
| (SEQ ID NO: 13) Ec_mhpE gene |
| AtgaACGGTAAAAACTTTATATCTCGGACGTCACATTGCGTGACGGTATGCACGCCATT CGTCATCAGTATTCGCTGGAAAACGTTCGCCAGATTGCCAAAGCACTGGACGATGCCCGC GTGGATTCGATTGAAGTGGCCCACGGCGACGGTTTGCAAGGTTCCAGCTTTAACTATGGT TTCGGCGCACATAGCGACCTTGAATGGATTGAAGCGGCGGCGGATGTGGTGAAGCACGCC AAAATCGCGACGTTGTTGCTGCCAGGAATCGGCACTATTCACGATCTGAAAAATGCCTGG CAGGCTGGCGCGCGGGTGGTTCGTGTGGCAACGCACTGTACCGAAGCTGATGTTTCCGCC CAGCATATTCAGTATGCCCGCGAGCTCGGAATGGACACCGTTGGTTTTCTGATGATGAGC CATATGACCACGCCGGAGAATCTCGCCAAGCAGGCAAAGCTGATGGAAGGCTACGGTGCG ACCTGTATTTATGTGGTGGATTCTGGCGGTGCGATGAACATGAGCGATATCCGTGACCGT TTCCGCGCCCTGAAAGCAGAGCTGAAACCAGAAACGCAAACTGGCATGCACGCTCACCAT AACCTGAGTCTTGGCGTGGCGAACTCTATCGCGGCGGTGGAAGAGGGCTGCGACCGAATC GACGCCAGCCTCGCGGGAATGGGCGCGGGCGCAGGTAACGCACCGCTGGAAGTGTTTATT GCCGCCGCGGATAAACTGGGCTGGCAGCATGGGACCGATCTCTATGCGTTAATGGATGCC GCCGACGACCTGGTGCGTCCGTTGCAGGATCGACCGGTACGAGTCGATCGCGAAACGCTG GCGCTGGGATACGCTGGTGTTTACTCGAGCTTCCTGCGTCACTGTGAAACGGCGGCGGCG CGTTATGGCTTAAGTGCGGTGGATATTCTCGTTGAGCTGGGCAAACGCCGGATGGTTGGC GGCCAGGAGGATATGATCGTTGACGTGGCGCTGGATCTGCGCAACAACAAATAA |

**[Table 14]**

| |
|---|
| (SEQ ID NO: 14) Ec_mhpE protein |
| MNGKKLYISDVTLRDGMHAIRHQYSLENVRQIAKALDDARVDSIEVAHGDGLQGSSFNYG FGAHSDLEWIEAAADVVKHAKIATLLLPGIGTIHDLKNAWQAGARVVRVATHCTEADVSA QHIQYARELGMDTVGFLMMSHMTTPENLAKQAKLMEGYGATCIYVVDSGGAMNMSDIRDR FRALKAELKPETQTGMHAHHNLSLGVANSIAAVEEGCDRIDASLAGMGAGAGNAPLEVFI AAADKLGWQHGTDLYALMDAADDLVRPLQDRPVRVDRETLALGYAGVYSSFLRHCETAAA RYGLSAVDILVELGKRRMVGGQEDMIVDVALDLRNNK |

**[Table 15]**

| |
|---|
| (SEQ ID NO: 15) Ec_garL gene |
| atgAATAACGATGTTTTCCCGAATAAATTCAAAGCCGCACTGGCTGCGAAACAGGTACAA ATTGGTTGCTGGTCAGCACTCTCTAACCCGATTAGCACTGAAGTTCTTGGTTTGGCTGGG TTTGACTGGCTGGTGCTGGATGGCGAACATGCGCCAAACGATATCTCCACGTTTATTCCG CAGTTAATGGCCTTGAAAGGCAGCGCCAGCGCCAGTAGTGCGAGTGCCGACCAACGAG CCGGTAATTATTAAGCGTCTTCTGGATATCGGTTTCTATAACTTCCTGATTCCTTTTGTA GAAACAAAAGAGGAAGCAGAGCTGGCGGTGGCATCAACCCGTTACCCACCGGAAGGCATT CGCGGCGTCTCCGTTTCTCACCGCGCCAATATGTTTGGCACCGTGGCGGATTATTTCGCT CAGTCGAACAAGAACATCACTATTCTGGTCCAGATAGAAAGTCAGCAGGGCGTAGATAAC GTCGATGCCATTGCCGCTACCGAAGGCGTAGACGGCATCTTCGTCGGCCCCAGCGATCTG GCCGCGGCATTAGGCCATCTCGGCAATGCATCACACCCGGATGTACAAAAAGCAATTCAG CACATTTTTAACCGTGCCAGCGCGCACGGCAAACCCAGCGGTATCCTCGCGCCGGTCGAA GCCGATGCGCGTCGTTATCTGGAATGGGGCGCGACGTTTGTGGCTGTCGGCAGCGATCTC GGCGTCTTCCGCTCTGCCACTCAGAAACTGGCTGATACCTTTAAAAAAtaa |

**[Table 16]**

| |
|---|
| (SEQ ID NO: 16) Ec_garL protein |
| |

**[Table 17]**

| |
|---|
| (SEQ ID NO: 17) Pp_galC gene |
| atgAGTACGCTCGTGGAAAAACCGGCGTTGTCCTTCGCAACATCGAGCGAGCGGGCGCG GGCCTGGTGGACGAACTGGCGGCGTACGGTGTTGCCACCATCCACGAGGCACAAGGGCGC AAAGGGTTGCTGGCGCCCTCGATATCGGCGATTCAGCAGGGCACTTCTGTCAGCGGCTCG GCCGTCACTGTGCTGGTGGCGCCCGGGGATAACTGGATGTTCCACGTTGCTGTTGAACAA TGCCGTCCTAGCGATGTGCTGGTAGTGGCACCCAGCTCTCCCTGTACCGACGGCTACTTT GGTGACCTGCTCGCAACCTCGCTCAAGGCGCATGGTGTTCGTGCGCTGGTGATCGATGCC GGGGTGCGCGATACCCGTACCCTTCGGCAAATGGGCTTCCCCGTATGGTCACGGGCGGTC AGCGCGCAAGGCACCATCAAGGAAACACTTGGGTCGGTCAATCTACCGATCATTTGCGGC GGGCAGCTGGTCAACCCCGGTGACGTGGTCGTGGCGGATGATGACGGTGTGGTTATTGTG CGTCGCGATGAGGTGACGCAGGTGCTGGAGGCGACCCGTCGGCGTGCTGATCTGGAAGAG CAGAAGGCGTTGCGCCTGGCCAGCGGCGAACTCGGTCTGGACATCTACAACATGCGTCCC CGCTTGGCCGAGAAGGGGTTACGGTACATCGACAGCCTGGATGAACTGGGAGAGtaa |

**[Table 18]**

| |
|---|
| (SEQ ID NO: 18) Pp_galC protein |
| MSTLVGKTGVVLRNIERAGAGLVDELAAYGVATIHEAQGRKGLLAPSISAIQQGTSVSGS AVTVLVAPGDNWMFHVAVEQCRPSDVLVVAPSSPCTDGYFGDLLATSLKAHGVRALVIDA GVRDTRTLRQMGFPVWSRAVSAQGTIKETLGSVNLPIICGGQLVNPGDVVVADDDGVVIV RRDEVTQVLEATRRRADLEEQKALRLASGELGLDIYNMRPRLAEKGLRYIDSLDELGE |

**[Table 19]**

| |
|---|
| (SEQ ID NO: 19) Ns_phdJ gene |
| atgACGTCACCCGCCGTGACATCCGCCGACATTACAGGGCTCGTTGGCATCGTGCCGACG CCCTCGAAGCCGGGCTCGGAAGCGCCGGACGCTGTAGACACCGTCGACCTCGACGAGACC GCGCGCATGGTCGAACTCATCGTCGCTTCCGGTGTTGACGTGCTGCTGACCAATGGCACC TTCGGCGAAGTCGCTACTCTCACTTATGAAGAACTACTGGCGTTCAACGACACGGTCATC CGTACGGTCGCTAATCGCATACCCGTCTTCTGCGGGGCCTCGACGCTGAACACCCGCGAC ACGATCGCCCGCAGCCTCGCCCTAATGGGGCTCGGTGCGAACGGTCTCTTCGTCGGGCGA CCAATGTGGCTACCTCTCGACGACGAGCAGCTGGTGTCCTACTACGCGGCCGTGTGCGAT GCCGTTCCGGCTGCCGCTGTCGTCGTCTACGACAACACTGGAGTGTTCAAGGGCAAAATC TCCTCCGCCGCGTACGCGGCGCTGGCCGAGATCCCGCAGATCGTGGCATCGAAGCATCTG GGCGTACTCTCGGGCAGCGATGCCTATGCGAGCGACCTGGCCGCTGTAAAGGCCGCTTT CCTCTGTTGCCCACCGCGGACAATTGGCTCCCCTCACTCGAGGCATTCCCCGGCGAAGTG CCGGCGGCTTGGAGCGGTGATGTGGCCTGCGGCCCGAACCCGTGATGGCGCTCCGCCGC GCGATCGCGGAGGGACTCTGGGACGACGCCCGTGCCGTGCATGAAGACATCGCATGGGCC ACGGAGCCCCTGTTCCCGGGCGGCGATATAAGTAAGTTCATGCCATACAGCATCCAGATC GACCGGGCCGAGTTCGAGGCCGCCGGTTACATCGTGCCCGGCCCCAGCCGGCATCCCTAC |
| GGCACCGCCCCAGCCGCCTACCTCGAAGGGGGCGCCGAGGTCGGCCGCCGTTGGGCGGGC ATTCGCCAGAAGTACGTCGCAACTCTCGCCGAACCAtaa |

**[Table 20]**

| |
|---|
| (SEQ ID NO: 20) Ns_phdJ protein |
| |

**[Table 21]**

| |
|---|
| (SEQ ID NO: 21) Bx_bphI gene |
| atgAAGCTAGAAGGCAAAAAAGTCACCGTCCACGACATGACGCTGCGCGACGGCATGCAC CCCAAGCGCCACCAGATGACGCTGGAGCAAATGAAATCCATCGCCTGCGGCCTGGATGCC GCGGGCATCCCGCTGATCGAAGTCACCCACGGCGACGGCCTGGGCGGCTCCTCCGTCAAC TACGGCTTTCCGGCGCACAGCGACGAGGAATACCTGGGCGCCGTGATTCCGTTGATGAAG CAGGCCAAGGTCAGTGCCCTGCTCTTGCCCGGCATCGGCACCGTCGAACACCTGAAGATG GCCAAGGACCTGGGCGTGAACACCATCCGCGTGGCCACCCACTGCACCGAAGCCGATGTG TCGGAGCAGCACATCACCCAATCGCGCAAGCTGGGTCTGGACACCGTGGGCTTTTTGATG ATGGCGCACATGGCCAGCCCAGAAAAGCTGGTCAGCCAGGCACTCTTGATGCAAGGCTAC GGCGCCAACTGCATCTACGTCACCGACTCGGCCGGCTACATGCTGCCTGACGACGTGAAA GCGCGCCTGAGTGCCGTGCGTGCCGCGCTCAAACCCGAAACCGAGTTGGGCTTTCACGGC CATCACAACCTGGCCATGGGCGTCGCCAACTCGATCGCCGCGATCGAGGCCGGGGCCACC CGCATCGATGCCGCTGCCGCTGGCCTGGGTGCCGGCGCCGGCAACACGCCGATGGAGGTG TTCATCGCCGTATGCGCGCGCATGGGGATCGAAACCGGAGTCGATGTGTTCAAGATCCAG GACGTGGCCGAAGACCTGGTGGTGCCGATCATGGACCATGTCATCCGCATCGACCGCGAC TCGCTCACGCTGGGCTATGCCGGGGTCTATTCCAGCTTTCTGCTGTTTGCCAAACGCGCC AGTGCGAAGTACGGCGTGCCGGCGCGCGACATCCTGGTCGAGCTGGGACGGCGCGGCATG GTCGGCGGCCAGGAAGACATGATTGAAGACACCGCCATGACCATGGCACGTGAGCGCGGG TTGACCTTGACCGCCGCAtaa |

**[Table 22]**

| |
|---|
| (SEQ ID NO: 22) Bx_bphI protein |
| MKLEGKKVTVHDMTLRDGMHPKRHQMTLEQMKSIACGLDAAGIPLIEVTHGDGLGGSSVN YGFPAHSDEEYLGAVIPLMKQAKVSALLLPGIGTVEHLKMAKDLGVNTIRVATHCTEADV SEQHITQSRKLGLDTVGFLMMAHMASPEKLVSQALLMQGYGANCIYVTDSAGYMLPDDVK ARLSAVRAALKPETELGFHGHHNLAMGVANSIAAIEAGATRIDAAAAGLGAGAGNTPMEV FIAVCARMGIETGVDVFKIQDVAEDLVVPIMDHVIRIDRDSLTLGYAGVYSSFLLFAKRA SAKYGVPARDILVELGRRGMVGGQEDMIEDTAMTMARERGLTLTAA |

Amino acid sequence homologies can be determined using the algorithm BLAST [Pro. Natl. Acad. Sci. USA, 90, 5873 (1993)] and FASTA [Methods Enzymol, 183, 63 (1990)] by Karlin and Altschul. Based on the algorithm BLAST, programs called BLASTN and BLASTX have been developed [J. Mol. Biol., 215, 403 (1990)]. In addition, when an amino acid sequence is analyzed according to BLASTX based on BLAST, parameters are set to score = 50 and word length = 3, for example. To obtain gapped alignment, Gapped BLAST can be utilized as described in Altschul et al. (1997, Nucleic Acids Res. 25: 3389-3402). Alternatively, PSI-Blast or PHI-Blast can be used to perform an iterated search that detects a positional relationship (Id.) between molecules and a relationship between molecules sharing a common pattern. When using BLAST, Gapped BLAST, PSI-Blast, and PHI-Blast programs, default parameters of the respective programs can be used. These can be referred to at http://www.ncbi.nlm.nih.gov.

The activity of catalyzing an aldol reaction between pyruvic acid and a carbonyl compound can be calculated by, for example, performing this enzyme reaction at 25°C in 100 mM HEPES-NaOH buffer (pH 8.0) containing 2 mM magnesium chloride, and measuring an initial generation rate of an aldol compound produced from pyruvic acid and a carbonyl compound which are raw materials. A generation rate of the aldol compound can be calculated from, for example, changes of a concentration of the aldol compound itself over time. The concentration of the aldol compound can be measured by, for example, mixing an enzymatic reaction solution and a solution of 130 mM O-benzylhydroxylamine hydrochloride (pyridine: methanol: water = 33:15:2) at 1:5 to stop the reaction, detecting O-benzyloxime derivatives produced at that time from the aldol compound using high-performance liquid chromatography, and comparing it with a sample of a known concentration. Here, an activity by which 1 µmol of an aldol compound is formed per minute at 25°C is defined as 1 unit.

The "gene (hereinafter referred to as a hpal gene mutant) encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound" can be selected from, for example, DNA libraries of another organism species through PCR or hybridization using primers or probes designed according to pieces of information based on a base sequence of SEQ ID NO: 2. A gene thus selected has a high probability of encoding a protein having an activity of catalyzing an aldol reaction between pyruvic acid and a carbonyl compound.

The "gene (a rhmA gene mutant) encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a nanA gene mutant) encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (an eda gene mutant) encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a yjhH gene mutant) encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a dgoA gene mutant) encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (an mhpE gene mutant) encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a garL gene mutant) encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a galC gene mutant) encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a phdJ gene mutant) encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound" can be selected from, for example, DNA libraries of another organism species through a method similar to the above-mentioned method for selecting the hpal gene mutant.

The "gene (a bphl gene mutant) encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound" can be selected from, for example, DNA libraries of another organism species through a method similar to the above-mentioned method for selecting the hpal gene mutant.

### (1.1.3) Construction of recombinant vector for transformation

The first gene can be incorporated into an appropriate plasmid vector for transformation.

As a plasmid vector, a known plasmid vector can be used depending on hosts. The plasmid vector may include a gene that controls an autonomous replication function in a host, or may include a base sequence necessary for incorporation of a gene into a host chromosome.

A transformant can be produced using the obtained recombinant vector.

### (1.1.4) Transformation

As a transformation method, a known method can be used without limitation. Examples of such known methods include a calcium chloride/rubidium chloride method, a calcium phosphate method, DEAE-dextran-mediated transfection, an electric pulse method, and the like.

The obtained transformant may be cultured immediately after transformation using a medium generally used for culturing transformants.

A culture temperature may be any temperature as long as it is suitable for growing the transformant. A pH of the medium may be any pH as long as it is suitable for growing the transformant. A pH of the medium can be adjusted by a known method. A culture time may be any time as long as it is sufficient for growing the transformant.

When the transformant is produced using a recombinant vector as described above, the first gene may be respectively incorporated into a host chromosome, or may be present in the cytoplasm of a host in the form of a recombinant vector.

### (1.1.5) Disruption or deletion of host chromosome gene

A host may be a mutant strain or an artificial genetic recombinant in addition to a wild strain. When the host is a coryneform bacterium, the host may be a gene-disrupted strain or a gene-deleted strain obtained by disrupting or deleting at least one of genes originally possessed by coryneform bacteria of a wild strain, the genes being as follows: a lactate dehydrogenase (for example, ldhA) gene, a phosphoenolpyrvate carboxylase (for example, ppc) gene, a pyruvate carboxylase (for example, pyc) gene, a pyruvate dehydrogenase (for example, poxB) gene, a glutamate-pyruvate aminotransferase (for example, alaA) gene, an acetolactate synthase (for example, ilvB) gene, an N-succinyldiaminopimelate aminotransferase (for example, cg0931) gene, an S-(hydroxymethyl)mycothiol dehydrogenase (for example, adhE) gene, and an acetaldehyde dehydrogenase (for example, ald) gene. By using such a gene-disrupted strain or gene-deleted strain as a host, it is possible to improve productivity of α-keto acids or to suppress production of by-products.

Construction of a gene-disrupted strain or a gene-deleted strain can be performed by a known method.

### (1.1.6) Specific examples of α-keto acid-producing microorganisms

Specifically, as an α-keto acid-producing microorganism, a coryneform bacterium can be used which comprises the first gene selected from the following group consisting of:
(1-1) a gene encoding a protein that consists of an amino acid sequence set forth in SEQ ID NO: 2 and catalyzes an aldol reaction between pyruvic acid and a carbonyl compound;
(1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
(1-3) a gene encoding a protein that consists of an amino acid sequence set forth in SEQ ID NO: 4 and catalyzes an aldol reaction between pyruvic acid and a carbonyl compound;
(1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
(1-5) a gene encoding a protein that consists of an amino acid sequence set forth in SEQ ID NO: 6 and catalyzes an aldol reaction between pyruvic acid and a carbonyl compound; and
(1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound.

### (1.2) Culture step

An α-keto acid can be produced by culturing an α-keto acid-producing microorganism (for example, the above-described transformant) in the presence of a pyruvic acid-supplying compound and a carbonyl compound. The "culturing in the presence of a pyruvic acid-supplying compound and a carbonyl compound" can be performed by culture in a culture solution containing the pyruvic acid-supplying compound and the carbonyl compound. The "culturing in the presence of a pyruvic acid-supplying compound and a carbonyl compound" can be preferably performed by culture in a culture solution into which the pyruvic acid-supplying compound and the carbonyl compound are added.

Prior to the culture in the presence of a pyruvic acid-supplying compound and a carbonyl compound, it is preferable to culture the above-described transformant under aerobic conditions for its proliferation. Hereinafter, this culture under aerobic conditions will be referred to as proliferation culture, and the culture in the presence of a pyruvic acid-supplying compound and a carbonyl compound will be referred to as production culture. Specifically, for example, the culture under aerobic conditions means culture under conditions in which oxygen is supplied to a culture solution in a sufficient amount that enables proliferation of a transformant during a culture period. The culture under aerobic conditions can be performed by known methods. The culture under aerobic conditions can be realized by supplying oxygen to a culture solution by, for example, aeration, agitation, shaking, or a combination thereof. More specifically, the culture under aerobic conditions can be realized by shaking culture or aeration and agitation culture of a deep portion.

When the proliferation culture is performed prior to the production culture, the proliferation culture and subsequent production culture can be performed as follows, for example. After the proliferation culture, first, a container containing a culture solution used in the proliferation culture (hereinafter referred to as the culture solution for proliferation) and a transformant suspended in the culture solution for proliferation is centrifuged in a centrifugal separator, and thereby the transformant can be precipitated. Thereafter, the culture solution for proliferation is removed from the container, the separated transformant is suspended in a culture solution used in the production culture (hereinafter referred to as the culture solution for production), and production culture can be performed.

Alternatively, regarding the proliferation culture and subsequent production culture, after the proliferation culture, a pyruvic acid-supplying compound and a carbonyl compound are added to the culture solution for proliferation containing the transformant, aerobic conditions are changed to anaerobic conditions or microaerobic conditions, and thereby production culture can be performed without exchanging the culture solution. As described above, the proliferation culture and the production culture can be performed in succession.

In the present specification, the term "culture" means maintaining a transformant under specifically controlled conditions suitable for growth of the transformant. During a culture period, the transformant may or may not proliferate. Accordingly, the term "culture" can also be rephrased as "incubation."

### (1.2.1) Culture solution for proliferation

As a culture solution for proliferation, it is possible to use a medium generally used for culturing transformants, for example, a known medium containing a carbon source, a nitrogen source, an inorganic salt, and the like.

A culture temperature in the proliferation culture may be any temperature as long as it is a temperature suitable for proliferation of transformants. A pH of the culture solution for proliferation may be any pH as long as it is suitable for proliferation of transformants. A pH of the culture solution for proliferation can be adjusted by a known method. A culture time in the proliferation culture may be any time as long as it is a sufficient time for proliferation of transformants.

### (1.2.2) Culture solution for production

As a culture solution for production, it is possible to use a culture solution containing a pyruvic acid-supplying compound, a carbonyl compound, and other necessary components. As the other necessary components, it is possible to use, for example, inorganic salts, carbon sources other than saccharides, or nitrogen sources. A concentration of each of components in a culture solution described in the present specification refers to a final concentration thereof in the culture solution at a time point when each of the components was added.

As a pyruvic acid-supplying compound, it is possible to use a compound selected from pyruvic acid and saccharides. The pyruvic acid-supplying compound may be pyruvic acid itself, or may be a saccharide that has been converted into pyruvic acid in a living body of a microorganism. As the pyruvic acid-supplying compound, it is possible to use pyruvic acid, saccharides, or a combination thereof.

As the saccharides, any saccharide can be used as long as it is a saccharide that can be introduced into a living body by a transformant and can be converted into pyruvic acid. Specific examples of saccharides include monosaccharides such as glucose, fructose, mannose, xylose, arabinose, and galactose; disaccharides such as sucrose, maltose, lactose, cellobiose, xylobiose, and trehalose; polysaccharides such as starch; molasses; and the like. Among them, monosaccharides are preferred, and fructose and glucose are more preferred. Furthermore, saccharides containing glucose as a constituent monosaccharide, specifically, disaccharides such as sucrose, oligosaccharides containing glucose as a constituent monosaccharide, and polysaccharides containing glucose as a constituent monosaccharide are also preferable. As the saccharides, one kind may be used, or two or more kinds may be mixed and used. In addition, saccharides can be added in a form of a saccharified solution (containing a plurality of types of saccharides such as glucose and xylose) which is obtained by saccharifying, with saccharification enzymes, non-edible agricultural waste such as rice straw, bagasse, and corn stover, and energy crops such as switchgrass, napier grass, and miscanthus.

A concentration of the pyruvic acid-supplying compound in the culture solution for production can be as high as possible while being within a range not inhibiting production of α-keto acid. A concentration of the pyruvic acid-supplying compound in the culture solution for production is preferably within a range of 0.1% to 40% (w/v), and is more preferably within a range of 1% to 20% (w/v). Furthermore, a pyruvic acid-supplying compound can be additionally added according to a decrease of the pyruvic acid-supplying compound accompanying production of α-keto acid.

As the carbonyl compound, it is possible to use a carbonyl compound represented by General Formula (1).

General Formula (1): R₁₀₁C(O)R₁₀₂,

where R₁₀₁ and R₁₀₂ are each independently hydrogen or an alkyl group having 1 to 5 carbon atoms.

According to one example, as the carbonyl compound, it is possible to use a carbonyl compound in which, in General Formula (1), R₁₀₁ is hydrogen or an alkyl group having 1 to 5 carbon atoms, and R₁₀₂ is hydrogen.

According to another example, as the carbonyl compound, it is possible to use a carbonyl compound in which, in General Formula (1), R₁₀₁ is a methyl group, and R₁₀₂ is an alkyl group having 1 to 5 carbon atoms.

Specifically, as the carbonyl compound, it is possible to use the following carbonyl compounds (where the following R₁₀₁'s and R₁₀₂'s are respectively in General Formula (1)).
A carbonyl compound in which R₁₀₁ is hydrogen and R₁₀₂ is hydrogen (that is, formaldehyde);
A carbonyl compound in which R₁₀₁ is a methyl group and R₁₀₂ is hydrogen (that is, acetaldehyde);
A carbonyl compound in which R₁₀₁ is an ethyl group and R₁₀₂ is hydrogen (that is, propionaldehyde);
A carbonyl compound in which R₁₀₁ is a propyl group and R₁₀₂ is hydrogen (that is, butyraldehyde), specifically, a carbonyl compound in which R₁₀₁ is an n-propyl group and R₁₀₂ is hydrogen (that is, n-butyraldehyde), or a carbonyl compound in which R₁₀₁ is an isopropyl group and R₁₀₂ is hydrogen (that is, isobutylaldehyde); and
A carbonyl compound in which R₁₀₁ is a pentyl group and R₁₀₂ is hydrogen (that is, valeraldehyde), specifically, a carbonyl compound in which R₁₀₁ is an n-pentyl group and R₁₀₂ is hydrogen (that is, n-valeraldehyde).

Furthermore, as the carbonyl compound, it is possible to use a carbonyl compound in which both R₁₀₁ and R₁₀₂ are methyl groups in General Formula (1) (that is, acetone).

As the carbonyl compound, one kind may be used, or two or more kinds may be mixed and used.

A concentration of the carbonyl compound in the culture solution for production is preferably within a range of 0.001% to 10% (w/v), and is more preferably within a range of 0.01% to 1% (w/v). Furthermore, a carbonyl compound can be additionally added according to a decrease of the carbonyl compound accompanying production of α-keto acid.

When one kind of carbonyl compound is used in the culture solution for production, it is possible to easily perform separation and purification to be described later in the section "(1.2.4) Recovery of α-keto acid."

Furthermore, inorganic salts can be added as necessary. As the inorganic salts, it is possible to use phosphoric salts, sulfate salts, and salts of metals such as magnesium, potassium, manganese, iron, and zinc. Specifically, it is possible to use primary potassium phosphate, dipotassium phosphate, magnesium sulfate, sodium chloride, ferrous nitrate, iron(II) sulfate, manganese sulfate, zinc sulfate, cobalt sulfate, calcium carbonate, and the like. As the inorganic salts, one kind may be used, or two or more kinds may be mixed and used. A concentration of the inorganic salts in the culture solution for production varies depending on inorganic salts used, but generally, it can be set to about 0.01% to 1% (w/v).

Furthermore, carbon sources other than saccharides can be added as necessary. As the carbon sources other than saccharides, it is possible to use sugar alcohols such as mannitol, sorbitol, xylitol, glycerol, and glycerin; organic acids such as acetic acid, citric acid, lactic acid, fumaric acid, maleic acid, and gluconic acid; hydrocarbons such as n-paraffins; and the like.

Furthermore, nitrogen sources can be added as necessary. As the nitrogen sources, it is possible to use inorganic or organic ammonium salts such as ammonium chloride, ammonium sulfate, ammonium nitrate, and ammonium acetate; urea; ammonia water; nitrate salts such as sodium nitrate and potassium nitrate; and the like. In addition, it is also possible to use corn steep liquor; meat extract; yeast extract; soybean hydrolysates; protein hydrolysates such as peptone, NZ-amine, and decomposed products of casein; nitrogen-containing organic compounds such as amino acid; and the like. As the nitrogen sources, one kind may be used, or two or more kinds may be mixed and used. A concentration of the nitrogen sources in the culture solution for production varies depending on nitrogen compounds used, but generally, it can be set to about 0.1% to 10% (w/v).

In addition, vitamins can be added as necessary. Examples of vitamins include biotin, thiamine (vitamin B1), pyridoxine (vitamin B6), pantothenic acid, inositol, nicotinic acid, and the like.

As a specific culture solution for production, it is possible to use a medium containing glucose, formaldehyde, potassium dihydrogen phosphate, dipotassium hydrogen phosphate, magnesium sulfate, iron(II) sulfate, and manganese(II) sulfate, and having a pH of 6.0.

As a specific culture solution for production, it is also possible to use a medium containing pyruvic acid, formaldehyde, potassium dihydrogen phosphate, dipotassium hydrogen phosphate, magnesium sulfate, iron(II) sulfate, manganese(II) sulfate, and 4-morpholinopropanesulfonic acid.

Furthermore, as the culture solution for production, it is possible to use a culture solution obtained by adding a pyruvic acid-supplying compound and a carbonyl compound to a base culture solution having the same composition as a culture solution for proliferation used in the proliferation culture. When a sufficient amount of the pyruvic acid-supplying compound is present in the above-mentioned base culture solution, it is not necessary to add the pyruvic acid-supplying compound.

### (1.2.3) Conditions for production culture

A culture temperature in the production culture may be any temperature as long as it is a temperature suitable for production of α-keto acid. A culture temperature in the production culture is preferably about 20°C to 50°C, and is more preferably about 25°C to 47°C. It is possible to efficiently produce an α-keto acid when a temperature is within the above-mentioned temperature range. A pH of the culture solution for production may be any pH as long as it is suitable for production of α-keto acid. A pH of the culture solution for production is preferably maintained within a range of about 6 to 8. A pH of the culture solution for production can be adjusted using an inorganic or organic acid; an alkaline solution such as an aqueous solution of potassium hydroxide; urea; calcium carbonate; ammonia; a pH buffer solution containing 4-morpholinopropanesulfonic acid; or the like. A pH of the culture solution for production can be appropriately adjusted as necessary even during the production culture. A culture time in the proliferation culture may be any time as long as it is a time sufficient for a transformant to produce an α-keto acid. A culture time in the production culture is preferably about 3 hours to 7 days, and is more preferably about 1 to 3 days.

The production culture may be performed under aerobic conditions, or may be performed under anaerobic conditions or microaerobic conditions. This is because the above-described transformant has a pathway that is for producing an α-keto acid and works under both aerobic conditions and anaerobic conditions or microaerobic conditions. The pathway for producing an α-keto acid will be described in the section "(1.3) Actions and effects" to be described later.

### <Anaerobic conditions or microaerobic conditions>

The production culture is preferably performed under anaerobic conditions or microaerobic conditions.

When a transformant is cultured under anaerobic conditions or microaerobic conditions, the transformant does not proliferate substantially, and thereby α-keto acid can be produced more efficiently.

The "anaerobic conditions or microaerobic conditions" refer to, for example, conditions in which a concentration of dissolved oxygen in a culture solution for production is within a range of 0 to 2 ppm. The "anaerobic conditions or microaerobic conditions" preferably refer to condition in which a concentration of dissolved oxygen in a culture solution for production is within a range of 0 to 1 ppm, and more preferably refer to conditions in which a concentration of dissolved oxygen in a culture solution for production is within a range of 0 to 0.5 ppm. A concentration of dissolved oxygen in a culture solution can be measured using, for example, a dissolved oxygen meter.

Strictly speaking, the term "anaerobic conditions" is a technical term that refers to conditions in which electron acceptors such as dissolved oxygen and oxides (for example, nitric acid) are not present. Meanwhile, as preferred conditions for the production culture, it is not necessary to adopt the strict conditions as above as long as transformants do not proliferate because there is no sufficient amount of these electron acceptors or because of the nature of electron acceptors, more amount of carbon sources supplied, which is supposed to be used for proliferation of transformants, is used for production of α-keto acid, and thereby production efficiency can be improved. That is, as preferred conditions for the production culture, electron acceptors such as dissolved oxygen and oxides (for example, nitric acid) may be present in a culture solution for production as long as proliferation of transformants can be inhibited and production efficiency for α-keto acid can be increased. Accordingly, the expression "anaerobic conditions or microaerobic conditions" used in the present specification includes such states.

Anaerobic conditions or microaerobic conditions can be realized by, for example, not causing a gas containing oxygen to pass through so that an amount of oxygen supplied from a gas-liquid interface is insufficient for proliferation of transformants. Alternatively, these conditions can be realized by aerating a culture solution for production with an inert gas, specifically, a nitrogen gas. Alternatively, these conditions can be realized by sealing a container used for the production culture.

### <High density conditions>

The production culture is preferably performed in a state in which a transformant is suspended at a high density in a culture solution for production. When the transformant is cultured in such a state, α-keto acid can be produced more efficiently.

The "state in which a transformant is suspended at a high density in a culture solution for production" refers to, for example, a state in which the transformant is suspended in the culture solution for production such that a wet bacterial cell weight% of the transformant is within a range of 1% to 50% (w/v). The "state in which a transformant is suspended at a high density in a culture solution for production" preferably refers to, for example, a state in which the transformant is suspended in the culture solution for production such that a wet bacterial cell weight% of the transformant is within a range of 3% to 30% (w/v).

The production culture is more preferably performed under anaerobic conditions or microaerobic conditions, and in a state in which a transformant is suspended at a high density in a culture solution for production.

### (1.2.4) Recovery of α-keto acid

As described above, when the transformant containing the first gene is cultured in a culture solution for production, α-keto acid is produced in the culture solution for production.

Specifically, when a carbonyl compound represented by General Formula (1) is used in a culture solution for production, it is possible to produce α-keto acid represented by General Formula (2).

General Formula (1): R₁₀₁C(O)R₁₀₂,

where R₁₀₁ and R₁₀₂ are each independently hydrogen or an alkyl group having 1 to 5 carbon atoms.

General Formula (2): R₁₀₁C(R₁₀₂)(OH)CH₂COCOOH,

where R₁₀₁ and R₁₀₂ in General Formula (2) are respectively the same groups as R₁₀₁ and R₁₀₂ in General Formula (1).

More specifically, by using a carbonyl compound in which R₁₀₁ is hydrogen and R₁₀₂ is hydrogen in General Formula (1), it is possible to produce an α-keto acid in which R₁₀₁ is hydrogen and R₁₀₂ is hydrogen in General Formula (2) (that is, 4-hydroxy-2-oxobutyric acid).

By using a carbonyl compound in which R₁₀₁ is a methyl group and R₁₀₂ is hydrogen in General Formula (1), it is possible to produce an α-keto acid in which R₁₀₁ is a methyl group and R₁₀₂ is hydrogen in General Formula (2) (that is, 4-hydroxy-2-oxovaleric acid).

By using a carbonyl compound in which R₁₀₁ is an ethyl group and R₁₀₂ is hydrogen in General Formula (1), it is possible to produce an α-keto acid in which R₁₀₁ is an ethyl group and R₁₀₂ is hydrogen in General Formula (2) (that is, 4-hydroxy-2-oxocaproic acid).

By using a carbonyl compound in which R₁₀₁ is a propyl group and R₁₀₂ is hydrogen in General Formula (1), it is possible to produce an α-keto acid in which R₁₀₁ is a propyl group and R₁₀₂ is hydrogen in General Formula (2) (that is, 4-hydroxy-2-oxoenanthic acid). Specifically, by using a carbonyl compound in which R₁₀₁ is an n-propyl group and R₁₀₂ is hydrogen in General Formula (1), it is possible to produce α-keto acid in which R₁₀₁ is an n-propyl group and R₁₀₂ is hydrogen in General Formula (2) (that is, 4-hydroxy-2-oxon-heptanoic acid). Furthermore, specifically, by using a carbonyl compound in which R₁₀₁ is an isopropyl group and R₁₀₂ is hydrogen in General Formula (1), it is possible to produce an α-keto acid in which R₁₀₁ is an isopropyl group and R₁₀₂ is hydrogen in General Formula (2) (that is, 4-hydroxy-2-oxoisoenanthic acid).

By using a carbonyl compound in which R₁₀₁ is a pentyl group and R₁₀₂ is hydrogen in General Formula (1), it is possible to produce an α-keto acid in which R₁₀₁ is a pentyl group and R₁₀₂ is hydrogen in General Formula (2) (that is, 4-hydroxy-2-oxocaprylic acid). Specifically, by using a carbonyl compound in which R₁₀₁ is an n-pentyl group and R₁₀₂ is hydrogen in General Formula (1), it is possible to produce an α-keto acid in which R₁₀₁ is an n-pentyl group and R₁₀₂ is hydrogen in General Formula (2) (that is, 4-hydroxy-2-oxon-caprylic acid).

By using a carbonyl compound in which both R₁₀₁ and R₁₀₂ are methyl groups in General Formula (1), it is possible to produce an α-keto acid in which both R₁₀₁ and R₁₀₂ are methyl groups in General Formula (2) (that is, 4-methyl-4-hydroxy-2-oxovaleric acid).

The above-described α-keto acids can be recovered by recovering a culture solution for production, but the α-keto acids can also be separated from the culture solution for production and purified by known methods. Examples of such known methods include a crystallization method, a chromatography method, an ion exchange resin method, an adsorption and elution method using activated carbon, a solvent extraction method, and the like.

In addition, when a plurality of types of carbonyl compounds is used, a plurality of types of α-keto acids is produced in a culture solution. A plurality of types of α-keto acids can be separated from each other by crystallization or chromatography.

### (1.3) Actions and effects

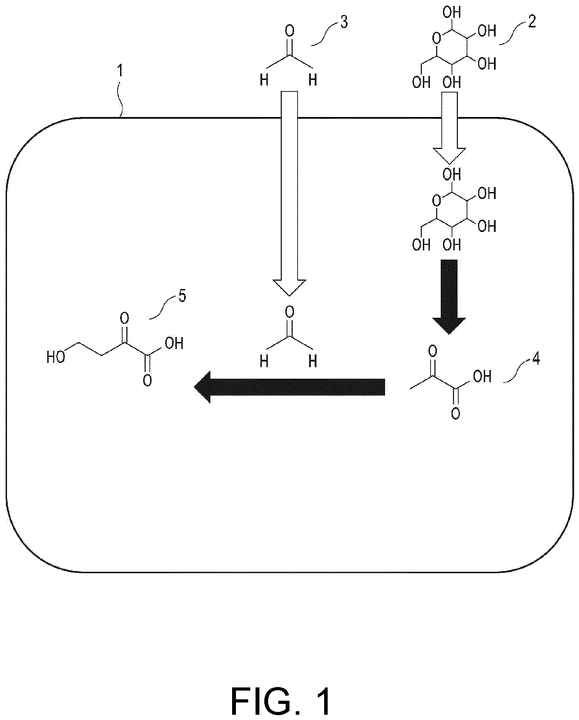

FIG. 1 schematically shows a process in which an α-keto acid-producing microorganism produces an α-keto acid. The process shown in FIG. 1 illustrates a case as an example in which a pyruvic acid-supplying compound is glucose, a carbonyl compound is formaldehyde, and α-ketoic acid is 4-hydroxy-2-oxobutyric acid.

As shown in FIG. 1, first, an α-keto acid-producing microorganism 1 introduces a glucose 2 and a formaldehyde 3. Next, the glucose 2 is converted into a pyruvic acid 4 through a glycolytic system. Next, the pyruvic acid 4 and the formaldehyde 3 are converted into a 4-hydroxy-2-oxobutyric acid 5 by an "enzyme that catalyzes an aldol reaction between pyruvic acid and aldehyde," which is an enzyme encoded by the first gene. Accordingly, 4-hydroxy-2-oxobutyric acid is produced.

FIG. 1 illustrates the case in which aldehyde is used as a carbonyl compound, but even in a case where ketone is used as a carbonyl compound, a reaction of converting ketone and pyruvic acid into α-keto acid proceeds by an "enzyme that catalyzes an aldol reaction between pyruvic acid and a carbonyl compound." This is because both aldehydes and ketones can serve as substrates for the same reaction in organic chemical reactions such as enzymatic reactions because both aldehydes and ketones belong to the same type of compound (that is, a carbonyl compound).

As described above, the production method described above has been completed by finding a production process in which a key intermediate for synthesis of useful substances such as amino acids can be obtained by culturing a microorganism having a specific gene in the presence of a carbonyl compound. The finding that useful compounds could be obtained in the presence of the carbonyl compound was surprising because in general, carbonyl compounds are harmful to growth of microorganisms. Furthermore, since the carbonyl compound is introduced into a microbial metabolism system in this case, the finding that an intermediate not produced in a normal microbial metabolism system could be obtained can be expected as a technique to meet future needs, which are to produce various compounds from biological raw materials. In fact, as will be described later in the present specification, the development in which various useful substances could be produced through this intermediate indicates a part of a possibility that various compounds can be produced by the technique of the present invention.

### (2.) Method for producing 1,3-diol

A method for producing a 1,3-diol includes a step of culturing a microorganism containing the following genes in the presence of a pyruvic acid-supplying compound selected from pyruvic acid and saccharides, and a carbonyl compound represented by General Formula (3) to produce a 1,3-diol represented by General Formula (4).
(a) First gene encoding an enzyme that catalyzes an aldol reaction between pyruvic acid and a carbonyl compound,
(b) Second gene encoding an enzyme that catalyzes a decarboxylation reaction of an α-keto acid, and
(c) Third gene encoding an enzyme that catalyzes a reduction reaction of an aldehyde

   General Formula (3): R₁₀₃C(O)R₁₀₄,

   where R₁₀₃ and R₁₀₄ are each independently hydrogen or an alkyl group having 1 to 5 carbon atoms, provided that in a case where R₁₀₃ is hydrogen, R₁₀₄ is not hydrogen,

   General Formula (4): R₁₀₃C(R₁₀₄)(OH)CH₂CH₂OH,

   where R₁₀₃ and R₁₀₄ in General Formula (4) are respectively the same groups as R₁₀₃ and R₁₀₄ in General Formula (3).

In the following description, the above-mentioned microorganism containing the first, second, and third genes is also referred to as a "1,3-diol-producing microorganism." The 1,3-diol-producing microorganism may be a microorganism that naturally has the first, second, and third genes, or may be a microorganism obtained by transforming a host with the first, second, and third genes. Hereinafter, a case in which the 1,3-diol-producing microorganism is a transformant will be described as an example.

### (2.1) Transformant

### (2.1.1) Host

As a host, it is possible to use a host similar to the host described in the section "(1.1.1) Host."

Next, the first, second, and third genes introduced into the host will be described.

### (2.1.2) First gene

As the first gene, it is possible to use a gene similar to the first gene described in the section "(1.1.2) First gene."

### (2.1.3) Second gene

The second gene encodes an "enzyme that catalyzes a decarboxylation reaction of α-keto acids." The "enzyme that catalyzes a decarboxylation reaction of α-keto acids" refers to an enzyme having an activity of 1 mU/mg protein or more when measured using an enzymatic reaction solution containing purified proteins and α-keto acids (an initial substrate concentration 1 mM) as an enzymatic reaction solution according to a measurement method to be described later. The second gene encodes, for example, an enzyme that catalyzes a decarboxylation reaction of α-keto acids obtained by the aldol reaction catalyzed by the protein encoded by the first gene. An α-keto acid in the "enzyme that catalyzes a decarboxylation reaction of α-keto acids" is, for example, an α-keto acid expressed by the general formula (2) described in the section "(1.) Method for producing α-keto acid". The "enzyme that catalyzes a decarboxylation reaction of α-keto acids" is, for example, a decarboxylase.

The second gene may be a gene encoding decarboxylases exemplified below.
(b1) Pyruvate decarboxylase (EC number 4.1.1.1),
(b2) Benzoylformate decarboxylase (EC number 4.1.1.7), and
(b3) Indolepyruvate decarboxylase (EC number 4.1.1.74).

Examples of decarboxylases included in (b1) include a pdc protein. The pdc protein is preferably derived from *Zymomonas mobilis.*

Examples of decarboxylases included in (b2) include an mdlC protein. The mdlC protein is preferably derived from *Pseudomonas putida.*

Examples of decarboxylases included in (b3) include a kivD protein. The kivD protein is preferably derived from *Lactococcus lactis.*

Preferably, the second gene is selected from the following group consisting of:
(2-1) a gene encoding a protein (that is, an mdlC protein derived from *Pseudomonas putida*) that consists of an amino acid sequence set forth in SEQ ID NO: 24 and catalyzes a decarboxylation reaction of α-keto acids; and
(2-2) a gene encoding a protein that consists of an amino acid sequence in which one or a plurality of amino acids is deleted, substituted, or added in SEQ ID NO: 24, and has an activity of catalyzing a decarboxylation reaction of α-keto acids.

A "gene encoding a protein that consists of an amino acid sequence in which one or a plurality of amino acids is deleted, substituted, or added in SEQ ID NO: 24, and has an activity of catalyzing a decarboxylation reaction of α-keto acids" may be a "gene encoding a protein that consists of an amino acid sequence showing a homology of 80% or more, preferably 90% or more, more preferably 95% or more, even more preferably 97% or more, still more preferably 98% or more, and yet more preferably 99% or more to an amino acid sequence set forth in SEQ ID NO: 24, which is a protein having an activity of catalyzing a decarboxylation reaction of α-keto acids."

A UniProtKB accession number of an mdlC protein derived from *Pseudomonas putida* is, for example, P20906.

The second gene is more preferably an mdlC gene derived from *Pseudomonas putida* consisting of a base sequence set forth in SEQ ID NO: 23.

A base sequence set forth in SEQ ID NO: 23 and an amino acid sequence set forth in SEQ ID NO: 24 are as follows.

**[Table 23]**

| |
|---|
| (SEQ ID NO: 23) Pp_mdlC gene |
| atgGCTTCGGTACACGGCACCACATACGAACTCTTGCGACGTCAAGGCATCGATACGGTC TTCGGCAATCCTGGCTCGAACGAGCTCCCGTTTTGAAGGACTTTCCAGAGGACTTTCGA TACATCCTGGCTTTGCAGGAAGCGTGTGTGGTGGGCATTGCAGACGGCTATGCGCAAGCC AGTCGGAAGCCGGCTTTCATTAACCTGCATTCTGCTGCTGGTACCGGCAATGCTATGGGT GCACTCAGTAACGCCTGGAACTCACATTCCCCGCTGATCGTCACTGCCGGCCAGCAGACC AGGGCGATGATTGGCGTTGAAGCTCTGCTGACCAACGTCGATGCCGCCAACCTGCCACGA CCACTTGTCAAATGGAGCTACGAGCCCGCAAGCGCAGCAGAAGTCCCTCATGCGATGAGC AGGGCTATCCATATGGCAAGCATGGCGCCACAAGGCCCTGTCTATCTTTCGGTGCCATAT GACGATTGGGATAAGGATGCTGATCCTCAGTCCCACCACCTTTTTGATCGCCATGTCAGT TCATCAGTACGCCTGAACGACCAGGATCTCGATATTCTGGTGAAAGCTCTCAACAGCGCA |
| TCCAACCCGGCGATCGTCCTGGGCCCGGACGTCGACGCAGCAAATGCGAACGCAGACTGC GTCATGTTGGCCGAACGCCTCAAAGCTCCGGTTTGGGTTGCGCCATCCGCTCCACGCTGC CCATTCCCTACCCGTCATCCTTGCTTCCGTGGATTGATGCCAGCTGGCATCGCAGCGATT TCTCAGCTGCTCGAAGGTCACGATGTGGTTTTGGTAATCGGCGCTCCAGTGTTCCGTTAC CACCAATACGACCCAGGTCAATATCTCAAACCTGGCACGCGATTGATTTCGGTGACCTGC GACCCGCTCGAAGCTGCACGCGCGCCAATGGGCGATGCGATCGTGGCAGACATTGGTGCG ATGGCTAGCGCTCTTGCCAACTTGGTTGAAGAGAGCAGCCGCCAGCTCCCAACTGCAGCT CCGGAACCCGCGAAGGTTGACCAAGACGCTGGCCGACTTCACCCAGAGACAGTGTTCGAC ACACTGAACGACATGGCCCCGGAGAATGCGATTTACCTGAACGAGTCGACTTCAACGACC GCCCAAATGTGGCAGCGCCTGAACATGCGCAACCCTGGTAGCTACTACTTCTGTGCAGCT GGCGGACTGGGCTTCGCCCTGCCTGCAGCAATTGGCGTTCAACTCGCAGAACCCGAGCGA CAAGTCATCGCCGTCATTGGCGACGGATCGGCGAACTACAGCATTAGTGCGTTGTGGACT GCAGCTCAGTACAACATCCCCACTATCTTCGTGATCATGAACAACGGCACCTACGGTGCG TTGCGATGGTTTGCCGGCGTTCTCGAAGCAGAAAACGTTCCTGGGCTGGATGTGCCAGGG ATCGACTTCCGCGCACTCGCCAAGGGCTATGGTGTCCAAGCGCTGAAAGCCGACAACCTT GAGCAGCTCAAGGGTTCGCTACAAGAAGCGCTTTCTGCCAAAGGCCCGGTACTTATCGAA GTAAGCACCGTAAGCCCGGTGAAGtga |

**[Table 24]**

| |
|---|
| (SEQ ID NO: 24) Pp_mdlC protein |
| |

The activity of catalyzing a decarboxylation reaction of α-keto acids can be calculated by, for example, performing this enzyme reaction at 30°C in 100 mM potassium phosphate buffer (pH 6.0) containing 0.5 mM thiamine pyrophosphate, 1 mM magnesium chloride, 10 mM NADH, and an excess amount of alcohol dehydrogenase, and measuring an initial generation rate of an alcohol compound produced by sequential reduction of an aldehyde compound generated from an α-keto acid which is a raw material. A generation rate of the alcohol compound can be calculated from, for example, changes of a concentration of the alcohol compound itself over time. The concentration of the alcohol compound can be measured by, for example, detecting the alcohol compound using high-performance liquid chromatography, and comparing it with a sample of a known concentration. Here, an activity by which 1 µmol of an α-keto acid is decarboxylated per minute at 30°C is defined as 1 unit.

The "gene (an mdlC gene mutant) encoding a protein that consists of an amino acid sequence in which one or a plurality of amino acids is deleted, substituted, or added in SEQ ID NO: 24, and has an activity of catalyzing a decarboxylation reaction of α-keto acids" can be selected from, for example, DNA libraries of another organism species through a method similar to the above-mentioned method for selecting the hpal gene mutant.

### (2.1.4) Third gene

The third gene encodes an "enzyme that catalyzes a reduction reaction of aldehydes." The "enzyme that catalyzes a reduction reaction of aldehydes" refers to an enzyme having an activity of 10 mU/mg protein or more when measured using an enzymatic reaction solution containing purified proteins and aldehydes (an initial substrate concentration 1 mM) as an enzymatic reaction solution according to a measurement method to be described later. The third gene encodes, for example, an enzyme that catalyzes a reduction reaction of aldehydes obtained by the decarboxylation reaction catalyzed by the protein encoded by the second gene. An aldehyde in the "enzyme that catalyzes a reduction reaction of aldehydes" is, for example, 3-hydroxybutyraldehyde, 3-hydroxyvaleraldehyde, 3-hydroxyhexyl aldehyde, 3-hydroxyisohexyl aldehyde, 3-hydroxyheptyl aldehyde or 3-methyl-3-hydroxybutyraldehyde. The "enzyme that catalyzes a reduction reaction of aldehydes" is, for example, an alcohol dehydrogenase.

The third gene may be, for example, a gene encoding 1,3-propanediol dehydrogenase (EC number 1.1.1.202).

Examples of 1,3-propanediol dehydrogenase include a dhaT protein or a lpo protein. The dhaT protein is preferably derived from *Klebsiella pneumoniae.* The lpo protein is preferably derived from *Lactobacillus reuteri.*

Preferably, the third gene is selected from the following group consisting of:
(3-1) a gene encoding a protein (that is, a dhaT protein derived from *Klebsiella pneumoniae*) that consists of an amino acid sequence set forth in SEQ ID NO: 26 and catalyzes a reduction reaction of aldehydes; and
(3-2) a gene encoding a protein that consists of an amino acid sequence in which one or a plurality of amino acids is deleted, substituted, or added in SEQ ID NO: 26, and has an activity of catalyzing a reduction reaction of aldehydes.

A "gene encoding a protein that consists of an amino acid sequence in which one or a plurality of amino acids is deleted, substituted, or added in SEQ ID NO: 26, and has an activity of catalyzing a reduction reaction of aldehydes" may be a "gene encoding a protein that consists of an amino acid sequence showing a homology of 80% or more, preferably 90% or more, more preferably 95% or more, even more preferably 97% or more, still more preferably 98% or more, and yet more preferably 99% or more to an amino acid sequence set forth in SEQ ID NO: 26, which is a protein having an activity of catalyzing a reduction reaction of aldehydes."

A UniProtKB accession number of a dhaT protein derived from *Klebsiella pneumoniae* is, for example, Q59477.

The third gene is more preferably a dhaT gene derived from *Klebsiella pneumoniae* consisting of a base sequence set forth in SEQ ID NO: 25.

A base sequence set forth in SEQ ID NO: 25 and an amino acid sequence set forth in SEQ ID NO: 26 are as follows.

**[Table 25]**

| |
|---|
| (SEQ ID NO: 25) Kp_dhaT gene |
| atgAGCTATCGTATGTTCGATTATCTGGTGCCAAACGTTAACTTTTTGGCCCCAACGCC ATTTCCGTAGTCGGCGAACGCTGCCAGCTGCTGGGGGGAAAAAGCCCTGCTGGTCACC GACAAAGGCCTGCGGGCAATTAAAGATGGCGCGGTGGACAAAACCCTGCATTATCTGCGG GAGGCCGGGATCGAGGTGGCGATCTTTGACGGCGTCGAGCCGAACCCGAAAGACACCAAC GTGCGCGACGGCCTCGCTGTGTTTCGCCGCGAACAGTGCGACATCATCGTCACCGTGGGC GGCGGCAGCCCGCACGATTGCGGCAAAGGCATCGGCATCGCCGCCACCCATGAGGGCGAT CTGTACCAGTATGCCGGAATCGAGACCCTGACCAACCCGCTGCCGCCTATCGTCGCGGTC AATACCACCGCCGGCACCGCCAGCGAGGTCACCCGCCACTGCGTCCTGACCAACACCGAA ACCAAAGTGAAGTTTGTGATCGTCAGCTGGCGCAACCTGCCGTCGGTCTCTATCAACGAT CCACTGCTGATGATCGGTAAACCGGCCGCCCTGACCGCGGCGACCGGGATGGATGCCCTG ACCCACGCCGTAGAGGCCTATATCTCCAAAGACGCTAACCCGGTGACGGACGCCGCCGCC ATGCAGGCGATCCGCCTCATCGCCCGCAACCTGCGCCAGGCCGTGGCCCTCGGCAGCAAT CTGCAGGCGCGGGAAAACATGGCCTATGCCTCTCTGCTGGCCGGGATGGCTTTCAATAAC GCCAACCTCGGCTACGTGCACGCCATGGCACACCAGCTGGGCGGCCTGTACGACATGCCG CACGGCGTGGCCAACGCTGTCCTGCTGCCGCATGTGGCGCGCTACAACCTGATCGCCAAC CCGGAGAAATTCGCCGATATTGCTGAACTGATGGGCGAAAATATCACCGGACTGTCCACT CTCGACGCGGCGGAAAAAGCCATCGCCGCTATCACGCGTCTGTCGATGGATATCGGTATT CCGCAGCATCTGCGCGATCTGGGAGTAAAAGAGGCCGACTTCCCCTACATGGCGGAGATG GCTCTGAAAGACGGCAATGCGTTCTCGAACCCGCGTAAAGGCAACGAGCAGGAGATTGCC GCGATTTTCCGCCAGGCATTCtaa |

**[Table 26]**

| |
|---|
| (SEQ ID NO: 26) Kp_dhaT protein |
| MSYRMFDYLVPNVNFFGPNAISVVGERCQLLGGKKALLVTDKGLRAIKDGAVDKTLHYLR EAGIEVAIFDGVEPNPKDTNVRDGLAVFRREQCDIIVTVGGGSPHDCGKGIGIAATHEGD LYQYAGIETLTNPLPPIVAVNTTAGTASEVTRHCVLTNTETKVKFVIVSWRNLPSVSIND PLLMIGKPAALTAATGMDALTHAVEAYISKDANPVTDAAAMQAIRLIARNLRQAVALGSN LQARENMAYASLLAGMAFNNANLGYVHAMAHQLGGLYDMPHGVANAVLLPHVARYNLIAN PEKFADIAELMGENITGLSTLDAAEKAIAAITRLSMDIGIPQHLRDLGVKEADFPYMAEM ALKDGNAFSNPRKGNEQEIAAIFRQAF |

The activity of catalyzing a reduction reaction of aldehydes can be calculated by, for example, performing the enzyme reaction at 30°C in 50 mM MES-NaOH buffer (pH 6.5) containing 0.2 mM NADH, and measuring an initial consumption rate of NADH consumed when an aldehyde that is a raw material is reduced to an alcohol compound. The initial consumption rate of NADH can be calculated by, for example, measuring a rate of decrease in absorbance at 340 nm which is caused due to NADH in an enzymatic reaction solution. Here, an activity by which 1 µmol of an aldehyde is reduced per minute at 30°C is defined as 1 unit.

The "gene (a dhaT gene mutant) encoding a protein that consists of an amino acid sequence in which one or a plurality of amino acids is deleted, substituted, or added in SEQ ID NO: 26, and has an activity of catalyzing a reduction reaction of aldehydes" can be selected from, for example, DNA libraries of another organism species through a method similar to the above-mentioned method for selecting the hpal gene mutant.

### (2.1.5) Construction of recombinant vector for transformation

The first, second, and third genes can be incorporated into an appropriate plasmid vector for transformation.

As the plasmid vector, it is possible to use a plasmid vector similar to the plasmid vector described in the section "(1.1.3) Construction of recombinant vector for transformation."

The first, second, and third genes may be respectively inserted into individual plasmid vectors or may be inserted into the same plasmid vector. A transformant can be produced using the obtained recombinant vector.

### (2.1.6) Transformation

As a transformation method, it is possible to use a known method without limitation. As such a known method, it is possible to use a method similar to the method described in the section "(1.1.4) Transformation."

In a case where a transformant is produced using a recombinant vector as described above, the first, second, and third genes may be respectively inserted into chromosomes of a host, or may be present in cytoplasm of the host in the form of recombinant vectors.

### (2.1.7) Disruption or deletion of chromosomal gene of host

The host may be a wild strain, a mutant strain thereof, or an artificial gene recombinant. In a case where the host is a coryneform bacterium, for the host, it is possible to use a gene-disrupted strain or gene-deleted strain similar to the gene-disrupted strain or gene-deleted strain described in the section "(1.1.5) Disruption or deletion of chromosomal gene of host." Furthermore, as a method for producing a gene-disrupted strain or gene-deleted strain, it is possible to use a method similar to the method described in the section "(1.1.5) Disruption or deletion of chromosomal gene of host." By using such a gene-disrupted strain or gene-deleted strain as a host, it is possible to improve productivity for 1,3-diol or to inhibit production of by-products.

### (2.1.8) Specific examples of 1,3-diol-producing microorganisms

Specifically, as a 1,3-diol-producing microorganism, a coryneform bacterium can be used which comprises:
the first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
the second gene selected from the group consisting of:
   (2-1) a gene encoding a protein that consists of an amino acid sequence set forth in SEQ ID NO: 24 and catalyzes a decarboxylation reaction of α-keto acids; and
   (2-2) a gene encoding a protein that consists of an amino acid sequence in which one or a plurality of amino acids is deleted, substituted, or added in SEQ ID NO: 24, and has an activity of catalyzing a decarboxylation reaction of α-keto acids; and
the third gene selected from the group consisting of:
   (3-1) a gene encoding a protein that consists of an amino acid sequence set forth in SEQ ID NO: 26 and catalyzes a reduction reaction of aldehydes; and
   (3-2) a gene encoding a protein that consists of an amino acid sequence in which one or a plurality of amino acids is deleted, substituted, or added in SEQ ID NO: 26, and has an activity of catalyzing a reduction reaction of aldehydes.

### (2.2) Culture step

A 1,3-diol can be produced by culturing a 1,3-diol-producing microorganism (for example, the above-described transformant) in the presence of a pyruvic acid-supplying compound and a carbonyl compound. The "culturing in the presence of a pyruvic acid-supplying compound and a carbonyl compound" can be performed by culture in a culture solution containing the pyruvic acid-supplying compound and the carbonyl compound. The "culturing in the presence of a pyruvic acid-supplying compound and a carbonyl compound" can be preferably performed by culture in a culture solution into which the pyruvic acid-supplying compound and the carbonyl compound are added.

Prior to the culture in the presence of a pyruvic acid-supplying compound and a carbonyl compound, it is preferable to perform the proliferation culture described in the section "(1.2) Culture step."

When the proliferation culture is performed prior to the production culture, the proliferation culture and subsequent production culture can be performed as described in the section "(1.2) Culture step."

### (2.2.1) Culture solution for proliferation

As a culture solution for proliferation, it is possible to use a culture solution similar to the culture solution for proliferation described in the section "(1.2.1) Culture solution for proliferation."

### (2.2.2) Culture solution for production

As a culture solution for production, it is possible to use a culture solution containing a pyruvic acid-supplying compound, a carbonyl compound, and other necessary components. As the other necessary components, it is possible to use, for example, inorganic salts, carbon sources other than saccharides, or nitrogen sources.

As the pyruvic acid-supplying compound, it is possible to use the pyruvic acid-supplying compound described in the section "(1.2.2) Culture solution for production."

As the carbonyl compound, it is possible to use a carbonyl compound represented by General Formula (3).

General Formula (3): R₁₀₃C(O)R₁₀₄,

where R₁₀₃ and R₁₀₄ are each independently hydrogen or an alkyl group having 1 to 5 carbon atoms, provided that in a case where R₁₀₃ is hydrogen, R₁₀₄ is not hydrogen.

According to one example, as the carbonyl compound, it is possible to use a carbonyl compound in which, in General Formula (3), R₁₀₃ is hydrogen or an alkyl group having 1 to 5 carbon atoms, and R₁₀₄ is hydrogen.

According to another example, as the carbonyl compound, it is possible to use a carbonyl compound in which, in General Formula (3), R₁₀₃ is a methyl group, and R₁₀₄ is an alkyl group having 1 to 5 carbon atoms.

Specifically, as the carbonyl compound, it is possible to use the following carbonyl compounds (where the following R₁₀₃'s and R₁₀₄'s are respectively in General Formula (1)).
A carbonyl compound in which R₁₀₃ is a methyl group and R₁₀₄ is hydrogen (that is, acetaldehyde);
A carbonyl compound in which R₁₀₃ is an ethyl group and R₁₀₄ is hydrogen (that is, propionaldehyde);
A carbonyl compound in which R₁₀₃ is a propyl group and R₁₀₄ is hydrogen (that is, butyraldehyde), specifically, a carbonyl compound in which R₁₀₃ is an n-propyl group and R₁₀₄ is hydrogen (that is, n-butyraldehyde), or a carbonyl compound in which R₁₀₃ is an isopropyl group and R₁₀₄ is hydrogen (that is, isobutylaldehyde); and
A carbonyl compound in which R₁₀₃ is a pentyl group and R₁₀₄ is hydrogen (that is, valeraldehyde), specifically, a carbonyl compound in which R₁₀₃ is an n-pentyl group and R₁₀₄ is hydrogen (that is, n-valeraldehyde).

Furthermore, as the carbonyl compound, it is possible to use a ketone in which both R₁₀₃ and R₁₀₄ are methyl groups in General Formula (3) (that is, acetone).

As the carbonyl compound, one kind may be used, or two or more kinds may be mixed and used.

A concentration of the carbonyl compound in the culture solution for production is preferably within a range of 0.001% to 10% (w/v), and is more preferably within a range of 0.01% to 1% (w/v). Furthermore, a carbonyl compound can be additionally added according to a decrease of the carbonyl compound accompanying production of 1,3-diol.

When one kind of carbonyl compound is used in the culture solution for production, it is possible to easily perform separation and purification to be described later in the section "(2.2.4) Recovery of 1,3-diol."

As other necessary components, it is possible to use components similar to the other necessary components described in the section "(1.2.2) Culture solution for production."

As a specific culture solution for production, it is possible to use a medium containing glucose, acetaldehyde, potassium dihydrogen phosphate, dipotassium hydrogen phosphate, magnesium sulfate, iron(II) sulfate, and manganese(II) sulfate, and having a pH of 6.0.

Furthermore, as the culture solution for production, it is possible to use a culture solution obtained by adding a pyruvic acid-supplying compound and a carbonyl compound to a base culture solution having the same composition as a culture solution for proliferation used in the proliferation culture. When a sufficient amount of the pyruvic acid-supplying compound is present in the above-mentioned base culture solution, it is not necessary to add the pyruvic acid-supplying compound.

### (2.2.3) Conditions for production culture

A culture temperature in the production culture may be any temperature as long as it is a temperature suitable for production of 1,3-diol. A culture temperature in the production culture is preferably about 20°C to 50°C, and is more preferably about 25°C to 47°C. It is possible to efficiently produce a 1,3-diol when a temperature is within the above-mentioned temperature range. A pH of the culture solution for production may be any pH as long as it is suitable for production of 1,3-diol. A pH of the culture solution for production is preferably maintained within a range of about 6 to 8. A pH of the culture solution for production can be adjusted using an inorganic or organic acid; an alkaline solution such as an aqueous solution of potassium hydroxide; urea; calcium carbonate; ammonia; a pH buffer solution containing 4-morpholinopropanesulfonic acid; or the like. A pH of the culture solution for production can be appropriately adjusted as necessary even during the production culture. A culture time in the production culture may be any time as long as it is a time sufficient for a transformant to produce a 1,3-diol. A culture time in the production culture is preferably about 3 hours to 7 days, and is more preferably about 1 to 3 days.

The production culture may be performed under aerobic conditions, or may be performed under anaerobic conditions or microaerobic conditions. This is because the above-described transformant has a pathway that is for producing a 1,3-diol and works under both aerobic conditions and anaerobic conditions or microaerobic conditions. The pathway for producing a 1,3-diol will be described in the section "(2.3) Actions and effects" to be described later.

### <Anaerobic conditions or microaerobic conditions>

The production culture is preferably performed under anaerobic conditions or microaerobic conditions.

When a transformant is cultured under anaerobic conditions or microaerobic conditions, the transformant does not proliferate substantially, and thereby 1,3-diol can be produced more efficiently.

The "anaerobic conditions or microaerobic conditions" can be set to the same conditions as the "anaerobic conditions or microaerobic conditions" described in the section "(1.2.3) Conditions for production culture."

### <High density conditions>

The production culture is preferably performed in a state in which a transformant is suspended at a high density in a culture solution for production. When the transformant is cultured in such a state, 1,3-diol can be produced more efficiently.

The "state in which a transformant is suspended at a high density in a culture solution for production" can be set to the same state as the "state in which a transformant is suspended at a high density in a culture solution for production" described in the section "(1.2.3) Conditions for production culture."

The production culture is more preferably performed under anaerobic conditions or microaerobic conditions, and in a state in which a transformant is suspended at a high density in a culture solution for production.

### (2.2.4) Recovery of 1,3-diol

As described above, when the transformant containing the first, second, and third genes is cultured in a culture solution for production, 1,3-diol is produced in the culture solution for production.

Specifically, when a carbonyl compound represented by General Formula (3) is used in a culture solution for production, it is possible to produce 1,3-diol represented by General Formula (4).

General Formula (3): R₁₀₃C(O)R₁₀₄,

where R₁₀₃ and R₁₀₄ are each independently hydrogen or an alkyl group having 1 to 5 carbon atoms, provided that both R₁₀₃ and R₁₀₄ are not hydrogen,

General Formula (4): R₁₀₃C(R₁₀₄)(OH)CH₂CH₂OH,

where R₁₀₃ and R₁₀₄ in General Formula (4) are respectively the same groups as R₁₀₃ and R₁₀₄ in General Formula (3).

More specifically, by using a carbonyl compound in which R₁₀₃ is a methyl group and R₁₀₄ is hydrogen in General Formula (3), it is possible to produce a 1,3-diol in which R₁₀₃ is a methyl group and R₁₀₄ is hydrogen in General Formula (4) (that is, 1,3-butanediol).

By using a carbonyl compound in which R₁₀₃ is an ethyl group and R₁₀₄ is hydrogen in General Formula (3), it is possible to produce a 1,3-diol in which R₁₀₃ is an ethyl group and R₁₀₄ is hydrogen in General Formula (4) (that is, 1,3-pentanediol).

By using a carbonyl compound in which R₁₀₃ is a propyl group and R₁₀₄ is hydrogen in General Formula (3), it is possible to produce a 1,3-diol in which R₁₀₃ is a propyl group and R₁₀₄ is hydrogen in General Formula (4) (that is, 1,3-hexanediol). Specifically, by using a carbonyl compound in which R₁₀₃ is an n-propyl group and R₁₀₄ is hydrogen in General Formula (3), it is possible to produce a 1,3-diol in which R₁₀₃ is an n-propyl group and R₁₀₄ is hydrogen in General Formula (4) (that is, 1,3-n-hexanediol). Furthermore, specifically, by using a carbonyl compound in which R₁₀₃ is an isopropyl group and R₁₀₃ is hydrogen in General Formula (3), it is possible to produce a 1,3-diol in which R₁₀₃ is an isopropyl group and R₁₀₄ is hydrogen in General Formula (4) (that is, 4-methyl-pentane-1,3-diol).

By using a carbonyl compound in which R₁₀₃ is a pentyl group and R₁₀₄ is hydrogen in General Formula (3), it is possible to produce a 1,3-diol in which R₁₀₃ is a pentyl group and R₁₀₄ is hydrogen in General Formula (4) (that is, 1,3-heptanediol). Specifically, by using a carbonyl compound in which R₁₀₃ is an n-pentyl group and R₁₀₄ is hydrogen in General Formula (3), it is possible to produce a 1,3-diol in which R₁₀₃ is an n-pentyl group and R₁₀₄ is hydrogen in General Formula (4) (that is, 1,3-n-heptanediol).

By using a carbonyl compound in which both R₁₀₃ and R₁₀₄ are methyl groups in General Formula (3), it is possible to produce a 1,3-diol in which both R₁₀₃ and R₁₀₄ are methyl groups in General Formula (4) (that is, 3-methyl-butane-1,3-diol).

The above-described 1,3-diols can be recovered by recovering a culture solution for production, but the 1,3-diols can also be separated from the culture solution for production and purified by known methods. Examples of such known methods include a distillation method, a concentration method, an ion exchange resin method, an adsorption and elution method using activated carbon, a solvent extraction method, a crystallization method, and the like.

From the culture solution for production, it is possible to recover substances such as α-keto acid produced by an aldol reaction catalyzed by the enzyme encoded by the first gene.

In addition, when a plurality of types of carbonyl compounds is used, a plurality of types of 1,3-diols is produced in a culture solution. A plurality of types of 1,3-diols can be separated from each other by distillation or chromatography.

### (2.3) Actions and effects

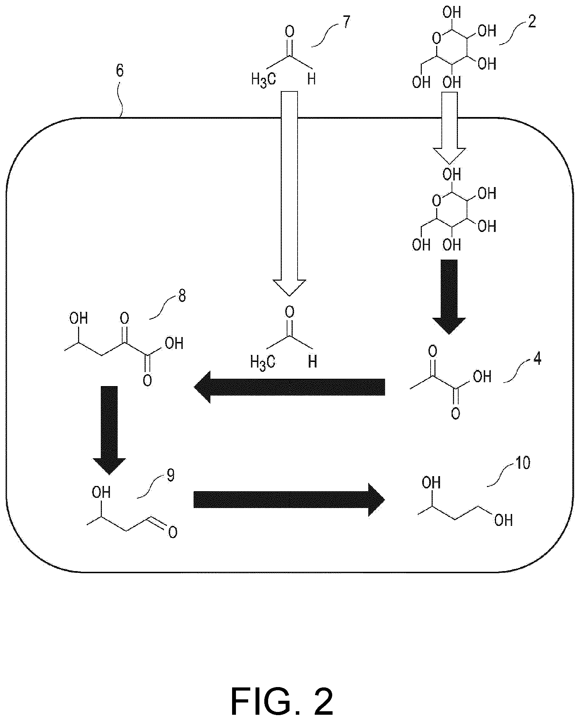

FIG. 2 schematically shows a process in which a 1,3-diol-producing microorganism produces a 1,3-diol. The process shown in FIG. 2 illustrates a case as an example in which a pyruvic acid-supplying compound is glucose, a carbonyl compound is acetaldehyde, and 1,3-diol is 1,3-butanediol.

As shown in FIG. 2, first, a 1,3-diol-producing microorganism 6 introduces a glucose 2 and an acetaldehyde 7. Next, the glucose 2 is converted into a pyruvic acid 4 through a glycolytic system. Next, the pyruvic acid 4 and the acetaldehyde 7 are converted into a 4-hydroxy-2-oxovaleric acid 8 by an "enzyme that catalyzes an aldol reaction between pyruvic acid and aldehyde," which is an enzyme encoded by the first gene. Next, the 4-hydroxy-2-oxovaleric acid 8 is converted into a 3-hydroxybutyraldehyde 9 by an "enzyme that catalyzes a decarboxylation reaction of an α-keto acid," which is an enzyme encoded by the second gene. Next, the 3-hydroxybutyraldehyde 9 is converted into 1,3-butanediol 10 by an "enzyme that catalyzes a reduction reaction of aldehyde," which is an enzyme encoded by the third gene. Accordingly, 1,3-butanediol is produced.

Alternatively, as another embodiment, the culture solution for production may not contain acetaldehyde only in a case of producing 1,3-butanediol in the method for producing a 1,3-diol described in the section "(2.) Method for producing 1,3-diol."

That is, the method for producing 1,3-butanediol according to the present embodiment includes a step of culturing the 1,3-diol-producing microorganism in the presence of a pyruvic acid-supplying compound.

The present embodiment can be performed according to the same procedure as in the above-described "Method for producing 1,3-diol" except that acetaldehyde is not added to the culture solution for production.

The inventors of the present invention think the reason why 1,3-butanediol can be produced even when the culture solution for production does not contain an acetaldehyde as follows. First, a pyruvic acid-supplying compound in the culture solution for production is introduced into a living body by the 1,3-diol-producing microorganism. Next, in a case where the pyruvic acid-supplying compound is a pyruvic acid, a part of the pyruvic acid is converted into an acetaldehyde by the enzyme encoded by the second gene. In a case where the pyruvic acid-supplying compound is a saccharide, the saccharide is converted into a pyruvic acid, and thereafter, a part of the pyruvic acid is converted into an acetaldehyde by the enzyme encoded by the second gene. Next, the acetaldehyde and the pyruvic acid are converted into a 4-hydroxy-2-oxovaleric acid by the enzyme encoded by the first gene. Next, the 4-hydroxy-2-oxovaleric acid is converted into a 3-hydroxybutyraldehyde by the enzyme encoded by the second gene. Next, the 3-hydroxybutyraldehyde is converted into 1,3-butanediol. As described above, the 1,3-diol-producing microorganism can produce an acetaldehyde in a living body. Accordingly, 1,3-butanediol is produced even when the culture solution for production does not contain an acetaldehyde. The acetaldehyde produced by the 1,3-diol-producing microorganism may be secreted into a culture solution by the 1,3-diol-producing microorganism.

### (3.) Method for producing 2,4-dihydroxycarboxylic acid

A method for producing a 2,4-dihydroxycarboxylic acid includes a step of culturing a microorganism containing the following genes in the presence of a pyruvic acid-supplying compound selected from pyruvic acid and saccharides, and a carbonyl compound represented by General Formula (1) to produce a 2,4-dihydroxycarboxylic acid represented by General Formula (5).
(a) First gene encoding an enzyme that catalyzes an aldol reaction between pyruvic acid and a carbonyl compound, and
(d) Fourth gene encoding an enzyme that catalyzes a reduction reaction of an α-keto acid

   General Formula (1): R₁₀₁C(O)R₁₀₂,

   where R₁₀₁ and R₁₀₂ are each independently hydrogen or an alkyl group having 1 to 5 carbon atoms,

   General Formula (5): R₁₀₁C(R₁₀₂)(OH)CH₂CH(OH)COOH,

   where R₁₀₁ and R₁₀₂ in General Formula (5) are respectively the same groups as R₁₀₁ and R₁₀₂ in General Formula (1).

In the following description, the above-mentioned microorganism containing the first and fourth genes is also referred to as a "2,4-dihydroxycarboxylic acid-producing microorganism." The 2,4-dihydroxycarboxylic acid-producing microorganism may be a microorganism that naturally has the first and fourth genes, or may be a microorganism obtained by transforming a host with the first and fourth genes. Hereinafter, a case in which the 2,4-dihydroxycarboxylic acid-producing microorganism is a transformant will be described as an example.

### (3.1.) Transformant

### (3.1.1) Host

As a host, it is possible to use a host similar to the host described in the section "(1.1.1) Host."

Next, the first and fourth genes to be introduced into the host will be described.

### (3.1.2) First gene

As the first gene, it is possible to use a gene similar to the first gene described in the section "(1.1.2) First gene."

### (3.1.3) Fourth gene

The fourth gene encodes an "enzyme that catalyzes a reduction reaction of an α-keto acid." The "enzyme that catalyzes a reduction reaction of an α-keto acid" refers to an enzyme having an activity of 10 mU/mg or more of protein when measuring the activity according to a measurement method to be described later using an enzymatic reaction solution containing purified protein and α-keto acid (initial concentration of substrate: 1 mM) as an enzymatic reaction solution. The fourth gene encodes, for example, the enzyme that catalyzes a reduction reaction of an α-keto acid obtained by an aldol reaction catalyzed by a protein encoded by the first gene. The α-keto acid referred to in the phrase "enzyme that catalyzes a reduction reaction of an α-keto acid" is, for example, an α-keto acid represented by General Formula (2) described in the section "(1.) Method for producing α-keto acid." The "enzyme that catalyzes a reduction reaction of an α-keto acid" is, for example, a dehydrogenase.

The fourth gene may be a gene encoding, for example, lactate dehydrogenase (EC number: 1.1.1.27).

Examples of lactate dehydrogenases include ldhA protein.

The ldhA protein is preferably a protein derived from *Lactococcus lactis.* The "protein derived from *Lactococcus lactis"* may be a protein isolated from *Lactococcus lactis,* or may be a mutant of the isolated protein as long as an activity of the protein is maintained. The ldhA protein is more preferably a protein derived from a *Lactococcus lactis* strain NBRC 100676. The ldhA protein is even more preferably an ldhA protein which is derived from the *Lactococcus lactis* strain NBRC 100676 and in which the 85th glutamine residue from an N-terminal is substituted by a cysteine residue.

The fourth gene is preferably selected from the group consisting of the following genes.
(4-1) Gene encoding a protein that consists of an amino acid sequence set forth in SEQ ID NO: 28 and catalyzes a reduction reaction of an α-keto acid (that is, the ldhA protein which is derived from the *Lactococcus lactis* strain NBRC 100676 and in which the 85th glutamine residue from an N-terminal is substituted by a cysteine residue), and
(4-2) Gene encoding a protein that consists of an amino acid sequence in which one or a plurality of amino acids is deleted, substituted, or added in SEQ ID NO: 28, and has an activity of catalyzing a reduction reaction of an α-keto acid

The "gene encoding a protein that consists of an amino acid sequence in which one or a plurality of amino acids is deleted, substituted, or added in SEQ ID NO: 28, and has an activity of catalyzing a reduction reaction of an α-keto acid" may be a "gene encoding a protein that consists of an amino acid sequence homologous to the amino acid sequence set forth in SEQ ID NO: 28 by 80% or more, preferably by 90% or more, more preferably by 95% or more, even more preferably by 97% or more, still more preferably by 98% or more, and further more preferably by 99% or more, and has an activity of catalyzing a reduction reaction of an α-keto acid."

The "gene encoding a protein that consists of an amino acid sequence in which one or a plurality of amino acids is deleted, substituted, or added in SEQ ID NO: 28, and has an activity of catalyzing a reduction reaction of an α-keto acid" preferably encodes a protein that maintains a cysteine residue at the 85th residue from the N-terminal.

The fourth gene is more preferably an ldhA protein that is derived from the *Lactococcus lactis* strain NBRC 100676 and consists of a base sequence set forth in SEQ ID NO: 27. The base sequence set forth in SEQ ID NO: 27 encodes a protein having a cysteine residue at the 85th residue from the N-terminal.

The base sequence set forth in SEQ ID NO: 27 and the amino acid sequence set forth in SEQ ID NO: 28 are as follows.

**[Table 27]**

| |
|---|
| (SEQ ID NO: 27) Ll_ldhA Q85C gene |
| atgGCTGATAAACAACGTAAGAAAGTTATCCTTGTTGGTGACGGTGCTGTAGGTTCATCA TACGCTTTTGCCCTTGTTAACCAAGGAATTGCACAAGAATTAGGTATTGTTGACCTTTTT AAAGAAAAAACTCAAGGGGATGCAGAAGACCTTTCTCATGCCTTGGCATTTACATCACCT AAAAAGATTTACTCTGCAGACTACTCTGATGCAAGCGACGCTGACCTCGTTGTCTTGACT TCTGGTGCTCCATGCAAACCAGGTGAAACTCGTCTTGACCTTGTTGAAAAAAATCTTCGT ATTACTAAAGATGTTGTAACTAAAATTGTTGCTTCAGGATTCAAAGGAATCTTCCTCGTT GCTGCTAACCCAGTTGACATCTTGACATACGCAACTTGGAAATTCTCTGGTTTCCCTAAA AACCGTGTTGTAGGTTCAGGTACTTCACTTGATACTGCACGTTTTCGTCAAGCATTGGCT GAAAAAGTTGACGTTGATGCTCGTTCAATCCACGCATACATCATGGGTGAACATGGTGAC TCAGAATTTGCTGTTTGGTCACACGCTAACGTTGCTGGTGTTAAATTGGAACAATGGTTC CAAGAAAATGACTACCTTAACGAAGCAGAAATCGTTGAATTGTTTGAATCTGTACGTGAT GCAGCTTACTCAATCATCGCTAAAAAAGGTGCAACATTCTACGGTGTGGCTGTAGCCCTT GCTCGTATTACTAAAGCAATTCTTGATGATGAACATGCAGTACTTCCTGTATCAGTATTC CAAGATGGACAATATGGCGTAAGCGACTGCTACCTTGGTCAACCAGCTGTAGTTGGTGCT GAAGGTGTTGTTAACCCAATTCACATTCCATTGAACGATGCTGAAATGCAAAAAATGGAA GCTTCTGGAGCTCAATTGAAAGCTATCATCGATGAGGCTTTTGCTAAAGAAGAATTTGCT TCTGCAGTTAAAAACtaa |

**[Table 28]**

| |
|---|
| (SEQ ID NO: 28) Ll_ldhA Q85C protein |
| MADKQRKKVILVGDGAVGSSYAFALVNQGIAQELGIVDLFKEKTQGDAEDLSHALAFTSP KKIYSADYSDASDADLVVLTSGAPCKPGETRLDLVEKNLRITKDVVTKIVASGFKGIFLV AANPVDILTYATWKFSGFPKNRVVGSGTSLDTARFRQALAEKVDVDARSIHAYIMGEHGD SEFAVWSHANVAGVKLEQWFQENDYLNEAEIVELFESVRDAAYSIIAKKGATFYGVAVAL ARITKAILDDEHAVLPVSVFQDGQYGVSDCYLGQPAVVGAEGVVNPIHIPLNDAEMQKME ASGAQLKAIIDEAFAKEEFASAVKN |

The activity of catalyzing a reduction reaction of an α-keto acid can be calculated by, for example, causing the enzymatic reaction at 30°C in a 60 mM HEPES-NaOH buffer (pH 7.0) containing 0.25 mM NADH, 50 mM potassium chloride, 5 mM magnesium chloride, 5 mM magnesium chloride, and 5 mM D-fructose 1,6-bisphosphate, and measuring an initial consumption rate of NADH consumed when the raw material α-keto acid is reduced. The initial consumption rate of NADH can be calculated by, for example, measuring a rate of decrease in absorbance at 340 nm derived from NADH in an enzymatic reaction solution. In this case, an activity of reducing 1 µmol of α-keto acid at 30°C for 1 minute is regarded as one unit.

The "gene (ldhA gene mutant) encoding a protein that consists of an amino acid sequence in which one or a plurality of amino acids is deleted, substituted, or added in SEQ ID NO: 28, and has an activity of catalyzing a reduction reaction of an α-keto acid" can be selected from a DNA library of animals of another species by the same method as the above-described method of selecting an hpal gene mutant.

### (3.1.4) Construction of recombinant vector for transformation

The first and fourth genes can be incorporated into an appropriate plasmid vector for transformation.

As the plasmid vector, it is possible to use a plasmid vector similar to the plasmid vector described in the section "(1.1.3) Construction of recombinant vector for transformation."

The first and fourth genes may be respectively inserted into individual plasmid vectors or may be inserted into the same plasmid vector. A transformant can be produced using the obtained recombinant vector.

### (3.1.5) Transformation

As a transformation method, it is possible to use a known method without limitation. As such a known method, it is possible to use a method similar to the method described in the section "(1.1.4) Transformation."

In a case where a transformant is produced using a recombinant vector as described above, the first and fourth genes may be respectively inserted into chromosomes of a host, or may be present in cytoplasm of the host in the form of recombinant vectors.

### (3.1.6) Disruption or deletion of chromosomal gene of host

The host may be a wild strain, a mutant strain thereof, or an artificial gene recombinant. In a case where the host is a coryneform bacterium, for the host, it is possible to use a gene-disrupted strain or gene-deleted strain similar to the gene-disrupted strain or gene-deleted strain described in the section "(1.1.5) Disruption or deletion of chromosomal gene of host." Furthermore, as a method for producing a gene-disrupted strain or gene-deleted strain, it is possible to use a method similar to the method described in the section "(1.1.5) Disruption or deletion of chromosomal gene of host." By using such a gene-disrupted strain or gene-deleted strain as a host, it is possible to improve productivity for 1,3-diol or to inhibit production of by-products.

### (3.1.7) Specific examples of 2,4-dihydroxycarboxylic acid-producing microorganisms

Specifically, as a 2,4-dihydroxycarboxylic acid-producing microorganism, a coryneform bacterium can be used which comprises:
the first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound; and
the fourth gene selected from the group consisting of:
   (4-1) a gene encoding a protein that consists of an amino acid sequence set forth in SEQ ID NO: 28 and catalyzes a reduction reaction of α-keto acids; and
   (4-2) a gene encoding a protein that consists of an amino acid sequence in which one or a plurality of amino acids is deleted, substituted, or added in SEQ ID NO: 28, and has an activity of catalyzing a reduction reaction of α-keto acids.

### (3.2) Culture step

A 2,4-dihydroxycarboxylic acid can be produced by culturing a 2,4-dihydroxycarboxylic acid-producing microorganism (for example, the above-described transformant) in the presence of a pyruvic acid-supplying compound and a carbonyl compound. The "culturing in the presence of a pyruvic acid-supplying compound and a carbonyl compound" can be performed by culture in a culture solution containing the pyruvic acid-supplying compound and the carbonyl compound. The "culturing in the presence of a pyruvic acid-supplying compound and a carbonyl compound" can preferably be performed by culture in a culture solution into which the pyruvic acid-supplying compound and the carbonyl compound are added.

Prior to the culture in the presence of a pyruvic acid-supplying compound and a carbonyl compound, it is preferable to perform the proliferation culture described in the section "(1.2) Culture step."

When the proliferation culture is performed prior to the production culture, the proliferation culture and subsequent production culture can be performed as described in the section "(1.2) Culture step."

### (3.2.1) Culture solution for proliferation

As a culture solution for proliferation, it is possible to use a culture solution similar to the culture solution for proliferation described in the section "(1.2.1) Culture solution for proliferation."

### (3.2.2) Culture solution for production

As a culture solution for production, it is possible to use a culture solution similar to the culture solution for production described in the section "(1.2.2) Culture solution for production."

As a specific culture solution for production, it is possible to use a medium containing glucose, formaldehyde, potassium dihydrogen phosphate, dipotassium hydrogen phosphate, magnesium sulfate, iron(II) sulfate, and manganese(II) sulfate, and having a pH of 7.0.

When one kind of carbonyl compound is used in the culture solution for production, it is possible to easily perform separation and purification to be described later in the section "(3.2.4) Recovery of 2,4-dihydroxycarboxylic acid."

### (3.2.3) Conditions for production culture

A culture temperature in the production culture may be any temperature as long as it is a temperature suitable for production of 2,4-dihydroxycarboxylic acid. A culture temperature in the production culture is preferably about 20°C to 50°C, and is more preferably about 25°C to 47°C. It is possible to efficiently produce a 2,4-dihydroxycarboxylic acid when a temperature is within the above-mentioned temperature range. A pH of the culture solution for production may be any pH as long as it is suitable for production of 2,4-dihydroxycarboxylic acid. A pH of the culture solution for production is preferably maintained within a range of about 6 to 8. A pH of the culture solution for production can be adjusted using an inorganic or organic acid; an alkaline solution such as an aqueous solution of potassium hydroxide; urea; calcium carbonate; ammonia; a pH buffer solution containing 4-morpholinopropanesulfonic acid; or the like. A pH of the culture solution for production can be appropriately adjusted as necessary even during the production culture. A culture time in the production culture may be any time as long as it is a time sufficient for a transformant to produce a 2,4-dihydroxycarboxylic acid. A culture time in the production culture is preferably about 3 hours to 7 days, and is more preferably about 1 to 3 days.

The production culture may be performed under aerobic conditions, or may be performed under anaerobic conditions or microaerobic conditions. This is because the above-described transformant has a pathway that is for producing a 2,4-dihydroxycarboxylic acid and works under both aerobic conditions and anaerobic conditions or microaerobic conditions. The pathway for producing a 2,4-dihydroxycarboxylic acid will be described in the section "(3.3) Actions and effects" to be described later.

### <Anaerobic conditions or microaerobic conditions>

The production culture is preferably performed under anaerobic conditions or microaerobic conditions.

When a transformant is cultured under anaerobic conditions or microaerobic conditions, the transformant does not proliferate substantially, and thereby 2,4-dihydroxycarboxylic acid can be produced more efficiently.

The "anaerobic conditions or microaerobic conditions" can be set to the same conditions as the "anaerobic conditions or microaerobic conditions" described in the section "(1.2.3) Conditions for production culture."

### <High density conditions>

The production culture is preferably performed in a state in which a transformant is suspended at a high density in a culture solution for production. When the transformant is cultured in such a state, 2,4-dihydroxycarboxylic acid can be produced more efficiently.

The "state in which a transformant is suspended at a high density in a culture solution for production" can be set to the same state as the "state in which a transformant is suspended at a high density in a culture solution for production" described in the section "(1.2.3) Conditions for production culture."

The production culture is more preferably performed under anaerobic conditions or microaerobic conditions, and in a state in which a transformant is suspended at a high density in a culture solution for production.

### (3.2.4) Recovery of 2,4-dihydroxycarboxylic acid

As described above, when the transformant containing the first and fourth genes is cultured in a culture solution for production, 2,4-dihydroxycarboxylic acid is produced in the culture solution for production.

Specifically, when a carbonyl compound represented by General Formula (1) is used in a culture solution for production, it is possible to produce 2,4-dihydroxycarboxylic acid represented by General Formula (5).

General Formula (1): R₁₀₁C(O)R₁₀₂,

where R₁₀₁ and R₁₀₂ are each independently hydrogen or an alkyl group having 1 to 5 carbon atoms,

General Formula (5): R₁₀₁C(R₁₀₂)(OH)CH₂CH(OH)COOH,

where R₁₀₁ and R₁₀₂ in General Formula (5) are respectively the same groups as R₁₀₁ and R₁₀₂ in General Formula (1).

More specifically, by using a carbonyl compound in which R₁₀₁ is hydrogen and R₁₀₂ is hydrogen in General Formula (1), it is possible to produce a 2,4-dihydroxycarboxylic acid in which R₁₀₁ is hydrogen and R₁₀₂ is hydrogen in General Formula (5) (that is, 2,4-dihydroxybutyric acid).

By using a carbonyl compound in which R₁₀₁ is a methyl group and R₁₀₂ is hydrogen in General Formula (1), it is possible to produce a 2,4-dihydroxycarboxylic acid in which R₁₀₁ is a methyl group and R₁₀₂ is hydrogen in General Formula (5) (that is, 2,4-dihydroxyvaleric acid).

By using a carbonyl compound in which R₁₀₁ is an ethyl group and R₁₀₂ is hydrogen in General Formula (1), it is possible to produce a 2,4-dihydroxycarboxylic acid in which R₁₀₁ is an ethyl group and R₁₀₂ is hydrogen in General Formula (5) (that is, 2,4-dihydroxycaproic acid).

By using a carbonyl compound in which R₁₀₁ is a propyl group and R₁₀₂ is hydrogen in General Formula (1), it is possible to produce a 2,4-dihydroxycarboxylic acid in which R₁₀₁ is a propyl group and R₁₀₂ is hydrogen in General Formula (5) (that is, 2,4-dihydroxyenanthic acid). Specifically, by using a carbonyl compound in which R₁₀₁ is an n-propyl group and R₁₀₂ is hydrogen in General Formula (1), it is possible to produce 2,4-dihydroxycarboxylic acid in which R₁₀₁ is an n-propyl group and R₁₀₂ is hydrogen in General Formula (5) (that is, 2,4-dihydroxy-n-heptanoic acid). Furthermore, specifically, by using a carbonyl compound in which R₁₀₁ is an isopropyl group and R₁₀₂ is hydrogen in General Formula (1), it is possible to produce 2,4-dihydroxycarboxylic acid in which R₁₀₁ is an isopropyl group and R₁₀₂ is hydrogen in General Formula (5) (that is, 2,4-dihydroxyisoenanthic acid).

By using a carbonyl compound in which R₁₀₁ is a pentyl group and R₁₀₂ is hydrogen in General Formula (1), it is possible to produce a 2,4-dihydroxycarboxylic acid in which R₁₀₁ is a pentyl group and R₁₀₂ is hydrogen in General Formula (5) (that is, 2,4-dihydroxycaprylic acid). Specifically, by using a carbonyl compound in which R₁₀₁ is an n-pentyl group and R₁₀₂ is hydrogen in General Formula (1), it is possible to produce 2,4-dihydroxycarboxylic acid in which R₁₀₁ is an n-pentyl group and R₁₀₂ is hydrogen in General Formula (5) (that is, 2,4-dihydroxy-n-caprylic acid).

More specifically, by using a carbonyl compound in which both R₁₀₁ and R₁₀₂ are methyl groups in General Formula (1), it is possible to produce 2,4-dihydroxycarboxylic acid in which both R₁₀₁ and R₁₀₂ are methyl groups in General Formula (5) (that is, 4-methyl-2,4-dihydroxyvaleric acid).

The above-described 2,4-dihydroxycarboxylic acids can be recovered by recovering a culture solution for production, but the 2,4-dihydroxycarboxylic acids can also be separated from the culture solution for production and purified by known methods. Examples of such known methods include a crystallization method, a chromatography method, an ion exchange resin method, an adsorption and elution method using activated carbon, a solvent extraction method, and the like.

From the culture solution for production, it is possible to recover substances such as α-keto acid produced by an aldol reaction catalyzed by the enzyme encoded by the first gene.

In addition, when a plurality of types of carbonyl compounds is used, a plurality of types of 2,4-dihydroxycarboxylic acids is produced in a culture solution. A plurality of types of 2,4-dihydroxycarboxylic acids can be separated from each other by crystallization or chromatography.

### (3.3) Actions and effects

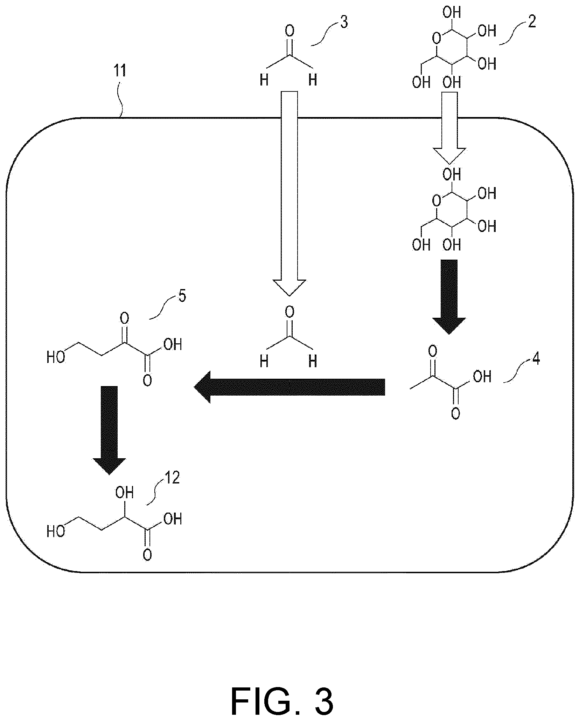

FIG. 3 schematically shows a process in which a 2,4-dihydroxycarboxylic acid-producing microorganism produces a 2,4-dihydroxycarboxylic acid. The process shown in FIG. 3 illustrates a case as an example in which a pyruvic acid-supplying compound is glucose, a carbonyl compound is formaldehyde, and 2,4-dihydroxycarboxylic acid is 2,4-dihydroxybutyric acid.

As shown in FIG. 3, first, a 2,4-dihydroxycarboxylic acid-producing microorganism 11 introduces a glucose 2 and a formaldehyde 3. Next, the glucose 2 is converted into a pyruvic acid 4 through a glycolytic system. Next, the pyruvic acid 4 and the formaldehyde 3 are converted into a 4-hydroxy-2-oxobutyric acid 5 by an "enzyme that catalyzes an aldol reaction between pyruvic acid and aldehyde," which is an enzyme encoded by the first gene. Next, the 4-hydroxy-2-oxobutyric acid 5 is converted into a 2,4-dihydroxybutyric acid 12 by an "enzyme that catalyzes a reduction reaction of an α-keto acid," which is an enzyme encoded by the fourth gene. Accordingly, 2,4-dihydroxybutyric acid is produced.

### (4.) Method for producing 2-amino-4-hydroxycarboxylic acid (biological method)

A method for producing a 2-amino-4-hydroxycarboxylic acid includes a step of culturing a microorganism containing the following genes in the presence of a pyruvic acid-supplying compound selected from pyruvic acid and saccharides, and a carbonyl compound represented by General Formula (1) to produce a 2-amino-4-hydroxycarboxylic acid represented by General Formula (6).
(a) First gene encoding an enzyme that catalyzes an aldol reaction between pyruvic acid and a carbonyl compound, and
(e) Fifth gene encoding an enzyme that catalyzes a reductive amination reaction of an α-keto acid

   General Formula (1): R₁₀₁C(O)R₁₀₂,

   where R₁₀₁ and R₁₀₂ are each independently hydrogen or an alkyl group having 1 to 5 carbon atoms,

   General Formula (6): R₁₀₁C(R₁₀₂)(OH)CH₂CH(NH₂)COOH,

   where R₁₀₁ and R₁₀₂ in General Formula (6) are respectively the same groups as R₁₀₁ and R₁₀₂ in General Formula (1).

In the following description, the above-mentioned microorganism containing the first and fifth genes is also referred to as a "2-amino-4-hydroxycarboxylic acid-producing microorganism." The 2-amino-4-hydroxycarboxylic acid-producing microorganism may be a microorganism that naturally has the first and fifth genes, or may be a microorganism obtained by transforming a host with the first and fifth genes. Hereinafter, a case in which the 2-amino-4-hydroxycarboxylic acid-producing microorganism is a transformant will be described as an example.

### (4.1) Transformant

### (4.1.1) Host

As a host, it is possible to use a host similar to the host described in the section "(1.1.1) Host."

Next, the first and fifth genes introduced into the host will be described.

### (4.1.2) First gene

As the first gene, it is possible to use a gene similar to the first gene described in the section "(1.1.2) First gene."

### (4.1.3) Fifth gene

The fifth gene encodes an "enzyme that catalyzes a reductive amination reaction of an α-keto acid." The "enzyme that catalyzes a reductive amination reaction of an α-keto acid" refers to an enzyme having an activity of 10 mU/mg or more of protein when measuring the activity according to a measurement method to be described later using an enzymatic reaction solution containing purified protein and α-keto acid (initial concentration of substrate: 1 mM) as an enzymatic reaction solution. The fifth gene encodes, for example, the enzyme that catalyzes a reductive amination reaction of an α-keto acid obtained by an aldol reaction catalyzed by a protein encoded by the first gene. The α-keto acid referred to in the phrase "enzyme that catalyzes a reductive amination reaction of an α-keto acid" is, for example, an α-keto acid represented by General Formula (2) described in the section "(1.) Method for producing α-keto acid." The "enzyme that catalyzes a reductive amination reaction of an α-keto acid" is, for example, glutamate/phenylalanine/leucine/valine dehydrogenase (IPR006095). The term "glutamate/phenylalanine/leucine/valine dehydrogenase" has been used in the technical field of the present specification as a term meaning a dehydrogenase family including glutamate dehydrogenase, phenylalanine dehydrogenase, leucine dehydrogenase, and valine dehydrogenase. According to one example, the "glutamate/phenylalanine/leucine/valine dehydrogenase" is at least one dehydrogenase selected from the group consisting of glutamate dehydrogenase, phenylalanine dehydrogenase, leucine dehydrogenase, and valine dehydrogenase.

The fifth gene may be a gene encoding glutamate/phenylalanine/leucine/valine dehydrogenase and exemplified below.
(d1) Leucine dehydrogenase (EC number: 1.4.1.9);
(d2) Phenylalanine dehydrogenase (EC number: 1.4.1.20); and
(d3) Valine dehydrogenase (EC numbers: 1.4.1.8 and 1.4.1.23)

Examples of dehydrogenases included in (d1) include leuDH protein. The leuDH protein is preferably derived from *Lysinibacillus sphaericus, Bacillus cereus,* or *Geobacillus stearothermophillus.*

Examples of dehydrogenases included in (d2) include phedh protein. The phedh protein is preferably derived from *Bacillus badius.*

Examples of dehydrogenases included in (d3) include valdh protein. The valdh protein is preferably derived from *Streptomyces fradiae.*

The fifth gene is preferably selected from the group consisting of the following genes.
(5-1) Gene encoding a protein that consists of an amino acid sequence set forth in SEQ ID NO: 30 and catalyzes a reductive amination reaction of an α-keto acid (that is, leuDH protein derived from *Lysinibacillus sphaericus*),
(5-2) Gene encoding a protein that consists of an amino acid sequence in which one or a plurality of amino acids is deleted, substituted, or added in SEQ ID NO: 30, and has an activity of catalyzing a reductive amination reaction of an α-keto acid,
(5-3) Gene encoding a protein that consists of an amino acid sequence set forth in SEQ ID NO: 32 and catalyzes a reductive amination reaction of an α-keto acid (that is, phedh protein derived from *Bacillus badius*),
(5-4) Gene encoding a protein that consists of an amino acid sequence in which one or a plurality of amino acids is deleted, substituted, or added in SEQ ID NO: 32, and has an activity of catalyzing a reductive amination reaction of an α-keto acid,
(5-5) Gene encoding a protein that consists of an amino acid sequence set forth in SEQ ID NO: 34 and catalyzes a reductive amination reaction of an α-keto acid (that is, valdh protein derived from *Streptomyces fradiae*), and
(5-6) Gene encoding a protein that consists of an amino acid sequence in which one or a plurality of amino acids is deleted, substituted, or added in SEQ ID NO: 34, and has an activity of catalyzing a reductive amination reaction of an α-keto acid.

The "gene encoding a protein that consists of an amino acid sequence in which one or a plurality of amino acids is deleted, substituted, or added in SEQ ID NO: 30, and has an activity of catalyzing a reductive amination reaction of an α-keto acid" may be a "gene encoding a protein that consists of an amino acid sequence homologous to the amino acid sequence set forth in SEQ ID NO: 30 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The "gene encoding a protein that consists of an amino acid sequence in which one or a plurality of amino acids is deleted, substituted, or added in SEQ ID NO: 32, and has an activity of catalyzing a reductive amination reaction of an α-keto acid" may be a "gene encoding a protein that consists of an amino acid sequence homologous to the amino acid sequence set forth in SEQ ID NO: 32 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The "gene encoding a protein that consists of an amino acid sequence in which one or a plurality of amino acids is deleted, substituted, or added in SEQ ID NO: 34, and has an activity of catalyzing a reductive amination reaction of an α-keto acid" may be a "gene encoding a protein that consists of an amino acid sequence homologous to the amino acid sequence set forth in SEQ ID NO: 34 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The fifth gene is more preferably the following genes.

An leuDH gene that is derived from *Lysinibacillus sphaericus* and consists of a base sequence set forth in SEQ ID NO: 30,

A phedh gene that is derived from *Bacillus badius* and consists of a base sequence set forth in SEQ ID NO: 32, and

A valdh gene that is derived from *Streptomyces fradiae* and consists of a base sequence set forth in SEQ ID NO: 34.

A base sequence set forth in SEQ ID NO: 29 and an amino acid sequence set forth in SEQ ID NO: 30 are as follows.

**[Table 29]**

| |
|---|
| (SEQ ID NO: 29) Ls_leuDH gene |
| atgGAAATCTTCAAGTATATGGAAAAGTATGATTATGAACAATTGGTATTTTGCCAAGAC GAAGCATCTGGGTTAAAAGCGATTATCGCTATCCATGACACAACACTTGGACCAGCATTA GGTGGTGCTCGTATGTGGACCTACGCGACAGAAGAAAATGCGATTGAGGATGCATTAAGA TTAGCACGCGGGATGACATATAAAAATGCAGCTGCTGGTTTAAACCTTGGCGGTGGAAAA ACGGTCATTATTGGGGACCCATTTAAAGATAAAAACGAAGAAATGTTCCGTGCTTTAGGT CGTTTCATTCAAGGATTAAACGGTCGCTATATTACCGCTGAAGATGTTGGTACAACCGTA ACAGATATGGATTTAATCCATGAGGAAACAAATTACGTTACAGGTATATCGCCAGCGTTT GGTTCATCGGGTAATCCTTCACCAGTAACTGCTTATGGCGTTTATCGTGGCATGAAAGCA GCGGCGAAAGAAGCATTTGGTACGGATATGCTAGAAGGTCGTACTATATCGGTACAAGGG CTAGGAAACGTAGCTTACAAGCTTTGCGAGTATTTACATAATGAAGGTGCAAAACTTGTA GTAACAGATATTAACCAAGCGGCTATTGATCGTGTTGTCAATGATTTTGGCGCTACAGCA GTTGCACCTGATGAAATCTATTCACAAGAAGTCGATATTTTCTCACCGTGTGCACTTGGC GCAATTTTAAATGACGAAACGATTCCGCAATTAAAAGCAAAAGTTATTGCTGGTTCTGCT AATAACCAACTACAAGATTCACGACATGGAGATTATTTACACGAGCTAGGCATTGTTTAT GCACCTGACTATGTCATTAATGCAGGTGGTGTAATAAATGTCGCGGACGAATTATATGGC TATAATCGTGAACGAGCGTTGAAACGTGTAGATGGTATTTACGATAGTATTGAAAAAATC TTTGAAATTTCCAAACGTGATAGTATTCCAACATATGTTGCGGCAAATCGTTTGGCAGAA GAACGTATTGCTCGTGTAGCGAAATCGCGTAGTCAGTTCTTAAAAAATGAAAAAAATATT TTGAACGGCCGTtaa |

**[Table 30]**

| |
|---|
| (SEQ ID NO: 30) Ls_leuDH protein |
| MEIFKYMEKYDYEQLVFCQDEASGLKAIIAIHDTTLGPALGGARMWTYATEENAIEDALR LARGMTYKNAAAGLNLGGGKTVIIGDPFKDKNEEMFRALGRFIQGLNGRYITAEDVGTTV TDMDLIHEETNYVTGISPAFGSSGNPSPVTAYGVYRGMKAAAKEAFGTDMLEGRTISVQG LGNVAYKLCEYLHNEGAKLVVTDINQAAIDRVVNDFGATAVAPDEIYSQEVDIFSPCALG AILNDETIPQLKAKVIAGSANNQLQDSRHGDYLHELGIVYAPDYVINAGGVINVADELYG YNRERALKRVDGIYDSIEKIFEISKRDSIPTYVAANRLAEERIARVAKSRSQFLKNEKNI LNGR |

A base sequence set forth in SEQ ID NO: 31 and an amino acid sequence set forth in SEQ ID NO: 32 are as follows.

**[Table 31]**

| |
|---|
| (SEQ ID NO: 31) Bb_phedh gene |
| atgAGCTTAGTAGAAAAACATCCATCATAAAAGATTTCACTCTTTTGAAAAAATGTCT GAACATGAACAAGTTGTTTTTGCAACGATCCGGCGACAGGACTAAGGGCCATTATCGCT ATTCATGACACCACACTCGGACCTGCGCTCGGCGGCTGCCGCATGCAGCCTTATAACAGT GTGGAAGAAGCATTGGAAGATGCTCTTCGCCTTTCCAAAGGAATGACTTACAAATGCGCG GCGTCCGATGTCGACTTTGGCGGCGGAAAAGCAGTCATTATCGGTGATCCGCAGAAAGAT AAATCTCCAGAACTGTTCCGCGCGTTTGGCCAATTTGTTGATTCGCTTGGCGGCCGTTTC TATACAGGTACTGATATGGGAACGAATATGGAAGATTTCATTCACGCCATGAAAGAAACA AACTGCATTGTTGGGGTGCCGGAAGCTTACGGCGGCGGAGATTCCTCTATTCCAACT GCCATGGGTGTCCTGTACGGCATTAAAGCAACCAACAAAATGTTGTTTGGCAAGGACGAT CTTGGCGGCGTCACTTATGCCATTCAAGGACTTGGCAAAGTAGGCTACAAAGTAGCGGAA GGGCTGCTCGAAGAAGGTGCTCATTTATTTGTAACGGATATTAACGAGCAAAGCTTGGAG GCTATCCAGGAAAAGCAAAAACAACATCCGGTTCTGTCACGGTAGTAGCGAGCGATGAA ATTTATTCCCAGGAAGCCGATGTGTTCGTTCCGTGTGCATTTGGCGGCGTTGTTAATGAT GAAACGATGAAGCAGTTCAAGGTGAAAGCAATCGCCGGTTCAGCCAACAATCAGCTGCTT ACGGAGGATCACGGCAGACAGCTTGCAGACAAAGGCATTCTGTATGCTCCGGATTATATT GTTAACTCTGGCGGTCTGATCCAAGTAGCCGACGAATTGTATGAGGTGAACAAAGAACGC GTGCTTGCGAAGACGAAGCATATTTACGACGCAATTCTTGAAGTGTACCAGCAAGCGGAA TTAGATCAAATCACCACAATGGAAGCAGCCAACAGAATGTGTGAGCAAAGAATGGCGGCA AGAGGCCGACGCAACAGCTTCTTTACTTCTTCTGTTAAGCCAAAATGGGATATTCGCAAC taa |

**[Table 32]**

| |
|---|
| (SEQ ID NO: 32) Bb_phedh protein |
| MSLVEKTSIIKDFTLFEKMSEHEQVVFCNDPATGLRAIIAIHDTTLGPALGGCRMQPYNS VEEALEDALRLSKGMTYKCAASDVDFGGGKAVIIGDPQKDKSPELFRAFGQFVDSLGGRF YTGTDMGTNMEDFIHAMKETNCIVGVPEAYGGGGDSSIPTAMGVLYGIKATNKMLFGKDD LGGVTYAIQGLGKVGYKVAEGLLEEGAHLFVTDINEQSLEAIQEKAKTTSGSVTVVASDE IYSQEADVFVPCAFGGVVNDETMKQFKVKAIAGSANNQLLTEDHGRQLADKGILYAPDYI VNSGGLIQVADELYEVNKERVLAKTKHIYDAILEVYQQAELDQITTMEAANRMCEQRMAA RGRRNSFFTSSVKPKWDIRN |

A base sequence set forth in SEQ ID NO: 33 and an amino acid sequence set forth in SEQ ID NO: 34 are as follows.

**[Table 33]**

| |
|---|
| (SEQ ID NO: 33) Sf_valdh gene |
| atgACCGACGCGTCCACCCACCGCCGCCGACGACCTCGGCGCCCTCTCCACCCTCTTC CGCTCCGAGCAGGGCGGCCACGAGCGGTCCTGCTCTGCCAGGACCGCGAGAGCGGCCTC AAGGCCGTCATCGCCCTCCACTCGACCGCCCTGGGCCCGGCCCTCGGCGGCACCCGCTTC CATGCCTACGCCTCCGACGAGGAGGCCGTGCTCGACGCCCTGAACCTCGCCCGCGGCATG TCGTACAAGAACGCCCTCGCCGGGCTGCCGCACGGCGGCGGCAAGGCCGTGATCATCGGC AGCCCCGCCCCGGTCTCCGAGGGCGGGCTGAAGAGCGAGGCGCTGCTCCGCGCGTACGGC CGCTTCGTCGCCTCCCTGGACGGCCGTTACGTCACGGCCTGTGACGTCGGCACGTATGTC GCCGACATGGACGTCGTCGCCCGCGAGTGCCGGTGGACCACCGGCCGCTCCCCGGAGAAC GGCGGCGCCGGCGACTCCTCCGTCCTCACCGCCTTCGGCGTCTTCCAGGGCATGCGGGCC AGCGCTCAGGCGCTGTGGGGCGAGCCCACCCTGCGCGGCCGGACCGTCGGCGTGGCCGGG GTCGGCAAGGTCGGCCACCACCTGGTGGACCACCTGGTGGAGGACGGTGCCCGGGTCGTG GTCACCGATGTGCGGCCGGAGAGCGTGGAGCGGGTGCGCGCCCGGCACCCGCGGGTGGTG GCCGTGCCCGACACGGAGTCCCTGATCCGCGCGGACCTGGACGTCTACGCCCCCTGCGCC CTCGGCGGTGCCCTGAACGACGACACGGTGCCCGCGCTGACCGCGCGCGTCGTCTGCGGC GCGGCCAACAACCAGCTCGCCCACCCGGGCGTCGAAAAGGACCTCGCCGGCCGCGGCATC CTGTACGCGCCCGACTACGTCGTCAACGCCGGTGGCGTGATCCAGGTCGCCGACGAGCTG CTCGGTTTTGATTTCGACCGGGCGAAGGCCAAGGCGGCGCAGATCTTCGACACGACGCTG GCCATATTCGACCGCGCCAGGACCGACGGCGTCCCGCCCGCCGTCGCCGCCGACCGGATC GCCGAGCAGCGCATGGCGGAGGCCCGCACCGTCtaa |

**[Table 34]**

| |
|---|
| (SEQ ID NO: 34) Sf_valdh protein |
| MTDASHPTAADDLGALSTLFRSEQGGHERVLLCQDRESGLKAVIALHSTALGPALGGTRF HAYASDEEAVLDALNLARGMSYKNALAGLPHGGGKAVIIGSPAPVSEGGLKSEALLRAYG RFVASLDGRYVTACDVGTYVADMDVVARECRWTTGRSPENGGAGDSSVLTAFGVFQGMRA SAQALWGEPTLRGRTVGVAGVGKVGHHLVDHLVEDGARVVVTDVRPESVERVRARHPRVV AVPDTESLIRADLDVYAPCALGGALNDDTVPALTARVVCGAANNQLAHPGVEKDLAGRGI LYAPDYVVNAGGVIQVADELLGFDFDRAKAKAAQIFDTTLAIFDRARTDGVPPAVAADRI AEQRMAEARTV |

The activity of catalyzing a reductive amination reaction of an α-keto acid can be calculated by, for example, causing the enzymatic reaction at 25°C in a 100 mM HEPES-KOH buffer (pH 7.5) containing 0.2 mM NADH and 200 mM ammonium chloride, and measuring an initial consumption rate of NADH consumed when the raw material α-keto acid is reduced to α-amino acid. The initial consumption rate of NADH can be calculated by, for example, measuring a rate of decrease in absorbance at 340 nm derived from NADH in an enzymatic reaction solution. In this case, an activity of reducing 1 µmol of α-keto acid to α-amino acid at 25°C for 1 minute is regarded as one unit.

The "gene (leuDH gene mutant) encoding a protein that consists of an amino acid sequence in which one or a plurality of amino acids is deleted, substituted, or added in SEQ ID NO: 30, and has an activity of catalyzing a reductive amination reaction of an α-keto acid" can be selected from a DNA library of animals of another species by the same method as the above-described method of selecting an hpal gene mutant.

The "gene (phedh gene mutant) encoding a protein that consists of an amino acid sequence in which one or a plurality of amino acids is deleted, substituted, or added in SEQ ID NO: 32, and has an activity of catalyzing a reductive amination reaction of an α-keto acid" can be selected from a DNA library of animals of another species by the same method as the above-described method of selecting an hpal gene mutant.

The "gene (valdh gene mutant) encoding a protein that consists of an amino acid sequence in which one or a plurality of amino acids is deleted, substituted, or added in SEQ ID NO: 34, and has an activity of catalyzing a reductive amination reaction of an α-keto acid" can be selected from a DNA library of animals of another species by the same method as the above-described method of selecting an hpal gene mutant.

### (4.1.4) Construction of recombinant vector for transformation

The first and fifth genes can be incorporated into an appropriate plasmid vector for transformation.

As the plasmid vector, it is possible to use a plasmid vector similar to the plasmid vector described in the section "(1.1.3) Construction of recombinant vector for transformation."

The first and fifth genes may be respectively inserted into individual plasmid vectors or may be inserted into the same plasmid vector. A transformant can be produced using the obtained recombinant vector.

### (4.1.5) Transformation

As a transformation method, it is possible to use a known method without limitation. As such a known method, it is possible to use a method similar to the method described in the section "(1.1.4) Transformation."

In a case where a transformant is produced using a recombinant vector as described above, the first and fifth genes may be respectively inserted into chromosomes of a host, or may be present in cytoplasm of the host in the form of recombinant vectors.

### (4.1.6) Disruption or deletion of chromosomal gene of host

The host may be a wild strain, a mutant strain thereof, or an artificial gene recombinant. In a case where the host is a coryneform bacterium, for the host, it is possible to use a gene-disrupted strain or gene-deleted strain similar to the gene-disrupted strain or gene-deleted strain described in the section "(1.1.5) Disruption or deletion of chromosomal gene of host." Furthermore, as a method for producing a gene-disrupted strain or gene-deleted strain, it is possible to use a method similar to the method described in the section "(1.1.5) Disruption or deletion of chromosomal gene of host." By using such a gene-disrupted strain or gene-deleted strain as a host, it is possible to improve productivity for 2-amino-4-hydroxycarboxylic acid or to inhibit production of by-products.

### (4.1.7) Specific examples of 2-amino-4-hydroxycarboxylic acid-producing microorganisms

Specifically, as a 2-amino-4-hydroxycarboxylic acid-producing microorganism, a coryneform bacterium can be used which comprises:
the first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound; and
the fifth gene selected from the group consisting of:
   (5-1) a gene encoding a protein that consists of an amino acid sequence set forth in SEQ ID NO: 30 and catalyzes a reductive amination reaction of α-keto acids;
   (5-2) a gene encoding a protein that consists of an amino acid sequence in which one or a plurality of amino acids is deleted, substituted, or added in SEQ ID NO: 30, and has an activity of catalyzing a reductive amination reaction of α-keto acids;
   (5-3) a gene encoding a protein that consists of an amino acid sequence set forth in SEQ ID NO: 32 and catalyzes a reductive amination reaction of α-keto acids;
   (5-4) a gene encoding a protein that consists of an amino acid sequence in which one or a plurality of amino acids is deleted, substituted, or added in SEQ ID NO: 32, and has an activity of catalyzing a reductive amination reaction of α-keto acids;
   (5-5) a gene encoding a protein that consists of an amino acid sequence set forth in SEQ ID NO: 34 and catalyzes a reductive amination reaction of α-keto acids; and
   (5-6) a gene encoding a protein that consists of an amino acid sequence in which one or a plurality of amino acids is deleted, substituted, or added in SEQ ID NO: 34, and has an activity of catalyzing a reductive amination reaction of α-keto acids.

### (4.2) Culture step

A 2-amino-4-hydroxycarboxylic acid can be produced by culturing a 2,4-dihydroxycarboxylic acid-producing microorganism (for example, the above-described transformant) in the presence of a pyruvic acid-supplying compound and a carbonyl compound. The "culturing in the presence of a pyruvic acid-supplying compound and a carbonyl compound" can be performed by culture in a culture solution containing the pyruvic acid-supplying compound and the carbonyl compound. The "culturing in the presence of a pyruvic acid-supplying compound and a carbonyl compound" can preferably be performed by culture in a culture solution into which the pyruvic acid-supplying compound and the carbonyl compound are added.

Prior to the culture in the presence of a pyruvic acid-supplying compound and a carbonyl compound, it is preferable to perform the proliferation culture described in the section "(1.2) Culture step."

When the proliferation culture is performed prior to the production culture, the proliferation culture and subsequent production culture can be performed as described in the section "(1.2) Culture step."

### (4.2.1) Culture solution for proliferation

As a culture solution for proliferation, it is possible to use a culture solution similar to the culture solution for proliferation described in the section "(1.2.1) Culture solution for proliferation."

### (4.2.2) Culture solution for production

As a culture solution for production, it is possible to use a culture solution similar to the culture solution for production described in the section "(1.2.2) Culture solution for production."

As a specific culture solution for production, it is possible to use a medium containing glucose, formaldehyde, ammonium sulfate, potassium dihydrogen phosphate, dipotassium hydrogen phosphate, magnesium sulfate, iron(II) sulfate, and manganese(II) sulfate, and having a pH of 7.0.

When one kind of carbonyl compound is used in the culture solution for production, it is possible to easily perform separation and purification to be described later in the section "(4.2.4) Recovery of 2-amino-4-hydroxycarboxylic acid."

### (4.2.3) Conditions for production culture

A culture temperature in the production culture may be any temperature as long as it is a temperature suitable for production of 2-amino-4-hydroxycarboxylic acid. A culture temperature in the production culture is preferably about 20°C to 50°C, and is more preferably about 25°C to 47°C. It is possible to efficiently produce a 2-amino-4-hydroxycarboxylic acid when a temperature is within the above-mentioned temperature range. A pH of the culture solution for production may be any pH as long as it is suitable for production of 2-amino-4-hydroxycarboxylic acid. A pH of the culture solution for production is preferably maintained within a range of about 6 to 8. A pH of the culture solution for production can be adjusted using an inorganic or organic acid; an alkaline solution such as an aqueous solution of potassium hydroxide; urea; calcium carbonate; ammonia; a pH buffer solution containing 4-morpholinopropanesulfonic acid; or the like. A pH of the culture solution for production can be appropriately adjusted as necessary even during the production culture. A culture time in the production culture may be any time as long as it is a time sufficient for a transformant to produce a 2-amino-4-hydroxycarboxylic acid. A culture time in the production culture is preferably about 3 hours to 7 days, and is more preferably about 1 to 3 days.

The production culture may be performed under aerobic conditions, or may be performed under anaerobic conditions or microaerobic conditions. This is because the above-described transformant has a pathway that is for producing a 2-amino-4-hydroxycarboxylic acid and works under both aerobic conditions and anaerobic conditions or microaerobic conditions. The pathway for producing a 2-amino-4-hydroxycarboxylic acid will be described in the section "(4.3) Actions and effects" to be described later.

### <Anaerobic conditions or microaerobic conditions>

The production culture is preferably performed under anaerobic conditions or microaerobic conditions.

When a transformant is cultured under anaerobic conditions or microaerobic conditions, the transformant does not proliferate substantially, and thereby 2-amino-4-hydroxycarboxylic acid can be produced more efficiently.

The "anaerobic conditions or microaerobic conditions" can be set to the same conditions as the "anaerobic conditions or microaerobic conditions" described in the section "(1.2.3) Conditions for production culture."

### <High density conditions>

The production culture is preferably performed in a state in which a transformant is suspended at a high density in a culture solution for production. When the transformant is cultured in such a state, 2-amino-4-hydroxycarboxylic acid can be produced more efficiently.

The "state in which a transformant is suspended at a high density in a culture solution for production" can be set to the same state as the "state in which a transformant is suspended at a high density in a culture solution for production" described in the section "(1.2.3) Conditions for production culture."

The production culture is more preferably performed under anaerobic conditions or microaerobic conditions, and in a state in which a transformant is suspended at a high density in a culture solution for production.

### (4.2.4) Recovery of 2-amino-4-hydroxycarboxylic acid

As described above, when the transformant containing the first and fifth genes is cultured in a culture solution for production, 2-amino-4-hydroxycarboxylic acid is produced in the culture solution for production.

Specifically, when a carbonyl compound represented by General Formula (1) is used in a culture solution for production, it is possible to produce 2-amino-4-hydroxycarboxylic acid represented by General Formula (6).

General Formula (1): R₁₀₁C(O)R₁₀₂,

where R₁₀₁ and R₁₀₂ are each independently hydrogen or an alkyl group having 1 to 5 carbon atoms,

General Formula (6): R₁₀₁C(R₁₀₂)(OH)CH₂CH(NH₂)COOH,

where R₁₀₁ and R₁₀₂ in General Formula (6) are respectively the same groups as R₁₀₁ and R₁₀₂ in General Formula (1).

More specifically, by using a carbonyl compound in which R₁₀₁ is hydrogen and R₁₀₂ is hydrogen in General Formula (1), it is possible to produce a 2-amino-4-hydroxycarboxylic acid in which R₁₀₁ is hydrogen and R₁₀₂ is hydrogen in General Formula (6) (that is, 2-amino-4-hydroxybutyric acid).

By using a carbonyl compound in which R₁₀₁ is a methyl group and R₁₀₂ is hydrogen in General Formula (1), it is possible to produce a 2-amino-4-hydroxycarboxylic acid in which R₁₀₁ is a methyl group and R₁₀₂ is hydrogen in General Formula (6) (that is, 2-amino-4-hydroxyvaleric acid).

By using a carbonyl compound in which R₁₀₁ is an ethyl group and R₁₀₂ is hydrogen in General Formula (1), it is possible to produce a 2-amino-4-hydroxycarboxylic acid in which R₁₀₁ is an ethyl group and R₁₀₂ is hydrogen in General Formula (6) (that is, 2-amino-4-hydroxycaproic acid).

By using a carbonyl compound in which R₁₀₁ is a propyl group and R₁₀₂ is hydrogen in General Formula (1), it is possible to produce a 2-amino-4-hydroxycarboxylic acid in which R₁₀₁ is a propyl group and R₁₀₂ is hydrogen in General Formula (6) (that is, 4-hydroxy-2-oxoenanthic acid). Specifically, by using a carbonyl compound in which R₁₀₁ is an n-propyl group and R₁₀₂ is hydrogen in General Formula (1), it is possible to produce a 2-amino-4-hydroxycarboxylic acid in which R₁₀₁ is an n-propyl group and R₁₀₂ is hydrogen in General Formula (6) (that is, 2-amino-4-hydroxy-n-heptanoic acid). Furthermore, specifically, by using a carbonyl compound in which R₁₀₁ is an isopropyl group and R₁₀₂ is hydrogen in General Formula (1), it is possible to produce a 2-amino-4-hydroxycarboxylic acid in which R₁₀₁ is an isopropyl group and R₁₀₂ is hydrogen in General Formula (6) (that is, 2-amino-4-hydroxyisoenanthic acid).

By using a carbonyl compound in which R₁₀₁ is a pentyl group and R₁₀₂ is hydrogen in General Formula (1), it is possible to produce a 2-amino-4-hydroxycarboxylic acid in which R₁₀₁ is a pentyl group and R₁₀₂ is hydrogen in General Formula (6) (that is, 2-amino-4-hydroxycaprylic acid). Specifically, by using a carbonyl compound in which R₁₀₁ is an n-pentyl group and R₁₀₂ is hydrogen in General Formula (1), it is possible to produce a 2-amino-4-hydroxycarboxylic acid in which R₁₀₁ is an n-pentyl group and R₁₀₂ is hydrogen in General Formula (6) (that is, 2-amino-4-hydroxy-n-caprylic acid).

More specifically, by using a carbonyl compound in which both R₁₀₁ and R₁₀₂ are methyl groups in General Formula (1), it is possible to produce a 2-amino-4-hydroxycarboxylic acid in which both R₁₀₁ and R₁₀₂ are methyl groups in General Formula (6) (that is, 4-methyl-2-amino-4-hydroxyvaleric acid).

2-Amino-4-hydroxybutyric acid, which is obtained in a case of using a carbonyl compound in which R₁₀₁ is hydrogen and R₁₀₂ is hydrogen in General Formula (1), is also called homoserine. Homoserine is, for example, L-homoserine. In the case of using a carbonyl compound in which R₁₀₁ is hydrogen and R₁₀₂ is hydrogen in General Formula (1), homoserine derivatives are produced in a culture solution for production in addition to homoserine. Examples of homoserine derivatives include threonine and 2-aminobutyric acid.

Threonine is produced from homoserine by, for example, an enzyme that converts homoserine into threonine (for example, a homoserine kinase and a threonine synthase) and is held by the 2-amino-4-hydroxycarboxylic acid-producing microorganism. Threonine is, for example, L-threonine.

2-Aminobutyric acid is produced from threonine by, for example, an enzyme that converts threonine into 2-aminobutyric acid (for example, a combination of a threonine deaminase and any of an amino acid dehydrogenase and an amino group transferase) and is held by the 2-amino-4-hydroxycarboxylic acid-producing microorganism. The amino acid dehydrogenase is, for example, glutamate/phenylalanine/leucine/valine dehydrogenase. Glutamate/phenylalanine/leucine/valine dehydrogenase is specifically leucine dehydrogenase.

The above-described 2-amino-4-hydroxycarboxylic acids and homoserine derivatives can be recovered by recovering a culture solution for production, but the 2-amino-4-hydroxycarboxylic acids can also be separated from the culture solution for production and purified by known methods. Examples of such known methods include a crystallization method, a chromatography method, an ion exchange resin method, an adsorption and elution method using activated carbon, a solvent extraction method, and the like.

From the culture solution for production, it is possible to recover substances such as α-keto acid produced by an aldol reaction catalyzed by the enzyme encoded by the first gene.

As described above, in the case of using a carbonyl compound in which R₁₀₁ is hydrogen and R₁₀₂ is hydrogen in General Formula (1), homoserine, threonine and 2-aminobutyric acid are produced in a culture solution. Homoserine, threonine, and 2-aminobutyric acid can be separated from each other by crystallization or chromatography.

In addition, when a plurality of types of carbonyl compounds is used, a plurality of types of 2-amino-4-hydroxycarboxylic acids is produced in a culture solution. A plurality of types of 2-amino-4-hydroxycarboxylic acids can be separated from each other by crystallization or chromatography.

### (4.3) Actions and effects

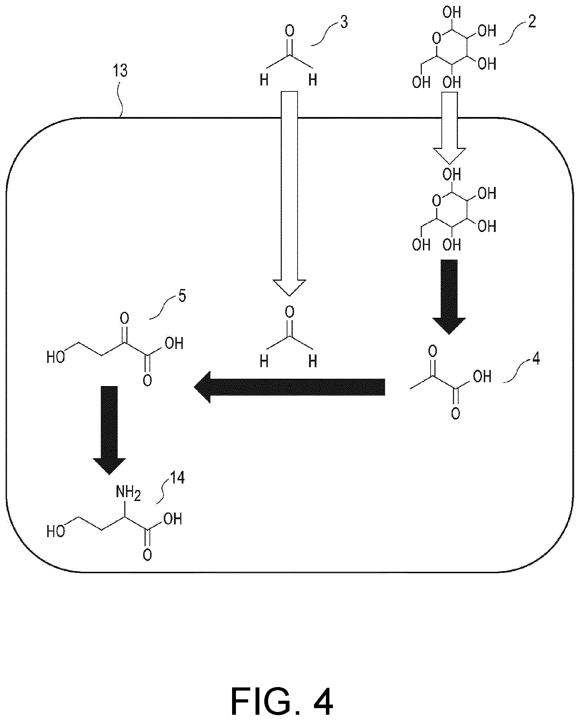

FIG. 4 schematically shows a process in which a 2-amino-4-hydroxycarboxylic acid-producing microorganism produces a 2-amino-4-hydroxycarboxylic acid. The process shown in FIG. 4 illustrates a case as an example in which a pyruvic acid-supplying compound is glucose, a carbonyl compound is formaldehyde, and 2-amino-4-hydroxycarboxylic acid is homoserine.

As shown in FIG. 4, first, a 2-amino-4-hydroxycarboxylic acid-producing microorganism 13 introduces a glucose 2 and a formaldehyde 3. Next, the glucose 2 is converted into a pyruvic acid 4 through a glycolytic system. Next, the pyruvic acid 4 and the formaldehyde 3 are converted into a 4-hydroxy-2-oxobutyric acid 5 by an "enzyme that catalyzes an aldol reaction between pyruvic acid and aldehyde," which is an enzyme encoded by the first gene. Next, the 4-hydroxy-2-oxobutyric acid 5 is converted into a homoserine 14 by an "enzyme that catalyzes a reductive amination reaction of an α-keto acid," which is an enzyme encoded by the fifth gene. Accordingly, homoserine is produced.

### (5.) Method for producing 2-amino-4-hydroxycarboxylic acid (chemical method)

The method for producing a 2-amino-4-hydroxycarboxylic acid by a biological method using microorganisms was described in the section "(4.) Method for producing 2-amino-4-hydroxycarboxylic acid." This method can also be performed by a chemical method without using microorganisms.

That is, a method for producing a 2-amino-4-hydroxycarboxylic acid includes a step of reacting an α-keto acid represented by General Formula (2), a nitrogen source, and a reducing agent in the presence of glutamate/phenylalanine/leucine/valine dehydrogenase to produce a 2-amino-4-hydroxycarboxylic acid represented by General Formula (6).

General Formula (2): R₁₀₁C(R₁₀₂)(OH)CH₂COCOOH,

where R₁₀₁ and R₁₀₂ are each independently hydrogen or an alkyl group having 1 to 5 carbon atoms,

General Formula (6): R₁₀₁C(R₁₀₂)(OH)CH₂CH(NH₂)COOH,

where R₁₀₁ and R₁₀₂ in General Formula (6) are respectively the same groups as R₁₀₁ and R₁₀₂ in General Formula (2).

Reaction of an α-keto acid, a nitrogen source, and a reducing agent in the presence of glutamate/phenylalanine/leucine/valine dehydrogenase can be performed in, for example, a reaction solution containing α-keto acid, a nitrogen source, a reducing agent, glutamate/phenylalanine/leucine/valine dehydrogenase, and a buffer. Hereinafter, for a reaction of an α-keto acid, a nitrogen source, and a reducing agent in the presence of glutamate/phenylalanine/leucine/valine dehydrogenase, a case in which the reaction is performed in the reaction solution will be described as an example.

### (5.1) Reaction solution

Each of components contained in the reaction solution will be described below. A concentration of each of the components in the reaction solution described in the present specification refers to a final concentration thereof in the reaction solution at a time point when each of the components was added.

### (5.1.1) α-Keto acid

An α-keto acid is represented by General Formula (2).

General Formula (2): R₁₀₁C(R₁₀₂)(OH)CH₂COCOOH,

where R₁₀₁ and R₁₀₂ are each independently hydrogen or an alkyl group having 1 to 5 carbon atoms.

According to one example, as the α-keto acid, it is possible to use an α-keto acid in which, in General Formula (2), R₁₀₁ is hydrogen or an alkyl group having 1 to 5 carbon atoms, and R₁₀₂ is hydrogen.

According to another example, as the α-keto acid, it is possible to use an α-keto acid in which, in General Formula (2), R₁₀₁ is a methyl group, and R₁₀₂ is an alkyl group having 1 to 5 carbon atoms.

Specifically, as the α-keto acid represented by General Formula (2), it is possible to use the following α-keto acids (where the following R₁₀₁'s and R₁₀₂'s are respectively in General Formula (2)).

An α-keto acid in which R₁₀₁ is hydrogen and R₁₀₂ is hydrogen (that is, 4-hydroxy-2-oxobutyric acid);

An α-keto acid in which R₁₀₁ is a methyl group and R₁₀₂ is hydrogen (that is, 4-hydroxy-2-oxovaleric acid);

An α-keto acid in which R₁₀₁ is an ethyl group and R₁₀₂ is hydrogen (that is, 4-hydroxy-2-oxocaproic acid);

An α-keto acid in which R₁₀₁ is a propyl group and R₁₀₂ is hydrogen (that is, 4-hydroxy-2-oxoenanthic acid), specifically, an α-keto acid in which R₁₀₁ is an n-propyl group and R₁₀₂ is hydrogen (that is, 4-hydroxy-2-oxo-n-heptanoic acid), or an α-keto acid in which R₁₀₁ is an isopropyl group and R₁₀₂ is hydrogen (that is, 4-hydroxy-2-oxoisoenanthic acid); and

An α-keto acid in which R₁₀₁ is a pentyl group and R₁₀₂ is hydrogen (that is, 4-hydroxy-2-oxocaprylic acid), specifically, an α-keto acid in which R₁₀₁ is an n-pentyl group and R₁₀₂ is hydrogen (that is, 4-hydroxy-2-oxo-n-caprylic acid)

In addition, as the α-keto acid represented by General Formula (2), it is possible to use an α-keto acid in which both R₁₀₁ and R₁₀₂ are methyl groups in General Formula (2) (that is, 4-methyl-4-hydroxy-2-oxovaleric acid).

As the α-keto acid, one kind may be used, or two or more kinds may be mixed and used.

A concentration of the α-keto acid in the reaction solution can be as high as possible while being within a range not inhibiting production of 2-amino-4-hydroxycarboxylic acid. A concentration of the α-keto acid in the reaction solution is preferably within a range of 0.1 mM to 1 M, and is more preferably within a range of 1 mM to 300 mM. Furthermore, an α-keto acid can be additionally added according to a decrease of the α-keto acid accompanying production of 2-amino-4-hydroxycarboxylic acid.

When one kind of α-keto acid is used in the reaction solution, it is possible to easily perform separation and purification to be described later in the section "(5.3) Recovery of 2-amino-4-hydroxycarboxylic acid."

### (5.1.2) Nitrogen source

As the nitrogen source, it is possible to use any compound that supplies an amino group in a reductive amination reaction catalyzed by glutamate/phenylalanine/leucine/valine dehydrogenase. Specifically, as the nitrogen source, it is possible to use ammonium salts that dissociate in the reaction solution and generate ammonium ions, such as ammonium chloride, ammonium sulfate, and ammonium carbonate.

A concentration of the nitrogen source in the reaction solution can be as high as possible while being within a range not inhibiting production of 2-amino-4-hydroxycarboxylic acid. A concentration of the nitrogen source in the reaction solution is preferably within a range of 0.1 mM to 1 M, and is more preferably within a range of 1 mM to 300 mM. Furthermore, a nitrogen source can be additionally added according to a decrease of the nitrogen source accompanying production of 2-amino-4-hydroxycarboxylic acid.

### (5.1.3) Reducing agent

As the reducing agent, it is possible to use any electron donor that can utilize glutamate/phenylalanine/leucine/valine dehydrogenase. For example, NADH and NADPH can be used.

A concentration of the reducing agent in the reaction solution is preferably within a range of 0.01 mM to 100 M, and is more preferably within a range of 0.1 mM to 10 mM. Furthermore, the reducing agent, which decreases as production of 2-amino-4-hydroxycarboxylic acid proceeds, can be regenerated using appropriate compounds and enzymes. Suitable compounds and enzymes are, for example, ethanol and alcohol dehydrogenase.

### (5.1.4) Glutamate/phenylalanine/leucine/valine dehydrogenase

As the glutamate/phenylalanine/leucine/valine dehydrogenase, it is possible to use any enzyme included in the glutamate/phenylalanine/leucine/valine dehydrogenase. According to one example, the "glutamate/phenylalanine/leucine/valine dehydrogenase" is at least one dehydrogenase selected from the group consisting of glutamate dehydrogenase, phenylalanine dehydrogenase, leucine dehydrogenase, and valine dehydrogenase.

As the glutamate/phenylalanine/leucine/valine dehydrogenase, it is possible to use dehydrogenases exemplified below.
(d1) Leucine dehydrogenase (EC number: 1.4.1.9);
(d2) Phenylalanine dehydrogenase (EC number: 1.4.1.20); and
(d3) Valine dehydrogenase (EC numbers: 1.4.1.8 and 1.4.1.23)

Examples of dehydrogenases included in (d1) include leuDH protein. The leuDH protein is preferably derived from *Lysinibacillus sphaericus, Bacillus cereus,* or *Geobacillus stearothermophillus.*

Examples of dehydrogenases included in (d2) include phedh protein. The phedh protein is preferably derived from *Bacillus badius.*

Examples of dehydrogenases included in (d3) include valdh protein. The valdh protein is preferably derived from *Streptomyces fradiae.*

The glutamate/phenylalanine/leucine/valine dehydrogenase is preferably selected from the group consisting of the following proteins.
(5'-1) Protein that consists of an amino acid sequence set forth in SEQ ID NO: 30 and catalyzes a reductive amination reaction of an α-keto acid;
(5'-2) Protein that consists of an amino acid sequence in which one or a plurality of amino acids is deleted, substituted, or added in SEQ ID NO: 30, and has an activity of catalyzing a reductive amination reaction of an α-keto acid;
(5'-3) Protein that consists of an amino acid sequence set forth in SEQ ID NO: 32 and catalyzes a reductive amination reaction of an α-keto acid;
(5'-4) Protein that consists of an amino acid sequence in which one or a plurality of amino acids is deleted, substituted, or added in SEQ ID NO: 32, and has an activity of catalyzing a reductive amination reaction of an α-keto acid;
(5'-5) Protein that consists of an amino acid sequence set forth in SEQ ID NO: 34 and catalyzes a reductive amination reaction of an α-keto acid; and
(5'-6) Protein that consists of an amino acid sequence in which one or a plurality of amino acids is deleted, substituted, or added in SEQ ID NO: 34, and has an activity of catalyzing a reductive amination reaction of an α-keto acid.

The "protein that consists of an amino acid sequence in which one or a plurality of amino acids is deleted, substituted, or added in SEQ ID NO: 30, and has an activity of catalyzing a reductive amination reaction of an α-keto acid" may be a "protein that consists of an amino acid sequence homologous to the amino acid sequence set forth in SEQ ID NO: 30 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The "protein that consists of an amino acid sequence in which one or a plurality of amino acids is deleted, substituted, or added in SEQ ID NO: 32, and has an activity of catalyzing a reductive amination reaction of an α-keto acid" may be a "protein that consists of an amino acid sequence homologous to the amino acid sequence set forth in SEQ ID NO: 32 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The "protein that consists of an amino acid sequence in which one or a plurality of amino acids is deleted, substituted, or added in SEQ ID NO: 34, and has an activity of catalyzing a reductive amination reaction of an α-keto acid" may be a "protein that consists of an amino acid sequence homologous to the amino acid sequence set forth in SEQ ID NO: 34 by 80% or more, preferably by 90% or more, more preferably by 95% or more, even more preferably by 97% or more, still more preferably by 98% or more, and further more preferably by 99% or more, and has an activity of catalyzing a reductive amination reaction of an α-keto acid."

The activity of catalyzing a reductive amination reaction of an α-keto acid can be measured by the same method as the method of measuring an activity described in the section "(4.1.3.) Fifth gene."

As the glutamate/phenylalanine/leucine/valine dehydrogenase, for example, a commercially available enzyme may be used, or an enzyme obtained by expressing a gene encoding glutamate/phenylalanine/leucine/valine dehydrogenase in a host such as *Escherichia coli* and a coryneform bacterium may be used. In the latter case, a cell disruption solution of a microorganism expressing the enzyme can be used as an enzyme, or an enzyme-containing solution obtained by purifying the cell disruption solution can be used as an enzyme.

A concentration of the glutamate/phenylalanine/leucine/valine dehydrogenase in the reaction solution can be as high as possible while being within a range not inhibiting production of 2-amino-4-hydroxycarboxylic acid. A concentration of the glutamate/phenylalanine/leucine/valine dehydrogenase in the reaction solution is preferably within a range of 0.01 ug/mL to 1 mg/mL, and is more preferably within a range of 0.1 to 100 ug/mL.

### (5.1.5) Buffer

A buffer contains, for example, a buffering agent and other necessary components.

As the buffering agent, any buffering agent can be used. As the buffering agent, it is possible to use, for example, HEPES or glycine. A concentration of the buffering agent in the reaction solution can be set within a range of 1 mM to 1 M.

Furthermore, inorganic salts and the like can be added as necessary.

### (5.2) Reaction conditions

A reaction temperature may be any temperature as long as it is a temperature suitable for production of 2-amino-4-hydroxycarboxylic acid. A reaction temperature is preferably about 4°C to 80°C and is more preferably about 20°C to 60°C. It is possible to efficiently produce a 2-amino-4-hydroxycarboxylic acid when a temperature is within the above-mentioned temperature range. A pH of the reaction solution may be any pH as long as it is a pH suitable for production of 2-amino-4-hydroxycarboxylic acid. A pH of the reaction solution is preferably maintained within a range of about 5 to 11. A pH of the reaction solution can be adjusted using hydrochloric acid, an aqueous solution of potassium hydroxide, or the like. A pH of the reaction solution can be appropriately adjusted as necessary even during a reaction. A reaction time may be any time as long as it is a sufficient time for production of 2-amino-4-hydroxycarboxylic acid. A reaction time is preferably about 1 minute to 10 days, and is more preferably about 1 hour to 3 days.

### (5.3) Recovery of 2-amino-4-hydroxycarboxylic acid

As described above, 2-amino-4-hydroxycarboxylic acid is produced by causing a reaction of an α-keto acid, a nitrogen source, and a reducing agent in the presence of glutamate/phenylalanine/leucine/valine dehydrogenase.

Specifically, when an α-keto acid represented by General Formula (2) is used, it is possible to produce 2-amino-4-hydroxycarboxylic acid represented by General Formula (6).

General Formula (2): R₁₀₁C(R₁₀₂)(OH)CH₂COCOOH,

where R₁₀₁ and R₁₀₂ are each independently hydrogen or an alkyl group having 1 to 5 carbon atoms,

General Formula (6): R₁₀₁C(R₁₀₂)(OH)CH₂CH(NH₂)COOH,

where R₁₀₁ and R₁₀₂ in General Formula (6) are respectively the same groups as R₁₀₁ and R₁₀₂ in General Formula (2).

More specifically, by using an α-keto acid in which R₁₀₁ is hydrogen and R₁₀₂ is hydrogen in General Formula (2), it is possible to produce a 2-amino-4-hydroxycarboxylic acid in which R₁₀₁ is hydrogen and R₁₀₂ is hydrogen in General Formula (6) (that is, 2-amino-4-hydroxybutyric acid).

By using an α-keto acid in which R₁₀₁ is a methyl group and R₁₀₂ is hydrogen in General Formula (2), it is possible to produce a 2-amino-4-hydroxycarboxylic acid in which R₁₀₁ is a methyl group and R₁₀₂ is hydrogen in General Formula (6) (that is, 2-amino-4-hydroxyvaleric acid).

By using an α-keto acid in which R₁₀₁ is an ethyl group and R₁₀₂ is hydrogen in General Formula (2), it is possible to produce a 2-amino-4-hydroxycarboxylic acid in which R₁₀₁ is an ethyl group and R₁₀₂ is hydrogen in General Formula (6) (that is, 2-amino-4-hydroxycaproic acid).

By using an α-keto acid in which R₁₀₁ is a propyl group and R₁₀₂ is hydrogen in General Formula (2), it is possible to produce a 2-amino-4-hydroxycarboxylic acid in which R₁₀₁ is a propyl group and R₁₀₂ is hydrogen in General Formula (6) (that is, 2-amino-4-hydroxyenanthic acid). Specifically, by using an α-keto acid in which R₁₀₁ is an n-propyl group and R₁₀₂ is hydrogen in General Formula (2), it is possible to produce a 2-amino-4-hydroxycarboxylic acid in which R₁₀₁ is an n-propyl group and R₁₀₂ is hydrogen in General Formula (6) (that is, 2-amino-4-hydroxy-n-heptanoic acid). Furthermore, specifically, by using an α-keto acid in which R₁₀₁ is an isopropyl group and R₁₀₂ is hydrogen in General Formula (2), it is possible to produce a 2-amino-4-hydroxycarboxylic acid in which R₁₀₁ is an isopropyl group and R₁₀₂ is hydrogen in General Formula (6) (that is, 2-amino-4-hydroxyisoenanthic acid).

By using an α-keto acid in which R₁₀₁ is a pentyl group and R₁₀₂ is hydrogen in General Formula (2), it is possible to produce a 2-amino-4-hydroxycarboxylic acid in which R₁₀₁ is an isopentyl group and R₁₀₂ is hydrogen in General Formula (6) (that is, 2-amino-4-hydroxycaprylic acid). Specifically, by using an α-keto acid in which R₁₀₁ is an n-pentyl group and R₁₀₂ is hydrogen in General Formula (2), it is possible to produce a 2-amino-4-hydroxycarboxylic acid in which R₁₀₁ is an n-pentyl group and R₁₀₂ is hydrogen in General Formula (6) (that is, 2-amino-4-hydroxy-n-caprylic acid).

By using an α-keto acid in which both R₁₀₁ and R₁₀₂ are methyl groups in General Formula (2), it is possible to produce a 2-amino-4-hydroxycarboxylic acid in which both R₁₀₁ and R₁₀₂ are methyl groups in General Formula (6) (that is, 4-methyl-2-amino-4-hydroxyvaleric acid).

The 2-amino-4-hydroxycarboxylic acids can be recovered by recovering a reaction solution, but the 2-amino-4-hydroxycarboxylic acids can also be separated from the reaction solution and purified by known methods. Examples of such known methods include a crystallization method, a chromatography method, an ion exchange resin method, an adsorption and elution method using activated carbon, a solvent extraction method, and the like.

In addition, when a plurality of types of α-keto acids is used, a plurality of types of 2-amino-4-hydroxycarboxylic acids is produced in a reaction solution. A plurality of types of 2-amino-4-hydroxycarboxylic acids can be separated from each other by a crystallization method or chromatography method.

### (5.4) Method for producing α-keto acid utilizing oxidative deamination reaction of glutamate/phenylalanine/leucine/valine dehydrogenase

The above-described method for producing a 2-amino-4-hydroxycarboxylic acid utilizes a reductive amination reaction of glutamate/phenylalanine/leucine/valine dehydrogenase.

In general, amino acid dehydrogenases such as glutamate/phenylalanine/leucine/valine dehydrogenase catalyze both of a reductive amination reaction and a reverse reaction thereof, that is, an oxidative deamination reaction, and there is a correlation between activity intensities of these dehydrogenases (for example, Biochim. Biophys. Acta 96, 248-262 (1965), J. Biol. Chem. 253, 5719-5725 (1978), Eur. J. Biochem. 100, 29-39 (1979)). Accordingly, it is possible to produce α-keto acid from 2-amino-4-hydroxycarboxylic acid by utilizing oxidative deamination of glutamate/phenylalanine/leucine/valine dehydrogenase.

Therefore, according to another embodiment, a method for producing an α-keto acid utilizing an oxidative deamination reaction of glutamate/phenylalanine/leucine/valine dehydrogenase is provided. That is, the method for producing an α-keto acid according to the present embodiment includes the following step.

A step of reacting a 2-amino-4-hydroxycarboxylic acid selected from a 2-amino-4-hydroxycarboxylic acid represented by General Formula (6), and an oxidizing agent in the presence of glutamate/phenylalanine/leucine/valine dehydrogenase to produce an α-keto acid represented by General Formula (2).

General Formula (6): R₁₀₁C(R₁₀₂)(OH)CH₂CH(NH₂)COOH,

where R₁₀₁ and R₁₀₂ are each independently hydrogen or an alkyl group having 1 to 5 carbon atoms,

General Formula (2): R₁₀₁C(R₁₀₂)(OH)CH₂COCOOH,

where R₁₀₁ and R₁₀₂ in General Formula (2) are respectively the same groups as R₁₀₁ and R₁₀₂ in General Formula (6).

Reaction of 2-amino-4-hydroxycarboxylic acid in the presence of glutamate/phenylalanine/leucine/valine dehydrogenase can be performed in, for example, a reaction solution containing 2-amino-4-hydroxycarboxylic acid, an oxidizing agent, glutamate/phenylalanine/leucine/valine dehydrogenase, and a buffer.

As the 2-amino-4-hydroxycarboxylic acid represented by General Formula (6), it is possible to use the 2-amino-4-hydroxycarboxylic acid represented by General Formula (6) and described in the section "(5.3) Recovery of 2-amino-4-hydroxycarboxylic acid."

As the 2-amino-4-hydroxycarboxylic acid, one kind may be used, or two or more kinds may be mixed and used.

When one kind of 2-amino-4-hydroxycarboxylic acid is used in a reaction solution, it is possible to easily perform separation and purification of α-keto acid, which will be described later.

As the glutamate/phenylalanine/leucine/valine dehydrogenase, it is possible to use the glutamate/phenylalanine/leucine/valine dehydrogenase described in the section "(5.1.4) Glutamate/phenylalanine/leucine/valine dehydrogenase."

As the oxidizing agent, it is possible to use any electron acceptor that can utilize glutamate/phenylalanine/leucine/valine dehydrogenase. For example, NAD⁺ and NADP⁺ can be used.

A concentration of the oxidizing agent in the reaction solution is preferably within a range of 0.01 mM to 100 M, and is more preferably within a range of 0.1 mM to 10 mM. Furthermore, the oxidizing agent, which decreases as production of α-keto acid proceeds, can be regenerated using appropriate compounds and enzymes. Suitable compounds and enzymes are, for example, pyruvic acid and lactate dehydrogenase.

As a buffer, it is possible to use the buffer described in the section "(5.1.5) Buffer."

A reaction temperature may be any temperature as long as it is a temperature suitable for production of α-keto acid. A reaction temperature is preferably about 4°C to 80°C and is more preferably about 20°C to 60°C. It is possible to efficiently produce an α-keto acid when a temperature is within the above-mentioned temperature range. A pH of the reaction solution may be any pH as long as it is suitable for production of α-keto acid. A pH of the reaction solution is preferably maintained within a range of about 5 to 11. A pH of the reaction solution can be adjusted using hydrochloric acid, an aqueous solution of potassium hydroxide, or the like. A pH of the reaction solution can be appropriately adjusted as necessary even during a reaction. A reaction time may be any time as long as it is a sufficient time for production of α-keto acid. A reaction time is preferably about 1 minute to 10 days, and is more preferably about 1 hour to 3 days.

As described above, α-keto acid is produced by causing a reaction of 2-amino-4-hydroxycarboxylic acid and an oxidizing agent in the presence of glutamate/phenylalanine/leucine/valine dehydrogenase.

Specifically, when a 2-amino-4-hydroxycarboxylic acid represented by General Formula (6) is used, it is possible to produce an α-keto acid represented by General Formula (2).

General Formula (6): R₁₀₁C(R₁₀₂)(OH)CH₂CH(NH₂)COOH,

where R₁₀₁ and R₁₀₂ are each independently hydrogen or an alkyl group having 1 to 5 carbon atoms,

General Formula (2): R₁₀₁C(R₁₀₂)(OH)CH₂COCOOH,

where R₁₀₁ and R₁₀₂ in General Formula (2) are respectively the same groups as R₁₀₁ and R₁₀₂ in General Formula (6).

More specifically, by using a 2-amino-4-hydroxycarboxylic acid in which R₁₀₁ is hydrogen and R₁₀₂ is hydrogen in General Formula (6), it is possible to produce an α-keto acid in which R₁₀₁ is hydrogen and R₁₀₂ is hydrogen in General Formula (2) (that is, 4-hydroxy-2-oxobutyric acid).

By using a 2-amino-4-hydroxycarboxylic acid in which R₁₀₁ is a methyl group and R₁₀₂ is hydrogen in General Formula (6), it is possible to produce an α-keto acid in which R₁₀₁ is a methyl group and R₁₀₂ is hydrogen in General Formula (2) (that is, 4-hydroxy-2-oxovaleric acid).

By using a 2-amino-4-hydroxycarboxylic acid in which R₁₀₁ is an ethyl group and R₁₀₂ is hydrogen in General Formula (6), it is possible to produce an α-keto acid in which R₁₀₁ is an ethyl group and R₁₀₂ is hydrogen in General Formula (2) (that is, 4-hydroxy-2-oxocaproic acid).

By using a 2-amino-4-hydroxycarboxylic acid in which R₁₀₁ is a propyl group and R₁₀₂ is hydrogen in General Formula (6), it is possible to produce an α-keto acid in which R₁₀₁ is a propyl group and R₁₀₂ is hydrogen in General Formula (2) (that is, 4-hydroxy-2-oxoenanthic acid). Specifically, by using a 2-amino-4-hydroxycarboxylic acid in which R₁₀₁ is an n-propyl group and R₁₀₂ is hydrogen in General Formula (6), it is possible to produce α-keto acid in which R₁₀₁ is an n-propyl group and R₁₀₂ is hydrogen in General Formula (2) (that is, 4-hydroxy-2-oxo-n-heptanoic acid). Furthermore, specifically, by using a 2-amino-4-hydroxycarboxylic acid in which R₁₀₁ is an isopropyl group and R₁₀₂ is hydrogen in General Formula (6), it is possible to produce an α-keto acid in which R₁₀₁ is an isopropyl group and R₁₀₂ is hydrogen in General Formula (2) (that is, 4-hydroxy-2-oxoisoenanthic acid).

By using a 2-amino-4-hydroxycarboxylic acid in which R₁₀₁ is a pentyl group and R₁₀₂ is hydrogen in General Formula (6), it is possible to produce an α-keto acid in which R₁₀₁ is a pentyl group and R₁₀₂ is hydrogen in General Formula (2) (that is, 4-hydroxy-2-oxocaprylic acid). Specifically, by using a 2-amino-4-hydroxycarboxylic acid in which R₁₀₁ is an n-pentyl group and R₁₀₂ is hydrogen in General Formula (6), it is possible to produce an α-keto acid in which R₁₀₁ is an n-pentyl group and R₁₀₂ is hydrogen in General Formula (2) (that is, 4-hydroxy-2-oxo-n-caprylic acid).

By using a 2-amino-4-hydroxycarboxylic acid in which both R₁₀₁ and R₁₀₂ are methyl groups in General Formula (6), it is possible to produce an α-keto acid in which both R₁₀₁ and R₁₀₂ are methyl groups in General Formula (2) (that is, 4-methyl-4-hydroxy-2-oxovaleric acid).

The above-described α-keto acids can be recovered by recovering a reaction solution, but the α-keto acids can also be separated from the reaction solution and purified by known methods. Examples of such known methods include a crystallization method, a chromatography method, an ion exchange resin method, an adsorption and elution method using activated carbon, a solvent extraction method, and the like.

In addition, when a plurality of types of 2-amino-4-hydroxycarboxylic acids is used, a plurality of types of α-keto acids is produced in a reaction solution. A plurality of types of α-keto acids can be separated from each other by crystallization or chromatography.

### (6.) Method for producing 2-amino-4-methylthiocarboxylic acid

A method for producing a 2-amino-4-methylthiocarboxylic acid includes a step of culturing a microorganism containing the following genes in the presence of a pyruvic acid-supplying compound selected from pyruvic acid and saccharides, methyl mercaptan, and a carbonyl compound represented by General Formula (1) to produce a 2-amino-4-methylthiocarboxylic acid represented by General Formula (7).
(a) First gene encoding an enzyme that catalyzes an aldol reaction between pyruvic acid and a carbonyl compound,
(e) Fifth gene encoding an enzyme that catalyzes a reductive amination reaction of an α-keto acid, and
(f) Sixth gene encoding an enzyme that catalyzes a reaction of substituting a phosphoric acid group contained in 2-amino-4-phosphonooxycarboxylic acid with a methylmercapto group contained in methyl mercaptan

   General Formula (1): R₁₀₁C(O)R₁₀₂,

   where R₁₀₁ and R₁₀₂ are each independently hydrogen or an alkyl group having 1 to 5 carbon atoms,

   General Formula (7): R₁₀₁C(R₁₀₂)(SCH₃)CH₂CH(NH₂)COOH,

   where R₁₀₁ and R₁₀₂ in General Formula (7) are respectively the same groups as R₁₀₁ and R₁₀₂ in General Formula (1).

In the following description, the above-mentioned microorganism containing the first, fifth, and sixth genes is also referred to as a "2-amino-4-methylthiocarboxylic acid-producing microorganism." The 2-amino-4-methylthiocarboxylic acid-producing microorganism may be a microorganism that naturally has the first, fifth, and sixth genes, or may be a microorganism obtained by transforming a host with the first, fifth, and sixth genes. Hereinafter, a case in which the 2-amino-4-methylthiocarboxylic acid-producing microorganism is a transformant will be described as an example.

### (6.1) Transformant

### (6.1.1) Host

As a host, it is possible to use a host similar to the host described in the section "(1.1.1) Host."

Next, the first, fifth and sixth genes introduced into the host will be described.

### (6.1.2) First gene

As the first gene, it is possible to use a gene similar to the first gene described in the section "(1.1.2) First gene."

### (6.1.3) Fifth gene

As the fifth gene, it is possible to use a gene similar to the first gene described in the section "(4.1.3) Fifth gene."

### (6.1.4) Sixth gene

The sixth gene encodes an "enzyme that catalyzes a reaction of substituting a phosphoric acid group contained in 2-amino-4-phosphonooxycarboxylic acid with a methylmercapto group contained in methyl mercaptan." The "enzyme that catalyzes a reaction of substituting a phosphoric acid group contained in 2-amino-4-phosphonooxycarboxylic acid with a methylmercapto group contained in methyl mercaptan" refers to an enzyme having an activity of 0.1 mU/mg or more of protein when measuring the activity according to a measurement method to be described later using an enzymatic reaction solution containing purified protein, 2-amino-4-phosphonooxycarboxylic acid (initial concentration of substrate: 1 mM), and methyl mercaptan (initial concentration of substrate: 5 mM) as an enzymatic reaction solution. The sixth gene encodes, for example, an enzyme that catalyzes a reaction of substituting a phosphoric acid group contained in 2-amino-4-phosphonooxycarboxylic acid, which is obtained via a reductive amination reaction catalyzed by a protein encoded by the fifth gene, with a methylmercapto group contained in methyl mercaptan. The "enzyme that catalyzes a reaction of substituting a phosphoric acid group contained in 2-amino-4-phosphonooxycarboxylic acid with a methylmercapto group contained in methyl mercaptan, is, for example, a transsulfation enzyme.

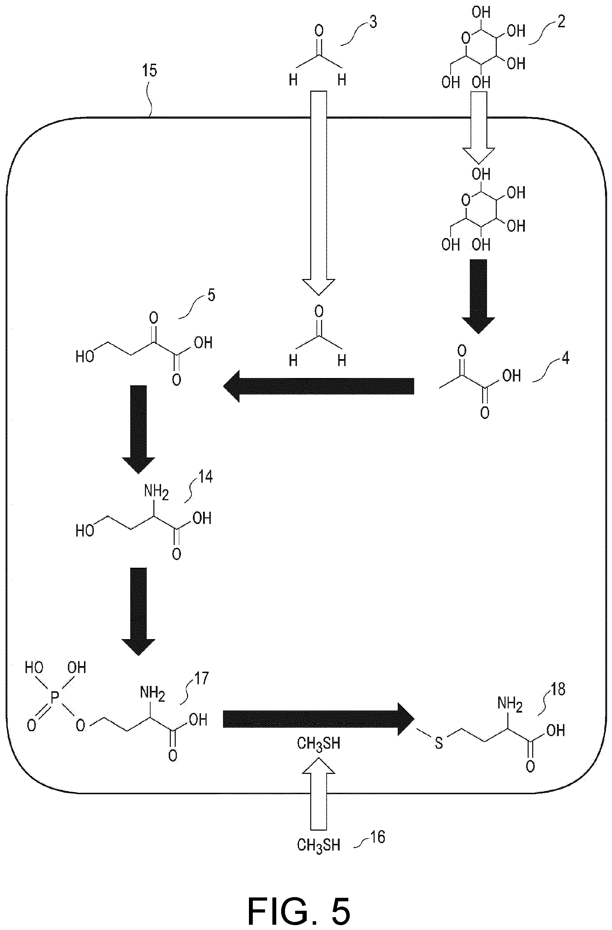

A reaction further occurs between the reaction catalyzed by the protein encoded by the fifth gene and the reaction catalyzed by the protein encoded by the sixth gene (refer to FIG. 5). Specifically, there is a reaction in which 2-amino-4-hydroxycarboxylic acid obtained by the reaction catalyzed by the protein encoded by the fifth gene is converted into 2-amino-4-phosphonooxycarboxylic acid by an enzyme (for example, homoserine kinase) which phosphorylates 2-amino-4-hydroxycarboxylic acid and is included in the 2-amino-4-methylthiocarboxylic acid-producing microorganism. 2-Amino-4-phosphonooxycarboxylic acid is used as a substrate for the reaction catalyzed by the protein encoded by the sixth gene.

The sixth gene may be, for example, a gene encoding cystathionine γ-synthase (EC number: 2.5.1.48).

Examples of cystathionine γ-synthases include CGS1 protein. The CGS1 protein is preferably derived from *Arabidopsis thaliana.* The CGS1 protein is more preferably a CGS1 mature protein derived from *Arabidopsis thaliana.* The CGS 1 mature protein derived from *Arabidopsis thaliana* is a protein in which 2nd to 68th amino acid residues from an N-terminal are deleted from a wild-type CGS1 protein derived from *Arabidopsis thaliana.*

The sixth gene is preferably selected from the group consisting of the following genes.
(6-1) Gene encoding a protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan (that is, a CGS1 mature protein derived from *Arabidopsis thaliana*), and
(6-2) Gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan.

The "gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan" may be a "gene encoding a protein that consists of an amino acid sequence homologous to the amino acid sequence set forth in SEQ ID NO: 36 by 80% or more, preferably by 90% or more, more preferably by 95% or more, even more preferably by 97% or more, still more preferably by 98% or more, and further more preferably by 99% or more, and has an activity of catalyzing a reaction of substituting a phosphoric acid group contained in 2-amino-4-phosphonooxycarboxylic acid with a methylmercapto group contained in methyl mercaptan."

A UniProtKB accession number of the CGS1 protein derived from *Arabidopsis thaliana* is P55217.

The sixth gene is more preferably a gene in which a site that becomes a mature protein in a CGS 1 gene, which is derived from *Arabidopsis thaliana* and consists of a base sequence set forth in SEQ ID NO: 35, is synthesized so that the protein is optimally expressed in coryneform bacteria.

A base sequence set forth in SEQ ID NO: 35 and an amino acid sequence set forth in SEQ ID NO: 36 are as follows.

**[Table 35]**

| |
|---|
| (SEQ ID NO: 35) At_CGS1 gene |
| atgGTGCGCCAGCTGTCCATCAAGGCACGCCGTAACTGCTCCAACATCGGCGTGGCACAG ATCGTGGCAGCAAAGTGGTCCAACAACCCATCCTCCGCACTGCCATCCGCAGCAGCAGCA GCCGCAACCTCCTCCGCATCCGCAGTGTCCTCCGCAGCATCCGCAGCTGCCGCATCCTCC GCTGCAGCAGCACCAGTGGCAGCAGCCCCACCAGTGGTGCTGAAGTCCGTGGATGAAGAG GTGGTGGTGGCAGAAGAAGGCATCCGCGAAAAGATCGGCTCCGTGCAGCTGACCGATTCC AAGCACTCCTTCTTGTCCTCCGATGGCTCCCTGACCGTGCACGCAGGCGAACGCTTGGGT CGCGGTATCGTGACCGATGCAATCACCACCCCAGTGGTGAACACCTCCGCATACTTTTTC AAGAAAACCGCAGAACTGATCGACTTCAAAGAAAAGCGCTCCGTGTCCTTCGAATACGGT CGCTACGGCAACCCAACCACCGTGGTGCTGGAAGATAAGATCTCCGCACTGGAAGGCGCA |
| GAATCCACCCTGGTGATGGCATCCGGCATGTGCGCATCCACCGTGATGCTGCTGGCCCTG GTGCCAGCAGGCGGTCACATCGTGACCACCACCGATTGCTACCGCAAGACCCGTATCTTC ATGGAAAACTTTCTGCCAAAGCTGGGCATCACCGTGACCGTGATCGATCCAGCAGATATC GCAGGCCTGGAAGCAGCAGTGAACGAATTCAAGGTGTCCCTGTTCTTCACCGAATCCCCA ACCAACCCATTCCTGCGCTGCGTGGATATCGAACTGGTGTCCAAGATCTGCCACAAGCGT GGCACCCTGGTGTGCATCGATGGCACCTTCGCAACCCCACTGAACCAGAAGGCACTGGCA CTTGGCGCAGATCTGGTGGTGCACTCCGCAACCAAGTACATCGGTGGCCACAACGATGTG CTGGCAGGCTGCATCTGCGGCTCCCTGAAGCTGGTGTCCGAAATCCGCAACCTGCACCAC GTGCTGGGTGGCACCCTGAACCCAAACGCAGCATACCTGATCATCCGTGGCATGAAAACC CTGCACTTGCGCGTGCAGCAGCAGAACTCCACCGCATTCCGCATGGCAGAAATCCTGGAA GCACACCCAAAGGTGTCCCACGTGTACTACCCAGGCCTGCCATCCCACCCAGAACACGAA CTGGCAAAGCGCCAGATGACCGGCTTCGGTGGCGTGGTGTCCTTCGAGATCGATGGCGAT ATCGAAACCACCATCAAGTTCGTGGATTCCCTGAAGATCCCATACATTGCACCATCCTTC GGTGGCTGCGAATCCATCGTGGATCAGCCAGCAATCATGTCCTACTGGGATCTCCCACAA GAAGAACGCCTGAAGTACGGCATCAAGGATAACCTGGTGCGCTTCTCCTTCGGCGTGGAA GATTTCGAAGATGTGAAGGCAGATATCCTGCAGGCACTCGAAGCAATCtaa |

**[Table 36]**

| |
|---|
| (SEQ ID NO: 36) At_CGS1 protein |
| MVRQLSIKARRNCSNIGVAQIVAAKWSNNPSSALPSAAAAAATSSASAVSSAASAAAASS AAAAPVAAAPPVVLKSVDEEVVVAEEGIREKIGSVQLTDSKHSFLSSDGSLTVHAGERLG RGIVTDAITTPVVNTSAYFFKKTAELIDFKEKRSVSFEYGRYGNPTTVVLEDKISALEGA ESTLVMASGMCASTVMLLALVPAGGHIVTTTDCYRKTRIFMENFLPKLGITVTVIDPADI AGLEAAVNEFKVSLFFTESPTNPFLRCVDIELVSKICHKRGTLVCIDGTFATPLNQKALA LGADLVVHSATKYIGGHNDVLAGCICGSLKLVSEIRNLHHVLGGTLNPNAAYLIIRGMKT LHLRVQQQNSTAFRMAEILEAHPKVSHVYYPGLPSHPEHELAKRQMTGFGGVVSFEIDGD IETTIKFVDSLKIPYIAPSFGGCESIVDQPAIMSYWDLPQEERLKYGIKDNLVRFSFGVE DFEDVKADILQALEAI |

The activity of catalyzing a reaction of substituting a phosphoric acid group contained in 2-amino-4-phosphonooxycarboxylic acid with a methylmercapto group contained in methyl mercaptan can be calculated by, for example, causing the enzymatic reaction at 25°C in a 100 mM HEPES-KOH buffer (pH 7.5) containing 1 mM dithiothreitol, and measuring an initial generation rate of inorganic phosphate ions liberated as the reaction proceeds. A trace amount of pyridoxal phosphate may be added as necessary. The generation rate of the inorganic phosphate ions can be calculated from, for example, a time change of a concentration of the inorganic phosphate ions. The concentration of the inorganic phosphate ions can be measured by, for example, mixing an enzymatic reaction solution and 20% (w/v) trichloroacetic acid at a ratio of 1: 2 to stop the reaction, and then performing a malachite green method. In this case, an activity of forming 1 µmol of a sulfide compound at 25°C for 1 minute is regarded as one unit.

The "gene (CGS1 gene mutant) encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan" can be selected from a DNA library of animals of another species by the same method as the above-described method of selecting an hpaI gene mutant.

### (6.1.5) Construction of recombinant vector for transformation

The first, fifth and sixth gene can be incorporated into an appropriate plasmid vector for transformation.

As the plasmid vector, it is possible to use a plasmid vector similar to the plasmid vector described in the section "(1.1.3) Construction of recombinant vector for transformation."

The first, fifth and sixth genes may be respectively inserted into individual plasmid vectors or may be inserted into the same plasmid vector. A transformant can be produced using the obtained recombinant vector.

### (6.1.6) Transformation

As a transformation method, it is possible to use a known method without limitation. As such a known method, it is possible to use a method similar to the method described in the section "(1.1.4) Transformation."

In a case where a transformant is produced using a recombinant vector as described above, the first, fifth and sixth genes may be respectively inserted into chromosomes of a host, or may be present in cytoplasm of the host in the form of recombinant vectors.

### (6.1.7) Disruption or deletion of chromosomal gene of host

The host may be a wild strain, a mutant strain thereof, or an artificial gene recombinant. In a case where the host is a coryneform bacterium, for the host, it is possible to use a gene-disrupted strain or gene-deleted strain similar to the gene-disrupted strain or gene-deleted strain described in the section "(1.1.5) Disruption or deletion of chromosomal gene of host." Furthermore, as a method for producing a gene-disrupted strain or gene-deleted strain, it is possible to use a method similar to the method described in the section "(1.1.5) Disruption or deletion of chromosomal gene of host." By using such a gene-disrupted strain or gene-deleted strain as a host, it is possible to improve productivity for 2-amino-4-methylthiocarboxylic acid or to inhibit production of by-products.

### (6.1.8) Specific examples of 2-amino-4-methylthiocarboxylic acid-produding microorganisms

Specifically, as a 2-amino-4-methylthiocarboxylic acid producing microorganism, a coryneform bacterium can be used which comprises:
the first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
the fifth gene selected from the group consisting of:
   (5-1) a gene encoding a protein that consists of an amino acid sequence set forth in SEQ ID NO: 30 and catalyzes a reductive amination reaction of α-keto acids;
   (5-2) a gene encoding a protein that consists of an amino acid sequence in which one or a plurality of amino acids is deleted, substituted, or added in SEQ ID NO: 30, and has an activity of catalyzing a reductive amination reaction of α-keto acids;
   (5-3) a gene encoding a protein that consists of an amino acid sequence set forth in SEQ ID NO: 32 and catalyzes a reductive amination reaction of α-keto acids;
   (5-4) a gene encoding a protein that consists of an amino acid sequence in which one or a plurality of amino acids is deleted, substituted, or added in SEQ ID NO: 32, and has an activity of catalyzing a reductive amination reaction of α-keto acids;
   (5-5) a gene encoding a protein that consists of an amino acid sequence set forth in SEQ ID NO: 34 and catalyzes a reductive amination reaction of α-keto acids; and
   (5-6) a gene encoding a protein that consists of an amino acid sequence in which one or a plurality of amino acids is deleted, substituted, or added in SEQ ID NO: 34, and has an activity of catalyzing a reductive amination reaction of α-keto acids; and
the sixth gene selected from the group consisting of:
   (6-1) Gene encoding a protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan, and
   (6-2) Gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan.

### (6.2) Culture step

A 2-amino-4-methylthiocarboxylic acid can be produced by culturing a 2-amino-4-methylthiocarboxylic acid-producing microorganism (for example, the above-described transformant) in the presence of a pyruvic acid-supplying compound, a carbonyl compound and methyl mercaptan. The "culturing in the presence of a pyruvic acid-supplying compound, a carbonyl compound and methyl mercaptan" can be performed by culture in a culture solution containing the pyruvic acid-supplying compound, a carbonyl compound and methyl mercaptan. The "culturing in the presence of a pyruvic acid-supplying compound, a carbonyl compound and methyl mercaptan" can be preferably performed by culture in a culture solution into which the pyruvic acid-supplying compound, a carbonyl compound and methyl mercaptan are added.

Prior to the culture in the presence of a pyruvic acid-supplying compound, a carbonyl compound and methyl mercaptan, it is preferable to perform the proliferation culture described in the section "(1.2) Culture step."

When the proliferation culture is performed prior to the production culture, the proliferation culture and subsequent production culture can be performed as follows, for example. After the proliferation culture, first, a container containing a culture solution used in the proliferation culture (hereinafter referred to as the culture solution for proliferation) and a transformant suspended in the culture solution for proliferation is centrifuged in a centrifugal separator, and thereby the transformant can be precipitated. Thereafter, the culture solution for proliferation is removed from the container, the separated transformant is suspended in a culture solution used in the production culture (hereinafter referred to as the culture solution for production), and production culture can be performed.

Alternatively, regarding the proliferation culture and subsequent production culture, after the proliferation culture, a pyruvic acid-supplying compound, a carbonyl compound and methyl mercaptan are added to the culture solution for proliferation containing the transformant, aerobic conditions are changed to anaerobic conditions or microaerobic conditions, and thereby production culture can be performed without exchanging the culture solution. As described above, the proliferation culture and the production culture can be performed in succession.

### (6.2.1) Culture solution for proliferation

As a culture solution for proliferation, it is possible to use a culture solution similar to the culture solution for proliferation described in the section "(1.2.1) Culture solution for proliferation."

### (6.2.2) Culture solution for production

As a culture solution for production, it is possible to use a culture solution containing a pyruvic acid-supplying compound, a carbonyl compound, methyl mercaptan and other necessary components. As the other necessary components, it is possible to use, for example, inorganic salts, carbon sources other than saccharides, or nitrogen sources.

As the pyruvic acid-supplying compound, it is possible to use the pyruvic acid-supplying compound described in the section "(1.2.2) Culture solution for production."

As the carbonyl compounds, it is possible to use the carbonyl compounds described in the section "(1.2.2) Culture solution for production."

When one kind of carbonyl compound is used in the culture solution for production, it is possible to easily perform separation and purification to be described later in the section "(6.2.4) Recovery of 2-amino-4-methylthiocarboxylic acid."

The concentration of methyl mercaptan in the culture solution for production is preferably in a range between 1 mM and 1 M, more preferably in a range between 10 mM and 300 mM. Furthermore, methyl mercaptan can be further added depending on the decrease in methyl mercaptan associated with the production of 2-amino-4-methylthiocarboxylic acid.

As other necessary components, it is possible to use components similar to the other necessary components described in the section "(1.2.2) Culture solution for production."

As a specific culture solution for production, it is possible to use a medium containing glucose, formaldehyde, methyl mercaptan, ammonium sulfate, potassium dihydrogen phosphate, dipotassium hydrogen phosphate, magnesium sulfate, iron(II) sulfate, and manganese(II) sulfate, and having a pH of 7.0.

Furthermore, as the culture solution for production, it is possible to use a culture solution obtained by adding a pyruvic acid-supplying compound, a carbonyl compound and methyl mercaptan to a base culture solution having the same composition as a culture solution for proliferation used in the proliferation culture. When a sufficient amount of the pyruvic acid-supplying compound is present in the above-mentioned base culture solution, it is not necessary to add the pyruvic acid-supplying compound.

### (6.2.3) Conditions for production culture

A culture temperature in the production culture may be any temperature as long as it is a temperature suitable for production of 2-amino-4-methylthiocarboxylic acid. A culture temperature in the production culture is preferably about 20°C to 50°C, and is more preferably about 25°C to 47°C. It is possible to efficiently produce a 2-amino-4-methylthiocarboxylic acid when a temperature is within the above-mentioned temperature range. A pH of the culture solution for production may be any pH as long as it is suitable for production of 2-amino-4-methylthiocarboxylic acid. A pH of the culture solution for production is preferably maintained within a range of about 6 to 8. A pH of the culture solution for production can be adjusted using an inorganic or organic acid; an alkaline solution such as an aqueous solution of potassium hydroxide; urea; calcium carbonate; ammonia; a pH buffer solution containing 4-morpholinopropanesulfonic acid; or the like. A pH of the culture solution for production can be appropriately adjusted as necessary even during the production culture. A culture time in the production culture may be any time as long as it is a time sufficient for a transformant to produce a 2-amino-4-methylthiocarboxylic acid. A culture time in the production culture is preferably about 3 hours to 7 days, and is more preferably about 1 to 3 days.

The production culture may be performed under aerobic conditions, or may be performed under anaerobic conditions or microaerobic conditions. This is because the above-described transformant has a pathway that is for producing a 2-amino-4-methylthiocarboxylic acid and works under both aerobic conditions and anaerobic conditions or microaerobic conditions. The pathway for producing a 2-amino-4-methylthiocarboxylic acid will be described in the section "(6.3) Actions and effects" to be described later.

### <Anaerobic conditions or microaerobic conditions>

The production culture is preferably performed under anaerobic conditions or microaerobic conditions.

When a transformant is cultured under anaerobic conditions or microaerobic conditions, the transformant does not proliferate substantially, and thereby 2-amino-4-methylthiocarboxylic acid can be produced more efficiently.

The "anaerobic conditions or microaerobic conditions" can be set to the same conditions as the "anaerobic conditions or microaerobic conditions" described in the section "(1.2.3) Conditions for production culture."

### <High density conditions>

The production culture is preferably performed in a state in which a transformant is suspended at a high density in a culture solution for production. When the transformant is cultured in such a state, 2-amino-4-methylthiocarboxylic acid can be produced more efficiently.

The "state in which a transformant is suspended at a high density in a culture solution for production" can be set to the same state as the "state in which a transformant is suspended at a high density in a culture solution for production" described in the section "(1.2.3) Conditions for production culture."

The production culture is more preferably performed under anaerobic conditions or microaerobic conditions, and in a state in which a transformant is suspended at a high density in a culture solution for production.

### (6.2.4) Recovery of 2-amino-4-methylthiocarboxylic acid

As described above, when the transformant containing the first, fifth, and sixth genes is cultured in a culture solution for production, 2-amino-4-methylthiocarboxylic acid is produced in the culture solution for production.

Specifically, when a carbonyl compound represented by General Formula (1) is used in a culture solution for production, it is possible to produce 2-amino-4-methylthiocarboxylic acid represented by General Formula (7).

General Formula (1): R₁₀₁C(O)R₁₀₂,

where R₁₀₁ and R₁₀₂ are each independently hydrogen or an alkyl group having 1 to 5 carbon atoms,

General Formula (7): R₁₀₁C(R₁₀₂)(SCH₃)CH₂CH(NH₂)COOH,

where R₁₀₁ and R₁₀₂ in General Formula (7) are respectively the same groups as R₁₀₁ and R₁₀₂ in General Formula (1).

More specifically, by using a carbonyl compound in which R₁₀₁ is hydrogen and R₁₀₂ is hydrogen in General Formula (1), it is possible to produce a 2-amino-4-methylthiocarboxylic acid in which R₁₀₁ is hydrogen and R₁₀₂ is hydrogen in General Formula (7) (that is, 2-amino-4-methylthiobutyric acid).

By using a carbonyl compound in which R₁₀₁ is a methyl group and R₁₀₂ is hydrogen in General Formula (1), it is possible to produce a 2-amino-4-methylthiocarboxylic acid in which R₁₀₁ is a methyl group and R₁₀₂ is hydrogen in General Formula (7) (that is, 2-amino-4-methylthiovaleric acid).

By using a carbonyl compound in which R₁₀₁ is an ethyl group and R₁₀₂ is hydrogen in General Formula (1), it is possible to produce a 2-amino-4-methylthiocarboxylic acid in which R₁₀₁ is an ethyl group and R₁₀₂ is hydrogen in General Formula (7) (that is, 2-amino-4-methylthiocaproic acid).

By using a carbonyl compound in which R₁₀₁ is a propyl group and R₁₀₂ is hydrogen in General Formula (1), it is possible to produce a 2-amino-4-methylthiocarboxylic acid in which R₁₀₁ is a propyl group and R₁₀₂ is hydrogen in General Formula (7) (that is, 2-amino-4-methylthioenanthic acid). Specifically, by using a carbonyl compound in which R₁₀₁ is an n-propyl group and R₁₀₂ is hydrogen in General Formula (1), it is possible to produce a 2-amino-4-methylthiocarboxylic acid in which R₁₀₁ is an n-propyl group and R₁₀₂ is hydrogen in General Formula (7) (that is, 2-amino-4-methylthio-n-heptanoic acid). Furthermore, specifically, by using a carbonyl compound in which R₁₀₁ is an isopropyl group and R₁₀₂ is hydrogen in General Formula (1), it is possible to produce a 2-amino-4-methylthiocarboxylic acid in which R₁₀₁ is an isopropyl group and R₁₀₂ is hydrogen in General Formula (7) (that is, 2-amino-4-methylthioisoenanthic acid).

By using a carbonyl compound in which R₁₀₁ is a pentyl group and R₁₀₂ is hydrogen in General Formula (1), it is possible to produce a 2-amino-4-methylthiocarboxylic acid in which R₁₀₁ is a pentyl group and R₁₀₂ is hydrogen in General Formula (7) (that is, 2-amino-4-methylthiocaprylic acid). Specifically, by using a carbonyl compound in which R₁₀₁ is an n-pentyl group and R₁₀₂ is hydrogen in General Formula (1), it is possible to produce a 2-amino-4-methylthiocarboxylic acid in which R₁₀₁ is an n-pentyl group and R₁₀₂ is hydrogen in General Formula (7) (that is, 2-amino-4-methylthio-n-caprylic acid).

By using a carbonyl compound in which both R₁₀₁ and R₁₀₂ are methyl groups in General Formula (1), it is possible to produce a 2-amino-4-methylthiocarboxylic acid in which both R₁₀₁ and R₁₀₂ are methyl groups in General Formula (7) (that is, 4-methyl-2-amino-4-methylthiovaleric acid).

2-Amino-4-methylthiobutyric acid is also called methionine. Methionine is, for example, L-methionine.

The above-described 2-amino-4-methylthiocarboxylic acids can be recovered by recovering a culture solution for production, but the 2-amino-4-methylthiocarboxylic acids can also be separated from the culture solution for production and purified by known methods. Examples of such known methods include a crystallization method, a chromatography method, an ion exchange resin method, an adsorption and elution method using activated carbon, a solvent extraction method, and the like.

From the culture solution for production, it is possible to recover substances such as α-keto acid produced by an aldol reaction catalyzed by the enzyme encoded by the first gene.

In addition, when a plurality of types of carbonyl compounds is used, a plurality of types of 2-amino-4-methylthiocarboxylic acids is produced in a culture solution. A plurality of types of 2-amino-4-methylthiocarboxylic acids can be separated from each other by crystallization or chromatography.

### (6.3) Actions and effects

FIG. 5 schematically shows a process in which a 2-amino-4-methylthiocarboxylic acid-producing microorganism produces a 2-amino-4-methylthiocarboxylic acid. The process shown in FIG. 5 illustrates a case as an example in which a pyruvic acid-supplying compound is glucose, a carbonyl compound is formaldehyde, and 2-amino-4-methylthiocarboxylic acid is methionine.

As shown in FIG. 5, first, a 2-amino-4-methylthiocarboxylic acid-producing microorganism 15 introduces a glucose 2, a formaldehyde 3, and methyl mercaptan 16. Next, the glucose 2 is converted into a pyruvic acid 4 through a glycolytic system. Next, the pyruvic acid 4 and the formaldehyde 3 are converted into a 4-hydroxy-2-oxobutyric acid 5 by an "enzyme that catalyzes an aldol reaction between pyruvic acid and aldehyde," which is an enzyme encoded by the first gene. Next, the 4-hydroxy-2-oxobutyric acid 5 is converted into a homoserine 14 by an "enzyme that catalyzes a reductive amination reaction of an α-keto acid," which is an enzyme encoded by the fifth gene. Next, the homoserine 14 is converted into an O-phosphohomoserine 17 by a homoserine kinase naturally contained in the 2-amino-4-methylthiocarboxylic acid-producing microorganism. The methyl mercaptan 16 and the O-phosphohomoserine 17 are converted into a methionine 18 by an "enzyme that converts O-phosphohomoserine into methionine," which is an enzyme encoded by the sixth gene. Accordingly, methionine is produced.

### (7.) Preferable embodiments

Preferable embodiments of the present invention are collectively shown below.
[A1] A method for producing an α-keto acid, including steps of
   culturing a microorganism including a gene encoding an enzyme that catalyzes an aldol reaction between pyruvic acid and a carbonyl compound
   in the presence of:
      a pyruvic acid-supplying compound selected from pyruvic acid and saccharides; and
      a carbonyl compound represented by the following General Formula (1):

         R₁₀₁C(O)R₁₀₂
      (wherein R₁₀₁ and R₁₀₂ are each independently hydrogen or an alkyl group having 1 to 5 carbon atoms), and
      producing an α-keto acid represented by the following General Formula (2):

         R₁₀₁C(R₁₀₂)(OH)CH₂COCOOH
      (wherein each of R₁₀₁ and R₁₀₂ in the general formula (2) above is the same group as R₁₀₁ and R₁₀₂ in the general formula (1) above).
[A2] The method according to [A1], in which R₁₀₂ is hydrogen in General Formulas (1) and (2).
[A3] The method according to [A1] or [A2], in which, in General Formulas (1) and (2), R₁₀₁ is hydrogen, a methyl group, an ethyl group, a propyl group, or a pentyl group, and R₁₀₂ is hydrogen.
[A4] The method according to any one of [A1] to [A3], in which, in General Formulas (1) and (2), R₁₀₁ is hydrogen, a methyl group, an ethyl group, an n-propyl group, an isopropyl group, or an n-pentyl group, and R₁₀₂ is hydrogen.
[A5] The method according to any one of [A1] to [A4], in which the culture is performed by culturing the microorganism in a culture solution containing the pyruvic acid-supplying compound and the carbonyl compound.
[A6] The method according to [A5], in which the culture solution is a culture solution into which the pyruvic acid-supplying compound and the carbonyl compound are added.
[A7] The method according to [A6], in which the pyruvic acid-supplying compound is added such that a concentration thereof in the culture solution is 0.1% to 40% (w/v), and is preferably 1% to 20% (w/v).
[A8] The method according to [A6] or [A7], in which the carbonyl compound is added such that a concentration thereof in the culture solution is 0.001% to 10% (w/v), and is preferably 0.01% to 1% (w/v). [A9] The method according to any one of [A1] to [A8], in which the pyruvic acid-supplying compound is a saccharide.
[A10] The method according to any one of [A1] to [A9], in which the saccharide is a monosaccharide such as fructose or glucose; a disaccharide such as sucrose; a polysaccharide such as a polysaccharide containing glucose as a constituent monosaccharide; or a saccharified solution obtained by treating plants (such as non-edible agricultural waste or energy crops) with saccharification enzymes.
[A11] The method according to any one of [A1] to [A10], in which the saccharide is glucose.
[A12] The method according to any one of [A1] to [A8], in which the pyruvic acid-supplying compound is pyruvic acid.
[A13] The method according to any one of [A1] to [A12], in which the culture is performed under anaerobic conditions or microaerobic conditions.
[A14] The method according to [A13], in which the culture is performed by culturing the microorganism in a culture solution containing the pyruvic acid-supplying compound and the carbonyl compound.
[A15] The method according to [A14], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[A16] The method according to any one of [A1] to [A15], in which the culture is performed in a state where the microorganism is suspended in the culture solution at a high density.
[A17] The method according to [A16], in which the culture is performed by culturing the microorganism in a culture solution containing the pyruvic acid-supplying compound and the carbonyl compound.
[A18] The method according to [A17], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[A19] The method according to any one of [A1] to [A18], in which the gene encodes an aldolase.
[A20] The method according to any one of [A1] to [A19], in which the gene encodes type I aldolase.
[A21] The method according to any one of [A1] to [A19], in which the gene encodes type II aldolase.
[A22] The method according to any one of [A1] to [A21], in which the gene encodes an aldolase selected from the group consisting of:
   (a1) 2-dehydro-3-deoxy-phosphogluconate aldolase;
   (a2) 2-dehydro-3-deoxy-6-phosphogluconate aldolase;
   (a3) 4-hydroxy-2-oxoglutarate aldolase;
   (a4) (4S)-4-hydroxyl-2-oxoglutarate aldolase;
   (a5) 2-dehydro-3-deoxy-D-pentonate aldolase;
   (a6) 2-dehydro-3-deoxy-D-gluconate aldolase;
   (a7) 3-deoxy-D-manno-octulosonate aldolase;
   (a8) 4-hydroxyl-2-oxovalerate aldolase;
   (a9) 4-(2-carboxyphenyl)-2-oxobut-3-enoate aldolase;
   (a10) 4-hydroxy-2-oxoheptanedioate aldolase;
   (a11) 2-dehydro-3-deoxyglucarate aldolase;
   (a12) 2-keto-3-deoxy-L-rhamnonate aldolase;
   (a13) 4-hydroxyl-4-methyl-2-oxoglutarate aldolase; and
   (a14) 4-hydroxy-2-oxohexanoate aldolase.
[A23] The method according to any one of [A1] to [A22], in which the gene encodes an aldolase selected from the group consisting of an eda protein, preferably an eda protein derived from *Escherichia coli;* a dgoA protein, preferably a dgoA protein derived from *Escherichia coli*; a yjhH protein, preferably a yjhH protein derived from *Escherichia coli*; a yagE protein, preferably a yagE protein derived from *Escherichia coli*; a nanA protein, preferably a nanA protein derived from *Escherichia coli*; an mhpE protein, preferably a mhpE protein derived from *Escherichia coli*; a hpaI protein, preferably a hpaI protein derived from *Escherichia coli*; a bphI protein, preferably a bphI protein derived from *Burkholderia xenovorans*; a phdJ protein, preferably a phdJ protein derived from the genus *Nocardioides*; a garL protein, preferably a garL protein derived from *Escherichia coli*; an rhmA protein, preferably an rhmA protein derived from *Escherichia coli*; and a galC protein, preferably, a galC protein derived from *Pseudomonas putida.*
[A24] The method according to any one of [A1] to [A23], in which the gene is selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound;
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[A25] The method according to any one of [A1] to [A24], in which the gene is selected from the group consisting of:
   a gene that consists of a base sequence set forth in SEQ ID NO: 1;
   a gene that consists of a base sequence set forth in SEQ ID NO: 3;
   a gene that consists of a base sequence set forth in SEQ ID NO: 5;
   a gene that consists of a base sequence set forth in SEQ ID NO: 7;
   a gene that consists of a base sequence set forth in SEQ ID NO: 9;
   a gene that consists of a base sequence set forth in SEQ ID NO: 11;
   a gene that consists of a base sequence set forth in SEQ ID NO: 13;
   a gene that consists of a base sequence set forth in SEQ ID NO: 15;
   a gene that consists of a base sequence set forth in SEQ ID NO: 17;
   a gene that consists of a base sequence set forth in SEQ ID NO: 19; and
   a gene that consists of a base sequence set forth in SEQ ID NO: 21.
[A26] The method according to any one of [A1] to [A25], in which the microorganism is an aerobic bacterium, is preferably a coryneform bacterium, is more preferably *Corynebacterium glutamicum.*
[A27] The method according to any one of [A1] to [A26], in which the microorganism is a microorganism transformed with the gene.
[A28] The method according to any one of [A1] to [A27], in which the microorganism is a gene-deleted strain from which at least one gene is deleted, the gene being selected from the group consisting of a lactate dehydrogenase gene, a phosphoenolpyrvate carboxylase gene, a pyruvate carboxylase gene, a pyruvate dehydrogenase gene, a glutamate-pyruvate aminotransferase gene, an acetolactate synthase gene, an N-succinyldiaminopimelate aminotransferase gene, an S-(hydroxymethyl)mycothiol dehydrogenase gene, and an acetaldehyde dehydrogenase gene.
[A29] The method according to any one of [A1] to [A28], further including culturing the microorganism under aerobic conditions before the culturing.
[A30] The method according to any one of [A1] to [A29], further including recovering the produced α-keto acids.
[B1] A method for manufacturing 1,3-diol, including steps of:
   culturing a microorganism including the following genes:
      (a) a first gene encoding an enzyme that catalyzes an aldol reaction between pyruvic acid and a carbonyl compound;
      (b) a second gene encoding an enzyme that catalyzes a decarboxylation reaction of α-keto acids; and
      (c) a third gene encoding an enzyme that catalyzes a reduction reaction of aldehydes,
   in the presence of:
      a pyruvic acid-supplying compound selected from pyruvic acid and saccharides; and
      a carbonyl compound expressed by the following general formula (3):

         R₁₀₃C(O)R₁₀₄
      (wherein R₁₀₃ and R₁₀₄ are each independently hydrogen or an alkyl having 1 to 5 carbon atoms, provided that when R₁₀₃ is hydrogen, R₁₀₄ is not hydrogen),
         and
      producing 1,3-diol expressed by the following general formula (4):

         R₁₀₃C(R₁₀₄)(OH)CH₂CH₂OH
      (wherein each of R₁₀₃ and R₁₀₄ in the general formula (4) above is the same group as R₁₀₃ and R₁₀₄ in the general formula (3) above).
[B2] The method according to [B1], in which R₁₀₄ in the general formulae (3) and (4) is hydrogen.
[B3] The method according to [B1] or [B2], in which R₁₀₃ in the general formulae (3) and (4) is a methyl, ethyl, propyl or pentyl group, and R₁₀₄ is hydrogen.
[B4] The method according to any one of [B1] to [B3], in which R₁₀₃ in the general formulae (3) and (4) is a methyl, ethyl, n-propyl or n-pentyl group, and R₁₀₄ is hydrogen.
[B5] The method according to any one of [B1] to [B4], in which the culture is performed by culturing the microorganism in a culture solution containing the pyruvic acid-supplying compound and the carbonyl compound.
[B6] The method according to [B5], in which the culture solution is a culture solution to which the pyruvic acid-supplying compound and the carbonyl compound are added.
[B7] The method according to [B6], in which the pyruvic acid-supplying compound is added to the culture solution so that a concentration thereof becomes 0.1 to 40 (w/v)%, preferably 1 to 20 (w/v)%.
[B8] The method according to [B6] or [B7], in which the a carbonyl compound in the culture solution so that a concentration thereof becomes 0.001 to 10 (w/v)%, preferably 0.01 to 1 (w/v)%.
[B9] The method according to any one of [B1] to [B8], in which the pyruvic acid-supplying compound is a saccharide.
[B10] The method according to any one of [B1] to [B9], in which the saccharide is a saccharified liquid obtained by treating, with saccharifying enzymes, monosaccharides such as fructose or glucose; disaccharides such as sucrose; polysaccharides such as a polysaccharide containing glucose as a constituent monosaccharide; or plants (non-edible agricultural waste or energy crops).
[B11] The method according to any one of [B1] to [B10], in which the saccharide is glucose.
[B12] The method according to any one of [B1] to [B8], in which the pyruvic acid-supplying compound is pyruvic acid.
[B13] The method according to any one of [B1] to [B12], in which the culture is performed under anaerobic conditions or microaerobic conditions.
[B14] The method according to [B13], in which the culture is performed by culturing the microorganism in a culture solution containing the pyruvic acid-supplying compound and the carbonyl compound.
[B15] The method according to [B14], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[B16] The method according to any one of [B1] to [B15], in which the culture is performed in a state where the microorganism is suspended in the culture solution at a high density.
[B17] The method according to [B16], in which the culture is performed by culturing the microorganism in a culture solution containing the pyruvic acid-supplying compound and the carbonyl compound.
[B18] The method according to [B17], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[B19] The method according to any one of [B1] to [B18], further comprising recovering the produced 1,3-diol.
[B20] A method for manufacturing 1,3-butanediol, including:
   culturing a microorganism including the following genes:
      (a) a first gene encoding an enzyme that catalyzes an aldol reaction between pyruvic acid and a carbonyl compound;
      (b) a second gene encoding an enzyme that catalyzes a decarboxylation reaction of α-keto acids; and
      (c) a third gene encoding an enzyme that catalyzes a reduction reaction of aldehydes,
   in the presence of a pyruvic acid-supplying compound selected from pyruvic acid and saccharides to produce 1,3-butanediol.
[B21] The method according to [B20], in which the culture is performed by culturing the microorganism in a culture solution containing the pyruvic acid-supplying compound.
[B22] The method according to [B21], in which the culture solution is a culture solution to which the pyruvic acid-supplying compound are added.
[B23] The method according to [B22], in which the pyruvic acid-supplying compound is added to the culture solution so that a concentration thereof becomes 0.1 to 40 (w/v)%, preferably 1 to 20 (w/v)%.
[B24] The method according to any one of [B20] to [B23], in which the pyruvic acid-supplying compound is a saccharide.
[B25] The method according to any one of [B20] to [B24], in which the saccharide is a saccharified liquid obtained by treating, with saccharifying enzymes, monosaccharides such as fructose or glucose; disaccharides such as sucrose; polysaccharides such as a polysaccharide containing glucose as a constituent monosaccharide; or plants (non-edible agricultural waste or energy crops).
[B26] The method according to any one of [B20] to [B25], in which the saccharide is glucose.
[B27] The method according to any one of [B20] to [B26], in which the culture is performed under anaerobic conditions or microaerobic conditions.
[B28] The method according to [B27], in which the culture is performed by culturing the microorganism in a culture solution containing the pyruvic acid-supplying compound.
[B29] The method according to [B28], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[B30] The method according to any one of [B20] to [B29], in which the culture is performed in a state where the microorganism is suspended in the culture solution at a high density.
[B31] The method according to [B30], in which the culture is performed by culturing the microorganism in a culture solution containing the pyruvic acid-supplying compound.
[B32] The method according to [B31], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[B33] The method according to any one of [B20] to [B23], further comprising recovering the produced 1,3-butanediol.
[B34] The method according to any one of [B1] to [B33], in which the first gene encodes an aldolase.
[B35] The method according to any one of [B1] to [B34], in which the first gene encodes a type I aldolase.
[B36] The method according to any one of [B1] to [B34], in which the first gene encodes a type II aldolase.
[B37] The method according to any one of [B1] to [B36], in which the first gene encodes an aldolase selected from the group consisting of:
   (a1) 2-dehydro-3-deoxy-phosphogluconate aldolase;
   (a2) 2-dehydro-3-deoxy-6-phosphogluconatealdolase;
   (a3) 4-hydroxy-2-oxoglutarate aldolase;
   (a4) (4S)-4-hydroxyl-2-oxoglutarate aldolase;
   (a5) 2-dehydro-3-deoxy-D-pentonate aldolase;
   (a6) 2-dehydro-3-deoxy-D-gluconate aldolase;
   (a7) 3-deoxy-D-manno-octulosonate aldolase;
   (a8) 4-hydroxyl-2-oxovalerate aldolase;
   (a9) 4-(2-carboxyphenyl)-2-oxobut-3-enoate aldolase;
   (a10) 4-hydroxy-2-oxoheptanedioate aldolase;
   (a11) 2-dehydro-3-deoxyglucarate aldolase;
   (a12) 2-keto-3-deoxy-L-rhamnonate aldolase;
   (a13) 4-hydroxyl-4-methyl-2-oxoglutarate aldolase; and
   (a14) 4-hydroxy-2-oxohexanoate aldolase.
[B38] The method according to any one of [B1] to [B37], in which the first gene encodes an aldolase selected from the group consisting of an eda protein, preferably an eda protein derived from *Escherichia coli*; a dgoA protein, preferably a dgoA protein derived from *Escherichia coli*; a yjhH protein, preferably a yjhH protein derived from *Escherichia coli*; a yagE protein, preferably a yagE protein derived from *Escherichia coli*; a nanA protein, preferably a nanA protein derived from *Escherichia coli*; an mhpE protein, preferably a mhpE protein derived from *Escherichia coli*; a hpaI protein, preferably a hpaI protein derived from *Escherichia coli*; a bphI protein, preferably a bphI protein derived from *Burkholderia xenovorans*; a phdJ protein, preferably a phdJ protein derived from the genus *Nocardioides*; a garL protein, preferably a garL protein derived from *Escherichia coli*; an rhmA protein, preferably an rhmA protein derived from *Escherichia coli*; and a galC protein, preferably, a galC protein derived from *Pseudomonas putida.*
[B39] The method according to any one of [B1] to [B38], in which the first gene is selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[B40] The method according to any one of [B1] to [B39], in which the first gene is selected from the group consisting of:
   a gene that consists of a base sequence set forth in SEQ ID NO: 1;
   a gene that consists of a base sequence set forth in SEQ ID NO: 3;
   a gene that consists of a base sequence set forth in SEQ ID NO: 5;
   a gene that consists of a base sequence set forth in SEQ ID NO: 7;
   a gene that consists of a base sequence set forth in SEQ ID NO: 9;
   a gene that consists of a base sequence set forth in SEQ ID NO: 11;
   a gene that consists of a base sequence set forth in SEQ ID NO: 13;
   a gene that consists of a base sequence set forth in SEQ ID NO: 15;
   a gene that consists of a base sequence set forth in SEQ ID NO: 17;
   a gene that consists of a base sequence set forth in SEQ ID NO: 19; and
   a gene that consists of a base sequence set forth in SEQ ID NO: 21.
[B41] The method according to any one of [B1] to [B40], in which the second gene encodes the enzyme that catalyzes the decarboxylation reaction of α-keto acids obtained by the aldol reaction.
[B42] The method according to any one of [B1] to [B41], in which the second gene encodes a decarboxylase.
[B43] The method according to any one of [B1] to [B42], in which the second gene encodes a decarboxylase selected from the group consisting of:
   (b1) pyruvate decarboxylase;
   (b2) benzoylformate decarboxylase; and
   (b3) indolepyruvate decarboxylase.
[B44] The method according to any one of [B1] to [B43], in which the second gene encodes a decarboxylase selected from the group consisting of a pdc protein, preferably a pdc protein derived from *Zymomonas mobilis*; a mdlC protein, preferably a mdlC protein derived from *Pseudomonas putida*; and a kivD protein, preferably a kivD protein derived from *Lactococcus lactis.*
[B45] The method according to any one of [B1] to [B44], in which the second gene encodes an mdlC protein, preferably an mdlC protein derived from *Pseudomonas putida.*
[B46] The method according to any one of [B1] to [B45], in which the second gene is selected from the group consisting of:
   (2-1) a gene encoding a protein that consists of an amino acid sequence set forth in SEQ ID NO: 24 and catalyzes a decarboxylation reaction of α-keto acids; and
   (2-2) a gene encoding a protein that consists of an amino acid sequence in which one or a plurality of amino acids is deleted, substituted, or added in SEQ ID NO: 24, and has an activity of catalyzing a decarboxylation reaction of α-keto acids.
[B47] The method according to any one of [B1] to [B46], in which the second gene is a gene consisting of a base sequence set forth in SEQ ID NO: 23.
[B48] The method according to any one of [B1] to [B47], in which the third gene encodes the enzyme that catalyzes a reduction reaction of an aldehyde that is a type different from the aldehyde that is a substrate of the aldol reaction catalyzed by the enzyme encoded by the first gene.
[B49] The method according to any one of [B1] to [B48], in which the third gene encodes the enzyme that catalyzes the reduction reaction of the aldehyde obtained by the decarboxylation reaction.
[B50] The method according to any one of [B1] to [B49], in which the third gene encodes an alcohol dehydrogenase.
[B51] The method according to any one of [B1] to [B50], in which the third gene encodes a 1,3-propanediol dehydrogenase.
[B52] The method according to any one of [B1] to [B51], in which the third gene encodes 1,3-propanediol dehydrogenase selected from the group consisting of a dhaT protein, preferably a dhaT protein derived from *Klebsiella pneumoniae*; and an lpo protein, preferably an lpo protein derived from *Lactobacillus reuteri.* [B53] The method according to any one of [B1] to [B52], in which the third gene is selected from the group consisting of:
   (3-1) a gene encoding a protein that consists of an amino acid sequence set forth in SEQ ID NO: 26 and catalyzes a reduction reaction of aldehydes; and
   (3-2) a gene encoding a protein that consists of an amino acid sequence in which one or a plurality of amino acids is deleted, substituted, or added in SEQ ID NO: 26, and has an activity of catalyzing a reduction reaction of aldehydes.
[B54] The method according to any one of [B1] to [B53], in which the third gene is a gene consisting of a base sequence set forth in SEQ ID NO: 25.
[B55] The method according to any one of [B1] to [B54], in which the microorganism is an aerobic bacterium, is preferably a coryneform bacterium, is more preferably bacteria belonging to the genus *Corynebacterium,* and is even more preferably *Corynebacterium glutamicum.*
[B55] The method according to any one of [B1] to [B55], in which the microorganism is a microorganism transformed with the first gene, the second gene, and the third gene.
[B57] The method according to any one of [B1] to [B56], in which the microorganism is a gene-deleted strain from which at least one gene is deleted, the gene being selected from the group consisting of a lactate dehydrogenase gene, a phosphoenolpyrvate carboxylase gene, a pyruvate carboxylase gene, a pyruvate dehydrogenase gene, a glutamate-pyruvate aminotransferase gene, an acetolactate synthase gene, an N-succinyldiaminopimelate aminotransferase gene, an S-(hydroxymethyl)mycothiol dehydrogenase gene, and an acetaldehyde dehydrogenase gene.
[B58] The method according to any one of [B1] to [B57], further including culturing the microorganism under aerobic conditions before the culturing.
[C1] A method for manufacturing 2,4-dihydroxycarboxylic acid, including steps of:
   culturing a microorganism including the following genes:
      (a) a first gene encoding an enzyme that catalyzes an aldol reaction between pyruvic acid and a carbonyl compound; and
      (b) a fourth gene encoding an enzyme that catalyzes a reduction reaction of α-keto acids;
   in the presence of:
      a pyruvic acid-supplying compound selected from pyruvic acid and saccharides; and
      a carbonyl compound expressed by the following general formula (1):

         R₁₀₁C(O)R₁₀₂
      (wherein R₁₀₁ and R₁₀₂ are each independently hydrogen or an alkyl having 1 to 5 carbon atoms), and
      producing 2,4-dihydroxycarboxylic acid expressed by the following general formula (5):

         R₁₀₁C(R₁₀₂)(OH)CH₂CH(OH)COOH
      (wherein each of R₁₀₁ and R₁₀₂ in the general formula (4) above is the same group as R₁₀₁ and R₁₀₂ in the general formula (1) above).
[C2] The method according to [C1], in which R₁₀₂ in the general formulae (1) and (5) is hydrogen.
[C3] The method according to [C1] or [C2], in which R₁₀₁ in the general formulae (1) and (5) is hydrogen, a methyl, ethyl, propyl or pentyl group, and R₁₀₂ is hydrogen.
[C4] The method according to any one of [C1] to [C3], in which R₁₀₁ and R₁₀₂ in the general formulae (1) and (5) are hydrogen.
[C5] The method according to any one of [C1] to [C4], in which the culture is performed by culturing the microorganism in a culture solution containing the pyruvic acid-supplying compound and the carbonyl compound.
[C6] The method according to [C5], in which the culture solution is a culture solution to which the pyruvic acid-supplying compound and the carbonyl compound are added.
[C7] The method according to [C6], in which the pyruvic acid-supplying compound is added to the culture solution so that a concentration thereof becomes 0.1 to 40 (w/v)%, preferably 1 to 20 (w/v)%.
[C8] The method according to [C6] or [C7], in which the a carbonyl compound in the culture solution so that a concentration thereof becomes 0.001 to 10 (w/v)%, preferably 0.01 to 1 (w/v)%.
[C9] The method according to any one of [C1] to [C8], in which the pyruvic acid-supplying compound is a saccharide.
[C10] The method according to any one of [C1] to [C9], in which the saccharide is a saccharified liquid obtained by treating, with saccharifying enzymes, monosaccharides such as fructose or glucose; disaccharides such as sucrose; polysaccharides such as a polysaccharide containing glucose as a constituent monosaccharide; or plants (non-edible agricultural waste or energy crops).
[C11] The method according to any one of [C1] to [C10], in which the saccharide is glucose.
[C12] The method according to any one of [C1] to [C11], in which the pyruvic acid-supplying compound is pyruvic acid.
[C13] The method according to any one of [C1] to [C12], in which the culture is performed under anaerobic conditions or microaerobic conditions.
[C14] The method according to [C13], in which the culture is performed by culturing the microorganism in a culture solution containing the pyruvic acid-supplying compound and the carbonyl compound.
[C15] The method according to [C14], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[C16] The method according to any one of [C1] to [C15], in which the culture is performed in a state where the microorganism is suspended in the culture solution at a high density.
[C17] The method according to [C16], in which the culture is performed by culturing the microorganism in a culture solution containing the pyruvic acid-supplying compound and the carbonyl compound.
[C18] The method according to [C17], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[C19] The method according to any one of [C1] to [C18], in which the first gene encodes an aldolase.
[C20] The method according to any one of [C1] to [C19], in which the first gene encodes a type I aldolase.
[C21] The method according to any one of [C1] to [C19], in which the first gene encodes a type II aldolase.
[C22] The method according to any one of [C1] to [C21], in which the first gene encodes an aldolase selected from the group consisting of:
   (a1) 2-dehydro-3-deoxy-phosphogluconate aldolase;
   (a2) 2-dehydro-3-deoxy-6-phosphogluconatealdolase;
   (a3) 4-hydroxy-2-oxoglutarate aldolase;
   (a4) (4S)-4-hydroxyl-2-oxoglutarate aldolase;
   (a5) 2-dehydro-3-deoxy-D-pentonate aldolase;
   (a6) 2-dehydro-3-deoxy-D-gluconate aldolase;
   (a7) 3-deoxy-D-manno-octulosonate aldolase;
   (a8) 4-hydroxyl-2-oxovalerate aldolase;
   (a9) 4-(2-carboxyphenyl)-2-oxobut-3-enoate aldolase;
   (a10) 4-hydroxy-2-oxoheptanedioate aldolase;
   (a11) 2-dehydro-3-deoxyglucarate aldolase;
   (a12) 2-keto-3-deoxy-L-rhamnonate aldolase;
   (a13) 4-hydroxyl-4-methyl-2-oxoglutarate aldolase; and
   (a14) 4-hydroxy-2-oxohexanoate aldolase.
[C23] The method according to any one of [C1] to [C22], in which the first gene encodes an aldolase selected from the group consisting of an eda protein, preferably an eda protein derived from *Escherichia coli*; a dgoA protein, preferably a dgoA protein derived from *Escherichia coli*; a yjhH protein, preferably a yjhH protein derived from *Escherichia coli*; a yagE protein, preferably a yagE protein derived from *Escherichia coli*; a nanA protein, preferably a nanA protein derived from *Escherichia coli*; an mhpE protein, preferably a mhpE protein derived from *Escherichia coli*; a hpaI protein, preferably a hpaI protein derived from *Escherichia coli*; a bphI protein, preferably a bphI protein derived from *Burkholderia xenovorans*; a phdJ protein, preferably a phdJ protein derived from the genus *Nocardioides*; a garL protein, preferably a garL protein derived from *Escherichia coli*; an rhmA protein, preferably an rhmA protein derived from *Escherichia coli*; and a galC protein, preferably, a galC protein derived from *Pseudomonas putida.*
[C24] The method according to any one of [C1] to [C23], in which the first gene is selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[C25] The method according to any one of [C1] to [C24], in which the first gene is selected from the group consisting of:
   a gene that consists of a base sequence set forth in SEQ ID NO: 1;
   a gene that consists of a base sequence set forth in SEQ ID NO: 3;
   a gene that consists of a base sequence set forth in SEQ ID NO: 5;
   a gene that consists of a base sequence set forth in SEQ ID NO: 7;
   a gene that consists of a base sequence set forth in SEQ ID NO: 9;
   a gene that consists of a base sequence set forth in SEQ ID NO: 11;
   a gene that consists of a base sequence set forth in SEQ ID NO: 13;
   a gene that consists of a base sequence set forth in SEQ ID NO: 15;
   a gene that consists of a base sequence set forth in SEQ ID NO: 17;
   a gene that consists of a base sequence set forth in SEQ ID NO: 19; and
   a gene that consists of a base sequence set forth in SEQ ID NO: 21.
[C26] The method according to any one of [C1] to [C23], in which the fourth gene encodes the enzyme that catalyzes a reduction reaction of an α-keto acid of α-keto acid obtained by the aldol reaction.
[C27] The method according to any one of [C1] to [C26], in which the fourth gene encodes dehydrogenase.
[C28] The method according to any one of [C1] to [C27], in which the fourth gene encodes lactate dehydrogenase.
[C29] The method according to any one of [C1] to [C28], in which the fourth gene encodes an ldhA protein, preferably an ldhA protein derived from *Lactococcus lactis,* more preferably an ldhA protein derived from a *Lactococcus lactis* strain NBRC 100676, and even more preferably an ldhA protein which is derived from the *Lactococcus lactis* strain NBRC 100676 and in which the 85th glutamine residue from an N-terminal is substituted by a cysteine residue.
[C30] The method according to any one of [C1] to [C29], in which the fourth gene is selected from the group consisting of the following genes:
   (4-1) gene encoding a protein that consists of an amino acid sequence set forth in SEQ ID NO: 28 and catalyzes a reduction reaction of an α-keto acid; and
   (4-2) gene encoding a protein that consists of an amino acid sequence in which one or a plurality of amino acids is deleted, substituted, or added in SEQ ID NO: 28, and has an activity of catalyzing a reduction reaction of an α-keto acid.
[C31] The method according to any one of [C1] to [C30], in which the gene encoding the protein that consists of an amino acid sequence in which one or a plurality of amino acids is deleted, substituted, or added in SEQ ID NO: 28, and has an activity of catalyzing a reduction reaction of an α-keto acid encodes a protein that maintains a cysteine residue at the 85th residue from the N-terminal.
[C32] The method according to any one of [C1] to [C31], in which the fourth gene is a gene consisting of a base sequence set forth in SEQ ID NO: 27.
[C33] The method according to any one of [C1] to [C32], in which the microorganism is an aerobic bacterium, is preferably a coryneform bacterium, is more preferably bacteria belonging to the genus *Corynebacterium,* and is even more preferably *Corynebacterium glutamicum.*
[C34] The method according to any one of [C1] to [C33], in which the microorganism is a microorganism transformed with the first gene and the fourth gene.
[C35] The method according to any one of [C1] to [C34], in which the microorganism is a gene-deleted strain from which at least one gene is deleted, the gene being selected from the group consisting of a lactate dehydrogenase gene, a phosphoenolpyrvate carboxylase gene, a pyruvate carboxylase gene, a pyruvate dehydrogenase gene, a glutamate-pyruvate aminotransferase gene, an acetolactate synthase gene, an N-succinyldiaminopimelate aminotransferase gene, an S-(hydroxymethyl)mycothiol dehydrogenase gene, and an acetaldehyde dehydrogenase gene.
[C36] The method according to any one of [C1] to [C35], further including culturing the microorganism under aerobic conditions before the culturing.
[C37] The method according to any one of [C1] to [C36], further including recovering the produced 2,4-dihydroxycarboxylic acid.
[D1] A method for manufacturing 2-amino-4-hydroxycarboxylic acid, including steps of:
   culturing a microorganism including the following genes:
      (a) a first gene encoding an enzyme that catalyzes an aldol reaction between pyruvic acid and a carbonyl compound; and
      (e) a fifth gene encoding an enzyme that catalyzes a reductive amination reaction of α-keto acids;
   in the presence of:
      a pyruvic acid-supplying compound selected from pyruvic acid and saccharides; and
      a carbonyl compound expressed by the following general formula (1):

         R₁₀₁C(O)R₁₀₂
      (wherein R₁₀₁ and R₁₀₂ are each independently hydrogen or an alkyl having 1 to 5 carbon atoms), and
      producing 2-amino-4-hydroxycarboxylic acid expressed by the following general formula (6):

         R₁₀₁C(R₁₀₂)(OH)CH₂CH(NH₂)COOH
      (wherein each of R₁₀₁ and R₁₀₂ in the general formula (6) above is the same group as R₁₀₁ and R₁₀₂ in the general formula (1) above).
[D2] The method according to [D1], in which R₁₀₂ in the general formulae (1) and (6) is hydrogen.
[D3] The method according to [D1] or [D2], in which R₁₀₁ in the general formulae (1) and (6) is hydrogen, a methyl, ethyl, propyl or pentyl group, and R₁₀₂ is hydrogen.
[D4] The method according to any one of [D1] to [D3], in which R₁₀₁ and R₁₀₂ in the general formulae (1) and (6) are hydrogen.
[D5] The method according to any one of [D1] to [D4], further including recovering the produced 2-amino-4-hydroxycarboxylic acid.
[D6] A method for manufacturing at least one of homoserine, threonine and 2-amino butyric acid, including steps of:
   culturing a microorganism including the following genes:
      (a) a first gene encoding an enzyme that catalyzes an aldol reaction between pyruvic acid and a carbonyl compound; and
      (e) a fifth gene encoding an enzyme that catalyzes a reductive amination reaction of α-keto acids,
   in the presence of:
      a pyruvic acid-supplying compound selected from pyruvic acid and saccharides; and formaldehyde (which is a carbonyl compound),
         and
      producing at least one of homoserine, threonine and 2-amino butyric acid.
[D7] The method according to [D6], further including recovering at least one of the produced homoserine, threonine and 2-amino butyric acid.
[D8] The method according to any one of [D1] to [D7], in which the culture is performed by culturing the microorganism in a culture solution containing the pyruvic acid-supplying compound and the carbonyl compound.
[D9] The method according to [D8], in which the culture solution is a culture solution to which the pyruvic acid-supplying compound and the carbonyl compound are added.
[D10] The method according to [D9], in which the pyruvic acid-supplying compound is added to the culture solution so that a concentration thereof becomes 0.1 to 40 (w/v)%, preferably 1 to 20 (w/v)%.
[D11] The method according to [D9] or [D10], in which the a carbonyl compound in the culture solution so that a concentration thereof becomes 0.001 to 10 (w/v)%, preferably 0.01 to 1 (w/v)%.
[D12] The method according to any one of [D1] to [D11], in which the pyruvic acid-supplying compound is a saccharide.
[D13] The method according to any one of [D1] to [D12], in which the saccharide is a saccharified liquid obtained by treating, with saccharifying enzymes, monosaccharides such as fructose or glucose; disaccharides such as sucrose; polysaccharides such as a polysaccharide containing glucose as a constituent monosaccharide; or plants (non-edible agricultural waste or energy crops).
[D14] The method according to any one of [D1] to [D13], in which the saccharide is glucose.
[D15] The method according to any one of [D1] to [C14], in which the pyruvic acid-supplying compound is pyruvic acid.
[D16] The method according to any one of [D1] to [D15], in which the culture is performed under anaerobic conditions or microaerobic conditions.
[D17] The method according to [C16], in which the culture is performed by culturing the microorganism in a culture solution containing the pyruvic acid-supplying compound and the carbonyl compound.
[D18] The method according to [D17], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[D19] The method according to any one of [D1] to [D18], in which the culture is performed in a state where the microorganism is suspended in the culture solution at a high density.
[D20] The method according to [D19], in which the culture is performed by culturing the microorganism in a culture solution containing the pyruvic acid-supplying compound and the carbonyl compound.
[D21] The method according to [D20], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[D22] The method according to any one of [D1] to [D21], in which the first gene encodes an aldolase.
[D23] The method according to any one of [D1] to [D22], in which the first gene encodes a type I aldolase.
[D24] The method according to any one of [D1] to [D22], in which the first gene encodes a type II aldolase.
[D25] The method according to any one of [D1] to [D24], in which the first gene encodes an aldolase selected from the group consisting of:
   (a1) 2-dehydro-3-deoxy-phosphogluconate aldolase;
   (a2) 2-dehydro-3-deoxy-6-phosphogluconatealdolase;
   (a3) 4-hydroxy-2-oxoglutarate aldolase;
   (a4) (4S)-4-hydroxyl-2-oxoglutarate aldolase;
   (a5) 2-dehydro-3-deoxy-D-pentonate aldolase;
   (a6) 2-dehydro-3-deoxy-D-gluconate aldolase;
   (a7) 3-deoxy-D-manno-octulosonate aldolase;
   (a8) 4-hydroxyl-2-oxovalerate aldolase;
   (a9) 4-(2-carboxyphenyl)-2-oxobut-3-enoate aldolase;
   (a10) 4-hydroxy-2-oxoheptanedioate aldolase;
   (a11) 2-dehydro-3-deoxyglucarate aldolase;
   (a12) 2-keto-3-deoxy-L-rhamnonate aldolase;
   (a13) 4-hydroxyl-4-methyl-2-oxoglutarate aldolase; and
   (a14) 4-hydroxy-2-oxohexanoate aldolase.
[D26] The method according to any one of [D1] to [D25], in which the first gene encodes an aldolase selected from the group consisting of an eda protein, preferably an eda protein derived from *Escherichia coli;* a dgoA protein, preferably a dgoA protein derived from *Escherichia coli;* a yjhH protein, preferably a yjhH protein derived from *Escherichia coli;* a yagE protein, preferably a yagE protein derived from *Escherichia coli;* a nanA protein, preferably a nanA protein derived from *Escherichia coli;* an mhpE protein, preferably a mhpE protein derived from *Escherichia coli;* a hpaI protein, preferably a hpaI protein derived from *Escherichia coli;* a bphI protein, preferably a bphI protein derived from *Burkholderia xenovorans;* a phdJ protein, preferably a phdJ protein derived from the genus *Nocardioides;* a garL protein, preferably a garL protein derived from *Escherichia coli;* an rhmA protein, preferably an rhmA protein derived from *Escherichia coli;* and a galC protein, preferably, a galC protein derived from *Pseudomonas putida.*
[D27] The method according to any one of [D1] to [D26], in which the first gene is selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[D28] The method according to any one of [D1] to [D27], in which the first gene is selected from the group consisting of:
   a gene that consists of a base sequence set forth in SEQ ID NO: 1;
   a gene that consists of a base sequence set forth in SEQ ID NO: 3;
   a gene that consists of a base sequence set forth in SEQ ID NO: 5;
   a gene that consists of a base sequence set forth in SEQ ID NO: 7;
   a gene that consists of a base sequence set forth in SEQ ID NO: 9;
   a gene that consists of a base sequence set forth in SEQ ID NO: 11;
   a gene that consists of a base sequence set forth in SEQ ID NO: 13;
   a gene that consists of a base sequence set forth in SEQ ID NO: 15;
   a gene that consists of a base sequence set forth in SEQ ID NO: 17;
   a gene that consists of a base sequence set forth in SEQ ID NO: 19; and
   a gene that consists of a base sequence set forth in SEQ ID NO: 21.
[D29] The method according to any one of [D1] to [D28], in which the fifth gene encodes the enzyme that catalyzes a reductive amination reaction of an α-keto acid obtained by the aldol reaction.
[D30] The method according to any one of [D1] to [D29], in which the fifth gene encodes glutamate/phenylalanine/leucine/valine dehydrogenase.
[D31] The method according to any one of [D1] to [D30], in which the fifth gene encodes at least one dehydrogenase selected from the group consisting of glutamate dehydrogenase, phenylalanine dehydrogenase, leucine dehydrogenase, and valine dehydrogenase.
[D32] The method according to any one of [D1] to [D31], in which the fifth gene encodes glutamate/phenylalanine/leucine/valine dehydrogenase selected from the group consisting of the following dehydrogenases:
   (d1) leucine dehydrogenase (EC number: 1.4.1.9);
   (d2) phenylalanine dehydrogenase (EC number: 1.4.1.20); and
   (d3) valine dehydrogenase (EC numbers: 1.4.1.8 and 1.4.1.23).
[D33] The method according to any one of [D1] to [D32], in which the fifth gene encodes aldolase selected from the group consisting of an leuDH protein, preferably, an leuDH protein derived from *Lysinibacillus sphaericus,* an leuDH protein derived from *Bacillus cereus,* or an leuDH protein derived from *Geobacillus stearothermophilus;* a phedh protein, preferably, a phedh protein derived from *Bacillus badius;* and a valdh protein, preferably, a valdh protein derived from *Streptomyces fradiae.*
[D34] The method according to any one of [D1] to [D33], in which the fifth gene is selected from the group consisting of the following genes:
   (5-1) gene encoding a protein that consists of an amino acid sequence set forth in SEQ ID NO: 30 and catalyzes a reductive amination reaction of an α-keto acid;
   (5-2) gene encoding a protein that consists of an amino acid sequence in which one or a plurality of amino acids is deleted, substituted, or added in SEQ ID NO: 30, and has an activity of catalyzing a reductive amination reaction of an α-keto acid;
   (5-3) gene encoding a protein that consists of an amino acid sequence set forth in SEQ ID NO: 32 and catalyzes a reductive amination reaction of an α-keto acid;
   (5-4) gene encoding a protein that consists of an amino acid sequence in which one or a plurality of amino acids is deleted, substituted, or added in SEQ ID NO: 32, and has an activity of catalyzing a reductive amination reaction of an α-keto acid;
   (5-5) gene encoding a protein that consists of an amino acid sequence set forth in SEQ ID NO: 34 and catalyzes a reductive amination reaction of an α-keto acid; and
   (5-6) gene encoding a protein that consists of an amino acid sequence in which one or a plurality of amino acids is deleted, substituted, or added in SEQ ID NO: 34, and has an activity of catalyzing a reductive amination reaction of an α-keto acid.
[D35] The method according to any one of [D1] to [D34], in which the fifth gene is selected from the group consisting of the following genes:
   gene consisting of a base sequence set forth in SEQ ID NO: 29;
   gene consisting of a base sequence set forth in SEQ ID NO: 31; and
   valdh gene consisting of a base sequence set forth in SEQ ID NO: 33.
[D36] The method according to any one of [D1] to [D35], in which the microorganism is an aerobic bacterium, is preferably a coryneform bacterium, is more preferably bacteria belonging to the genus *Corynebacterium,* and is even more preferably *Corynebacterium glutamicum.*
[D37] The method according to any one of [D1] to [D36], in which the microorganism is a microorganism transformed with the first gene and the fifth gene.
[D38] The method according to any one of [D1] to [D37], in which the microorganism is a gene-deleted strain from which at least one gene is deleted, the gene being selected from the group consisting of a lactate dehydrogenase gene, a phosphoenolpyrvate carboxylase gene, a pyruvate carboxylase gene, a pyruvate dehydrogenase gene, a glutamate-pyruvate aminotransferase gene, an acetolactate synthase gene, an N-succinyldiaminopimelate aminotransferase gene, an S-(hydroxymethyl)mycothiol dehydrogenase gene, and an acetaldehyde dehydrogenase gene.
[D39] The method according to any one of [D1] to [D38], further including culturing the microorganism under aerobic conditions before the culturing.
[E1] A method for manufacturing 2-amino-4-methylthiocarboxylic acid, including steps of:
   culturing a microorganism including the following genes:
      (a) a first gene encoding an enzyme that catalyzes an aldol reaction between pyruvic acid and a carbonyl compound;
      (e) a fifth gene encoding an enzyme that catalyzes a reductive amination reaction of α-keto acids; and
      (f) a sixth gene encoding an enzyme that catalyzes a reaction of substituting a phosphoric acid group contained in 2-amino-4-phosphonooxycarboxylic acid with a methylmercapto group contained in methyl mercaptan
   in the presence of:
      a pyruvic acid-supplying compound selected from pyruvic acid and saccharides;
      methyl mercaptan; and
      a carbonyl compound expressed by the following general formula (1):

         R₁₀₁C(O)R₁₀₂
      (wherein R₁₀₁ and R₁₀₂ are each independently hydrogen or an alkyl having 1 to 5 carbon atoms), and
      producing 2-amino-4-methylthiocarboxylic acid expressed by the following general formula (7):

         R₁₀₁C(R₁₀₂)(SCH₃)CH₂CH(NH₂)COOH
      (wherein each of R₁₀₁ and R₁₀₂ in the general formula (7) above is the same group as R₁₀₁ and R₁₀₂ in the general formula (1) above).
[E2] The method according to [E1], in which R₁₀₂ in the general formulae (1) and (7) is hydrogen.
[E3] The method according to [E1] or [E2], in which R₁₀₁ in the general formulae (1) and (7) is hydrogen, a methyl, ethyl, propyl or pentyl group, and R₁₀₂ is hydrogen.
[E4] The method according to any one of [E1] to [E3], in which R₁₀₁ and R₁₀₂ in the general formulae (1) and (7) are hydrogen.
[E5] The method according to any one of [D1] to [D4], in which the culture is performed by culturing the microorganism in a culture solution containing the pyruvic acid-supplying compound, the carbonyl compound and methyl mercaptan.
[E6] The method according to [E5], in which the culture solution is a culture solution to which the pyruvic acid-supplying compound, the carbonyl compound and methyl mercaptan are added.
[E7] The method according to [E6], in which the pyruvic acid-supplying compound is added to the culture solution so that a concentration thereof becomes 0.1 to 40 (w/v)%, preferably 1 to 20 (w/v)%.
[E8] The method according to [E6] or [E7], in which the a carbonyl compound in the culture solution so that a concentration thereof becomes 0.001 to 10 (w/v)%, preferably 0.01 to 1 (w/v)%.
[E9] The method according to any one of [E6] to [E8], in which methyl mercaptan is added to the culture solution so that a concentration thereof becomes 1 mM to 1 M, preferably 10 mM to 300 mM.
[E10] The method according to any one of [E1] to [E9], in which the pyruvic acid-supplying compound is a saccharide.
[E11] The method according to any one of [E1] to [E10], in which the saccharide is a saccharified liquid obtained by treating, with saccharifying enzymes, monosaccharides such as fructose or glucose; disaccharides such as sucrose; polysaccharides such as a polysaccharide containing glucose as a constituent monosaccharide; or plants (non-edible agricultural waste or energy crops).
[E12] The method according to any one of [E1] to [E11], in which the saccharide is glucose.
[E13] The method according to any one of [E1] to [E9], in which the pyruvic acid-supplying compound is pyruvic acid.
[E14] The method according to any one of [E1] to [E13], in which the culture is performed under anaerobic conditions or microaerobic conditions.
[E15] The method according to [E17], in which the culture is performed by culturing the microorganism in a culture solution containing the pyruvic acid-supplying compound, the carbonyl compound and methyl mercaptan.
[E16] The method according to [E15], in which the anaerobic conditions or the microaerobic conditions are conditions in which a concentration of dissolved oxygen in the culture solution is within a range of 0 to 2 ppm, preferably within a range of 0 to 1 ppm, and more preferably within the range of 0 to 0.5 ppm.
[E17] The method according to any one of [E1] to [E16], in which the culture is performed in a state where the microorganism is suspended in the culture solution at a high density.
[E18] The method according to [E17], in which the culture is performed by culturing the microorganism in a culture solution containing the pyruvic acid-supplying compound, the carbonyl compound and methyl mercaptan.
[E19] The method according to [E18], in which the state in which the microorganism is suspended in the culture solution at a high density is a state in which the microorganism is suspended in the culture solution so that a wet bacterial cell weight% of the microorganism is within a range of 1 to 50 (w/v)%, and is preferably a state in which the microorganism is suspended in the culture solution so that a wet bacterial cell weight% of the microorganism is within a range of 3 to 30 (w/v)%.
[E20] The method according to any one of [E1] to [E19], in which the first gene encodes an aldolase.
[E21] The method according to any one of [E1] to [E20], in which the first gene encodes a type I aldolase.
[E22] The method according to any one of [E1] to [E20], in which the first gene encodes a type II aldolase.
[E23] The method according to any one of [E1] to [E22], in which the first gene encodes an aldolase selected from the group consisting of:
   (a1) 2-dehydro-3-deoxy-phosphogluconate aldolase;
   (a2) 2-dehydro-3-deoxy-6-phosphogluconatealdolase;
   (a3) 4-hydroxy-2-oxoglutarate aldolase;
   (a4) (4S)-4-hydroxyl-2-oxoglutarate aldolase;
   (a5) 2-dehydro-3-deoxy-D-pentonate aldolase;
   (a6) 2-dehydro-3-deoxy-D-gluconate aldolase;
   (a7) 3-deoxy-D-manno-octulosonate aldolase;
   (a8) 4-hydroxyl-2-oxovalerate aldolase;
   (a9) 4-(2-carboxyphenyl)-2-oxobut-3-enoate aldolase;
   (a10) 4-hydroxy-2-oxoheptanedioate aldolase;
   (a11) 2-dehydro-3-deoxyglucarate aldolase;
   (a12) 2-keto-3-deoxy-L-rhamnonate aldolase;
   (a13) 4-hydroxyl-4-methyl-2-oxoglutarate aldolase; and
   (a14) 4-hydroxy-2-oxohexanoate aldolase.
[E24] The method according to any one of [E1] to [E23], in which the first gene encodes an aldolase selected from the group consisting of an eda protein, preferably an eda protein derived from *Escherichia coli;* a dgoA protein, preferably a dgoA protein derived from *Escherichia coli;* a yjhH protein, preferably a yjhH protein derived from *Escherichia coli;* a yagE protein, preferably a yagE protein derived from *Escherichia coli;* a nanA protein, preferably a nanA protein derived from *Escherichia coli;* an mhpE protein, preferably a mhpE protein derived from *Escherichia coli;* a hpaI protein, preferably a hpaI protein derived from *Escherichia coli;* a bphI protein, preferably a bphI protein derived from *Burkholderia xenovorans;* a phdJ protein, preferably a phdJ protein derived from the genus *Nocardioides;* a garL protein, preferably a garL protein derived from *Escherichia coli;* an rhmA protein, preferably an rhmA protein derived from *Escherichia coli;* and a galC protein, preferably, a galC protein derived from *Pseudomonas putida.*
[E25] The method according to any one of [E1] to [E24], in which the first gene is selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[E26] The method according to any one of [E1] to [E25], in which the first gene is selected from the group consisting of:
   a gene that consists of a base sequence set forth in SEQ ID NO: 1;
   a gene that consists of a base sequence set forth in SEQ ID NO: 3;
   a gene that consists of a base sequence set forth in SEQ ID NO: 5;
   a gene that consists of a base sequence set forth in SEQ ID NO: 7;
   a gene that consists of a base sequence set forth in SEQ ID NO: 9;
   a gene that consists of a base sequence set forth in SEQ ID NO: 11;
   a gene that consists of a base sequence set forth in SEQ ID NO: 13;
   a gene that consists of a base sequence set forth in SEQ ID NO: 15;
   a gene that consists of a base sequence set forth in SEQ ID NO: 17;
   a gene that consists of a base sequence set forth in SEQ ID NO: 19; and
   a gene that consists of a base sequence set forth in SEQ ID NO: 21.
[E27] The method according to any one of [E1] to [E26], in which the fifth gene encodes the enzyme that catalyzes a reductive amination reaction of an α-keto acid obtained by the aldol reaction.
[E28] The method according to any one of [E1] to [E27], in which the fifth gene encodes glutamate/phenylalanine/leucine/valine dehydrogenase.
[E29] The method according to any one of [E1] to [E28], in which the fifth gene encodes at least one dehydrogenase selected from the group consisting of glutamate dehydrogenase, phenylalanine dehydrogenase, leucine dehydrogenase, and valine dehydrogenase.
[E30] The method according to any one of [E1] to [E29], in which the fifth gene encodes glutamate/phenylalanine/leucine/valine dehydrogenase selected from the group consisting of the following dehydrogenases:
   (d1) leucine dehydrogenase (EC number: 1.4.1.9);
   (d2) phenylalanine dehydrogenase (EC number: 1.4.1.20); and
   (d3) valine dehydrogenase (EC numbers: 1.4.1.8 and 1.4.1.23).
[E31] The method according to any one of [E1] to [E30], in which the fifth gene encodes aldolase selected from the group consisting of an leuDH protein, preferably, an leuDH protein derived from Lysinibacillus sphaericus, an leuDH protein derived from Bacillus cereus, or an leuDH protein derived from Geobacillus stearothermophilus; a phedh protein, preferably, a phedh protein derived from Bacillus badius; and a valdh protein, preferably, a valdh protein derived from Streptomyces fradiae.
[E32] The method according to any one of [E1] to [E31], in which the fifth gene is selected from the group consisting of the following genes:
   (5-1) gene encoding a protein that consists of an amino acid sequence set forth in SEQ ID NO: 30 and catalyzes a reductive amination reaction of an α-keto acid;
   (5-2) gene encoding a protein that consists of an amino acid sequence in which one or a plurality of amino acids is deleted, substituted, or added in SEQ ID NO: 30, and has an activity of catalyzing a reductive amination reaction of an α-keto acid;
   (5-3) gene encoding a protein that consists of an amino acid sequence set forth in SEQ ID NO: 32 and catalyzes a reductive amination reaction of an α-keto acid;
   (5-4) gene encoding a protein that consists of an amino acid sequence in which one or a plurality of amino acids is deleted, substituted, or added in SEQ ID NO: 32, and has an activity of catalyzing a reductive amination reaction of an α-keto acid;
   (5-5) gene encoding a protein that consists of an amino acid sequence set forth in SEQ ID NO: 34 and catalyzes a reductive amination reaction of an α-keto acid; and
   (5-6) gene encoding a protein that consists of an amino acid sequence in which one or a plurality of amino acids is deleted, substituted, or added in SEQ ID NO: 34, and has an activity of catalyzing a reductive amination reaction of an α-keto acid.
[E33] The method according to any one of [E1] to [E32], in which the fifth gene is selected from the group consisting of the following genes:
   gene consisting of a base sequence set forth in SEQ ID NO: 30;
   gene consisting of a base sequence set forth in SEQ ID NO: 32; and
   valdh gene consisting of a base sequence set forth in SEQ ID NO: 34.
[E34] The method according to any one of [B1] to [B33], in which the sixth gene encodes a transsulfation enzyme.
[E35] The method according to any one of [E1] to [E34], in which the sixth gene encodes cystathionine γ-synthase.
[E36] The method according to any one of [E1] to [E35], in which the sixth gene encodes a CGS1 protein, preferably a CGS1 protein derived from *Arabidopsis thaliana,* and more preferably a CGS1 mature protein derived from *Arabidopsis thaliana.*
[E37] The method according to any one of [E1] to [E36], in which the sixth gene is selected from the group consisting of the following genes:
   (6-1) gene encoding a protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan; and
   (6-2) gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan.
[E38] The method according to any one of [E1] to [E37], in which the sixth gene is a gene consisting of a base sequence set forth in SEQ ID NO: 35.
[E39] The method according to any one of [E1] to [E38], in which the microorganism is an aerobic bacterium, preferably a coryneform bacterium, more preferably a bacterium belonging to the genus *Corynebacterium,* more preferably *Corynebacterium glutamicum.*
[E40] The method according to any one of [E1] to [E39], in which the microorganism is a microorganism transformed with the first, the fifth and the sixth genes.
[E41] The method according to any one of [E1] to [E43], in which the microorganism is a gene-deleted strain from which at least one gene is deleted, the gene being selected from the group consisting of a lactate dehydrogenase gene, a phosphoenolpyrvate carboxylase gene, a pyruvate carboxylase gene, a pyruvate dehydrogenase gene, a glutamate-pyruvate aminotransferase gene, an acetolactate synthase gene, an N-succinyldiaminopimelate aminotransferase gene, an S-(hydroxymethyl)mycothiol dehydrogenase gene, and an acetaldehyde dehydrogenase gene.
[E42] The method according to any one of [E1] to [E41], further including culturing the microorganism under aerobic conditions before the culturing.
[E43] The method according to any one of [E1] to [E42], further including recovering the produced 2-amino-4-methylthiocarboxylic acid.
[F1] A method for manufacturing 2-amino-4-hydroxycarboxylic acid, including steps of:
   reacting an α-keto acid expressed by the following general formula (2):

      R₁₀₁C(R₁₀₂)(OH)CH₂COCOOH
   (wherein R₁₀₁ and R₁₀₂ are each independently hydrogen or an alkyl having 1 to 5 carbon atoms),
   a nitrogen source and a reducing agent,
   in the presence of glutamate/phenylalanine/leucine/valine dehydrogenase to produce a 2-amino-4-hydroxycarboxylic acid represented by General Formula (6):

      R₁₀₁C(R₁₀₂)(OH)CH₂CH(NH₂)COOH
   (wherein each of R₁₀₁ and R₁₀₂ in General Formula (6) above is the same group as R₁₀₁ and R₁₀₂ in General Formula (2) above).
[F2] The method according to [F1], in which R₁₀₂ in General Formulae (2) and (6) is hydrogen.
[F3] The method according to [F1] or [F2], in which R₁₀₁ and R₁₀₂ in General Formulae (2) and (6) are hydrogen.
[F4] The method according to any one of [F1] to [F3], in which the reaction is performed in a reaction solution containing the α-keto acid represented by General Formula (2), the nitrogen source, the reducing agent, and glutamate/phenylalanine/leucine/valine dehydrogenase.
[F5] The method according to [F4], in which the reaction solution is a reaction solution into which the α-keto acid represented by General Formula (2), the nitrogen source, the reducing agent, and glutamate/phenylalanine/leucine/valine dehydrogenase are added.
[F6] The method according to [F5], in which the α-keto acid represented by General Formula (2) is added such that a concentration thereof in the reaction solution is 0.1 mM to 1 M and is preferably 1 mM to 300 mM.
[F7] The method according to [F5] or [F6], in which the nitrogen source is added such that a concentration thereof in the reaction solution is 0.1 mM to 1 M and is preferably 1 mM to 300 mM.
[F8] The method according to any one of [F5] to [F7], in which the reducing agent is added such that a concentration thereof in the reaction solution is 0.01 mM to 100 mM and is preferably 0.1 mM to 10 mM.
[F9] The method according to any one of [F5] to [F8], in which the glutamate/phenylalanine/leucine/valine dehydrogenase is added such that a concentration thereof in the reaction solution is 0.01 ug/mL to 1 mg/mL and is preferably 0.1 to 100 ug/mL.
[F10] The method according to any one of [F1] to [F19], in which the nitrogen source is an ammonium salt.
[F11] The method according to any one of [F1] to [F10], in which the nitrogen source is at least one ammonium salt selected from the group consisting of ammonium chloride, ammonium sulfate, and ammonium carbonate.
[F12] The method according to any one of [F1] to [F11], in which the reducing agent is at least one selected from the group consisting of NADH and NADPH.
[F13] The method according to any one of [F1] to [F12], in which the glutamate/phenylalanine/leucine/valine dehydrogenase is at least one dehydrogenase selected from the group consisting of glutamate dehydrogenase, phenylalanine dehydrogenase, leucine dehydrogenase, and valine dehydrogenase.
[F14] The method according to any one of [F1] to [F13], in which the glutamate/phenylalanine/leucine/valine dehydrogenase is selected from the group consisting of the following dehydrogenases:
   (d1) leucine dehydrogenase;
   (d2) phenylalanine dehydrogenase; and
   (d3) valine dehydrogenase.
[F15] The method according to any one of [F1] to [F14], in which the glutamate/phenylalanine/leucine/valine dehydrogenase is dehydrogenase selected from the group consisting of an leuDH protein, preferably, an leuDH protein derived from *Lysinibacillus sphaericus,* an leuDH protein derived from *Bacillus cereus,* or an leuDH protein derived from *Geobacillus stearothermophilus;* a phedh protein, preferably, a phedh protein derived from *Bacillus badius;* and a valdh protein, preferably, a valdh protein derived from *Streptomyces fradiae.*
[F16] The method according to any one of [F1] to [F15], in which the glutamate/phenylalanine/leucine/valine dehydrogenase is selected from the group consisting of the following proteins:
   (5'-1) protein that consists of an amino acid sequence set forth in SEQ ID NO: 30 and catalyzes a reductive amination reaction of an α-keto acid;
   (5'-2) protein that consists of an amino acid sequence in which one or a plurality of amino acids is deleted, substituted, or added in SEQ ID NO: 30, and has an activity of catalyzing a reductive amination reaction of an α-keto acid;
   (5'-3) protein that consists of an amino acid sequence set forth in SEQ ID NO: 32 and catalyzes a reductive amination reaction of an α-keto acid;
   (5'-4) protein that consists of an amino acid sequence in which one or a plurality of amino acids is deleted, substituted, or added in SEQ ID NO: 32, and has an activity of catalyzing a reductive amination reaction of an α-keto acid;
   (5'-5) protein that consists of an amino acid sequence set forth in SEQ ID NO: 34 and catalyzes a reductive amination reaction of an α-keto acid; and
   (5'-6) protein that consists of an amino acid sequence in which one or a plurality of amino acids is deleted, substituted, or added in SEQ ID NO: 34, and has an activity of catalyzing a reductive amination reaction of an α-keto acid.
[G1] A method for manufacturing α-keto acid, including steps of:
   reacting 2-amino-4-hydroxycarboxylic acid expressed by the following general formula (6):

      R₁₀₁C(R₁₀₂)(OH)CH₂CH(NH₂)COOH
   (wherein R₁₀₁ and R₁₀₂ are each independently hydrogen or an alkyl having 1 to 5 carbon atoms),
   and an oxidizing agent
   in the presence of glutamate/phenylalanine/leucine/valine dehydrogenase to produce an α-keto acid expressed by the following general formula (2):

      R₁₀₁C(R₁₀₂)(OH)CH₂COCOOH
   (wherein each of R₁₀₁ and R₁₀₂ in General Formula (2) above is the same group as R₁₀₁ and R₁₀₂ in General Formula (6) above).
[G2] The method according to [G1], in which R₁₀₂ in General Formulae (6) and (2) is hydrogen.
[G3] The method according to [G1] or [G2], in which R₁₀₁ and R₁₀₂ in General Formulae (6) and (2) are hydrogen.
[G4] The method according to any one of [G1] to [G3], in which the reaction is performed in a reaction solution containing the 2-amino-4-hydroxycarboxylic acid represented by General Formula (6), the oxidizing agent, and glutamate/phenylalanine/leucine/valine dehydrogenase.
[G5] The method according to [G4], in which the reaction solution is a reaction solution into which the 2-amino-4-hydroxycarboxylic acid represented by General Formula (6), the oxidizing agent, and the glutamate/phenylalanine/leucine/valine dehydrogenase are added.
[G6] The method according to [G5], in which the 2-amino-4-hydroxycarboxylic acid represented by General Formula (6) is added such that a concentration thereof in the reaction solution is 0.1 mM to 1 M and is preferably 1 mM to 300 mM.
[G7] The method according to [G51] or [G6], in which the oxidizing agent is added such that a concentration thereof in the reaction solution is 0.01 mM to 100 mM and is preferably 0.1 mM to 10 mM.
[G8] The method according to any one of [G5] to [G7], in which the glutamate/phenylalanine/leucine/valine dehydrogenase is added such that a concentration thereof in the reaction solution is 0.01 ug/mL to 1 mg/mL and is preferably 0.1 to 100 ug/mL.
[G9] The method according to any one of [G1] to [G8], in which the oxidizing agent is at least one selected from the group consisting of NAD⁺ and NADP⁺.
[G10] The method according to any one of [G1] to [G9], in which the glutamate/phenylalanine/leucine/valine dehydrogenase is at least one dehydrogenase selected from the group consisting of glutamate dehydrogenase, phenylalanine dehydrogenase, leucine dehydrogenase, and valine dehydrogenase.
[G11] The method according to any one of [G1] to [G10], in which the glutamate/phenylalanine/leucine/valine dehydrogenase is selected from the group consisting of the following dehydrogenases:
   (d1) leucine dehydrogenase;
   (d2) phenylalanine dehydrogenase; and
   (d3) valine dehydrogenase.
[G12] The method according to any one of [G1] to [G11], in which the glutamate/phenylalanine/leucine/valine dehydrogenase is dehydrogenase selected from the group consisting of an leuDH protein, preferably, an leuDH protein derived from Lysinibacillus sphaericus, an leuDH protein derived from Bacillus cereus, or an leuDH protein derived from Geobacillus stearothermophilus; a phedh protein, preferably, a phedh protein derived from Bacillus badius; and a valdh protein, preferably, a valdh protein derived from Streptomyces fradiae.
[G13] The method according to any one of [G1] to [G12], in which the glutamate/phenylalanine/leucine/valine dehydrogenase is selected from the group consisting of the following proteins:
   (5'-1) protein that consists of an amino acid sequence set forth in SEQ ID NO: 30 and catalyzes a reductive amination reaction of an α-keto acid;
   (5'-2) protein that consists of an amino acid sequence in which one or a plurality of amino acids is deleted, substituted, or added in SEQ ID NO: 30, and has an activity of catalyzing a reductive amination reaction of an α-keto acid;
   (5'-3) protein that consists of an amino acid sequence set forth in SEQ ID NO: 32 and catalyzes a reductive amination reaction of an α-keto acid;
   (5'-4) protein that consists of an amino acid sequence in which one or a plurality of amino acids is deleted, substituted, or added in SEQ ID NO: 32, and has an activity of catalyzing a reductive amination reaction of an α-keto acid;
   (5'-5) protein that consists of an amino acid sequence set forth in SEQ ID NO: 34 and catalyzes a reductive amination reaction of an α-keto acid; and
   (5'-6) protein that consists of an amino acid sequence in which one or a plurality of amino acids is deleted, substituted, or added in SEQ ID NO: 34, and has an activity of catalyzing a reductive amination reaction of an α-keto acid.
[H1] A coryneform bacterium comprising a first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
[H2] A coryneform bacterium comprising a first gene selected from the group consisting of:
   (1-1) a gene encoding a protein that consists of an amino acid sequence set forth in SEQ ID NO: 2 and catalyzes an aldol reaction between pyruvic acid and a carbonyl compound;
   (1-2) a gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound;
   (1-3) a gene encoding a protein that consists of an amino acid sequence set forth in SEQ ID NO: 4 and catalyzes an aldol reaction between pyruvic acid and a carbonyl compound;
   (1-4) a gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound;
   (1-5) a gene encoding a protein that consists of an amino acid sequence set forth in SEQ ID NO: 6 and catalyzes an aldol reaction between pyruvic acid and a carbonyl compound; and
   (1-6) a gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound;
   (1-7) a gene encoding a protein that consists of an amino acid sequence set forth in SEQ ID NO: 8 and catalyzes an aldol reaction between pyruvic acid and a carbonyl compound; and
   (1-8) a gene encoding a protein that consists of an amino acid sequence in which one or a plurality of amino acids is deleted, substituted, or added in SEQ ID NO: 8, and has an activity of catalyzing an aldol reaction between pyruvic acid and a carbonyl compound.
   (1-9) a gene encoding a protein that consists of an amino acid sequence set forth in SEQ ID NO: 10 and catalyzes an aldol reaction between pyruvic acid and a carbonyl compound;
   (1-10) a gene encoding a protein that consists of an amino acid sequence in which one or a plurality of amino acids is deleted, substituted, or added in SEQ ID NO: 10, and has an activity of catalyzing an aldol reaction between pyruvic acid and a carbonyl compound.
   (1-11) a gene encoding a protein that consists of an amino acid sequence set forth in SEQ ID NO: 12 and catalyzes an aldol reaction between pyruvic acid and a carbonyl compound;
   (1-12) a gene encoding a protein that consists of an amino acid sequence in which one or a plurality of amino acids is deleted, substituted, or added in SEQ ID NO: 12, and has an activity of catalyzing an aldol reaction between pyruvic acid and a carbonyl compound;
   (1-13) a gene encoding a protein that consists of an amino acid sequence set forth in SEQ ID NO: 14 and catalyzes an aldol reaction between pyruvic acid and a carbonyl compound;
   (1-14) a gene encoding a protein that consists of an amino acid sequence in which one or a plurality of amino acids is deleted, substituted, or added in SEQ ID NO: 14, and has an activity of catalyzing an aldol reaction between pyruvic acid and a carbonyl compound;
   (1-15) a gene encoding a protein that consists of an amino acid sequence set forth in SEQ ID NO: 16 and catalyzes an aldol reaction between pyruvic acid and a carbonyl compound;
   (1-16) a gene encoding a protein that consists of an amino acid sequence in which one or a plurality of amino acids is deleted, substituted, or added in SEQ ID NO: 16, and has an activity of catalyzing an aldol reaction between pyruvic acid and a carbonyl compound;
   (1-17) a gene encoding a protein that consists of an amino acid sequence set forth in SEQ ID NO: 18 and catalyzes an aldol reaction between pyruvic acid and a carbonyl compound;
   (1-18) a gene encoding a protein that consists of an amino acid sequence in which one or a plurality of amino acids is deleted, substituted, or added in SEQ ID NO: 18, and has an activity of catalyzing an aldol reaction between pyruvic acid and a carbonyl compound;
   (1-19) a gene encoding a protein that consists of an amino acid sequence set forth in SEQ ID NO: 20 and catalyzes an aldol reaction between pyruvic acid and a carbonyl compound;
   (1-20) a gene encoding a protein that consists of an amino acid sequence in which one or a plurality of amino acids is deleted, substituted, or added in SEQ ID NO: 20, and has an activity of catalyzing an aldol reaction between pyruvic acid and a carbonyl compound;
   (1-21) a gene encoding a protein that consists of an amino acid sequence set forth in SEQ ID NO: 22 and catalyzes an aldol reaction between pyruvic acid and a carbonyl compound; and
   (1-22) a gene encoding a protein that consists of an amino acid sequence in which one or a plurality of amino acids is deleted, substituted, or added in SEQ ID NO: 22, and has an activity of catalyzing an aldol reaction between pyruvic acid and a carbonyl compound.
[H3] The coryneform bacterium according to [H1] or [H2], in which the gene, which encodes the protein that consists of an amino acid sequence set forth in SEQ ID NO: 2 and catalyzes an aldol reaction between pyruvic acid and a carbonyl compound, is a gene consisting of a base sequence set forth in SEQ ID NO: 1.
[H4] The coryneform bacterium according to any one of [H1] to [H3], in which the gene, which encodes the protein that consists of an amino acid sequence set forth in SEQ ID NO: 4 and catalyzes an aldol reaction between pyruvic acid and a carbonyl compound, is a gene consisting of a base sequence set forth in SEQ ID NO: 3.
[H5] The coryneform bacterium according to any one of [H1] to [H4], in which the gene, which encodes the protein that consists of an amino acid sequence set forth in SEQ ID NO: 6 and catalyzes an aldol reaction between pyruvic acid and a carbonyl compound, is a gene consisting of a base sequence set forth in SEQ ID NO: 5.
[H6] The coryneform bacterium according to any one of [H1] to [H5], in which the gene, which encodes the protein that consists of an amino acid sequence set forth in SEQ ID NO: 8 and catalyzes an aldol reaction between pyruvic acid and a carbonyl compound, is a gene consisting of a base sequence set forth in SEQ ID NO: 7.
[H7] The coryneform bacterium according to any one of [H1] to [H6], in which the gene, which encodes the protein that consists of an amino acid sequence set forth in SEQ ID NO: 10 and catalyzes an aldol reaction between pyruvic acid and a carbonyl compound, is a gene consisting of a base sequence set forth in SEQ ID NO: 9.
[H8] The coryneform bacterium according to any one of [H1] to [H7], in which the gene, which encodes the protein that consists of an amino acid sequence set forth in SEQ ID NO: 12 and catalyzes an aldol reaction between pyruvic acid and a carbonyl compound, is a gene consisting of a base sequence set forth in SEQ ID NO: 11.
[H9] The coryneform bacterium according to any one of [H1] to [H8], in which the gene, which encodes the protein that consists of an amino acid sequence set forth in SEQ ID NO: 14 and catalyzes an aldol reaction between pyruvic acid and a carbonyl compound, is a gene consisting of a base sequence set forth in SEQ ID NO: 13.
[H10] The coryneform bacterium according to any one of [H1] to [H9], in which the gene, which encodes the protein that consists of an amino acid sequence set forth in SEQ ID NO: 16 and catalyzes an aldol reaction between pyruvic acid and a carbonyl compound, is a gene consisting of a base sequence set forth in SEQ ID NO: 15.
[H11] The coryneform bacterium according to any one of [H1] to [H10], in which the gene, which encodes the protein that consists of an amino acid sequence set forth in SEQ ID NO: 18 and catalyzes an aldol reaction between pyruvic acid and a carbonyl compound, is a gene consisting of a base sequence set forth in SEQ ID NO: 17.
[H12] The coryneform bacterium according to any one of [H1] to [H11], in which the gene, which encodes the protein that consists of an amino acid sequence set forth in SEQ ID NO: 20 and catalyzes an aldol reaction between pyruvic acid and a carbonyl compound, is a gene consisting of a base sequence set forth in SEQ ID NO: 19.
[H13] The coryneform bacterium according to any one of [H1] to [H12], in which the gene, which encodes the protein that consists of an amino acid sequence set forth in SEQ ID NO: 22 and catalyzes an aldol reaction between pyruvic acid and a carbonyl compound, is a gene consisting of a base sequence set forth in SEQ ID NO: 21.
[H14] The coryneform bacterium according to any one of [H1] to [H13], in which the coryneform bacterium is a bacterial species included in the genus *Corynebacterium,* is preferably *Corynebacterium glutamicum,* and is more preferably a coryneform bacterium with an accession number of NITE BP-02780, NITE BP-02781, NITE BP-02782, NITE BP-02783, or NITE BP-02784.
[H15] The method according to any one of [H1] to [H14], in which the coryneform bacterium is a coryneform bacterium transformed by the first gene.
[H16] The coryneform bacterium according to any one of [H1] to [H15], further including:
   a second gene selected from the group consisting of the following genes,
      (2-1) gene encoding a protein that consists of an amino acid sequence set forth in SEQ ID NO: 24 and catalyzes a decarboxylation reaction of an α-keto acid, and
      (2-2) gene encoding a protein that consists of an amino acid sequence in which one or a plurality of amino acids is deleted, substituted, or added in SEQ ID NO: 24, and has an activity of catalyzing a decarboxylation reaction of an α-keto acid; and
   a third gene selected from the group consisting of the following genes,
      (3-1) gene encoding a protein that consists of an amino acid sequence set forth in SEQ ID NO: 26 and catalyzes a reduction reaction of an aldehyde, and
      (3-2) gene encoding a protein that consists of an amino acid sequence in which one or a plurality of amino acids is deleted, substituted, or added in SEQ ID NO: 26, and has an activity of catalyzing a reduction reaction of an aldehyde.
[H17] The coryneform bacterium according to [H16], in which the gene, which encodes the protein that consists of an amino acid sequence set forth in SEQ ID NO: 24 and catalyzes a decarboxylation reaction of an α-keto acid, is a gene consisting of a base sequence set forth in SEQ ID NO: 23.
[H18] The coryneform bacterium according to [H16] or [H17], in which the gene, which encodes the protein that consists of an amino acid sequence set forth in SEQ ID NO: 26 and catalyzes a reduction reaction of aldehyde, is a gene consisting of a base sequence set forth in SEQ ID NO: 25.
[H19] The coryneform bacterium according to any one of [H16] to [H18], in which the coryneform bacterium is a bacterial species included in the genus *Corynebacterium,* is preferably *Corynebacterium glutamicum,* and is more preferably a coryneform bacterium with an accession number of NITE BP-02780 or NITE BP-02781.
[H20] The method according to any one of [H16] to [H19], in which the coryneform bacterium is a coryneform bacterium transformed by the first gene, the second gene, and the third gene.
[H21] The coryneform bacterium according to any one of [H1] to [H20], further including:
   a fourth gene selected from the group consisting of the following genes,
   (4-1) gene encoding a protein that consists of an amino acid sequence set forth in SEQ ID NO: 28 and catalyzes a reduction reaction of an α-keto acid, and
   (4-2) gene encoding a protein that consists of an amino acid sequence in which one or a plurality of amino acids is deleted, substituted, or added in SEQ ID NO: 28, and has an activity of catalyzing a reduction reaction of an α-keto acid.
[H21] The coryneform bacterium according to [H20], in which the gene, which encodes the protein that consists of an amino acid sequence set forth in SEQ ID NO: 28 and catalyzes a reduction reaction of an α-keto acid, is a gene consisting of a base sequence set forth in SEQ ID NO: 27.
[H22] The coryneform bacterium according to [H20] or [H21], in which the coryneform bacterium is a bacterial species included in the genus *Corynebacterium,* is preferably *Corynebacterium glutamicum,* and is more preferably a coryneform bacterium with an accession number of NITE BP-02782.
[H23] The method according to any one of [H20] to [H22], in which the coryneform bacterium is a coryneform bacterium transformed by the first gene and the fourth gene.
[H24] The coryneform bacterium according to any one of [H1] to [H23], further including:
   a fifth gene selected from the group consisting of the following genes,
   (5-1) gene encoding a protein that consists of an amino acid sequence set forth in SEQ ID NO: 30 and catalyzes a reductive amination reaction of an α-keto acid,
   (5-2) gene encoding a protein that consists of an amino acid sequence in which one or a plurality of amino acids is deleted, substituted, or added in SEQ ID NO: 30, and has an activity of catalyzing a reductive amination reaction of an α-keto acid,
   (5-3) gene encoding a protein that consists of an amino acid sequence set forth in SEQ ID NO: 32 and catalyzes a reductive amination reaction of an α-keto acid,
   (5-4) gene encoding a protein that consists of an amino acid sequence in which one or a plurality of amino acids is deleted, substituted, or added in SEQ ID NO: 32, and has an activity of catalyzing a reductive amination reaction of an α-keto acid,
   (5-5) gene encoding a protein that consists of an amino acid sequence set forth in SEQ ID NO: 34 and catalyzes a reductive amination reaction of an α-keto acid, and
   (5-6) gene encoding a protein that consists of an amino acid sequence in which one or a plurality of amino acids is deleted, substituted, or added in SEQ ID NO: 34, and has an activity of catalyzing a reductive amination reaction of an α-keto acid.
[H25] The coryneform bacterium according to [H24], in which the gene, which encodes the protein that consists of an amino acid sequence set forth in SEQ ID NO: 30 and catalyzes a reductive amination reaction of an α-keto acid, is a gene consisting of a base sequence set forth in SEQ ID NO: 29.
[H26] The coryneform bacterium according to [H24] or [H25], in which the gene, which encodes the protein that consists of an amino acid sequence set forth in SEQ ID NO: 32 and catalyzes a reductive amination reaction of an α-keto acid, is a gene consisting of a base sequence set forth in SEQ ID NO: 31.
[H27] The coryneform bacterium according to any one of [H24] to [H26], in which the gene, which encodes the protein that consists of an amino acid sequence set forth in SEQ ID NO: 34 and catalyzes a reductive amination reaction of an α-keto acid, is a gene consisting of a base sequence set forth in SEQ ID NO: 33.
[H28] The coryneform bacterium according to any one of [H24] to [H27], in which the coryneform bacterium is a bacterial species included in the genus *Corynebacterium,* is preferably *Corynebacterium glutamicum,* and is more preferably a coryneform bacterium with an accession number of NITE BP-02783.
[H29] The method according to any one of [H24] to [H28], in which the coryneform bacterium is a coryneform bacterium transformed by the first gene and the fifth gene.
[H30] The coryneform bacterium according to any one of [H1] to [H29], further including:
   a fifth gene selected from the group consisting of the following genes,
      (5-1) gene encoding a protein that consists of an amino acid sequence set forth in SEQ ID NO: 30 and catalyzes a reductive amination reaction of an α-keto acid,
      (5-2) gene encoding a protein that consists of an amino acid sequence in which one or a plurality of amino acids is deleted, substituted, or added in SEQ ID NO: 30, and has an activity of catalyzing a reductive amination reaction of an α-keto acid,
      (5-3) gene encoding a protein that consists of an amino acid sequence set forth in SEQ ID NO: 32 and catalyzes a reductive amination reaction of an α-keto acid,
      (5-4) gene encoding a protein that consists of an amino acid sequence in which one or a plurality of amino acids is deleted, substituted, or added in SEQ ID NO: 32, and has an activity of catalyzing a reductive amination reaction of an α-keto acid,
      (5-5) gene encoding a protein that consists of an amino acid sequence set forth in SEQ ID NO: 34 and catalyzes a reductive amination reaction of an α-keto acid, and
      (5-6) gene encoding a protein that consists of an amino acid sequence in which one or a plurality of amino acids is deleted, substituted, or added in SEQ ID NO: 34, and has an activity of catalyzing a reductive amination reaction of an α-keto acid; and
   a sixth gene selected from the group consisting of the following genes,
      (6-1) gene encoding a protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan, and
      (6-2) gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan.
[H31] The coryneform bacterium according to [H30], in which the gene, which encodes the protein that consists of an amino acid sequence set forth in SEQ ID NO: 30 and catalyzes a reductive amination reaction of an α-keto acid, is a gene consisting of a base sequence set forth in SEQ ID NO: 29.
[H32] The coryneform bacterium according to [H30] or [H31], in which the gene, which encodes the protein that consists of an amino acid sequence set forth in SEQ ID NO: 32 and catalyzes a reductive amination reaction of an α-keto acid, is a gene consisting of a base sequence set forth in SEQ ID NO: 31.
[H33] The coryneform bacterium according to any one of [H30] to [H32], in which the gene, which encodes the protein that consists of an amino acid sequence set forth in SEQ ID NO: 34 and catalyzes a reductive amination reaction of an α-keto acid, is a gene consisting of a base sequence set forth in SEQ ID NO: 33.
[H34] The coryneform bacterium according to any one of [H30] to [H33], in which the gene, which encodes the protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan, is a gene consisting of a base sequence set forth in SEQ ID NO: 35.
[H35] The coryneform bacterium according to any one of [H30] to [H34], in which the coryneform bacterium is a bacterial species included in the genus *Corynebacterium,* is preferably *Corynebacterium glutamicum,* and is more preferably a coryneform bacterium with an accession number of NITE BP-02784.
[H36] The method according to any one of [H30] to [H35], in which the coryneform bacterium is a coryneform bacterium transformed by the first gene and the fifth gene.

### [Examples]

### 1. Materials and methods

### (1-1) Medium

Reagents were purchased from FUJIFILM Wako Pure Chemical Corporation unless otherwise indicated.

### (A medium)

Urea 2.0 g, ammonium sulfate 7.0 g, potassium dihydrogen phosphate 0.50 g, dipotassium hydrogen phosphate 0.50 g, magnesium sulfate heptahydrate 0.50 g, Yeast Extract (manufactured by Difco) 2.0 g, Casamino Acids Vitamin Assay (manufactured by Difco) 7.0 g, iron(II) sulfate heptahydrate 6.0 mg, manganese(II) sulfate-hydrate 4.2 mg, D (+)-biotin 0.20 mg, and thiamine hydrochloride 0.20 mg were dissolved in 0.92 L of water, and a pH was adjusted to 7.0 using a 5 M aqueous potassium hydroxide solution. The mixture was autoclave-sterilized at 121°C for 20 minutes and cooled to room temperature. Thereafter, 80 mL of a 50% (w/v) autoclave-sterilized aqueous glucose solution was added thereto, and thereby a liquid medium was prepared.

### (A agar medium)

Urea 2.0 g, ammonium sulfate 7.0 g, potassium dihydrogen phosphate 0.50 g, dipotassium hydrogen phosphate 0.50 g, magnesium sulfate heptahydrate 0.50 g, Yeast Extract (manufactured by Difco) 2.0 g, Casamino Acids Vitamin Assay (manufactured by Difco) 7.0 g, iron(II) sulfate heptahydrate 6.0 mg, manganese(II) sulfate-hydrate 4.2 mg, D (+)-biotin 0.20 mg, and thiamine hydrochloride 0.20 mg were dissolved and suspend in 0.92 L of water, and a pH was adjusted to 7.0 using a 5 M aqueous potassium hydroxide solution. The mixture was autoclave-sterilized at 121°C for 20 minutes and cooled to 50°C. Thereafter, 80 mL of a 50% (w/v) autoclave-sterilized aqueous glucose solution was added thereto. The mixture was dispensed to a Petri dish, and then cooled and solidified, and thereby a solid medium was prepared.

### (BA medium)

Urea 2.0 g, ammonium sulfate 7.0 g, potassium dihydrogen phosphate 0.50 g, dipotassium hydrogen phosphate 0.50 g, magnesium sulfate heptahydrate 0.50 g, Yeast Extract (manufactured by Difco) 2.0 g, Casamino Acids Vitamin Assay (manufactured by Difco) 7.0 g, iron(II) sulfate heptahydrate 6.0 mg, manganese(II) sulfate-hydrate 4.2 mg, D (+)-biotin 0.20 mg, thiamine hydrochloride 0.20 mg, and 4-morpholinopropanesulfonic acid 21 g were dissolved in 0.92 L of water, and a pH was adjusted to 7.0 using a 5 M aqueous potassium hydroxide solution. The mixture was autoclave-sterilized at 121°C for 20 minutes and cooled to room temperature. Thereafter, 80 mL of a 50% (w/v) autoclave-sterilized aqueous glucose solution was added thereto, and thereby a liquid medium was prepared.

### (LB medium)

Bacto Tryptone (manufactured by Difco) 10 g, Yeast Extract (manufactured by Difco) 5.0 g, and sodium chloride 10 g were dissolved in 1 L of water, and a pH was adjusted to 7.0 using a 5 M aqueous sodium hydroxide solution. Thereafter, autoclave sterilization was performed at 121°C for 20 minutes, and thereby a liquid medium was prepared.

### (LB agar medium)

Bacto Tryptone (manufactured by Difco) 10 g, Yeast Extract (manufactured by Difco) 5.0 g, sodium chloride 10 g, and agar 15 g were dissolved and suspended in 1 L of water, and a pH was adjusted to 7.0 using a 5 M aqueous sodium hydroxide solution. Thereafter, the mixture was autoclave-sterilized at 121°C for 20 minutes and cooled to 50°C. Thereafter, the mixture was dispensed to a Petri dish, and then cooled and solidified, and thereby a solid medium was prepared.

### (Suc agar medium)

Bacto Tryptone (manufactured by Difco) 10 g, Yeast Extract (manufactured by Difco) 5.0 g, sodium chloride 10 g, and Agar 15 g were dissolved and suspended in 0.5 L of water, and a pH was adjusted to 7.0 using a 5 M aqueous sodium hydroxide solution. The mixture was autoclave-sterilized at 121°C for 20 minutes and cooled to 50°C. Thereafter, 0.5 L of a 20% (w/v) autoclave-sterilized aqueous sucrose solution was added thereto. The mixture was dispensed to a Petri dish, and then cooled and solidified, and thereby a solid medium was prepared.

### (BHIS medium)

Brain Heart Infusion (manufactured by Difco) 36 g and sorbitol 91 g were dissolved in 1 L of water, and a pH was adjusted to 7.0 using a 5 M aqueous sodium hydroxide solution. Thereafter, autoclave sterilization was performed at 121°C for 20 minutes, and thereby a liquid medium was prepared.

### (BHIS-GIT medium)

Brain Heart Infusion (manufactured by Difco) 36 g, glycine 8.0 g, isoniazid 1.3 g, Tween 80 3 mL, and sorbitol 91 g were dissolved in 1 L of water, and a pH was adjusted to 7.0 using a 5 M aqueous sodium hydroxide solution. Thereafter, autoclave sterilization was performed at 121°C for 20 minutes, and thereby a liquid medium was prepared.

### (1-2) Buffers and reagents

### (TE buffer)

Trishydroxymethylaminomethane 1.2 g and ethylenediamine-N,N,N',N'-tetraacetic acid disodium salt dihydrate 0.37 g were dissolved in 1 L of water, and a pH was adjusted to 8.0 using 6M hydrochloric acid. Thereafter, autoclave sterilization was performed at 121°C for 20 minutes, and thereby a buffer solution was obtained.

### (TG buffer)

Trishydroxymethylaminomethane 0.12 g and glycerol 10 g were dissolved in 1 L of water, and a pH was adjusted to 7.5 using 6M hydrochloric acid. Thereafter, autoclave sterilization was performed at 121°C for 20 minutes, and thereby a buffer solution was prepared.

### (10% Glycerol aqueous solution)

100 g of glycerol was dissolved in 1 L of water, and then autoclave-sterilized at 121°C for 20 minutes. Thereby, an aqueous solution was prepared.

### (BR buffer)

Potassium dihydrogen phosphate 0.50 g, dipotassium hydrogen phosphate 0.50 g, magnesium sulfate heptahydrate 0.50 g, iron(II) sulfate heptahydrate 6.0 mg, and manganese(II) sulfate-hydrate 4.2 mg were dissolved in 1.0 L of water, and a pH was adjusted to 7.0 using a 5 M aqueous potassium hydroxide solution. Thereby, a buffer solution was obtained.

### (BS Buffer)

Potassium dihydrogen phosphate 0.50 g, dipotassium hydrogen phosphate 0.50 g, magnesium sulfate heptahydrate 0.50 g, iron(II) sulfate heptahydrate 6.0 mg, manganese(II) sulfate-hydrate 4.2 mg and 4-morpholinopropanesulfonic acid 21g were dissolved in 1.0 L of water, and pH was adjusted to 7.0 using a 5 M aqueous potassium hydroxide solution to obtain BS Buffer solution.

### (BT Buffer)

Ammonium sulfate 7.0 g, potassium dihydrogen phosphate 0.50 g, dipotassium hydrogen phosphate 0.50 g, magnesium sulfate heptahydrate 0.50 g, iron(II) sulfate heptahydrate 6.0 mg and manganese(II) sulfate-hydrate 4.2 mg were dissolved in 1.0 L of water, and pH was adjusted to 7.0 using a 5 M aqueous potassium hydroxide solution to obtain BT buffer solution.

### (1-3) Definition of analysis conditions and experimental method

### (Extraction and purification of DNA fragments after agarose gel electrophoresis)

DNA fragments were extracted and purified using NucleoSpin (registered trademark) Gel and PCR Clean-up (manufactured by MACHEREY-NAGEL GmbH & Co. KG) according to the attached instructions.

### (Plasmid extraction)

Plasmid extraction and purification were performed using NucleoSpin (registered trademark) Plasmid EasyPure (manufactured by MACHEREY-NAGEL GmbH & Co. KG) according to the attached instructions.

### (PCR method)

PCR was performed according to the attached instructions using any of the following DNA polymerases and reagents attached thereto: PrimeSTAR (registered trademark) HS DNA Polymerase (manufactured by Takara Bio Inc.), PrimeSTAR (registered trademark) Max DNA Polymerase (manufactured by Takara Bio Inc.), and Tks Gflex™ DNA Polymerase (manufactured by Takara Bio Inc.).

### (Liquid chromatography for organic acid analysis)

An organic acid analysis system which is manufactured by Shimadzu Corporation and is configured of the following devices was used. The devices are: a System controller: CBM-20A, a liquid feeding unit: LC-20AB, an online degassing unit: DGU-20A3R, an autoinjector: SIL-20AC, a column oven: CTO-20AC, and a photodiode array detector: SPD-M20A.

Analysis conditions are as follows.
Guard column: TSKgel OApak-P (manufactured by Tosoh Corporation)
Analysis column: TSKgel OApak-A (manufactured by Tosoh Corporation)
Column oven temperature: 40°C
Mobile phase: 0.75 mM sulfuric acid
Flow rate: 1.0 mL/min

### (Liquid chromatography for sugar analysis)

A sugar analysis system which is manufactured by Shimadzu Corporation and configured of the following devices was used. The devices are: a System controller: CBM-20A, a liquid feeding unit: LC-20AB, an online degassing unit: DGU-20A3R, an autoinjector: SIL-20AC, a column oven: CTO-20AC, a refractive index detector: RID-10A.

Analysis conditions are as follows.
Pretreatment: Desalting Cartridge (manufactured by Bio-Rad Laboratories)
Analysis column: Aminex HPX-87P (manufactured by Bio-Rad Laboratories)
Column oven temperature: 85°C
Mobile phase: water
Flow rate: 0.6 mL/min.

### (Liquid chromatography for analysis of amino acid)

An amino acid analysis system configured of the following instruments and manufactured by Shimadzu Corporation was used. System controller: CBM-20A, liquid transfer unit: one unit of LC-20AB and two units of LC-20AD, flow path switching valve: FCV-11AL, online degassing unit: DGU-20A3R, auto injector: SIL-20AC, column oven: CTO-20AC, and fluorescence detector: RF-20AXs.

The analysis was performed using an ammonia trap column ISC-30/S 0504 NA (manufactured by Shimadzu Corporation) and an analytical column Shim-pack AMINO-NA (manufactured by Shimadzu Corporation) according to Na-type rapid conditions described in the Shimadzu High-Performance Liquid Chromatography Prominence Amino Acid Analysis System Instruction Manual as analytical conditions.

### (Gas Chromatography Mass Spectrometry (GCMS))

GCMS was performed using a gas chromatogram quadrupole mass spectrometer (7890B GC/5977A MSD, manufactured by Agilent Technologies, Inc.) and a multifunctional autosampler (MPS2-xt, manufactured by GERSTEL K.K.) under the following conditions.

### <Heat desorption sampling conditions>

Heating conditions: 50°C (0.5 min) → 50°C/min → 300°C (10 min)
Trap temperature: -100°C → 12°C/s → 300°C

### <Gas chromatograph conditions>

Separation column: DB-5ht [30 m x 0.25 mm ID, 0.10 µm, manufactured by Agilent Technologies, Inc.]
Temperature conditions: 40°C (5 min) → 10°C/min → 320°C (12 min)

### <Mass spectrometry conditions>

Ionization method: Electron Ionization (EI)
Measurement mass range: m/z = 29-550

### (Genomic DNA extraction)

The microorganisms were liquid-cultured according to the attached instructions of the source were recovered by centrifugation and frozen at -80°C. Bacterial cell pellets were thawed at room temperature, 537 µL of a 10 mg/ml lysozyme aqueous solution was added thereto, and the mixture was shaken at 37°C for 1 hour. Next, 30 µL of a 0.5 M EDTA aqueous solution, 30 µL of a 10% SDS aqueous solution, and 3 µL of a 20 mg/ml proteinase K aqueous solution were added, and the mixture was shaken at 37°C for 1 hour. Thereafter, 100 µL of a 5M sodium chloride aqueous solution and 80 µL of a 10% hexadecyltrimethylammonium bromide aqueous solution (in 0.7M sodium chloride) were added, and the mixture was heated at 65°C for 10 minutes. After cooling to room temperature, 780 µL of chloroform-isoamyl alcohol (24:1) was added and gently mixed. Centrifugation was performed at 15000 × g and 4°C for 5 minutes to recover 600 µL of an aqueous layer. 600 µL of phenol-chloroform-isoamyl alcohol (25:24:1) was added thereto and gently mixed. Centrifugation was performed at 15000 × g and 4°C for 5 minutes to recover 500 µL of an aqueous layer. 300 µL of isopropanol was added thereto and centrifuged at 15000 × g and 4°C for 5 minutes, and then the supernatant was removed. Genomic DNA obtained as a precipitate was washed with 500 µL of 70% ethanol and centrifuged at 15000 × g and 4°C for 5 minutes. This washing was repeated twice, drying was performed at room temperature, and then the obtained genomic DNA was dissolved in 100 µL of TE buffer.

### (Electroporation for transforming strain derived from Corynebacterium glutamicum ATCC13032)

A glycerol stock of a strain derived from Corynebacterium glutamicum ATCC13032 to be transformed was inoculated into the A agar medium under aseptic conditions and cultured at 33°C. Bacterial cells grown on the agar medium were inoculated into 10 mL of the A medium and shaking-cultured at 33°C, and thereby a seed culture solution was obtained. This seed culture solution was inoculated into 100 mL of the BHIS medium prepared in a 500 mL flask so that a turbidity was 0.2. The mixture was shaken at 33°C and cultured until a turbidity reached 0.5 to 0.7. This culture solution was centrifuged at 4°C and 5500 × g for 20 minutes, and the supernatant was removed. The recovered bacterial cells were washed with 10 mL of TG buffer, centrifuged at 4°C and 5500 x g for 5 minutes, and the supernatant was removed. This washing was performed twice. Thereafter, the cells were washed with 10 mL of a 10% glycerol aqueous solution and centrifuged at 4°C and 5500 × g for 5 minutes, and the supernatant was removed. The obtained bacterial cell pellets were suspended in 1 mL of a 10% glycerol aqueous solution, dispensed to a sterilized 1.5 mL tube by 150 µL, frozen using liquid nitrogen, and stored at -80°C as competent cells.

In a case of gene deletion and gene replacement on genomic DNA, 150 µL of competent cells were thawed on ice, and 500 ng of plasmid was added thereto. These cells were transferred to a 0.2 cm-gap sterile electroporation cuvette (manufactured by Bio-Rad), and electroporation was performed at 25 µF, 2000, 2500 V using a Gene Pulser Xcell™ electroporation system (manufactured by Bio-Rad). The bacterial cell suspension was diluted with 1 mL of the BHIS medium, incubated at 46°C for 6 minutes, and then incubated at 33°C for 1 hour. This bacterial cell suspension was inoculated into an A agar medium containing an appropriate antibiotic substance, and a desired transformant was selected.

In a case where a gene overexpression plasmid was introduced, 50 µL of competent cells was thawed on ice, and 200 ng of plasmid was added thereto. These cells were transferred to a 0.2 cm-gap sterile electroporation cuvette (manufactured by Bio-Rad), and electroporation was performed at 25 µF, 2000, 2500 V using a Gene Pulser Xcell™ electroporation system (manufactured by Bio-Rad). The bacterial cell suspension was diluted with 1 mL of the BHIS medium, incubated at 46°C for 6 minutes, and then incubated at 33°C for 1 hour. This bacterial cell suspension was inoculated into an A agar medium containing an appropriate antibiotic substance, and a desired transformant was selected.

### (Colony PCR)

Reagents other than a template DNA were mixed according to the attached instructions of TaKaRa Ex Taq (registered trademark) (manufactured by Takara Bio Inc.) using a combination of primers capable of amplifying a target DNA sequence. A small amount of a colony of a bacterial strain produced in the agar medium was suspended in this mixture, and using DNA present therein as a template, PCR was performed according to the attached instructions. PCR products were analyzed by agarose gel electrophoresis, and it was confirmed that a target plasmid was retained, or that the corresponding DNA sequence on the genome was deleted or substituted.

### (Turbidity measurement)

A turbidity of the culture solution of Escherichia coli and Corynebacterium glutamicum was measured at a wavelength of 610 nm using UItrospec 8000 (manufactured by GE Healthcare).

### (1-4) Details of primer DNAs, bacterial cells, and plasmids

Details of primer DNAs, bacterial cells, and plasmids are shown in Tables 37 to 67 below.

| Plasmid ID | Target gene | Target vector | | Template | DNA ID | DNA sequences |
|---|---|---|---|---|---|---|
| pGE015 | sacB | pHSG299 | F | pNIC28-Bsa4 | GEP007 | GGGGAAGCTTGACGTCCACATATACCTG CC (SEQ ID NO: 37) |
| | | | R | | GEP008 | ATTCGGATCCGTATCCACCTTTAC (SEQ ID NO: 38) |

**[Table 38]**

| DNA ID | DNA sequences |
|---|---|
| GEP808 | TGAACAGATACCATTTGCCGTTCATT (SEQ ID NO: 39) |
| GEP809 | AATGGTATCTGTTCACTGACTCCCGC (SEQ ID NO: 40) |

**[Table 39]**

| Plasmid ID | Deletion target | Target vector | | Template | DNA ID | DNA sequences |
|---|---|---|---|---|---|---|
| pGE020 | ppc | pGE015 (EcoRI) | Up-F | ATCC1303 2 genome | GEP132 | CCATGATTACGAATTCGGGAAACTTTTT TAAGAAA (SEQ ID NO: 41) |
| | | | Up-R | ATCC1303 2 genome | GEP133 | TAACTACTTTAAACACTCTT (SEQ ID NO: 42) |
| | | | Down-F | ATCC1303 2 genome | GEP134 | TGTTTAAAGTAGTTATCCAGCCGGCTGG GTAGTAC (SEQ ID NO: 43) |
| | | | Down-R | ATCC1303 2 genome | GEP135 | TACCGAGCTCGAATTGAAGTATTCAAGG GGATTTC (SEQ ID NO: 44) |
| pGE033 | ldhA | pGE015 (EcoRI) | Up-F | ATCC1303 2 genome | GEP169 | GACGGCCAGTGAATTTTTCATACGACCA CGGGCTA (SEQ ID NO: 45) |
| | | | Up-R | ATCC1303 2 genome | GEP170 | GACAATCTTGTTACCGACGG (SEQ ID NO: 46) |
| | | | Down-F | ATCC1303 2 genome | GEP215 | GGTAACAAGATTGTCCATTCCGCAAATA CCCTGCG (SEQ ID NO: 47) |
| | | | Down-R | ATCC1303 2 genome | GEP216 | TACCGAGCTCGAATTGGGACGTTGATGA CGCTGCC (SEQ ID NO: 48) |
| pGE177 | pyc | pGE015 (EcoRI) | Up-F | ATCC1303 2 genome | GEP615 | GACGGCCAGTGAATTGTTGATGACTGTTGGTT CCA (SEQ ID NO: 49) |
| | | | Up-R | ATCC1303 2 genome | GEP616 | CTCGAGTAGAGTAATTATTCCTTTCA (SEQ ID NO: 50) |
| | | | Down-F | ATCC1303 2 genome | GEP617 | ATTACTCTACTCGAGACCTTTCTGTAAAAAGC CCC (SEQ ID NO: 51) |
| | | | Down-R | ATCC1303 2 genome | GEP618 | TACCGAGCTCGAATTCACGATCCGCCGGGCAG CCT (SEQ ID NO: 52) |
| pGE191 | poxB | pGE015 (EcoRI) | Up-F | ATCC1303 2 genome | GEP406 | GACGGCCAGTGAAAACGTTAATGAGGAAAAC CG (SEQ ID NO: 53) |
| | | | Up-R | ATCC1303 2 genome | GEP407 | AATTAATTGTTCTGCGTAGC (SEQ ID NO: 54) |
| | | | Down-F | ATCC1303 2 genome | GEP698 | GCAGAACAATTAATTCTCGAGTCGAACATAAG GAATATTCC (SEQ ID NO: 55) |
| | | | Down-R | ATCC1303 2 genome | GEP409 | TACCGAGCTCGAATTTTCCAGGTACGGAAAGT GCC (SEQ ID NO: 56) |

**[Table 41]**

| Plasmid ID | Deletion target | Target vector | | Template | DNA ID | DNA sequences |
|---|---|---|---|---|---|---|
| pGE210 | alaA | pGE015 (EcoRI) | Up-F | ATCC1303 2 genome | GEP810 | GACGGCCAGTGAATTTTTCACCGAAGTGACAC CTA (SEQ ID NO: 57) |
| | | | Up-R | ATCC1303 2 genome | GEP811 | CTCGAGCCGCTCAATGTTGCCACTTT (SEQ ID NO: 58) |
| | | | Down-F | ATCC1303 2 genome | GEP812 | ATTGAGCGGCTCGAGTAGTTGTTAGGATTCAC CAC (SEQ ID NO: 59) |
| | | | Down-R | ATCC1303 2 genome | GEP813 | TACCGAGCTCGAATTGTTCCCTGGAAATTGTT TGC (SEQ ID NO: 60) |
| pGE228 | ilvB | pGE015 (EcoRI) | Up-F | ATCC1303 2 genome | GEP956 | GACGGCCAGTGAATTACCGCACGCGACCAGTT ATT (SEQ ID NO: 61) |
| | | | Up-R | ATCC1303 2 genome | GEP957 | GCTAGCGACTTTCTGGCTCCTTTACT (SEQ ID NO: 62) |
| | | | Down-F | ATCC1303 2 genome | GEP958 | CAGAAAGTCGCTAGCGGAGAGACCCAAGATG GCTA (SEQ ID NO: 63) |
| | | | Down-R | ATCC1303 2 genome | GEP959 | TACCGAGCTCGAATTCTCGATGTCGTTGGTGA AGA (SEQ ID NO: 64) |

**[Table 42]**

| Plasmid ID | Deletion target | Target vector | | Template | DNA ID | DNA sequences |
|---|---|---|---|---|---|---|
| pGE253 | cg0931 | pGE015 (EcoRI) | Up-F | ATCC1303 2 genome | GEP1132 | GACGGCCAGTGAATTAGCAGGGACGAAAGTC GGGA (SEQ ID NO: 65) |
| | | | Up-R | ATCC1303 2 genome | GEP1133 | CTCGAGGCAGCTACTATATTTGATCC (SEQ ID NO: 66) |
| | | | Down-F | ATCC1303 2 genome | GEP1134 | AGTAGCTGCCTCGAGTTTGAACAGGTTGTTGG GGG (SEQ ID NO: 67) |
| | | | Down-R | ATCC1303 2 genome | GEP1135 | TACCGAGCTCGAATTATCGACGGCAAAACGCC CAA (SEQ ID NO: 68) |
| pGE1171 | adhE | pGE209 (KpnI,BamHI) | Up-F | ATCC1303 2 genome | GEP2606 | GTGAATTCGAGCTCGCAGAAGCAGATCTTGCA ATC (SEQ ID NO: 69) |
| | | | Up-R | ATCC1303 2 genome | GEP2607 | CTTCCTCGAGAATTCCAGGCACTACAGTGCTC (SEQ ID NO: 70) |
| | | | Down-F | ATCC1303 2 genome | GEP2608 | GAATTCTCGAGGAAGAGGCTTTCAACACCATG (SEQ ID NO: 71) |
| | | | Down-R | ATCC1303 2 genome | GEP2609 | AGGTGGATACGGATCGGAACAAAGTGTCTCC AGAG (SEQ ID NO: 72) |

**[Table 43]**

| Plasmid ID | Deletion target | Target vector | | Template | DNA ID | DNA sequences |
|---|---|---|---|---|---|---|
| pGE1218 | adhE | pGE209 (KpnI,BamHI) | Up-F | ATCC1303 2 genome | GEP2688 | GTGAATTCGAGCTCGATTCAGGATTTGCTTCG CGACG (SEQ ID NO: 73) |
| | | | Up-R | ATCC1303 2 genome | GEP2607 | CTTCCTCGAGAATTCCAGGCACTACAGTGCTC (SEQ ID NO: 70) |
| | | | Down-F | ATCC1303 2 genome | GEP2608 | GAATTCTCGAGGAAGAGGCTTTCAACACCATG (SEQ ID NO: 71) |
| | | | Down-R | ATCC1303 2 genome | GEP2689 | AGGTGGATACGGATCACAGTGTCCGGATCATC TGCTG (SEQ ID NO: 74) |
| pGE1172 | ald | pGE209 (KpnI,BamHI) | Up-F | ATCC1303 2 genome | GEP2610 | GTGAATTCGAGCTCGCCGAAACCTCAAAGAAT CCC (SEQ ID NO: 75) |
| | | | Up-R | ATCC1303 2 genome | GEP2611 | GGTACTCGAGTCCTGGATTTGCGTAGACAGTC (SEQ ID NO: 76) |
| | | | Down-F | ATCC1303 2 genome | GEP2612 | CAGGACTCGAGTACCAGCAGACCAAGAACCT G (SEQ ID NO: 77) |
| | | | Down-R | ATCC1303 2 genome | GEP2613 | AGGTGGATACGGATCGATCTCCAGAGGTTTCA AGC (SEQ ID NO: 78) |

**[Table 44]**

| Plasmid ID | Deletion target | Target vector | | Template | DNA ID | DNA sequences |
|---|---|---|---|---|---|---|
| pGE1221 | ald | pGE209 (KpnI,BamHI) | Up-F | ATCC1303 2 genome | GEP2692 | gtgaattcgagctcgAACGCCAGATGGTCGTACTTTGC (SEQ ID NO: 79) |
| | | | Up-R | ATCC1303 2 genome | GEP2611 | GGTACTCGAGTCCTGGATTTGCGTAGACAGTC (SEQ ID NO: 76) |
| | | | Down-F | ATCC1303 2 genome | GEP2612 | CAGGACTCGAGTACCAGCAGACCAAGAACCT G (SEQ ID NO: 77) |
| | | | Down-R | ATCC1303 2 genome | GEP2693 | aggtggatacggatcTTCAACACCGCTGATTTCCTACG (SEQ ID NO: 80) |

**[Table 45]**

| Plasmid ID | Target gene | Target vector | | Template | DNA ID | DNA sequences |
|---|---|---|---|---|---|---|
| pGE403 | KnR | | F | pHSG298 | GEP433 | CGGTACCGTCGACGATATCGAGGTCTGCCTCGTG AAGAA (SEQ ID NO: 81) |
| | | | R | | GEP434 | GCCTTTTTACGGTTCGATTTATTCAACAAAGCCG C (SEQ ID NO: 82) |
| | pUCori | | F | pHSG298 | GEP437 | GAACCGTAAAAAGGCCGCGTTGCTGGCGTTTTTC C (SEQ ID NO: 83) |
| | | | R | | GEP438 | GCGGCCGCGTAGAAAAGATCAAAGGATCTTCTTG A (SEQ ID NO: 84) |
| | pCGlori | | F | pCG1 | GEP445 | TTTCTACGCGGCCGCCCATGGTCGTCACAGAGCT G (SEQ ID NO: 85) |
| | | | R | | GEP446 | TCGTCGACGGTACCGGATCCTTGGGAGCAGTCCT TGTGCG (SEQ ID NO: 86) |

**[Table 46]**

| Plasmid ID | Target gene | Target vector | | Template | DNA ID | DNA sequences |
|---|---|---|---|---|---|---|
| pGE409 | PgapA | pGE403 | F | ATCC1303 2 genome | GEP472 | CTGCTCCCAAGGATCGAAGAAATTTAGATGATTG A (SEQ ID NO: 87) |
| | | | R | | GEP478 | CCGTCGACGATATCATATGGTGTCTCCTCTAAAG ATTGT (SEQ ID NO: 88) |
| | TrrnB | pGE403 | F | pFLAG-CTC | GEP467 | TGATATCGTCGACGGATCCTGCCTGGCGGCAGTA GCGCG (SEQ ID NO: 89) |
| | | | R | | GEP468 | GAGGCAGACCTCGATAAAAAGGCCATCCGTCAG GA (SEQ ID NO: 90) |

**[Table 47]**

| Plasmid ID | Target gene | Target vector | | Template | DNA ID | DNA sequences |
|---|---|---|---|---|---|---|
| pGE1031 | Ec_hpaI | pET15b (NdeI) | F | BL21(DE3) genome | GEP2268 | CGCGCGGCAGCCATATGGAAAACAGTTTTAAAG CG (SEQ ID NO: 91) |
| | | | R | | GEP2269 | GGATCCTCGAGCATAttaATACACGCCGGGCTTAA TC (SEQ ID NO: 92) |
| pGE1143 | Ec_hpaI | pGE409 | F | pGE1031 | GEP2519 | AGAGGAGACACCATATGGGCAGCAGCCATCATC ATC (SEQ ID NO: 93) |
| | | | R | | GEP2518 | CCGCCAGGCAGGATCCTTAATACACGCCGGGCTT AATC (SEQ ID NO: 94) |
| pGE1258 | Ec_rhmA | pGE409 | F | DH5alpha genome | GEP2765 | AGAGGAGACACCATatgAACGCATTATTAAGCAA (SEQ ID NO: 99) |
| | | | R | | GEP2766 | CCGCCAGGCAGGATCagatcttaATAACTACCTTTTAT GCG (SEQ ID NO: 100) |
| pGE1226 | Ec_nanA | pGE409 (NdeI, BamHI) | F | DH5alpha genome | GEP2722 | AGAGGAGACACCATatgGCAACGAATTTACGTGG (SEQ ID NO: 101) |
| | | | R | | GEP2723 | CCGCCAGGCAGGATCctTaCCCGCGCTCTTGCATC AAC (SEQ ID NO: 102) |

**[Table 48]**

| Plasmid ID | Target gene | Target vector | | Template | DNA ID | DNA sequences |
|---|---|---|---|---|---|---|
| pGE1185 | Δ adhE:: hpaI | pGE1171 (XhoI) | F | pGE1143 | GEP2637 | GCCTGGAATTCTCGAGAAGGACTGCTCCCAAGGA TCG (SEQ ID NO: 95) |
| | | | R | | GEP2638 | AAGCCTCTTCCTCGATCTTCACGAGGCAGACCTC (SEQ ID NO: 96) |
| pGE1186 | Δ ald:: hpaI | pGE1172 (XhoI) | F | pGE1143 | GEP2639 | AAATCCAGGACTCGAGAAGGACTGCTCCCAAGG ATCG (SEQ ID NO: 97) |
| | | | R | | GEP2640 | TCTGCTGGTACTCGATCTTCACGAGGCAGACCTC (SEQ ID NO: 98) |
| pGE1302 | Δald:: rhmA | pGE1221 (XhoI) | F | pGE1258 | GEP2639 | AAATCCAGGACTCGAGAAGGACTGCTCCCAAGG ATCG (SEQ ID NO: 97) |
| | | | R | | GEP2640 | TCTGCTGGTACTCGATCTTCACGAGGCAGACCTC (SEQ ID NO: 98) |

**[Table 49]**

| Plasmid ID | Target gene | Target vector | | Template | DNA ID | DNA sequences |
|---|---|---|---|---|---|---|
| pGE1304 | ΔadhE::rh mA | pGE1218 (XhoI) | F | pGE1258 | GEP2637 | GCCTGGAATTCTCGAGAAGGACTGCTCCCAAGGA TCG (SEQ ID NO: 95) |
| | | | R | | GEP2638 | AAGCCTCTTCCTCGATCTTCACGAGGCAGACCTC (SEQ ID NO: 96) |
| pGE1272 | Δ adhE:: nanA | pGE1218 (XhoI) | F | pGE1226 | GEP2637 | GCCTGGAATTCTCGAGAAGGACTGCTCCCAAGGA TCG (SEQ ID NO: 95) |
| | | | R | | GEP2638 | AAGCCTCTTCCTCGATCTTCACGAGGCAGACCTC (SEQ ID NO: 96) |
| pGE1273 | Δ ald:: nanA | pGE1221 (XhoI) | F | pGE1226 | GEP2639 | AAATCCAGGACTCGAGAAGGACTGCTCCCAAGG ATCG (SEQ ID NO: 97) |
| | | | R | | GEP2640 | TCTGCTGGTACTCGATCTTCACGAGGCAGACCTC (SEQ ID NO: 98) |

**[Table 50]**

| Plasmid ID | Target gene | Target vector | | Template | DNA ID | DNA sequences |
|---|---|---|---|---|---|---|
| pGE402 | KnR | | F | pHSG298 | GEP433 | CGGTACCGTCGACGATATCGAGGTCTGCCTCGTG AAGAA (SEQ ID NO: 81) |
| | | | R | | GEP434 | GCCTTTTTACGGTTCGATTTATTCAACAAAGCCG C (SEQ ID NO: 82) |
| | pUCori | | F | pHSG298 | GEP437 | GAACCGTAAAAAGGCCGCGTTGCTGGCGTTTTTC C (SEQ ID NO: 83) |
| | | | R | | GEP438 | GCGGCCGCGTAGAAAAGATCAAAGGATCTTCTTG A (SEQ ID NO: 84) |
| | pCASElori | | F | pCASE1 | GEP443 | TTTCTACGCGGCCGCCACTGGAAGGGTTCTTCAG G (SEQ ID NO: 103) |
| | | | R | | GEP444 | TCGTCGACGGTACCGGATCCCTGACTTGGTTACG ATGGAC (SEQ ID NO: 104) |

**[Table 51]**

| Plasmid ID | Target gene | Target vector | | Template | DNA ID | DNA sequences |
|---|---|---|---|---|---|---|
| pGE411 | PldhA | pGE402 | F | ATCC1303 2 genome | GEP474 | ACCAAGTCAGGGATCGCGGAACTAGCTCTGCAAT G (SEQ ID NO: 105) |
| | | | R | | GEP477 | CCGTCGACGATATCATATGCGATCCCACTTCCTG ATTTC (SEQ ID NO: 106) |
| | TrrnB | pGE402 | F | pFLAG-CTC | GEP467 | TGATATCGTCGACGGATCCTGCCTGGCGGCAGTA GCGCG (SEQ ID NO: 89) |
| | | | R | | GEP468 | GAGGCAGACCTCGATAAAAAGGCCATCCGTCAG GA (SEQ ID NO: 90) |

**[Table 52]**

| Plasmid ID | Target gene | Target vector | | Template | DNA ID | DNA sequences |
|---|---|---|---|---|---|---|
| pGE619 (=pGES002) | SpR | | F | pCR8/GW/TOP O invitrogen | GEP435 | CGGTACCGTCGACGATATCTCTAGACCAGCCAGG ACAGA (SEQ ID NO: 107) |
| | | | R | | GEP436 | GCCTTTTTACGGTTCCTCGAGGGTTATTTGCCGAC (SEQ ID NO: 108) |
| | pUCori | | F | pHSG298 | GEP437 | GAACCGTAAAAAGGCCGCGTTGCTGGCGTTTTTC C (SEQ ID NO: 83) |
| | | | R | | GEP438 | GCGGCCGCGTAGAAAAGATCAAAGGATCTTCTTG A (SEQ ID NO: 84) |
| | pCASElori | | F | pCASE1 | GEP443 | TTTCTACGCGGCCGCCACTGGAAGGGTTCTTCAG G (SEQ ID NO: 103) |
| | | | R | | GEP444 | TCGTCGACGGTACCGGATCCCTGACTTGGTTACG ATGGAC (SEQ ID NO: 104) |

**[Table 53]**

| Plasmid ID | Target gene | Target vector | | Template | DNA ID | DNA sequences |
|---|---|---|---|---|---|---|
| pGE945-2 | PldhA | pGE619 | F | ATCC13032 genome | GEP474 | ACCAAGTCAGGGATCGCGGAACTAGCTCTGCAAT G (SEQ ID NO: 105) |
| | | | R | | GEP477 | CCGTCGACGATATCATATGCGATCCCACTTCCTG ATTTC (SEQ ID NO: 106) |
| | TrrnB | pGE619 | F | pFLAG-CTC | GEP467 | TGATATCGTCGACGGATCCTGCCTGGCGGCAGTA GCGCG (SEQ ID NO: 89) |
| | | | R | | GEP2897 | GGCTGGTCTAGAGATAAAAAGGCCATCCGTCAG GA (SEQ ID NO: 109) |

**[Table 54]**

| Plasmid ID | Target gene | Target Vector | | Template | Oligo DNA | DNA sequences |
|---|---|---|---|---|---|---|
| pGE1161 | Pp_mdlC | pET15b (Ndel) | F | NBRC 14164 genome | GEP2550 | CGCGCGGCAGCCATATGGCTTCGGTACACGGCAC (SEQ ID NO: 110) |
| | | | R | | GEP2551 | GGATCCTCGAGCATATCACTTCACCGGGCTTACG GTG (SEQ ID NO: 111) |
| pGE1150 | Kp_dhaT | pET15b (Ndel) | F | pHA12-dhaBT | GEP2529 | CGCGCGGCAGCCATATGAGCTATCGTATGTTCGA TTATC (SEQ ID NO: 114) |
| | | | R | | GEP2530 | GGATCCTCGAGCATATTAGAATGCCTGGCGGAAA ATCG (SEQ ID NO: 115) |
| pGE1274 | L1_1dhA | pET15b (Ndel) | F | NBRC 100676 genome | GEP2799 | CGCGCGGCAGCCATATGGCTGATAAACAACGTA AGAAA (SEQ ID NO: 116) |
| | | | R | | GEP2800 | GGATCCTCGAGCATATTAGTTTTTAACTGCAGAA GCAA (SEQ ID NO: 117) |

**[Table 55]**

| Plasmid ID | Target gene | Target Vector | | Template | Oligo DNA | DNA sequences |
|---|---|---|---|---|---|---|
| pGE1148 | Ls_leudh | pET15b (Ndel) | F | NBRC 3525 genome | GEP2525 | CGCGCGGCAGCCATATGGAAATCTTCAAGTATAT G (SEQ ID NO: 122) |
| | | | R | | GEP2526 | GGATCCTCGAGCATATTAACGGCCGTTCAAAATA TTTTTT (SEQ ID NO: 123) |
| pGE1175 | Sf_valdh | pET15b (Ndel) | F | ATCC19609 | GEP2600 | CGCGCGGCAGCCATATGACCGACGCGTCCCACCC CAC (SEQ ID NO: 126) |
| | | | R | | GEP2601 | GGATCCTCGAGCATAttaGACGGTGCGGCCTCCG CCATG (SEQ ID NO: 127) |
| pGE1237 | Bb_phedh | pET15b (Ndel) | F | NBRC15713 | GEP2678 | CGCGCGGCAGCCATATGAGCTTAGTAGAAAAAA C (SEQ ID NO: 128) |
| | | | R | | GEP2679 | GGATCCTCGAGCATATTAGTTGCGAATATCCCAT TTTG (SEQ ID NO: 129) |
| pGE479 | AtCGS | pGE411 | F | pMK-AtCGS (pGE233) | GEP1053 | AAGTGGGATCGCATATGGTGCGCCAGCTGTC (SEQ ID NO: 130) |
| | | | R | | GEP1054 | GTCGACGATATCATATTAGATTGCTTCGAGTGCC TGCAG (SEQ ID NO: 131) |

**[Table 56]**

| Plasmid ID | Target gene | Target Vector | | Template | Oligo DNA | DNA sequences |
|---|---|---|---|---|---|---|
| pGE1223 | Ec_eda | pGE409 | F | DH5alpha genome | GEP2712 | AGAGGAGACACCATatgAAAAACTGGAAAACAAG (SEQ ID NO: 132) |
| | | | R | | GEP2713 | CCGCCAGGCAGGATCcttaCAGCTTAGCGCCTTCTA (SEQ ID NO: 133) |
| pGE1224 | Ec_yjhH | pGE409 | F | DH5alpha genome | GEP2714 | AGAGGAGACACCATatgAAAAAATTCAGCGGCAT (SEQ ID NO: 134) |
| | | | R | | GEP2715 | CCGCCAGGCAGGATCctTaGACTGGTAAAATGCCC TGC (SEQ ID NO: 135) |
| pGE1225 | Ec_dgoA | pGE409 | F | DH5alpha genome | GEP2720 | AGAGGAGACACCATatgCAGTGGCAAACTAAACT (SEQ ID NO: 136) |
| | | | R | | GEP2721 | CCGCCAGGCAGGATCctTaTTGCACTGCCTCTCGA TAC (SEQ ID NO: 137) |
| pGE1227 | Ec_mhpFE | pGE409 | F | DH5alpha genome | GEP2726 | AGAGGAGACACCATatgAGTAAGCGTAAAGTCGC (SEQ ID NO: 138) |
| | | | R | | GEP2727 | CCGCCAGGCAGGATCcTTATTTGTTGTTGCGCAG AT (SEQ ID NO: 139) |

**[Table 57]**

| Plasmid ID | Target gene | Target Vector | | Template | Oligo DNA | DNA sequences |
|---|---|---|---|---|---|---|
| pGE1239 | Ns_phdJ | pGE409 | F | NBRC 109351 genome | GEP2716 | AGAGGAGACACCATaTGACGTCACCCGCCGTGAC (SEQ ID NO: 144) |
| | | | R | | GEP2717 | CCGCCAGGCAGGATCctTATGGTTCGGCGAGAGTT GC (SEQ ID NO: 145) |
| pGE1259 | Ec_garL | pGE409 | F | DH5alpha genome | GEP2767 | AGAGGAGACACCATatgAATAACGATGTTTTCCC (SEQ ID NO: 140) |
| | | | R | | GEP2768 | CCGCCAGGCAGGATCcttaTTTTTTAAAGGTATCAG (SEQ ID NO: 141) |
| pGE1260 | Pp_galC | pGE409 | F | NBRC14164 genome | GEP2769 | AGAGGAGACACCATaTGAGTACGCTCGTGGGAAA (SEQ ID NO: 142) |
| | | | R | | GEP2770 | CCGCCAGGCAGGATCctTACTCTCCCAGTTCATCC AG (SEQ ID NO: 143) |
| pGE1262 | Bx_bphJI | pGE409 | F | DSMZ 17367 genome | GEP2771 | AGAGGAGACACCATaTGACGAAAAAAATCAAAT G (SEQ ID NO: 146) |
| | | | R | | GEP2772 | CCGCCAGGCAGGATCcttATGCGGCGGTCAAGGTC AAC (SEQ ID NO: 147) |

**[Table 58]**

| Oligo DNA | DNA sequences |
|---|---|
| GEP2803 | GCTCCATGCAAACCAGGTGAAACTCGT (SEQ ID NO: 118) |
| GEP2804 | TGGTTTGCATGGAGCACCAGAAGTCAA (SEQ ID NO: 119) |

**[Table 59]**

| Plasmid ID | Target gene | Target vector | | Template | Oligo DNA | DNA sequences |
|---|---|---|---|---|---|---|
| pGE1197 | Pp_mdlC | pGE409 (Ndel) | F | pGE1161 | GEP2653 | AGAGGAGACACCATATGGCTTCGGTACACG GCAC (SEQ ID NO: 112) |
| | | | R | | GEP2654 | GTCGACGATATCATATCACTTCACCGGGCTT ACGGT (SEQ ID NO: 113) |
| pGE1190 | Kp_dhaT | pGE945-2 (Ndel, BamHI) | F | pGE1150 | ligation | |
| | | | R | | | |
| pGE1286 | L1_1dhA Q85C | pGE945 (Ndel, BamHI) | F | pGE1275 | GEP2801 | AAGTGGGATCGCATATGGCTGATAAACAAC GTAAGAAA (SEQ ID NO: 120) |
| | | | R | | GEP2802 | CCGCCAGGCAGGATCCTTAGTTTTTAACTGC AGAAGCAA (SEQ ID NO: 121) |

**[Table 60]**

| Plasmid ID | Target gene | Target vector | | Template | Oligo DNA | DNA sequences |
|---|---|---|---|---|---|---|
| pGE1203 | Ls_leudh | pGE945 (Ndel, BamHI) | F | pGE1148 | GEP2655 | AAGTGGGATCGCATATGGAAATCTTCAAGTA TATGG (SEQ ID NO: 124) |
| | | | R | | GEP2650 | CCGCCAGGCAGGATCCTTAACGGCCGTTCAA AATATTTTT (SEQ ID NO: 125) |
| pGE513 | AtCGS | pGE449 (Ndel, BamHI) | F | | ligation | |
| | | | R | | | |

**[Table 61]**

| Strain ID | Host strain | Plasmid |
|---|---|---|
| ATCC1303 2 | - | - |
| GES007 | Δppc | - |
| GES048 | ΔppcΔldhA | - |
| GES1041 | ΔppcΔldhAΔadhE | - |
| GES1070 | ΔppcΔldhAΔadhEΔald | - |
| GES1052 | ΔppcΔldhA | - |
| | ΔadhEΔald::PgapA-hpaI | |
| GES1063 | ΔppcΔldhA | - |
| | ΔadhE::PgapA-hpaIΔald::PgapA-hpaI | |
| GES1188 | ΔppcΔldhA | - |
| | ΔadhEΔald::PgapA-nanA | |
| GES1223 | ΔppcΔldhA | - |
| | ΔadhE::PgapA-nanAΔald::PgapA-nanA | |
| GES1215 | ΔppcΔldhA | - |
| | ΔadhEΔald::PgapA-rhmA | |
| GES1242 | ΔppcΔldhA | - |
| | ΔadhE::PgapA-rhmAΔald:: PgapA-rhmA | |
| GES1206 | ΔppcΔldhA | pGE1197 PgapA-Pp8_mdlC (pCG1, Km) |
| | ΔadhEΔald | pGE1190 PldhA-Kp_dhaT (pCASE1, Sp) |

**[Table 62]**

| Strain ID | Host strain | Plasmid |
|---|---|---|
| GES1077 | ΔppcΔldhA | pGE1197 PgapA-Pp_mdlC (pCG1, Km) |
| | ΔadhE::PgapA-hpaIΔald::PgapA-hpaI | pGE1190 PldhA-Kp_dhaT (pCASE1, Sp) |
| GES1253 | ΔppcΔldhA | pGE1197 PgapA-Pp_mdlC (pCG1, Km) |
| | ΔadhE::PgapA-nanAΔald::PgapA-nanA | pGE1190 PldhA-Kp_dhaT (pCASE1, Sp) |
| GES1282 | ΔppcΔldhA | pGE1197 PgapA-Pp_mdlC (pCG1, Km) |
| | ΔadhE::PgapA-rhmAΔald::PgapA-rhmA | pGE1190 PldhA-Kp_dhaT (pCASE1, Sp) |

**[Table 63]**

| Strain ID | Host strain | Plasmid |
|---|---|---|
| GES385 | ΔppcΔldhA | - |
| | Δpyc | |
| GES388 | ΔppcΔldhA | - |
| | ΔpycΔpoxB | |
| GES393 | ΔppcΔldhA | - |
| | ΔpycΔpoxBΔalaA | |
| GES405 | ΔppcΔldhA | - |
| | ΔpycΔpoxBΔalaAΔilvB | |
| GES435 | ΔppcΔldhA | - |
| | ΔpycΔpoxBΔalaAΔilvBΔcg0931 | |
| GES1042 | ΔppcΔldhA | - |
| | ΔpycΔpoxBΔalaAΔilvBΔcg0931 | |
| | ΔadhE | |
| GES1211 | ΔppcΔldhA | - |
| | ΔpycΔpoxBΔalaAΔilvBΔcg0931 | |
| | ΔadhEΔald | |
| GES1053 | ΔppcΔldhA | - |
| | ΔpycΔpoxBΔalaAΔilvBΔcg0931 | |
| | AadhEAald::PgapA-hpaI | |

**[Table 64]**

| Strain ID | Host strain | Plasmid |
|---|---|---|
| GES1064 | ΔppcΔldhA | - |
| | ΔpycΔpoxBΔalaAΔilvBΔcg0931 | |
| | ΔadhE::PgapA-hpaIΔald::PgapA-hpaI | |
| GES1189 | ΔppcΔldhA | - |
| | ΔpycΔpoxBΔalaAΔilvBΔcg0931 | |
| | ΔadhEΔald::PgapA-nanA | |
| GES1233 | ΔppcΔldhA | - |
| | ΔpycΔpoxBΔalaAΔilvBΔcg0931 | |
| | ΔadhE::PgapA-nanAΔald::PgapA-nanA | |
| GES1068 | ΔppcΔldhA | pGE1197 PgapA-Pp_mdlC (pCG1, Km) |
| | ΔpycΔpoxBΔalaAΔilvBΔcg0931 | |
| | ΔadhE::PgapA-hpaIΔald::PgapA-hpaI | pGE1190 PldhA-Kp_dhaT (pCASE1, Sp) |
| GES1254 | ΔppcΔldhA | pGE1197 PgapA-Pp_mdlC (pCG1, Km) |
| | ΔpycΔpoxBΔalaAΔilvBΔcg0931 | |
| | ΔadhE::PgapA-nanAΔald:: PgapA-nanA | pGE1190 PldhA-Kp_dhaT (pCASE1, Sp) |

**[Table 65]**

| Strain ID | Host strain | Plasmid |
|---|---|---|
| GES1294 | ΔppcΔldhA | pGE1286 PldhA-Ll_ldhA Q85C (pCASE1, Sp) |
| | ΔpycΔpoxBΔalaAΔilvBΔcg0931 | |
| | ΔadhEΔald | |
| GES1232 | ΔppcΔldhA | pGE1286 PldhA-Ll_ldhA Q85C (pCASE1, Sp) |
| | ΔpycΔpoxBΔalaAΔilvBΔcg0931 | |
| | ΔadhE::PgapA-hpaIΔald::PgapA-hpaI | |
| GES1295 | ΔppcΔldhA | pGE1203 PldhA-Ls_leudh (pCASE1, Sp) |
| | ΔpycΔpoxBΔalaAΔilvBΔcg0931 | |
| | ΔadhEAald | |
| GES1073 | ΔppcΔldhA | pGE1203 PldhA-Ls_leudh (pCASE1, Sp) |
| | ΔpycΔpoxBΔalaAΔilvBΔcg0931 | |
| | ΔadhE::PgapA-hpaIΔald::PgapA-hpaI | |
| GES1097 | ΔppcΔldhA | pGE1203 PldhA-Ls_leudh (pCASE1, Sp) |
| | Δ pycΔpoxBΔalaAΔilvBΔcg0931 | |
| | Δ adhE::PgapA-hpaIΔald::PgapA-hpaI | pGE513 PgapA-AtCGS1 (pCG1, Km) |

**[Table 66]**

| Strain ID | Host strain | Plasmid |
|---|---|---|
| GES1009 | ΔppcΔldhA | pGE1143 PgapA-Ec_hpaI (pCG1, Km) |
| | ΔpycΔpoxBΔalaAΔilvBΔcg0931 | |
| GES1119 | ΔppcΔldhA | pGE1223 PgapA-Ec_eda (pCG1, Km) |
| | ΔpycΔpoxBΔalaAΔilvBΔcg0931 | |
| GES 1120 | ΔppcΔldhA | pGE1224 PgapA-Ec_yjhH (pCG1, Km) |
| | ΔpycΔpoxBΔalaAΔilvBΔcg0931 | |
| GES1121 | ΔppcΔldhA | pGE1225 PgapA-Ec_dgoA (pCG1, Km) |
| | ΔpycΔpoxBΔalaAΔilvBΔcg0931 | |
| GES 1122 | ΔppcΔldhA | pGE1226 PgapA-Ec_nanA (pCG1, Km) |
| | ΔpycΔpoxBΔalaAΔilvBΔcg0931 | |
| GES 1123 | ΔppcΔldhA | pGE1227 PgapA-Ec_mhpFE (pCG1, Km) |
| | ΔpycΔpoxBΔalaAΔilvBΔcg0931 | |
| GES1127 | ΔppcΔldhA | pGE1239 PgapA-Ns_phdJ (pCG1, Km) |
| | ΔpycΔpoxBΔalaAΔilvBΔcg0931 | |
| GES1154 | ΔppcΔldhA | pGE1258 PgapA-Ec_rhmA (pCG1, Km) |
| | ΔpycΔpoxBΔalaAΔilvBΔcg0931 | |
| GES1155 | ΔppcΔldhA | pGE1259 PgapA-Ec_garL (pCG1, Km) |
| | ΔpycΔpoxBΔalaAΔilvBΔcg0931 | |
| GES1156 | ΔppcΔldhA | pGE1260 PgapA-Pp_galC (pCG1, Km) |
| | ΔpycΔpoxBΔalaAΔilvBΔcg0931 | |
| GES1157 | ΔppcΔldhA | pGE1262 PgapA-Bx_bphJI (pCG1, Km) |
| | ΔpycΔpoxBΔalaAΔilvBΔcg0931 | |

**[Table 67]**

| Strain ID | Host strain | Plasmid |
|---|---|---|
| GES1007 | BL21(DE3) | pGE1148 |
| GES1057 | BL21(DE3) | pGE1175 |
| GES1136 | BL21(DE3) | pGE1237 |

### 2. Plasmid construction

### (2-1) Gene deletion

### 2-1-(1) Construction of plasmid for gene disruption

Using pNIC28-Bsa4 (manufactured by Source BioScience) as a template, and using DNAs of base sequences represented by GEP007 (SEQ ID NO: 37) and GEP008 (SEQ ID NO: 38), a DNA fragment [Mol. Microbiol., 6, 1195 (1992)] containing a sacB gene derived from Bacillus subtilis was amplified by the PCR method. This PCR product was treated with BamHI and PstI, and after performing agarose gel electrophoresis, the DNA fragment was extracted and purified. In addition, a plasmid pHSG299 (manufactured by Takara Bio Inc.) having a gene conferring resistance to kanamycin was treated with BamHI and PstI, and after performing agarose gel electrophoresis, the DNA fragment was extracted and purified. These two DNA fragments were ligated using a DNA Ligation Kit, <Mighty Mix> (manufactured by Takara Bio Inc.) according to the attached instructions, and thereby a plasmid pGE015 was obtained.

For the purpose of deleting a KpnI recognition site that pGE015 has on sacB, using pGE015 as a template, and using DNAs of base sequencess represented by GEP808 (SEQ ID NO: 39) and GEP809 (SEQ ID NO: 40), mutagenesis and plasmid amplification were carried out using a PrimeSTAR (registered trademark) Mutagenesis Basal Kit (manufactured by Takara Bio Inc.) according to the attached instructions. Thereby, pGE209 was obtained.

### 2-1-(2) Construction of plasmid for creating ppc-gene-disrupted strain

A Corynebacterium glutamicum ATCC13032 strain was obtained as a NBRC12168 strain from the Biotechnology Center of the National Institute of Technology and Evaluation, cultured according to the attached instructions, and then genomic DNA extraction was performed.

Using a genomic DNA of the ATCC13032 strain as a template, and using a combination of DNAs of base sequences represented by GEP132 (SEQ ID NO: 41) and GEP133 (SEQ ID NO: 42), and a combination of DNAs of base sequences represented by GEP134 (SEQ ID NO: 43) and GEP135 (SEQ ID NO: 44), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in a B medium containing 50 µg/ml of kanamycin, and plasmid extraction was performed using the obtained culture suspension. The plasmid thus obtained was designated as pGE020.

### 2-1-(3) Creation of ppc-gene-deleted strain

A Corynebacterium glutamicum ATCC13032 strain was transformed by electroporation using pGE020, and a kanamycin-resistant strain was selected on an A medium containing 25 µg/ml of kanamycin at 33°C. Next, the obtained kanamycin-resistant strain was spread on a Suc agar medium and cultured at 33°C. The grown colonies were further cultured in the A medium, and a strain lacking a ppc gene was selected by colony PCR. The strain thus obtained was named GES007.

### 2-1-(4) Construction of plasmid for creating ldhA-gene-disrupted strain

Using a genomic DNA of the ATCC13032 strain as a template, and using a combination of DNAs of base sequences represented by GEP169 (SEQ ID NO: 45) and GEP170 (SEQ ID NO: 46), and a combination of DNAs of base sequences represented by GEP215 (SEQ ID NO: 47) and GEP216 (SEQ ID NO: 48), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE033.

### 2-1-(5) Creation of ldhA-gene-deleted strain

Using pGE033, GES007 was transformed by electroporation, and a strain selected by the same operation as in the creation of GES007 was named GES048.

### 2-1-(6) Construction of plasmid for creating pyc-gene-disrupted strain

Using a genomic DNA of the ATCC13032 strain as a template, and using a combination of DNAs of base sequences represented by GEP615 (SEQ ID NO: 49) and GEP616 (SEQ ID NO: 50), and a combination of DNAs of base sequences represented by GEP617 (SEQ ID NO: 51) and GEP618 (SEQ ID NO: 52), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE177.

### 2-1-(7) Creation of pyc-gene-deleted strain

Using pGE177, GES048 was transformed by electroporation, and a strain selected by the same operation as in the creation of GES007 was named GES385.

### 2-1-(8) Construction of plasmid for creating poxB-gene-disrupted strain

Using a genomic DNA of the ATCC13032 strain as a template, and using a combination of DNAs of base sequences represented by GEP406 (SEQ ID NO: 53) and GEP407 (SEQ ID NO: 54), and a combination of DNAs of base sequences represented by GEP698 (SEQ ID NO: 55) and GEP409 (SEQ ID NO: 56), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE191.

### 2-1-(9) Creation of poxB-gene-deleted strain

Using pGE191, GES385 was transformed by electroporation, and a strain selected by the same operation as in the creation of GES007 was named GES388.

### 2-1-(10) Construction of plasmid for creating alaA-gene-disrupted strain

Using a genomic DNA of the ATCC13032 strain as a template, and using a combination of DNAs of base sequences represented by GEP810 (SEQ ID NO: 57) and GEP811 (SEQ ID NO: 58), and a combination of DNAs of base sequences represented by GEP812 (SEQ ID NO: 59) and GEP813 (SEQ ID NO: 60), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE210.

### 2-1-(11) Creation of alaA-gene-deleted strain

Using pGE210, GES388 was transformed by electroporation, and a strain selected by the same operation as in the creation of GES007 was named GES393.

### 2-1-(12) Construction of plasmid for creating ilvB-gene-disrupted strain

Using a genomic DNA of the ATCC13032 strain as a template, and using a combination of DNAs of base sequences represented by GEP956 (SEQ ID NO: 61) and GEP957 (SEQ ID NO: 62), and a combination of DNAs of base sequences represented by GEP958 (SEQ ID NO: 63) and GEP959 (SEQ ID NO: 64), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE228.

### 2-1-(13) Creation of ilvB-gene-deleted strain

Using pGE228, GES393 was transformed by electroporation, and a strain selected by the same operation as in the creation of GES007 was named GES405.

### 2-1-(14) Construction of plasmid for creating cg0931-gene-disrupted strain

Using a genomic DNA of the ATCC13032 strain as a template, and using a combination of DNAs of base sequences represented by GEP1132 (SEQ ID NO: 65) and GEP1133 (SEQ ID NO: 66), and a combination of DNAs of base sequences represented by GEP1134 (SEQ ID NO: 67) and GEP1135 (SEQ ID NO: 68), two types of PCR products were obtained by the PCR method, agarose gel electrophoresis was performed thereon, and then extraction and purification were performed. In addition, pGE015 was treated with EcoRI, agarose gel electrophoresis was performed thereon, and then extraction and purification were performed. These three DNA fragments were mixed, and a plasmid obtained by the same operation as in the creation of pGE020 was designated as pGE253.

### 2-1-(15) Creation of cg0931-gene-deleted strain

Using pGE253, GES405 was transformed by electroporation, and a strain selected by the same operation as in the creation of GES007 was named GES435.

### 2-1-(16) Construction of plasmid for creating adhE-gene-disrupted strain

Using a genomic DNA of the ATCC13032 strain as a template, and using a combination of DNAs of base sequences represented by GEP2606 (SEQ ID NO: 69) and GEP2607 (SEQ ID NO: 70), and a combination of DNAs of base sequences represented by GEP2608 (SEQ ID NO: 71) and GEP2609 (SEQ ID NO: 72), two types of PCR products were obtained by the PCR method, agarose gel electrophoresis was performed thereon, and then extraction and purification were performed. In addition, pGE209 was treated with KpnI and BamHI, agarose gel electrophoresis was performed thereon, and then extraction and purification were performed. These three DNA fragments were mixed, and a plasmid obtained by the same operation as in the creation of pGE020 was designated as pGE1171.

Using a genomic DNA of the ATCC13032 strain as a template, and using a combination of DNAs of base sequences represented by GEP2688 (SEQ ID NO: 73) and GEP2607 (SEQ ID NO: 70), and a combination of DNAs of base sequences represented by GEP2608 (SEQ ID NO: 71) and GEP2689 (SEQ ID NO: 74), two types of PCR products were obtained by the PCR method, agarose gel electrophoresis was performed thereon, and then extraction and purification were performed. In addition, pGE209 was treated with KpnI and BamHI, agarose gel electrophoresis was performed thereon, and then extraction and purification were performed. These three DNA fragments were mixed, and a plasmid obtained by the same operation as in the creation of pGE020 was designated as pGE1218.

### 2-1-(17) Creation of adhE-gene-deleted strain

Using pGE1171, GES048 was transformed by electroporation, and a strain selected by the same operation as in the creation of GES007 was named GES1041.

Using pGE1171, GES435 was transformed by electroporation, and a strain selected by the same operation as in the creation of GES007 was named GES1042.

### 2-1-(18) Construction of plasmid for creating ald-gene-disrupted strain

Using a genomic DNA of the ATCC13032 strain as a template, and using a combination of DNAs of base sequences represented by GEP2610 (SEQ ID NO: 75) and GEP2611 (SEQ ID NO: 76), and a combination of DNAs of base sequences represented by GEP2612 (SEQ ID NO: 77) and GEP2613 (SEQ ID NO: 78), two types of PCR products were obtained by the PCR method, agarose gel electrophoresis was performed thereon, and then extraction and purification were performed. In addition, pGE209 was treated with KpnI and BamHI, agarose gel electrophoresis was performed thereon, and then extraction and purification were performed. These three DNA fragments were mixed, and a plasmid obtained by the same operation as in the creation of pGE020 was designated as pGE1172.

Using a genomic DNA of the ATCC13032 strain as a template, and using a combination of DNAs of base sequences represented by GEP2692 (SEQ ID NO: 79) and GEP2611 (SEQ ID NO: 76), and a combination of DNAs of base sequences represented by GEP2612 (SEQ ID NO: 77) and GEP2693 (SEQ ID NO: 80), two types of PCR products were obtained by the PCR method, agarose gel electrophoresis was performed thereon, and then extraction and purification were performed. In addition, pGE209 was treated with KpnI and BamHI, agarose gel electrophoresis was performed thereon, and then extraction and purification were performed. These three DNA fragments were mixed, and a plasmid obtained by the same operation as in the creation of pGE020 was designated as pGE1221.

### 2-1-(19) Creation of ald-gene-deleted strain

Using pGE1172, GES1041 was transformed by electroporation, and a strain selected by the same operation as in the creation of GES007 was named GES1070.

Using pGE1172, GES1042 was transformed by electroporation, and a strain selected by the same operation as in the creation of GES007 was named GES1211.

### (2-2) Gene replacement

### 2-2-(1) Construction of pGE409

Using pHSG298 (manufactured by Takara Bio Inc.) as a template, and using a combination of DNAs of base sequences represented by GEP433 (SEQ ID NO: 81) and GEP434 (SEQ ID NO: 82), and a combination of DNAs of base sequences represented by GEP437 (SEQ ID NO: 83) and GEP438 (SEQ ID NO: 84), PCR products were obtained by the PCR method, agarose gel electrophoresis was performed thereon, and then extraction and purification were performed. These are DNA fragments respectively containing a kanamycin-resistant gene and a replication origin, pUCori for Escherichia coli.

A Corynebacterium glutamicum NBRC12169 strain was obtained from the Biotechnology Center of the National Institute of Technology and Evaluation and cultured according to the attached instructions, and the bacterial cells were recovered by centrifugation. The bacterial cell pellet was washed with 1 mL of a buffer solution of 10 mM cyclohexylene dinitrilotetraacetic acid and 25 mM Tris-HCI (pH 8.0), centrifuged at 6,000 × g and 4°C for 5 minutes, and recovered again. The bacterial cell pellet was suspended in 300 µL of Buffer A1 containing 15 mg/ml lysozyme (attached to NucleoSpin (registered trademark) Plasmid EasyPure manufactured by MACHEREY-NAGEL GmbH & Co. KG) and shaken at 37°C for 2 hours. Thereafter, a pCG1 plasmid was extracted and purified according to the attached instructions of NucleoSpin (registered trademark) Plasmid EasyPure. Using this as a template, and using a combination of DNAs of base sequences represented by GEP445 (SEQ ID NO: 85) and GEP446 (SEQ ID NO: 86), PCR products were obtained by the PCR method, agarose gel electrophoresis was performed thereon, and then extraction and purification were performed. This is a DNA fragment containing a replication origin, pCGlori for Corynebacterium.

The above three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. The plasmid thus obtained was designated as pGE403.

Using a genomic DNA of the ATCC13032 strain as a template, and using a combination of DNAs of base sequences represented by GEP472 (SEQ ID NO: 87) and GEP478 (SEQ ID NO: 88), PCR products were obtained by the PCR method, agarose gel electrophoresis was performed thereon, and then extraction and purification were performed. In addition, using pFLAG-CTC (manufactured by Sigma-Aldrich) as a template, and using a combination of DNAs of base sequences represented by GEP467 (SEQ ID NO: 89) and GEP468 (SEQ ID NO: 90), PCR products were obtained by the PCR method, agarose gel electrophoresis was performed thereon, and then extraction and purification were performed. These are DNA fragments respectively containing a gap A promoter (PgapA) and an E. coli-ribosomal-RNA-transcriptional terminator (TrrnB) of the ATCC 13032 strain. In addition, pGE403 was treated with BamHI and EcoRV, agarose gel electrophoresis was performed thereon, and then extraction and purification were performed. These three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. The plasmid thus obtained was designated as pGE409.

### 2-2-(2) Construction of pGE1185 (plasmid for creating ΔadhE::hpaI-substituted strain)

ECOS™ Competent E. Coli BL21 (DE3) (manufactured by NIPPON GENE CO., LTD.) was purchased, inoculated into an LB agar medium under aseptic conditions, and cultured at 37°C. The bacterial cells grown on the agar medium were inoculated into 10 mL of the LB medium, and the grown bacterial cells were recovered and subjected to genomic DNA extraction. Using a genomic DNA of the BL21 (DE3) strain as a template, and using a combination of DNAs of base sequences represented by GEP2268 (SEQ ID NO: 91) and GEP2269 (SEQ ID NO: 92), PCR products were obtained by the PCR method, agarose gel electrophoresis was performed thereon, and then extraction and purification were performed. This is a DNA fragment containing a hpaI gene (Ec_hpaI) of the Escherichia coli BL21 (DE3) strain. In addition, pET-15b (manufactured by Novagen) was treated with Ndel, agarose gel electrophoresis was performed thereon, and then extraction and purification were performed. These two DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 100 µg/ml of ampicillin. This transformant was cultured in an LB medium containing 100 µg/ml of ampicillin, and plasmid extraction was performed using the obtained culture suspension. In this manner, a plasmid pGE1031 in which Ec_hpaI was cloned into pET-15b was obtained.

Using pGE1031 as a template, and using a combination of DNAs of base sequences represented by GEP2519 (SEQ ID NO: 93) and GEP2518 (SEQ ID NO: 94), PCR products were obtained by the PCR method, agarose gel electrophoresis was performed thereon, and then extraction and purification were performed. In addition, pGE409 was treated with Ndel and BamHI, agarose gel electrophoresis was performed thereon, and then extraction and purification were performed. These two DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. In this manner, a plasmid pGE1143 in which Ec_hpaI was subcloned into pGE409 was obtained.

Using pGE1143 as a template, and using a combination of DNAs of base sequences represented by GEP2637 (SEQ ID NO: 95) and GEP2638 (SEQ ID NO: 96), PCR products were obtained by the PCR method, agarose gel electrophoresis was performed thereon, and then extraction and purification were performed. This is a DNA fragment containing PgapA-hpaI-TrrnB. In addition, pGE1171 was treated with Xhol, agarose gel electrophoresis was performed thereon, and then extraction and purification were performed. These two DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. The plasmid thus obtained was designated as pGE1185.

### 2-2-(3) Construction of pGE1186 (plasmid for creating Δald::hpaI-substituted strain)

Using pGE1143 as a template, and using a combination of DNAs of base sequences represented by GEP2639 (SEQ ID NO: 97) and GEP2640 (SEQ ID NO: 98), PCR products were obtained by the PCR method, agarose gel electrophoresis was performed thereon, and then extraction and purification were performed. This is a DNA fragment containing PgapA-hpaI-TrrnB. In addition, pGE1172 was treated with Xhol, agarose gel electrophoresis was performed thereon, and then extraction and purification were performed. These two DNA fragments were mixed, and a plasmid obtained by the same operation as in the creation of pGE1185 was designated as pGE1186.

### 2-2-(4) Creation of ΔadhE::hpaI- and Δald::hpaI-substituted strains

GES1041 was transformed by electroporation using pGE1186, and a kanamycin-resistant strain was selected on an A medium containing 25 µg/ml of kanamycin at 33°C. Next, the obtained kanamycin-resistant strain was spread on a Suc agar medium and cultured at 33°C. The grown colonies were further cultured in the A medium, and a strain in which the aid gene was substituted by PgapA-hpaI-TrrnB was selected by colony PCR. This was named GES1052. Furthermore, using pGE1185, GES1052 was transformed by electroporation, and a strain in which PgapA-hpaI-TrrnB was inserted at the position of the adhE gene was selected by the same operation as in the creation of GES1052. This was named GES1063.

Using pGE1186, GES1042 was transformed by electroporation, and a strain in which the aid gene was substituted by PgapA-hpaI-TrrnB was selected by the same operation as in the creation of GES 1052. This was named GES1053. Using pGE1185, GES1053 was transformed by electroporation, and a strain in which PgapA-hpaI-TrrnB was inserted at the position of the adhE gene was selected by the same operation as in the creation of GES1052. This was named GES1064.

### 2-2-(5) Construction of pGE1304 (plasmid for creating ΔadhE::rhmA-substituted strain)

E. Coli DH5α Competent Cells (manufactured by Takara Bio Inc.) was purchased, inoculated into an LB agar medium under aseptic conditions, and cultured at 37°C. The bacterial cells grown on the agar medium were inoculated into 10 mL of the LB medium, and the grown bacterial cells were recovered and subjected to genomic DNA extraction. Using a genomic DNA of the DH5α strain as a template, and using a combination of DNAs of base sequences represented by GEP2765 (SEQ ID NO: 99) and GEP2766 (SEQ ID NO: 100), PCR products were obtained by the PCR method, agarose gel electrophoresis was performed thereon, and then extraction and purification were performed. This is a DNA fragment containing a rhmA gene (Ec_rhmA) of the Escherichia coli DH5α strain. In addition, pGE409 was treated with Ndel and BamHI, agarose gel electrophoresis was performed thereon, and then extraction and purification were performed. These two DNA fragments were mixed, and a plasmid pGE1258 in which Ec_rhmA was cloned into pGE409 was obtained by the same operation as in the creation of GE1143.

Using pGE1258 as a template, and using a combination of DNAs of base sequences represented by GEP2637 (SEQ ID NO: 95) and GEP2638 (SEQ ID NO: 96), PCR products were obtained by the PCR method, agarose gel electrophoresis was performed thereon, and then extraction and purification were performed. This is a DNA fragment containing PgapA-rhmA-TrmB. In addition, pGE1218 was treated with Xhol, agarose gel electrophoresis was performed thereon, and then extraction and purification were performed. These two DNA fragments were mixed, and a plasmid obtained by the same operation as in the creation of pGE1185 was designated as pGE1304.

### 2-2-(6) Construction of pGE1302 (plasmid for creating Δald::rhmA-substituted strain)

Using pGE1258 as a template, and using a combination of DNAs of base sequences represented by GEP2639 (SEQ ID NO: 97) and GEP2640 (SEQ ID NO: 98), PCR products were obtained by the PCR method, agarose gel electrophoresis was performed thereon, and then extraction and purification were performed. In addition, pGE1221 was treated with Xhol, agarose gel electrophoresis was performed thereon, and then extraction and purification were performed. This is a DNA fragment containing PgapA-rhmA-TrmB. These two DNA fragments were mixed, and a plasmid obtained by the same operation as in the creation of pGE1185 was designated as pGE1302.

### 2-2-(7) Creation of ΔadhE::rhmA- and Δald::rhmA-substituted strains

Using pGE1302, GES1041 was transformed by electroporation, and a strain in which the aid gene was substituted by PgapA-rhmA-TrrnB was selected by the same operation as in the creation of GES 1052. This was named GES1215. Furthermore, using pGE1304, GES1215 was transformed by electroporation, and a strain in which PgapA-rhmA-TrrnB was inserted at the position of the adhE gene was selected by the same operation as in the creation of GES1052. This was named GES1242.

### 2-2-(8) Construction of pGE1272 (plasmid for creating ΔadhE::nanA-substituted strain)

E. Coli DH5α Competent Cells (manufactured by Takara Bio Inc.) was purchased, inoculated into an LB agar medium under aseptic conditions, and cultured at 37°C. The bacterial cells grown on the agar medium were inoculated into 10 mL of the LB medium, and the grown bacterial cells were recovered and subjected to genomic DNA extraction. Using a genomic DNA of the DH5α strain as a template, and using a combination of DNAs of base sequences represented by GEP2722 (SEQ ID NO: 101) and GEP2723 (SEQ ID NO: 102), PCR products were obtained by the PCR method, agarose gel electrophoresis was performed thereon, and then extraction and purification were performed. This is a DNA fragment containing a nanA gene (Ec_nanA) of the Escherichia coli DH5α strain. In addition, pGE409 was treated with Ndel and BamHI, agarose gel electrophoresis was performed thereon, and then extraction and purification were performed. These two DNA fragments were mixed, and a plasmid pGE1226 in which Ec_nanA was cloned into pGE409 was obtained by the same operation as in the creation of pGE1143.

Using pGE1226 as a template, and using a combination of DNAs of base sequences represented by GEP2637 (SEQ ID NO: 95) and GEP2638 (SEQ ID NO: 96), PCR products were obtained by the PCR method, agarose gel electrophoresis was performed thereon, and then extraction and purification were performed. This is a DNA fragment containing PgapA-nanA-TrrnB. In addition, pGE1218 was treated with Xhol, agarose gel electrophoresis was performed thereon, and then extraction and purification were performed. These two DNA fragments were mixed, and a plasmid obtained by the same operation as in the creation of pGE1185 was designated as pGE1272.

### 2-2-(9) Construction of pGE1273 (plasmid for creating Δald::nanA-substituted strain)

Using pGE1226 as a template, and using a combination of DNAs of base sequences represented by GEP2639 (SEQ ID NO: 97) and GEP2640 (SEQ ID NO: 98), PCR products were obtained by the PCR method, agarose gel electrophoresis was performed thereon, and then extraction and purification were performed. In addition, pGE1221 was treated with Xhol, agarose gel electrophoresis was performed thereon, and then extraction and purification were performed. This is a DNA fragment containing PgapA-nanA-TrrnB. These two DNA fragments were mixed, and a plasmid obtained by the same operation as in the creation of pGE1185 was designated as pGE1273.

### 2-2-(10) Creation of ΔadhE::nanA- and Δald::nanA-substituted strains

Using pGE1273, GES1041 was transformed by electroporation, and a strain in which the aid gene was substituted by PgapA-nanA-TrrnB was selected by the same operation as in the creation of GES 1052. This was named GES1188. Furthermore, using pGE1272, GES1188 was transformed by electroporation, and a strain in which PgapA-nanA-TrrnB was inserted at the position of the adhE gene was selected by the same operation as in the creation of GES1052. This was named GES1223.

Using pGE1273, GES1042 was transformed by electroporation, and a strain in which the aid gene was substituted by PgapA-nanA-TrrnB was selected by the same operation as in the creation of GES 1052. This was named GES1189. Furthermore, using pGE1272, GES1189 was transformed by electroporation, and a strain in which PgapA-nanA-TrrnB was inserted at the position of the adhE gene was selected by the same operation as in the creation of GES1052. This was named GES1233.

### 2-2-(11) Creation of ΔadhE Δald strains

Using pGE1273, GES1041 was transformed by electroporation, and a strain in which the aid gene was substituted by PgapA-nanA-TrrnB was selected by the same operation as in the creation of GES 1052. This was named GES1188. Furthermore, using pGE1272, GES1188 was transformed by electroporation, and a strain in which PgapA-nanA-TrrnB was inserted at the position of the adhE gene was selected by the same operation as in the creation of GES1052. This was named GES1223.

Using pGE1273, GES 1042 was transformed by electroporation, and a strain in which the aid gene was substituted by PgapA-nanA-TrrnB was selected by the same operation as in the creation of GES 1052. This was named GES1189. Furthermore, using pGE1272, GES1189 was transformed by electroporation, and a strain in which PgapA-nanA-TrrnB was inserted at the position of the adhE gene was selected by the same operation as in the creation of GES1052. This was named GES1233.

### (2-3) Overexpression plasmid

### 2-3-(1) Construction of pGE411

Using pHSG298 (manufactured by Takara Bio Inc.) as a template, and using a combination of DNAs of base sequences represented by GEP433 (SEQ ID NO: 81) and GEP434 (SEQ ID NO: 82), and a combination of DNAs of base sequences represented by GEP437 (SEQ ID NO: 83) and GEP438 (SEQ ID NO: 84), PCR products were obtained by the PCR method, agarose gel electrophoresis was performed thereon, and then extraction and purification were performed. These are DNA fragments respectively containing a kanamycin-resistant gene and a replication origin, pUCori for Escherichia coli.

A Corynebacterium casei strain JCM12072 was obtained from RIKEN BioResource Research Center and cultured according to the attached instructions, and the bacterial cells were recovered by centrifugation. The bacterial cell pellet was suspended in 1 mL of a buffer solution of 10 mM cyclohexylene dinitrilotetraacetic acid and 25 mM Tris-HCI (pH 8.0), centrifuged at 6,000 × g and 4°C for 5 minutes, and recovered again. The bacterial cell pellet was suspended in 300 µL of Buffer A1 containing 15 mg/ml lysozyme (attached to NucleoSpin (registered trademark) Plasmid EasyPure manufactured by MACHEREY-NAGEL GmbH & Co. KG) and shaken at 37°C for 2 hours. Thereafter, a pCASE1 plasmid was extracted and purified according to the attached instructions of NucleoSpin (registered trademark) Plasmid EasyPure. Using this as a template, and using a combination of DNAs of base sequences represented by GEP443 (SEQ ID NO: 103) and GEP444 (SEQ ID NO: 104), PCR products were obtained by the PCR method, agarose gel electrophoresis was performed thereon, and then extraction and purification were performed. This is a DNA fragment containing a replication origin, pCASE1ori for Corynebacterium.

The above three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. The plasmid thus obtained was designated as pGE402.

Using a genomic DNA of the ATCC13032 strain as a template, and using a combination of DNAs of base sequences represented by GEP474 (SEQ ID NO: 105) and GEP477 (SEQ ID NO: 106), PCR products were obtained by the PCR method, agarose gel electrophoresis was performed thereon, and then extraction and purification were performed. In addition, using pFLAG-CTC (manufactured by Sigma-Aldrich) as a template, and using a combination of DNAs of base sequences represented by GEP467 (SEQ ID NO: 89) and GEP468 (SEQ ID NO: 90), PCR products were obtained by the PCR method, agarose gel electrophoresis was performed thereon, and then extraction and purification were performed. These are DNA fragments respectively containing a ldhA promoter (PldhA) and an E. coli-ribosomal-RNA-transcriptional terminator (TrrnB) of the ATCC 13032 strain. In addition, pGE402 was treated with BamHI and EcoRV, agarose gel electrophoresis was performed thereon, and then extraction and purification were performed. These three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. The plasmid thus obtained was designated as pGE411.

### 2-3-(2) Construction of pGE945-2

Using pCR8/GW/TOPO (manufactured by Invitrogen) as a template, and using a combination of DNAs of base sequences represented by GEP435 (SEQ ID NO: 107) and GEP436 (SEQ ID NO: 108), PCR products were obtained by the PCR method, agarose gel electrophoresis was performed thereon, and then extraction and purification were performed. In addition, using pHSG298 (manufactured by Takara Bio Inc.) as a template, and using a combination of DNAs of base sequences represented by GEP437 (SEQ ID NO: 83) and GEP438 (SEQ ID NO: 84), PCR products were obtained by the PCR method, agarose gel electrophoresis was performed thereon, and then extraction and purification were performed. These are DNA fragments respectively containing a spectinomycin-resistant gene and a replication origin, pUCori for Escherichia coli.

The above two DNA fragments, and the DNA fragments containing pCASElori obtained when creating pGE402 were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 100 µg/ml of spectinomycin. This transformant was cultured in an LB medium containing 100 µg/ml of spectinomycin, and plasmid extraction was performed using the obtained culture suspension. The plasmid thus obtained was designated as pGE619.

Using a genomic DNA of the ATCC13032 strain as a template, and using a combination of DNAs of base sequences represented by GEP474 (SEQ ID NO: 105) and GEP477 (SEQ ID NO: 106), PCR products were obtained by the PCR method, agarose gel electrophoresis was performed thereon, and then extraction and purification were performed. In addition, using pFLAG-CTC (manufactured by Sigma-Aldrich) as a template, and using a combination of DNAs of base sequences represented by GEP467 (SEQ ID NO: 89) and GEP2897 (SEQ ID NO: 109), PCR products were obtained by the PCR method, agarose gel electrophoresis was performed thereon, and then extraction and purification were performed. These are DNA fragments respectively containing a ldhA promoter (PldhA) and an E. coli-ribosomal-RNA-transcriptional terminator (TrrnB) of the ATCC 13032 strain. In addition, pGE619 was treated with BamHI and EcoRV, agarose gel electrophoresis was performed thereon, and then extraction and purification were performed. These three DNA fragments were mixed, and a binding reaction of the DNA fragments was performed using an In-Fusion (registered trademark) HD Cloning Kit according to the attached instructions. Using the reaction products, E. coli DH5α Competent Cells (manufactured by Takara Bio Inc.) were transformed according to the attached instructions. A target transformant was selected by culturing on an LB agar medium containing 100 µg/ml of spectinomycin. This transformant was cultured in an LB medium containing 100 µg/ml of spectinomycin, and plasmid extraction was performed using the obtained culture suspension. The plasmid thus obtained was designated as pGE945-2.

### 2-3-(3) Construction of Pp_mdlC overexpression plasmid

A Pseudomonas putida NBRC14164 strain was obtained from the Biotechnology Center of the National Institute of Technology and Evaluation, cultured according to the attached instructions, and then genomic DNA extraction was performed. Using a genomic DNA of the NBRC14164 strain as a template, and using a combination of DNAs of base sequences represented by GEP2550 (SEQ ID NO: 110) and GEP2551 (SEQ ID NO: 111), PCR products were obtained by the PCR method, agarose gel electrophoresis was performed thereon, and then extraction and purification were performed. This is a DNA fragment containing a mdlC gene (Pp_mdlC) of the Pseudomonas putida NBRC14164 strain. In addition, pET-15b (manufactured by Novagen) was treated with Ndel, agarose gel electrophoresis was performed thereon, and then extraction and purification were performed. These two DNA fragments were mixed, and a plasmid pGE 1161 in which Pp_mdlC was cloned into pET-15b was obtained by the same operation as in the creation of pGE1031.

Using pGE1161 as a template, and using a combination of DNAs of base sequences represented by GEP2653 (SEQ ID NO: 112) and GEP2654 (SEQ ID NO: 113), PCR products were obtained by the PCR method, agarose gel electrophoresis was performed thereon, and then extraction and purification were performed. In addition, pGE409 was treated with Ndel, agarose gel electrophoresis was performed thereon, and then extraction and purification were performed. These two DNA fragments were mixed, and a plasmid pGE1197 in which Pp_mdlC was subcloned into pGE409 was obtained by the same operation as in the creation of pGE1185.

### 2-3-(4) Construction of Kp_dhaT overexpression plasmid

A plasmid pHA12-dhaBT containing a gene derived from Klebsiella pneumoniae (DDBJ accession number: LC419022) was provided by Dr. Takahisa Tajima from Hiroshima University. Using this as a template, and using a combination of DNAs of base sequences represented by GEP2529 (SEQ ID NO: 114) and GEP2530 (SEQ ID NO: 115), PCR products were obtained by the PCR method, agarose gel electrophoresis was performed thereon, and then extraction and purification were performed. This is a DNA fragment containing a dhaT gene (Kp_dhaT) of Klebsiella pneumoniae. In addition, pET-15b (manufactured by Novagen) was treated with Ndel, agarose gel electrophoresis was performed thereon, and then extraction and purification were performed. These two DNA fragments were mixed, and a plasmid pGE1150 in which Kp_dhaT was cloned into pET-15b was obtained by the same operation as in the creation of pGE1161.

pGE1150 was treated with Ndel and BamHI, a 1.2 kb DNA fragment was subjected to agarose gel electrophoresis, and then extracted and purified. In addition, pGE945-2 was treated with Ndel and BamHI, agarose gel electrophoresis was performed thereon, and then extraction and purification were performed. These two DNA fragments were mixed, and a binding reaction of the DNA fragments was performed using a Ligation high Ver. 2 (manufactured by TOYOBO CO., LTD.) according to the attached instructions. Using the reaction products, E. coli DH5α Competent Cells (manufactured by Takara Bio Inc.) were transformed according to the attached instructions. A target transformant was selected by culturing on an LB agar medium containing 100 µg/ml of spectinomycin. This transformant was cultured in an LB medium containing 100 µg/ml of spectinomycin, and plasmid extraction was performed using the obtained culture suspension. In this manner, a plasmid pGE1190 in which Kp_dhaT was subcloned into pGE945-2 was obtained.

### 2-3-(5) Construction of plasmid for overexpression of Ll_ldhA Q85C

A *Lactococcus lactis* strain NBRC 100676 was obtained from Biological Resource Center of the National Institute of Technology and Evaluation. After culture was performed according to the attached instructions, genomic DNA was extracted. A PCR product was obtained by a PCR method using the genomic DNA of the strain NBRC 100676 as a template, and using a combination of DNAs having base sequences set forth in GEP2799 (SEQ ID NO: 116) and GEP2800 (SEQ ID NO: 117). This PCR product was subjected to agarose gel electrophoresis, and thereafter, extraction and purification were performed. This resulted product was a DNA fragment containing an ldhA gene (Ll_ldhA) of the *Lactococcus lactis* strain NBRC 100676. Furthermore, pET-15b (manufactured by Novagen) was treated with Ndel, and the resulted product was subjected to agarose gel electrophoresis. Thereafter, extraction and purification were performed. These two DNA fragments were mixed to obtain a plasmid pGE1274 in which Ll_ldhA was cloned into pET-15b by the same operation as in creation of pGE1031.

For the purpose of mutagenesis of lactate dehydrogenase derived from the *Lactococcus lactis* strain NBRC 100676, mutagenesis and plasmid amplification were performed using pGE1274 as a template, using DNA having a base sequence set forth in GEP2803 (SEQ ID NO: 118) and GEP2804 (SEQ ID NO: 119), and using a PrimeSTAR (registered trademark) Mutagenesis Basal Kit (manufactured by Takara Bio Inc.) according to its instructions. Thereby, a plasmid pGE1275 having a sequence of a mutant gene Ll_ldhA Q85C of Ll_ldhA was obtained.

A PCR product was obtained by a PCR method using pGE1275 as a template, and using a combination of DNAs having base sequences set forth in GEP2801 (SEQ ID NO: 120) and GEP2802 (SEQ ID NO: 121). This PCR product was subjected to agarose gel electrophoresis, and thereafter, extraction and purification were performed. Furthermore, pGE945-2 was treated with Ndel and BamHI, and the resulted product was subjected to agarose gel electrophoresis. Thereafter, extraction and purification were performed. These two DNA fragments were mixed, and a binding reaction of the DNA fragments was performed using an In-Fusion (registered trademark) HD Cloning Kit according to its instructions. The reaction product was transformed using *E. coli* DH5α Competent Cells (manufactured by Takara Bio Inc.) according to its instructions. A target transformant was selected by culturing on an LB agar medium containing 100 µg/ml of spectinomycin. This transformant was cultured in an LB medium containing 100 µg/ml of spectinomycin, and plasmid extraction was performed using the obtained culture suspension. In this manner, a plasmid pGE1286 in which Ll_ldhA Q85C was subcloned into pGE945-2 was obtained.

### 2-3-(6) Construction of plasmid for overexpression of Ls_leudh

A *Lysinibacillus sphaericus* strain NBRC 3525 was obtained from Biological Resource Center of the National Institute of Technology and Evaluation. After culture was performed according to the attached instructions, genomic DNA was extracted. A PCR product was obtained by a PCR method using the genomic DNA of the strain NBRC 3525 as a template, and using a combination of DNAs having base sequences set forth in GEP2525 (SEQ ID NO: 122) and GEP2526 (SEQ ID NO: 123). This PCR product was subjected to agarose gel electrophoresis, and thereafter, extraction and purification were performed. This resulted product was a DNA fragment containing an leudh gene (Ls_leudh) of the *Lysinibacillus sphaericus* strain NBRC 3525. Furthermore, pET-15b (manufactured by Novagen) was treated with Ndel, and the resulted product was subjected to agarose gel electrophoresis. Thereafter, extraction and purification were performed. These two DNA fragments were mixed to obtain a plasmid pGE1148 in which Ls_leudh was cloned into pET-15b by the same operation as in creation of pGE1031.

A PCR product was obtained by a PCR method using pGE1148 as a template, and using a combination of DNAs having base sequences set forth in GEP2655 (SEQ ID NO: 124) and GEP2650 (SEQ ID NO: 125). This PCR product was subjected to agarose gel electrophoresis, and thereafter, extraction and purification were performed. Furthermore, pGE945-2 was treated with Ndel and BamHI, and the resulted product was subjected to agarose gel electrophoresis. Thereafter, extraction and purification were performed. These two DNA fragments were mixed to obtain a plasmid pGE1203 in which Ls_leudh was subcloned into pGE945-2 by the same operation as in creation of pGE1286.

### 2-3-(7) Construction of plasmid for overexpression of Sf_valdh

A *Streptomyces fradiae* strain ATCC 19609 was obtained from the American Type Culture Collection. After culture was performed according to the attached instructions, genomic DNA was extracted. A PCR product was obtained by a PCR method using the genomic DNA of the strain ATCC 19609 as a template, and using a combination of DNAs having base sequences set forth in GEP2600 (SEQ ID NO: 126) and GEP2601 (SEQ ID NO: 127). This PCR product was subjected to agarose gel electrophoresis, and thereafter, extraction and purification were performed. This resulted product was a DNA fragment containing a valdh gene (Sf_valdh) of the *Streptomyces fradiae* strain ATCC 19609. Furthermore, pET-15b (manufactured by Novagen) was treated with Ndel, and the resulted product was subjected to agarose gel electrophoresis. Thereafter, extraction and purification were performed. These two DNA fragments were mixed to obtain a plasmid pGE1175 in which Sf_valdh was cloned into pET-15b by the same operation as in creation of pGE1031.

### 2-3-(8) Construction of plasmid for overexpression of Bb_phedh

A *Bacillus badius* strain NBRC 15713 was obtained from Biological Resource Center of the National Institute of Technology and Evaluation. After culture was performed according to the attached instructions, genomic DNA was extracted. A PCR product was obtained by a PCR method using the genomic DNA of the strain NBRC 15713 as a template, and using a combination of DNAs having base sequences set forth in GEP2678 (SEQ ID NO: 128) and GEP2679 (SEQ ID NO: 129). This PCR product was subjected to agarose gel electrophoresis, and thereafter, extraction and purification were performed. This resulted product was a DNA fragment containing a phedh gene (Bb_phedh) of the *Bacillus badius* strain NBRC 15713. Furthermore, pET-15b (manufactured by Novagen) was treated with Ndel, and the resulted product was subjected to agarose gel electrophoresis. Thereafter, extraction and purification were performed. These two DNA fragments were mixed to obtain a plasmid pGE1237 in which Bb_phedh was cloned into pET-15b by the same operation as in creation of pGE1031.

### 2-3-(9) Construction of plasmid for overexpression of AtCGS1

pMK-AtCGS was purchased from Thermo Fisher Scientific Inc. pMK-AtCGS is a plasmid containing synthetic DNA in which expression of a sequence after the 69th amino acid in an amino acid sequence encoded by a CGS1 gene of *Arabidopsis thaliana* (Biochem. J.331, 639-648 (1998), DDBJ accession number: U43709) is optimized in *Corynebacterium glutamicum.* A PCR product was obtained by a PCR method using this plasmid as a template, and using a combination of DNAs having base sequences set forth in GEP1053 (SEQ ID NO: 130) and GEP1054 (SEQ ID NO: 131). This PCR product was subjected to agarose gel electrophoresis, and thereafter, extraction and purification were performed. This resulted product was a DNA fragment containing a CGS1 gene (AtCGS1) of *Arabidopsis thaliana* with optimized expression in *Corynebacterium glutamicum.* Furthermore, pGE411 was treated with Ndel, and the resulted product was subjected to agarose gel electrophoresis. Thereafter, extraction and purification were performed. These two DNA fragments were mixed to obtain a plasmid pGE479 in which AtCGS1 was subcloned into pGE411 by the same operation as in creation of pGE1185.

pGE479 was treated with NdeI and BamHI, and a 1.5-kb DNA fragment was subjected to agarose gel electrophoresis. Thereafter, extraction and purification were performed. Furthermore, pGE409 was treated with Ndel and BamHI, and the resulted product was subjected to agarose gel electrophoresis. Thereafter, extraction and purification were performed. These two DNA fragments were mixed, and a binding reaction of the DNA fragments was performed using Ligation high Ver.2 (manufactured by TOYOBO CO., LTD.) according to its instructions. The reaction product was transformed using *E. coli* DH5α Competent Cells (manufactured by Takara Bio Inc.) according to its instructions. A target transformant was selected by culturing on an LB agar medium containing 50 µg/ml of kanamycin. This transformant was cultured in an LB medium containing 50 µg/ml of kanamycin, and plasmid extraction was performed using the obtained culture suspension. In this manner, a plasmid pGE513 in which AtCGS1 was subcloned into pGE409 was obtained.

### 2-3-(10) Construction of plasmids for overexpression of various aldolase enzymes

PCR products were obtained by a PCR method using the genomic DNA of the strain DH5α as a template, and using a combination of DNAs having base sequences set forth in GEP2712 (SEQ ID NO: 132) and GEP2713 (SEQ ID NO: 133), a combination of DNAs having base sequences set forth in GEP2714 (SEQ ID NO: 134) and GEP2715 (SEQ ID NO: 135), a combination of DNAs having base sequences set forth in GEP2720 (SEQ ID NO: 136) and GEP2721 (SEQ ID NO: 137), a combination of DNAs having base sequences set forth in GEP2726 (SEQ ID NO: 138) and GEP2727 (SEQ ID NO: 139), and a combination of DNAs having base sequences set forth in GEP2767 (SEQ ID NO: 140) and GEP2768 (SEQ ID NO: 141). These PCR products were subjected to agarose gel electrophoresis, and thereafter, extraction and purification were performed. These resulted products were respectively DNA fragments containing an eda gene (Ec_eda), a yjjJ gene (Ec_yjhH), a dgoA gene (Ec_dgoA), an mhpFE gene (Ec_mhpFE), and a garL gene (Ec_garL) of the *Escherichia coli* strain DH5α. Furthermore, pGE409 was treated with Ndel and BamHI, and the resulted product was subjected to agarose gel electrophoresis. Thereafter, extraction and purification were performed. A DNA fragment having various genes and a DNA fragment derived from pGE409 were mixed to obtain plasmids pGE1223, pGE1224, pGE1225, pGE1227, and pGE1259 in which Ec_eda, Ec_yjhH, Ec_dgoA, Ec_mhpFE, Ec_garL were respectively cloned into pGE409 by the same operation as in creation of pGE1185.

A PCR product was obtained by a PCR method using the genomic DNA of a *Pseudomonas putida* strain NBRC 14164 as a template, and using a combination of DNAs having base sequences set forth in GEP2769 (SEQ ID NO: 142) and GEP2770 (SEQ ID NO: 143). This PCR product was subjected to agarose gel electrophoresis, and thereafter, extraction and purification were performed. This resulted product was a DNA fragment containing a galC gene (Pp_galC) of the *Pseudomonas putida* strain NBRC 14164. Furthermore, pGE409 was treated with Ndel and BamHI, and the resulted product was subjected to agarose gel electrophoresis. Thereafter, extraction and purification were performed. These two DNA fragments were mixed to obtain a plasmid pGE1260 in which Pp_galC was cloned into pGE409 by the same operation as in creation of pGE1185.

A strain NBRC 109351 of the genus *Nocardioides* was obtained from Biological Resource Center of the National Institute of Technology and Evaluation. After culture was performed according to the attached instructions, genomic DNA was extracted. A PCR product was obtained by a PCR method using the genomic DNA of the strain NBRC 109351 as a template, and using a combination of DNAs having base sequences set forth in GEP2716 (SEQ ID NO: 144) and GEP2717 (SEQ ID NO: 145). This PCR product was subjected to agarose gel electrophoresis, and thereafter, extraction and purification were performed. This resulted product was a DNA fragment containing a phdJ gene (Ns_phdJ) of the strain NBRC 109351 of the genus *Nocardioides.* Furthermore, pGE409 was treated with Ndel and BamHI, and the resulted product was subjected to agarose gel electrophoresis. Thereafter, extraction and purification were performed. These two DNA fragments were mixed to obtain a plasmid pGE1239 in which Ns_phdJ was cloned into pGE409 by the same operation as in creation of pGE1185.

A *Burkholderia xenovorans* strain DSMZ 17367 was obtained from the Deutsche Sammlung von Mikroorganismen und Zellkulturen GmbH. After culture was performed according to the attached instructions, genomic DNA was extracted. A PCR product was obtained by a PCR method using the genomic DNA of the strain DSMZ 17367 as a template, and using a combination of DNAs having base sequences set forth in GEP2771 (SEQ ID NO: 146) and GEP2772 (SEQ ID NO: 147). This PCR product was subjected to agarose gel electrophoresis, and thereafter, extraction and purification were performed. This resulted product was a DNA fragment containing a bphJI gene (Bx_bphJI) of the *Burkholderia xenovorans* strain DSMZ 17367. Furthermore, pGE409 was treated with Ndel and BamHI, and the resulted product was subjected to agarose gel electrophoresis. Thereafter, extraction and purification were performed. These two DNA fragments were mixed to obtain a plasmid pGE1262 in which Bx_bphJI was cloned into pGE409 by the same operation as in creation of pGE1185.

### 3. Bacterial cell construction

### (3-1) Creation of strain into which Pp_mdlC and Kp_dhaT overexpression plasmid is introduced

GES1070 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1206.

GES1063 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1077.

GES1064 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1068.

Corynebacterium glutamicum GES1068 was deposited in NITE Patent Microorganisms Depositary, NPMD (2-5-8 Kazusakamatari, Kisarazu City, Chiba, Japan (postal code: 292-0818)) (Deposit Date: September 13, 2018, Accession Number: NITE BP-02780). GES1223 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1253.

GES1233 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1254.

Corynebacterium glutamicum GES1254 was deposited in NITE Patent Microorganisms Depositary, NPMD (2-5-8 Kazusakamatari, Kisarazu City, Chiba, Japan (postal code: 292-0818)) (Deposit Date: September 13, 2018, Accession Number: NITE BP-02781).

GES1242 was transformed by electroporation using pGE1197 and pGE1190, and a kanamycin/spectinomycin-resistant strain was selected on an A agar medium containing 25 µg/ml of kanamycin and 100 µg/ml of spectinomycin at 33°C. This was named GES1282.

### (3-2) Creation of strain into which Ll_ldhA Q85C overexpression plasmid is introduced

GES1064 was transformed by electroporation using pGE1286, and a spectinomycin-resistant strain was selected on an A agar medium containing 100 µg/ml of spectinomycin at 33°C. This was named GES1232.

Corynebacterium glutamicum GES1232 was deposited in NITE Patent Microorganisms Depositary, NPMD (2-5-8 Kazusakamatari, Kisarazu City, Chiba, Japan (postal code: 292-0818)) (Deposit Date: September 13, 2018, Accession Number: NITE BP-02782).

GES1211 was transformed by electroporation using pGE1286, and a spectinomycin-resistant strain was selected on an A agar medium containing 100 µg/ml of spectinomycin at 33°C. This was named GES1294.

### (3-3) Creation of strain into which Ls_leudh overexpression plasmid is introduced

GES1064 was transformed by electroporation using pGE1203, and a spectinomycin-resistant strain was selected on an A medium containing 100 µg/ml of spectinomycin at 33°C. This was named GES1073.

Corynebacterium glutamicum GES1073 was deposited in NITE Patent Microorganisms Depositary, NPMD (2-5-8 Kazusakamatari, Kisarazu City, Chiba, Japan (postal code: 292-0818)) (Deposit Date: September 13, 2018, Accession Number: NITE BP-02783).

GES1211 was transformed by electroporation using pGE1203, and a spectinomycin-resistant strain was selected on an A medium containing 100 µg/ml of spectinomycin at 33°C. This was named GES1295.

### (3-4) Creation of strain into which Ls_leudh and AtCGS1 expression plasmid is introduced

GES1064 was transformed by electroporation using pGE1203 and pGE513, and a kanamycin/spectinomycin-resistant strain was selected on an A agar medium containing 25 µg/ml of kanamycin and 100 µg/ml of spectinomycin at 33°C. This was named GES1097.

Corynebacterium glutamicum GES1097 was deposited in NITE Patent Microorganisms Depositary, NPMD (2-5-8 Kazusakamatari, Kisarazu City, Chiba, Japan (postal code: 292-0818)) (Deposit Date: September 13, 2018, Accession Number: NITE BP-02784).

### (3-5) Creation of strains into which different aldolase expression plasmids are introduced

GES435 was transformed by electroporation using each of pGE1143, pGE1223, pGE1224, pGE1225, pGE1226, pGE1227, pGE1239, pGE1258, pGE1259, pGE1260 and pGE1262, and kanamycin/spectinomycin-resistant strains were selected on an A agar medium containing 25 µg/ml of kanamycin at 33°C. These were named GES1009, GES1119, GES1120, GES1121, GES1122, GES1123, GES1127, GES1154, GES1155, GES1156 and GES1157, respectively.

### 4. Material production experiment

### (4-1) Production of 4-hydroxy-2-oxobutyric acid (HOB)

### 4-1-(1) Culture of GES1077

A glycerol stock of GES1077 was inoculated under aseptic conditions into an A agar medium containing 25 µg/ml of kanamycin and 100 µg/ml of spectinomycin, and cultured at 33°C. Bacterial cells grown on the agar medium were inoculated into 10 mL of a BA medium containing 25 µg/ml of kanamycin and 100 µg/ml of spectinomycin and shaking-cultured at 33°C, and thereby a seed culture solution was obtained. This seed culture was inoculated into 500 mL of a BA medium containing 25 µg/ml of kanamycin and 100 µg/ml of spectinomycin prepared in a 2 L flask so that a turbidity was 0.1. The mixture was shaken at 33°C and cultured overnight. This main culture solution was centrifuged at 4°C and 5500 × g for 15 minutes, and the supernatant was removed. The wet bacterial cells thus obtained were used for the following reaction.

### 4-1-(2) Production of HOB using GES1077

The wet bacterial cells prepared as described above were suspended in BR buffer so that a concentration thereof became 10% (w/v). A 50% (w/v) aqueous glucose solution and a 1.2 M aqueous formaldehyde solution were added to this bacterial cell suspension so that their concentrations respectively became 50 mM and 40 mM, and cultured at 33°C while adjusting a pH to 6.0 using a 1.0 M aqueous potassium hydroxide solution. At this concentration of the wet bacterial cells, anaerobic conditions or microaerobic conditions would naturally be achieved by keeping the level of stirring to the extent at which the aqueous potassium hydroxide solution is efficiently mixed and at performing no aeration. When the supernatant of the reaction solution was analyzed by liquid chromatography for organic acid analysis after 3 hours, 0.65 mM of HOB was produced.

### 4-1-(3) Comparative Example 1

The wet bacterial cells prepared as described above were suspended in BR buffer so that a concentration thereof became 10% (w/v). A 50% (w/v) aqueous glucose solution was added to this bacterial cell suspension so that its concentration became 50 mM, and the reaction was performed at 33°C while adjusting a pH to 6.0 using a 1.0 M aqueous potassium hydroxide solution. When the supernatant of the reaction suspension was analyzed by liquid chromatography for organic acid analysis after 3 hours, HOB was not detected.

### 4-1-(4) Comparative Example 2

For GES1206, the same experiment as in 4-1-(1) and 4-1-(2) was performed. When the supernatant of the reaction suspension was analyzed by liquid chromatography for organic acid analysis 3 hours after the reaction, HOB was not detected.

Based on the results of 4-1-(2), 4-1-(3), and 4-1-(4), it was found that when bacterial cells having a gene encoding a class II aldolase such as GES1077 are cultured in the presence of saccharides and aldehydes, pyruvic acid is generated from saccharides, and when the enzyme catalyzes the aldol reaction between this pyruvic acid and aldehydes, an aldol compound typified by HOB is generated.

### 4-1-(5) Culture of GES1282

Wet bacterial cells obtained by culturing a glycerol stock of GES1282 in the same manner as in 4-1-(1) were used in the following reaction.

### 4-1-(6) Production of HOB using GES1282

The wet bacterial cells prepared as described above were cultured in the same manner as in 4-1-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for organic acid analysis after 3 hours, 0.36 mM of HOB was produced.

### 4-1-(7) Comparative Example 1

The wet bacterial cells prepared as described above were cultured in the same manner as in 4-1-(3). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction suspension was analyzed by liquid chromatography for organic acid analysis after 3 hours, HOB was not detected.

Based on the results of 4-1-(6), 4-1-(7), and 4-1-(4), it was found that when bacterial cells having a gene encoding a class II aldolase such as GES1282 which is different from GES1077 are cultured in the presence of saccharides and aldehydes, pyruvic acid is also generated from saccharides, and when the enzyme catalyzes the aldol reaction between this pyruvic acid and aldehydes, an aldol compound typified by HOB is generated.

### 4-1-(8) Culture of GES1253

Wet bacterial cells obtained by culturing a glycerol stock of GES1253 in the same manner as in 4-1-(1) were used in the following reaction.

### 4-1-(9) Production of HOB using GES1253

The wet bacterial cells prepared as described above were cultured in the same manner as in 4-1-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for organic acid analysis after 3 hours, 0.34 mM of HOB was produced.

### 4-1-(10) Comparative Example 1

The wet bacterial cells prepared as described above were cultured in the same manner as in 4-1-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction suspension was analyzed by liquid chromatography for organic acid analysis after 3 hours, HOB was not detected.

Based on the results of 4-1-(9), 4-1-(10), and 4-1-(4), it was found that when bacterial cells having a gene encoding a class I aldolase such as GES1253 are cultured in the presence of saccharides and aldehydes, pyruvic acid is generated from saccharides, and when the enzyme catalyzes the aldol reaction between this pyruvic acid and aldehydes, an aldol compound typified by HOB is generated.

### 4-1-(11) Culture of GES1068 and GES1254

Wet bacterial cells obtained by culturing a glycerol stock of GES1068 and GES1254 in the same manner as in 4-1-(1) were used in the following reaction.

### 4-1-(12) Production of HOB using GES1068 and GES1254

The wet bacterial cells prepared as described above were cultured in the same manner as in 4-1-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for organic acid analysis after 3 hours, 1.2 mM of HOB was produced in the reaction using GES1068, and 0.42 mM of HOB was produced in the reaction using GES1254.

Based on the results of 4-1-(12), it was found that even when a microorganism having a genotype different from GES1077 and GES1253, such as GES1068 and GES1254, are cultured in the presence of saccharides and aldehydes, as long as it is a bacterial cell having a gene encoding a class II aldolase, pyruvic acid is also generated from saccharides, and when the enzyme catalyzes the aldol reaction between this pyruvic acid and aldehydes, an aldol compound typified by HOB is generated.

### (4-2) Production of 1,3-propanediol (PDO)

### 4-2-(1) Culture of GES1077 and GES1068

A glycerol stock of GES1077 and GES1068 was inoculated under aseptic conditions into an A agar medium containing 25 µg/ml of kanamycin and 100 µg/ml of spectinomycin, and cultured at 33°C. Bacterial cells grown on the agar medium were inoculated into 10 mL of a BA medium containing 25 µg/ml of kanamycin and 100 µg/ml of spectinomycin and shaking-cultured at 33°C, and thereby a seed culture solution was obtained. This seed culture was inoculated into 500 mL of a BA medium containing 25 µg/ml of kanamycin and 100 µg/ml of spectinomycin prepared in a 2 L flask so that a turbidity was 0.1. The mixture was shaken at 33°C and cultured overnight. This main culture solution was centrifuged at 4°C and 5500 × g for 15 minutes, and the supernatant was removed. The wet bacterial cells thus obtained were used for the following reaction.

### 4-2-(2) Production of PDO using GES1077 and GES1068

The wet bacterial cells prepared as described above were suspended in BR buffer so that a concentration thereof became 10% (w/v). A 50% (w/v) aqueous glucose solution and a 1.2 M aqueous formaldehyde solution were added to this bacterial cell suspension so that their concentrations respectively became 50 mM and 40 mM, and cultured at 33°C while adjusting a pH to 6.0 using a 1.0 M aqueous potassium hydroxide solution. In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for sugar analysis after 3 hours, 7.4 mM of PDO was produced in the reaction using GES1077, and 11.4 mM of PDO was produced in the reaction using GES1068.

Based on the above results, it was found that in a case where a bacterial cell, which has a gene encoding a class II aldolase such as GES1077 and GES1068 and which can produce HOB when being cultured in the presence of saccharides and formaldehydes, further has a gene encoding decarboxylase and alcohol dehydrogenase, HOB can be further converted to PDO, and thereby PDO can be manufactured.

### 4-2-(3) Culture of GES1282

Wet bacterial cells obtained by culturing a glycerol stock of GES1282 in the same manner as in 4-2-(1) were used in the following reaction.

### 4-2-(4) Production of PDO using GES1282

The wet bacterial cells prepared as described above were cultured in the same manner as in 4-2-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for sugar analysis after 6 hours, 11.4 mM of PDO was produced.

Based on the above results, it was found that in a case where a bacterial cell, which has a gene encoding a class II aldolase different from GES1077 and GES1068, such as GES 1282, and which can produce HOB when being cultured in the presence of saccharides and formaldehydes, further has a gene encoding decarboxylase and alcohol dehydrogenase, HOB can also be further converted to PDO, and thereby PDO can be manufactured.

### 4-2-(5) Culture of GES1253 and GES1254

Wet bacterial cells obtained by culturing a glycerol stock of GES1253 and GES1254 in the same manner as in 4-2-(1) were used in the following reaction.

### 4-2-(6) Production of PDO using GES1253 and GES1254

The wet bacterial cells prepared as described above were cultured in the same manner as in 4-2-(2). In the culture, stirring was performed to efficiently mix the aqueous potassium hydroxide solution, and aeration was not performed. By this operation, anaerobic conditions or microaerobic conditions were achieved at the above-mentioned concentration of the wet bacterial cells. When the supernatant of the reaction solution was analyzed by liquid chromatography for sugar analysis after 6 hours, 4.1 mM of PDO was produced in the reaction using GES1253, and 5.8 mM of PDO was produced in the reaction using GES1254.

Based on the above results, it was found that in a case where a bacterial cell, which has a gene encoding a class I aldolase such as GES1253 and GES1254 and which can produce HOB when being cultured in the presence of saccharides and formaldehydes, further has a gene encoding decarboxylase and alcohol dehydrogenase, HOB can be further converted to PDO, and thereby PDO can be manufactured.

### (4-3) Production of 1,3-butanediol (BDO)

### 4-3-(1) Culture of GES1068

Wet bacterial cells obtained by culturing from a glycerol stock of GES1068 in the same manner as in 4-2-(1) were used for the following reaction.

### 4-3-(2) Production of BDO using GES1068

The wet bacterial cells prepared as described above were suspended in a BR buffer such that a concentration thereof was 10% (w/v). An aqueous solution of 50% (w/v) glucose and an aqueous solution of 1.2 M acetaldehyde were respectively added to this bacterial cell suspension such that final concentrations were respectively set to 50 mM and 40 mM, and culture was performed at 33°C while maintaining a pH to 6.0 using an aqueous solution of 1.0 M potassium hydroxide. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 3 hours by liquid chromatography for sugar analysis, 8.2 mM of BDO was produced.

Based on the above results, it was found that BDO can be produced by culturing bacterial cells having a gene, such as GES1068, which encodes aldolase, decarboxylase, and alcohol dehydrogenase in the presence of saccharides and acetaldehyde.

In 4-2-(2), when the reaction was continued even after the reaction time exceeded 3 hours and a concentration of formaldehyde decreased, 3.5 mM of BDO was produced after 22 hours by the reaction in which GES1077 was used, and 2.7 mM of BDO was produced after 22 hours by the reaction in which GES1068 was used. It is thought that the reason for this is because when a concentration of formaldehyde decreases to a concentration at which formaldehyde cannot participate in an aldolase reaction, acetaldehyde is generated by a catalytic activity of mdlC (decarboxylase) against pyruvic acid, this causes an aldol reaction with another molecule of pyruvic acid, 4-hydroxy-2-oxovalerate (HOV) is generated, this 4-hydroxy-2-oxovalerate is further decarboxylated and reduced by a catalytic activity of mdlC and dhaT, and thereby BDO is generated. Accordingly, BDO can be produced by culturing bacterial cells having a gene encoding aldolase and further having a gene encoding decarboxylase and alcohol dehydrogenase using only saccharides as a carbon source. In fact, when culture was performed using GES1077 under conditions described in 4-2-(2) except that formaldehyde was not added, 0.60 mM of BDO was produced 22 hours after the culture.

### (4-4) Production of various 1,3-diols

### 4-4-(1) Culture of GES1068

Wet bacterial cells obtained by culturing from a glycerol stock of GES1068 in the same manner as in 4-2-(1) were used for the following reaction.

### 4-4-(2) Production of various 1,3-diols using GES1068

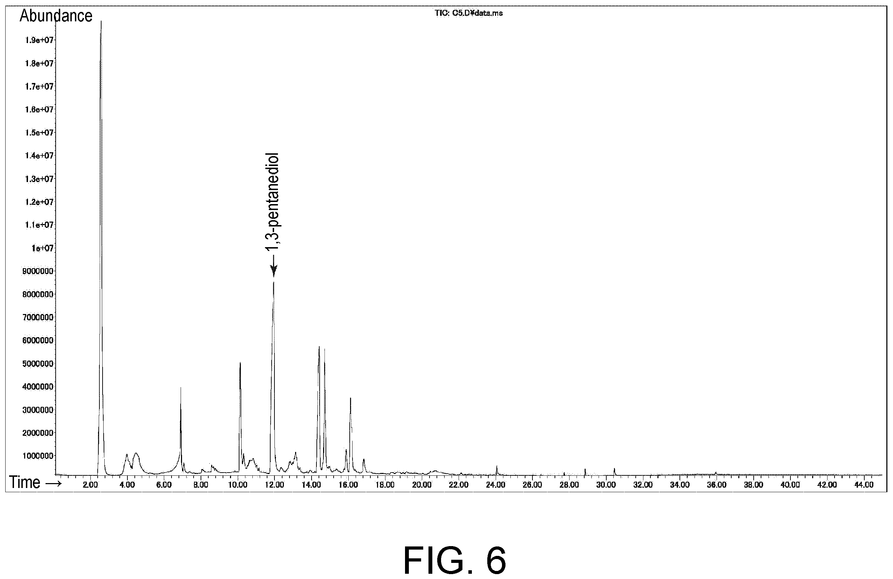

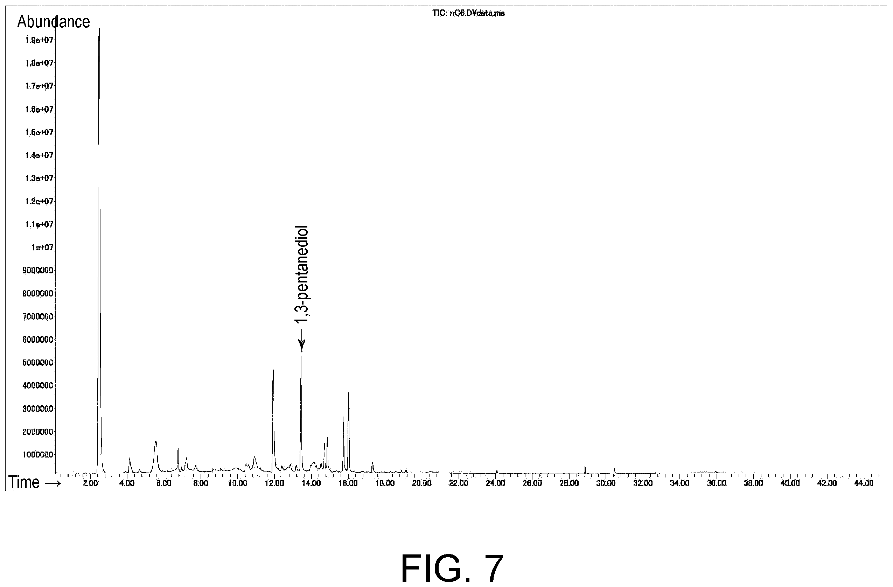

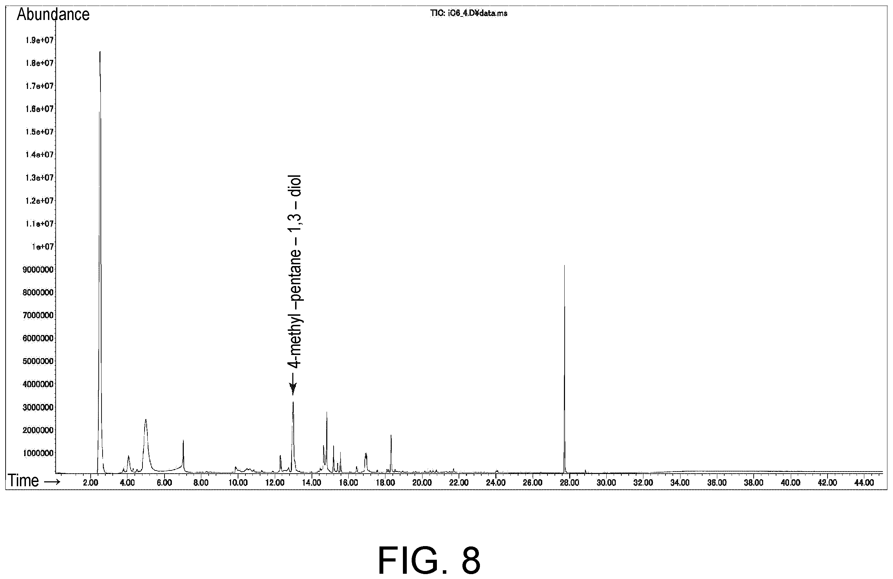

The wet bacterial cells prepared as described above were suspended in a BR buffer such that a concentration thereof was 10% (w/v). An aqueous solution of 50% (w/v) glucose and aldehydes (propionaldehyde, n-butyraldehyde, isobutyraldehyde, n-valeraldehyde) were respectively added to this bacterial cell suspension such that final concentrations were respectively set to 50 mM and 40 mM, and culture was performed at 33°C while maintaining a pH to 6.0 using an aqueous solution of 1.0 M potassium hydroxide. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 3 hours by GCMS, 1,3-pentanediol, 1,3-hexanediol, 4-methyl-pentane-1,3-diol, and 1,3-heptanediol respectively derived from propionaldehyde, n-butyraldehyde, isobutyraldehyde, and n-valeraldehyde were respectively confirmed in each of the reactions. FIG. 6 shows results of gas chromatography-mass spectrometry when propionaldehyde was used. FIG. 7 shows results of gas chromatography-mass spectrometry when n-butyraldehyde was used. FIG. 8 shows results of gas chromatography-mass spectrometry when isobutyraldehyde was used. FIG. 8 shows results of gas chromatography-mass spectrometry when n-valeraldehyde was used.

Based on the above results, it was found that it is possible to produce 1,3-pentanediol, 1,3-hexanediol, 4-methyl-pentane-1,3-diol, and 1,3-heptanediol by culturing bacterial cells having a gene, such as GES1068, which encodes aldolase, decarboxylase, and alcohol dehydrogenase in the presence of saccharides and propionaldehyde, or saccharides and n-butyraldehyde, or saccharides and isobutyraldehyde, or saccharides and n-valeraldehyde.

In addition, in general, the following is expected: bacterial cells having a gene, such as GES1068, which encodes aldolase, decarboxylase, and alcohol dehydrogenase are cultured in the presence of saccharides and aldehydes, pyruvic acid is produced from the saccharides, 4-hydroxy-2-oxocarboxylic acid, which is an aldol compound, is produced from the reaction of the pyruvic acid and the aldehydes, the 4-hydroxy-2-oxocarboxylic acid is converted into a 3-hydroxyaldehyde compound and then into 1,3-diol, and finally 1,3-diol is produced.

### (4-5) Production of 2,4-dihydroxybutyric acid (DHB)

### 4-5-(1) Culture of GES1232

A glycerol stock of GES1232 was inoculated into a A-agar medium containing 100 µg/mL of spectinomycin under aseptic conditions, and culture was performed at 33°C. Bacterial cells grown on the agar medium were inoculated into 10 mL of a BA medium containing 100 µg/mL of spectinomycin, and shaking culture was performed at 33°C to prepare a seed culture solution. This seed culture solution was inoculated into 500 mL of a BA medium containing 100 µg/mL of spectinomycin and prepared in a 2 L flask so that a turbidity was 0.1. Shaking culture was performed at 33°C overnight. This main culture solution was centrifuged at 5,500 xg at 4°C for 15 minutes to remove the supernatant. The wet bacterial cells thus obtained were used in the following reaction.

### 4-5-(2) Production of DHB using GES1232

The wet bacterial cells prepared as described above were suspended in a BR buffer such that a concentration thereof was 10% (w/v). An aqueous solution of 50% (w/v) glucose and an aqueous solution of 1.2 M formaldehyde were respectively added to this bacterial cell suspension such that final concentrations were respectively set to 100 mM and 40 mM, and culture was performed at 33°C while maintaining a pH to 7.0 using an aqueous solution of 1.0 M potassium hydroxide. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 3 hours by liquid chromatography for organic acid analysis, 11 mM of DHB was produced.

### 4-5-(3) Comparative Example 1

The wet bacterial cells prepared as described above were suspended in a BR buffer such that a concentration thereof was 10% (w/v). An aqueous solution of 50% (w/v) glucose was added to this bacterial cell suspension such that a final concentration was set to 100 mM, and a reaction was caused at 33°C while maintaining a pH to 7.0 using an aqueous solution of 1.0 M potassium hydroxide. When the supernatant of the reaction solution was analyzed after 3 hours by liquid chromatography for organic acid analysis, DHB was not detected.

### 4-5-(4) Comparative Example 2

For GES1294, the same experiments as in (1) and (2) were performed. When the supernatant of the reaction suspension was analyzed 3 hours after the reaction by liquid chromatography for organic acid analysis, DHB was not detected.

Based on the above results, the following was found: when bacterial cells, which have a gene, such as GES 1232, encoding aldolase and which are capable of producing HOB when being cultured in the presence of saccharides and formaldehyde, further have a gene encoding dehydrogenase having HOB reducing activity, HOB is further converted into DHB by culturing these bacterial cells in the presence of saccharides and formaldehyde, and thereby DHB can be produced.

### (4-6) Confirmation of activity against L-homoserine N-dehydrogenase

### 4-6-(1) Measurement of expression and oxidative deamination reaction activity of leucine dehydrogenase

Transformation was performed using pGE1148 and using ECOSTM Competent E. coli BL21 (DE3) (manufactured by NIPPON GENE CO., LTD.) according to its instructions, and the obtained strain was designated as GES1007. GES1007 was inoculated into an LB agar medium containing 100 µg/mL of ampicillin under aseptic conditions, and culture was performed at 37°C. Bacterial cells grown on the agar medium were inoculated into 10 mL of an LB medium containing 100 µg/mL of ampicillin, and shaking culture was performed at 37°C to prepare a seed culture solution. This seed culture solution was inoculated into 100 mL of an LB medium containing 100 µg/mL of ampicillin and prepared in a 500 mL flask so that a turbidity was 0.1. Shaking was performed at 37°C, and culture was performed until a turbidity was 0.7. IPTG was added so that a final concentration was 0.2 mM, and shaking culture was performed overnight at 16°C. This main culture solution was centrifuged at 6,000 xg at 4°C for 5 minutes to remove the supernatant. The wet bacterial cells thus obtained were once frozen at -80°C and then thawed at room temperature. The cells were suspended in 4.5 mL of a 50 mM sodium phosphate buffer (pH 8.0), 500 mM sodium chloride, and 10% glycerol, and 2.0 g of glass beads YGB01 Φ0.1 mm (manufactured by Yasui Kikai Corporation) was added thereinto. Bacterial cells were crushed at 2,500 rpm and 4°C using a multi-bead shocker MB1001C (S) (manufactured by Yasui Kikai Corporation). Centrifugal separation was performed at 18,000 xg at 4°C for 3 minutes, and the supernatant was recovered. When a protein concentration in this solution in which the cells were crushed was measured, it was 12 mg/mL.

Using this solution in which the bacterial cells were crushed, an oxidative deamination reaction activity against L-homoserine was measured. As the reaction conditions, an appropriately diluted solution in which the bacterial cells were crushed was mixed with 100 mM glycine-KCl-KOH (pH 10.0), 2.5 mM NAD⁺, and 50 mM L-homoserine, and a continuous increase in absorption by NADH at 340 nm was measured using a Varioskan LUX (manufactured by Thermo Fisher Scientific Inc.). As a result, a specific activity of this solution in which the bacterial cells were crushed against L-homoserine was 0.21 U/mg of protein.

### 4-6-(2) Measurement of expression and oxidative deamination reaction activity of valine dehydrogenase

Transformation was performed using pGE1175 and using ECOSTM Competent E. coli BL21 (DE3) (manufactured by NIPPON GENE CO., LTD.) according to its instructions, and the obtained strain was designated as GES 1057. When a protein concentration in a solution, in which the cells were crushed and which was obtained by subjecting GES1057 to the same operation as that of GES 1007, was measured, it was 4.2 mg/mL.

As a result of measuring an oxidative deamination reaction activity against L-homoserine in the same manner as in 4-6-(1) using this solution in which the bacterial cells were crushed, a specific activity of this solution in which the bacterial cells were crushed against L-homoserine was 0.21 U/mg of protein.

### 4-6-(3) Measurement of expression and oxidative deamination reaction activity of phenylalanine dehydrogenase

Transformation was performed using pGE1237 and using ECOSTM Competent E. coli BL21 (DE3) (manufactured by NIPPON GENE CO., LTD.) according to its instructions, and the obtained strain was designated as GES 1136. When a protein concentration in a solution, in which the cells were crushed and which was obtained by subj ecting GES1136 to the same operation as that of GES 1007, was measured, it was 11 mg/mL.

As a result of measuring an oxidative deamination reaction activity against L-homoserine in the same manner as in 4-6-(1) using this solution in which the bacterial cells were crushed, a specific activity of this solution in which the bacterial cells were crushed against L-homoserine was 0.045 U/mg of protein.

Based on the results of 4-6-(1), 4-6-(2), and 4-6-(3), it was found that leucine dehydrogenase, valine dehydrogenase, and phenylalanine dehydrogenase all have the activity of oxidizing L-homoserine to HOB. In general, amino acid dehydrogenase catalyzes both of an oxidative deamination reaction and a reductive amination reaction, and activity intensities thereof are correlated (for example, Biochim. Biophys. Acta 96, 248-262 (1965), J. Biol. Chem. 253, 5719-5725 (1978), Eur. J. Biochem. 100, 29-39 (1979)). Accordingly, it is expected that leucine dehydrogenase, valine dehydrogenase, and phenylalanine dehydrogenase all catalyze a reductive amination reaction from HOB to L-homoserine. For confirmation, leucine dehydrogenase was purified, and a reductive amination reaction of HOB was subjected to analysis of enzyme kinetics.

### 4-6-(4) Purification and reductive amination reaction activity of leucine dehydrogenase

The solution in which the bacterial cells were crushed and which was obtained in 4-6-(1) was subjected to a filtration treatment using a syringe filter with a pore size of 0.2 µm (manufactured by Sartorius AG). Fractionation was performed using an AKTA purifier system (manufactured by GE Healthcare) to which a HisTrap™ FF 1 mL column was connected, and using 50 mM sodium phosphate buffer (pH 8.0), 500 mM sodium chloride, and 10% glycerol as adsorption buffers, and 50 mM sodium phosphate buffer (pH 8.0), 500 mM sodium chloride, 10% glycerol, and 500 mM imidazole as elution buffers. Fractions containing leucine dehydrogenase identified by SDS-PAGE were recovered and concentrated using ultrafiltration. Thereafter, buffer substitution to 50 mM potassium phosphate (pH 7.4), 100 mM sodium chloride, 1 mM EDTA, and 10% glycerol was performed using a PD-10 column (manufactured by GE Healthcare). Reconcentration was performed using ultrafiltration, and thereby an aqueous solution of 5.9 mg/mL of leucine dehydrogenase was obtained.

Using the purified leucine dehydrogenase thus obtained, analysis of enzyme kinetics based on the Michaelis-Menten formula using HOB as a substrate was performed. As reaction conditions, 100 mM HEPES/KOH (pH 7.5), 200 mM ammonium chloride, 0.2 mM NADH, and 3.7 µg/mL leucine dehydrogenase were used, and a reaction rate at which a concentration of HOB was 3.13, 6.25, 12.5, 25.0, 50.0, and 100 mM was measured. A reaction rate was calculated by measuring a continuous decrease in absorption by NADH at 340 nm using a Varioskan LUX (manufactured by Thermo Fisher Scientific Inc.). As a result of calculating a constant of the analysis of enzyme kinetics by plotting the reaction rate at each concentration and fitting to the Michaelis-Menten formula, a Michaelis constant K_{M} for HOB was 7.7 mM, and a catalyst rotation speed k_{cat} was 2.7 sec⁻¹. Therefore, a specificity constant k_{cat}/K_{M} was 0.35 mM⁻¹ sec⁻¹.

Based on these results, it was found that leucine dehydrogenase exhibits a catalytic activity for a reductive amination reaction of HOB as expected. Based on these results, it is considered that valine dehydrogenase and phenylalanine dehydrogenase also exhibit a catalytic activity for a reductive amination reaction.

### (4-7) Production of amino acids

### 4-7-(1) Culture of GES1073

Wet bacterial cells obtained by culturing from a glycerol stock of GES1073 in the same manner as in 4-5-(1) were used for the following reaction.

### 4-7-(2) Production of amino acids using GES1073

The wet bacterial cells prepared as described above were suspended in a BT buffer such that a concentration thereof was 10% (w/v). An aqueous solution of 50% (w/v) glucose and an aqueous solution of 2.0 M formaldehyde were respectively added to this bacterial cell suspension such that final concentrations were respectively set to 2% (w/v) and 100 mM, and culture was performed at 33°C while maintaining a pH to 7.0 using an aqueous solution of 5.0 M ammonia. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 6 hours by liquid chromatography for amino acid analysis, 15.7 mM of L-homoserine, 1.3 mM of L-threonine, and 0.15 mM of 2-aminobutyric acid were produced.

### 4-7-(3) Comparative Example 1

The wet bacterial cells prepared as described above were suspended in a BT buffer such that a concentration thereof was 10% (w/v). An aqueous solution of 50% (w/v) glucose was added to this bacterial cell suspension such that a final concentration was set to 2% (w/v), and a reaction was caused at 33°C while maintaining a pH to 7.0 using an aqueous solution of 5.0 M ammonia. When the supernatant of the reaction solution was analyzed after 6 hours by liquid chromatography for amino acid analysis, an amount was below a limit for quantitative determination for all of L-homoserine, L-threonine, and 2-aminobutyric acid.

### 4-7-(4) Comparative Example 2

For GES1295, the same experiments as in 4-7-(1) and 4-7-(2) were performed. When the supernatant of the reaction suspension was analyzed 6 hours after the reaction by liquid chromatography for amino acid analysis, an amount was below a limit for quantitative determination for all of L-homoserine, L-threonine, and 2-aminobutyric acid.

Based on the above results, the following was found: when bacterial cells, which have a gene, such as GES 1073, encoding aldolase and which are capable of producing HOB when being cultured in the presence of saccharides and formaldehyde, further have a gene encoding dehydrogenase having an activity of reductive amination against HOB, HOB is further converted into L-homoserine by culturing these bacterial cells in the presence of saccharides and formaldehyde, and thereby L-homoserine can be produced. In addition, it was also found that L-homoserine can be produced because a part of L-homoserine is converted into L-threonine via O-phospho-L-homoserine (OPH) by an endogenous metabolic system. Furthermore, it was found that L-homoserine can be produced because L-threonine is dehydrated by the endogenous metabolic system and becomes 2-oxobutyric acid, and this 2-oxobutyric acid is converted into 2-aminobutyric acid by endogenous amino acid dehydrogenase or leucine dehydrogenase specifically possessed by GES1073.

### (4-8) Production of L-methionine

### 4-8-(1) Culture of GES1097

Wet bacterial cells obtained by culturing from a glycerol stock of GES1097 in the same manner as in 4-2-(1) were used for the following reaction.

### 4-8-(2) Production of amino acids using GES1097

The wet bacterial cells prepared as described above were suspended in a BT buffer such that a concentration thereof was 20% (w/v). An aqueous solution of 50% (w/v) glucose, an aqueous solution of 2.0 M formaldehyde, and an aqueous solution of 0.2 M methyl mercaptan (which was an aqueous solution obtained by diluting an aqueous solution of 15% methyl mercaptan sodium (manufactured by Tokyo Chemical Industry Co., Ltd.) with a BT buffer, and adjusting a pH to 8.0 using concentrated hydrochloric acid) were added to this bacterial cell suspension, and were further diluted with a BT buffer such that final concentrations were respectively adjusted to 2% (w/v), 100 mM, and 50 mM. In addition, in this case, a final concentration of the wet bacterial cells was set to 10% (w/v). Culture was caused at 33°C while maintaining a pH to 7.0 using an aqueous solution of 5.0 M ammonia. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 21 hours by liquid chromatography for amino acid analysis, 2.1 mM of L-methionine was produced. In addition, 8.6 mM of L-homoserine, 3.0 mM of L-threonine, and 0.86 mM of 2-aminobutyric acid were produced.

### 4-8-(3) Comparative Example 1

The wet bacterial cells prepared as described above were suspended in a BT buffer such that a concentration thereof was 20% (w/v). An aqueous solution of 50% (w/v) glucose and an aqueous solution of 2.0 M formaldehyde were respectively added to this bacterial cell suspension, and were further diluted with a BT buffer such that final concentrations were respectively adjusted to 2% (w/v) and 100 mM. In addition, in this case, a final concentration of the wet bacterial cells was set to 10% (w/v). A reaction was caused at 33°C while maintaining a pH to 7.0 using an aqueous solution of 5 N ammonia. When the supernatant of the reaction solution was analyzed after 21 hours by liquid chromatography for amino acid analysis, an amount of L-methionine was below a limit for quantitative determination. Meanwhile, 5.4 mM of L-homoserine, 5.6 mM of L-threonine, and 0.98 mM of 2-aminobutyric acid were produced.

### 4-8-(4) Comparative Example 2

For GES1073, the same experiments as in 4-8-(1) and 4-8-(2) were performed. When the supernatant of the reaction suspension was analyzed 21 hours after the reaction by liquid chromatography for amino acid analysis, an amount of L-methionine produced was below a limit for quantitative determination. Meanwhile, 12.8 mM of L-homoserine, 1.6 mM of L-threonine, and 0.40 mM of 2-aminobutyric acid were produced.

Based on the above results, the following was found: when bacterial cells, which have a gene, such as GES 1097, encoding aldolase and a gene encoding dehydrogenase having an activity of reductive amination against HOB, and which are capable of producing L-homoserine when being cultured in the presence of saccharides and formaldehyde, further have a gene encoding a synthase having an activity of binding OPH and methyl mercaptan, L-homoserine is converted into L-methionine via OPH by culturing these bacterial cells in the presence of saccharides, formaldehyde, and methyl mercaptan, and thereby L-methionine can be produced.

### 5. Production of HOB from pyruvic acid

### (5-1) Culture of GES1009, GES1119, GES1120, GES1121, GES1122, GES1123, GES1127, GES1154, GES1155, GES1156, and GES1157

Glycerol stocks of GES1009, GES1119, GES1120, GES1121, GES1122, GES1123, GES1127, GES1154, GES1155, GES1156, and GES1157 were inoculated into a A-agar medium containing 25 µg/mL of kanamycin under aseptic conditions, and culture was performed at 33°C. Bacterial cells grown on the agar medium were inoculated into 10 mL of a BA medium containing 25 µg/mL of kanamycin, and shaking culture was performed at 33°C overnight. This culture solution was centrifuged at 5,500 xg at 4°C for 15 minutes to remove the supernatant. The wet bacterial cells thus obtained were used in the following reaction.

### (5-2) Production of HOB from pyruvic acid using GES1009, GES1119, GES1120, GES1121, GES1122, GES1123, GES1127, GES1154, GES1155, GES1156, and GES1157

The wet bacterial cells prepared as described above were suspended in a BS buffer such that a concentration thereof was 10% (w/v). An aqueous solution of 1.0 M pyruvic acid and an aqueous solution of 1.2 M formaldehyde were added together into the bacterial cell suspension such that a final concentration was set to 40 mM, and culture was performed at 33°C. In the culture, agitation was performed so as to efficiently mix the aqueous solution of potassium hydroxide, and aeration was not performed. By this operation, anaerobic conditions or microaerobic conditions were achieved for the above-mentioned concentration of the wet bacterial cells. When the supernatant of the reaction solution was analyzed after 6 hours by liquid chromatography for organic acid analysis, 3.6 mM, 4.1 mM, 2.1 mM, 3.4 mM, 30.5 mM, 1.9 mM, 18.3 mM, 19.5 mM, 6.5 mM, 4.5 mM, and 2.0 mM of HOB were respectively produced in each of the reactions in which GES 1009, GES1119, GES1120, GES1121, GES1122, GES1123, GES1127, GES1154, GES1155, GES1156, and GES1157 were used.

### (5-3) Comparative Example 1

The wet bacterial cells prepared as described above were suspended in a BS buffer such that a concentration thereof was 10% (w/v). An aqueous solution of 1.0 M pyruvic acid was added into the bacterial cell suspension such that a final concentration was set to 40 mM, and a reaction was caused at 33°C. When the supernatant of the reaction solution was analyzed after 6 hours by liquid chromatography for organic acid analysis, HOB was not detected in any of the cases.

Based on the above results, it was found that, when the enzyme catalyzes an aldol reaction between pyruvic acid and formaldehyde, bacterial cells having genes, such as GES1009, GES1119, GES1120, GES1121, GES1122, GES1123, GES1127, GES1154, GES1155, GES1156, and GES1157, which encode aldolase, all produce aldol compounds such as an aldol compound represented by HOB.

### [Reference Documents]

様式第4　　　　　　　　　　　　　　　　　　　　　　　　　　　　　　　　　　　　　　　国際
特許手続上の微生物の寄託の
国際的承認に関するブダペスト条約
下記国際寄託当局によってブダペスト条約に
基づく規則7．1に従い発行される
原寄託についての受託証
BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名(名称)　Green Earth Institute 株式会社　代表取締役　伊原智人　様
DEPOSITOR　Name　Green Earth Institute Co.,Ltd. CEO Tomohito Ihara

住所　〒113-0033
東京都文京区本郷7-3-1　東京大学アントレプレナープラザ
Address　The University of Tokyo Entrepreneur Plaza
7-3-1 Hongo, Bunkyo-ku, Tokyo, 113-0033, Japan

I．微生物の表示　IDENTIFICATION OF THE MICROORGANISM
識別の表示：Identification reference given by the
DEPOSITOR:
GES1068 | 受託番号：Accession number given by the
INTERNATIONAL DEPOSITARY AUTHORITY:
NITE　BP－ 02780
II．科学的性質及び分類学上の位置　SCIENTIFIC DESCRIPTION AND/OR PROPOSED
TAXONOMIC DESIGNATION
I 欄の微生物には、次の事項が記載されていた。：The microorganism identified under I above was accompanied by:
■ 科学的性質　a scientific description
■ 分類学上の位置　a proposed taxonomic designation
III．受領及び受託　RECEIPT AND ACCEPTANCE
本国際寄託当局は、　2018 年 9 月 13日(原寄託日)に受領したＩ欄の微生物を受託した。
This International Depositary Authority accepts the microorganism identified under I above, which was received by it
on　September 13, 2018　(date of the original deposit).
IV．移管請求の受領　RECEIPT OF REQUEST FOR CONVERSION
本国際寄託当局は、I欄の微生物を　　年　　月　　日(国内寄託日)に受託しており、
ブダペスト条約に基づく国際寄託への移管請求を　　年　　月　　日に受領した。
The microorganism identified under I above was received by this International Depositary Authority
on　　　　(date of the original deposit) and a request to convert the original deposit to a deposit under the
Budapest Treaty was received by it on　　　　(date of receipt of request for conversion).
V．国際寄託当局　INTERNATIONAL DEPOSITARY AUTHORITY
独立行政法人製品評価技術基盤機構 特許微生物寄託センター
名称：　NITE Patent Microorganisms Depositary
Name:　National Institute of Technology and Evaluation
センター長　中川　恭好
Yasuyoshi Nakagawa, Director General
住所：　〒292-0818 千葉県木更津市かずさ鎌足2-5-8 122号室
Address:　#122, 2-5-8 Kazusakamatari, Kisarazu-shi, Chiba 292-0818, Japan
発行日：2018 年 10 月 2 日
Date:　October 2, 2018

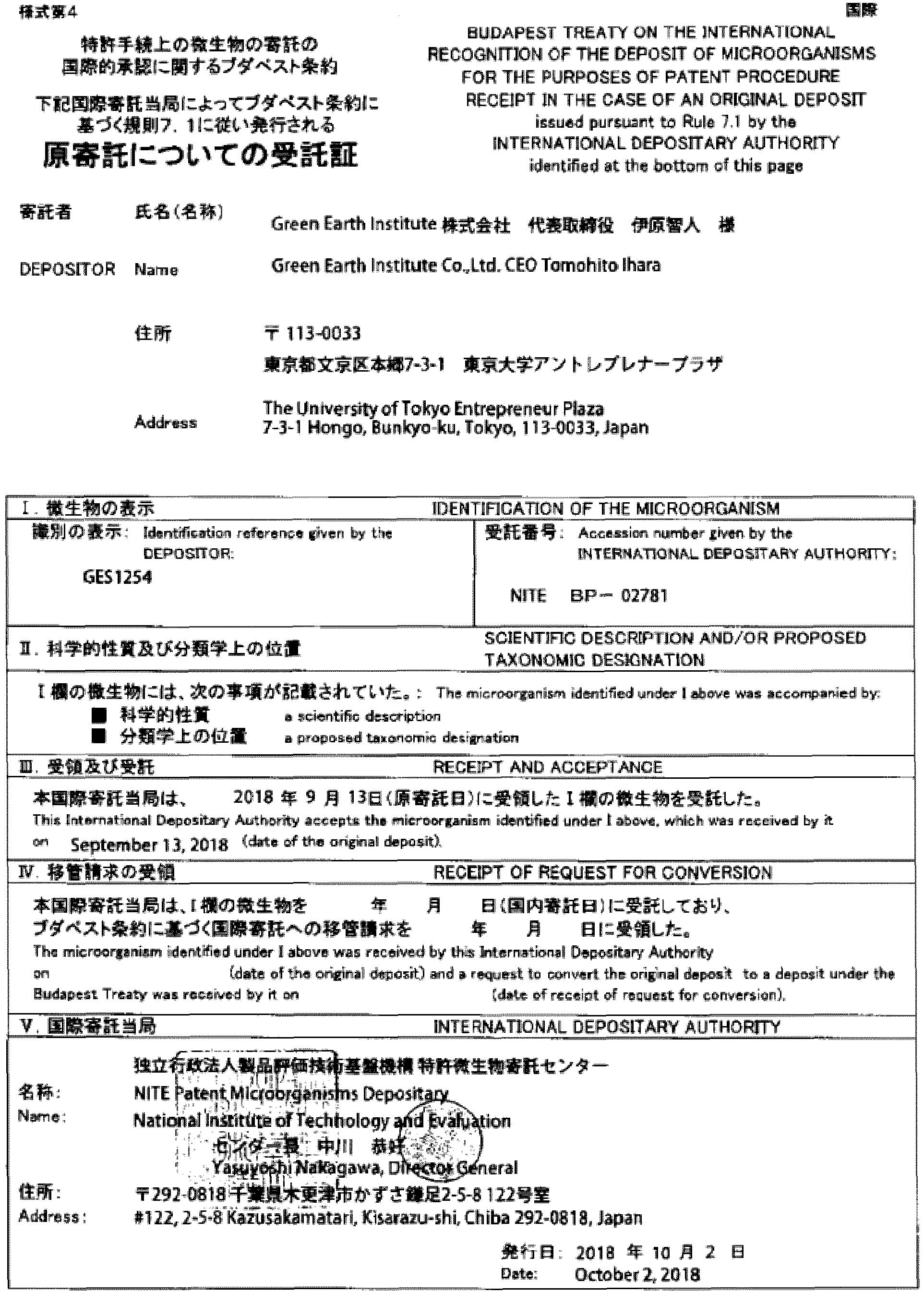
様式第4　　　　　　　　　　　　　　　　　　　　　　　　　　　　　　　　　　　　　　　　　　　　　　　国際

特許手続上の微生物の寄託の
国際的承認に関するブダペスト条約

下記国際寄託当局によってブダペスト条約に
基づく規則7．1に従い発行される
原寄託についての受託証

BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　Green Earth Institute 株式会社　代表取締役　伊原智人　様

DEPOSITOR　Name　Green Earth Institute Co.,Ltd. CEO Tomohito Ihara

住所　〒113-0033
東京都文京区本郷7-3-1　東京大学アントレプレナープラザ

Address　The University of Tokyo Entrepreneur Plaza
7-3-1 Hongo, Bunkyo-ku, Tokyo, 113-0033, Japan

I．微生物の表示 | IDENTIFICATION OF THE MICROORGANISM
識別の表示：Identification reference given by the
DEPOSITOR:
GES1254 | 受託番号：Accession number given by the
INTERNATIONAL DEPOSITARY AUTHORITY:
NITE　BP－02781

II．科学的性質及び分類学上の位置 | SCIENTIFIC DESCRIPTION AND/OR PROPOSED
TAXONOMIC DESIGNATION
I欄の微生物には、次の事項が記載されていた。：The microorganism identified under I above was accompanied by:
■ 科学的性質　a scientific description
■ 分類学上の位置　a proposed taxonomic designation

III．受領及び受託 | RECEIPT AND ACCEPTANCE
本国際寄託当局は、　2018 年 9 月 13日（原寄託日）に受領したI欄の微生物を受託した。
This International Depositary Authority accepts the microorganism identified under I above, which was received by it
on　September 13, 2018　(date of the original deposit).

IV．移管請求の受領 | RECEIPT OF REQUEST FOR CONVERSION
本国際寄託当局は、I欄の微生物を　　年　　月　　日（国内寄託日）に受託しており、
ブダペスト条約に基づく国際寄託への移管請求を　　年　　月　　日に受領した。
The microorganism identified under I above was received by this International Depositary Authority
on　　　　　　(date of the original deposit) and a request to convert the original deposit to a deposit under the
Budapest Treaty was received by it on　　　　　　(date of receipt of request for conversion).

V．国際寄託当局 | INTERNATIONAL DEPOSITARY AUTHORITY
独立行政法人製品評価技術基盤機構 特許微生物寄託センター
名称：　NITE Patent Microorganisms Depositary
Name：　National Institute of Technology and Evaluation
センター長　中川　恭好
Yasuyoshi Nakagawa, Director General
住所：　〒292-0818 千葉県木更津市かずさ鎌足2-5-8 122号室
Address：　#122, 2-5-8 Kazusakamatari, Kisarazu-shi, Chiba 292-0818, Japan

発行日：2018 年 10 月 2 日
Date:　October 2, 2018


様式第4　国際

特許手続上の微生物の寄託の
国際的承認に関するブダペスト条約

下記国際寄託当局によってブダペスト条約に
基づく規則7．1に従い発行される
原寄託についての受託証

BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　Green Earth Institute 株式会社　代表取締役　伊原智人　様

Green Earth Institute Co.,Ltd. CEO Tomohito Ihara
DEPOSITOR　Name

住所　〒113-0033
東京都文京区本郷7-3-1　東京大学アントレプレナープラザ

Address　The University of Tokyo Entrepreneur Plaza
7-3-1 Hongo, Bunkyo-ku, Tokyo, 113-0033, Japan

I．微生物の表示　IDENTIFICATION OF THE MICROORGANISM
識別の表示： Identification reference given by the
DEPOSITOR:
GES1232 | 受託番号： Accession number given by the
INTERNATIONAL DEPOSITARY AUTHORITY:
NITE　BP－ 02782

II．科学的性質及び分類学上の位置　SCIENTIFIC DESCRIPTION AND/OR PROPOSED
TAXONOMIC DESIGNATION
I 欄の微生物には、次の事項が記載されていた。： The microorganism identified under I above was accompanied by:
■ 科学的性質　a scientific description
■ 分類学上の位置　a proposed taxonomic designation

III．受領及び受託　RECEIPT AND ACCEPTANCE
本国際寄託当局は、　2018 年 9 月 13日（原寄託日）に受領した I 欄の微生物を受託した。
This International Depositary Authority accepts the microorganism identified under I above, which was received by it
on　September 13, 2018　(date of the original deposit).

IV．移管請求の受領　RECEIPT OF REQUEST FOR CONVERSION
本国際寄託当局は、I 欄の微生物を　年　月　日（国内寄託日）に受託しており、
ブダペスト条約に基づく国際寄託への移管請求を　年　月　日に受領した。
The microorganism identified under I above was received by this International Depositary Authority
on　(date of the original deposit) and a request to convert the original deposit to a deposit under the
Budapest Treaty was received by it on　(date of receipt of request for conversion).

V．国際寄託当局　INTERNATIONAL DEPOSITARY AUTHORITY
独立行政法人製品評価技術基盤機構 特許微生物寄託センター
名称：　NITE Patent Microorganisms Depositary
Name：　National Institute of Technology and Evaluation
センター長　中川　恭好
Yasuyoshi Nakagawa, Director General
住所：　〒292-0818 千葉県木更津市かずさ鎌足2-5-8 122号室
Address：　#122, 2-5-8 Kazusakamatari, Kisarazu-shi, Chiba 292-0818, Japan

発行日：　2018 年 10 月 2 日
Date:　October 2, 2018

様式第4　　　　　　　　　　　　　　　　　　　　　　　　　　　　　　　　　　　　国際

特許手続上の微生物の寄託の
国際的承認に関するブダペスト条約

下記国際寄託当局によってブダペスト条約に
基づく規則7．1に従い発行される
原寄託についての受託証

BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　Green Earth Institute 株式会社　代表取締役　伊原智人　様

DEPOSITOR　Name　Green Earth Institute Co.,Ltd. CEO Tomohito Ihara

住所　〒113-0033
東京都文京区本郷7-3-1　東京大学アントレプレナープラザ

Address　The University of Tokyo Entrepreneur Plaza
7-3-1 Hongo, Bunkyo-ku, Tokyo, 113-0033, Japan

Ⅰ．微生物の表示 | IDENTIFICATION OF THE MICROORGANISM
識別の表示：Identification reference given by the
DEPOSITOR:
GES1073 | 受託番号：Accession number given by the
INTERNATIONAL DEPOSITARY AUTHORITY:
NITE　BP－02783
Ⅱ．科学的性質及び分類学上の位置 | SCIENTIFIC DESCRIPTION AND/OR PROPOSED
TAXONOMIC DESIGNATION
Ⅰ欄の微生物には、次の事項が記載されていた。：The microorganism identified under I above was accompanied by:
■　科学的性質　　a scientific description
■　分類学上の位置　　a proposed taxonomic designation
Ⅲ．受領及び受託 | RECEIPT AND ACCEPTANCE
本国際寄託当局は、　2018 年 9 月 13日（原寄託日）に受領したⅠ欄の微生物を受託した。
This International Depositary Authority accepts the microorganism identified under I above, which was received by it
on　September 13, 2018　(date of the original deposit).
Ⅳ．移管請求の受領 | RECEIPT OF REQUEST FOR CONVERSION
本国際寄託当局は、Ⅰ欄の微生物を　　年　　月　　日（国内寄託日）に受託しており、
ブダペスト条約に基づく国際寄託への移管請求を　　年　　月　　日に受領した。
The microorganism identified under I above was received by this International Depositary Authority
on　　　　(date of the original deposit) and a request to convert the original deposit to a deposit under the
Budapest Treaty was received by it on　　　　(date of receipt of request for conversion).
Ⅴ．国際寄託当局 | INTERNATIONAL DEPOSITARY AUTHORITY
独立行政法人製品評価技術基盤機構 特許微生物寄託センター
名称：　NITE Patent Microorganisms Depositary
Name:　National Institute of Technology and Evaluation
センター長　中川　恭好
Yasuyoshi Nakagawa, Director General
住所：　〒292-0818 千葉県木更津市かずさ鎌足2-5-8 122号室
Address:　#122, 2-5-8 Kazusakamatari, Kisarazu-shi, Chiba 292-0818, Japan
発行日：2018 年 10 月 2 日
Date:　October 2, 2018

様式第4　　　　　　　　　　　　　　　　　　　　　　　　　　　　　　　　　　　　　　　　　　　　　　　　　　　　　　　　　　　国際
特許手続上の微生物の寄託の
国際的承認に関するブダペスト条約
下記国際寄託当局によってブダペスト条約に
基づく規則7．1に従い発行される
原寄託についての受託証
BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者　氏名（名称）　Green Earth Institute 株式会社　代表取締役　伊原智人　様
Green Earth Institute Co.,Ltd. CEO Tomohito Ihara
DEPOSITOR　Name

住所　〒113-0033
東京都文京区本郷7-3-1　東京大学アントレプレナープラザ
Address　The University of Tokyo Entrepreneur Plaza
7-3-1 Hongo, Bunkyo-ku, Tokyo, 113-0033, Japan

I．微生物の表示　IDENTIFICATION OF THE MICROORGANISM
識別の表示：Identification reference given by the
DEPOSITOR:
GES1097 | 受託番号：Accession number given by the
INTERNATIONAL DEPOSITARY AUTHORITY:
NITE　BP－02784
II．科学的性質及び分類学上の位置　SCIENTIFIC DESCRIPTION AND/OR PROPOSED
TAXONOMIC DESIGNATION
I欄の微生物には、次の事項が記載されていた。：The microorganism identified under I above was accompanied by:
■　科学的性質　a scientific description
■　分類学上の位置　a proposed taxonomic designation
III．受領及び受託　RECEIPT AND ACCEPTANCE
本国際寄託当局は、　2018 年 9 月 13日（原寄託日）に受領したI欄の微生物を受託した。
This International Depositary Authority accepts the microorganism identified under I above, which was received by it
on　September 13, 2018　(date of the original deposit).
IV．移管請求の受領　RECEIPT OF REQUEST FOR CONVERSION
本国際寄託当局は、I欄の微生物を　年　月　日（国内寄託日）に受託しており、
ブダペスト条約に基づく国際寄託への移管請求を　年　月　日に受領した。
The microorganism identified under I above was received by this International Depositary Authority
on　(date of the original deposit) and a request to convert the original deposit to a deposit under the
Budapest Treaty was received by it on　(date of receipt of request for conversion).
V．国際寄託当局　INTERNATIONAL DEPOSITARY AUTHORITY
独立行政法人製品評価技術基盤機構 特許微生物寄託センター
名称：　NITE Patent Microorganisms Depositary
Name:　National Institute of Technology and Evaluation
センター長　中川　恭好
Yasuyoshi Nakagawa, Director General
住所：　〒292-0818 千葉県木更津市かずさ鎌足2-5-8 122号室
Address:　#122, 2-5-8 Kazusakamatari, Kisarazu-shi, Chiba 292-0818, Japan
発行日：2018 年 10 月 2 日
Date:　October 2, 2018

## Claims

1. A method for producing an α-keto acid, the method comprising:
a step of culturing a microorganism containing a gene encoding an enzyme that catalyzes an aldol reaction between pyruvic acid and a carbonyl compound in the presence of a pyruvic acid-supplying compound selected from pyruvic acid and saccharides, and a carbonyl compound represented by General Formula (1) to produce an α-keto acid represented by General Formula (2),
General Formula (1): R₁₀₁C(O)R₁₀₂
where R₁₀₁ and R₁₀₂ are each independently hydrogen or an alkyl group having 1 to 5 carbon atoms, and
General Formula (2): R₁₀₁C(R₁₀₂)(OH)CH₂COCOOH
where R₁₀₁ and R₁₀₂ in General Formula (2) are respectively the same groups as R₁₀₁ and R₁₀₂ in General Formula (1).

2. The method for producing an α-keto acid according to claim 1, wherein the culture is performed under anaerobic conditions or microaerobic conditions.

3. The method for producing an α-keto acid according to claim 1 or 2, wherein the culture is performed in a state in which the microorganism is suspended in a culture solution at a high density.

4. The method for producing an α-keto acid according to any one of claims 1 to 3, wherein, in General Formulas (1) and (2), R₁₀₁ is hydrogen, a methyl group, an ethyl group, an n-propyl group, an isopropyl group, or an n-pentyl group, and R₁₀₂ is hydrogen.

5. A method for producing a 1,3-diol, the method comprising:
a step of culturing a microorganism containing the following genes in the presence of a pyruvic acid-supplying compound selected from pyruvic acid and saccharides, and a carbonyl compound represented by General Formula (3) to produce a 1,3-diol represented by General Formula (4),
(a) first gene encoding an enzyme that catalyzes an aldol reaction between pyruvic acid and a carbonyl compound,
(b) second gene encoding an enzyme that catalyzes a decarboxylation reaction of an α-keto acid, and
(c) third gene encoding an enzyme that catalyzes a reduction reaction of an aldehyde,
General Formula (3): R₁₀₃C(O)R₁₀₄
where R₁₀₃ and R₁₀₄ are each independently hydrogen or an alkyl group having 1 to 5 carbon atoms, provided that in a case where R₁₀₃ is hydrogen, R₁₀₄ is not hydrogen, and
General Formula (4): R₁₀₃C(R₁₀₄)(OH)CH₂CH₂OH
where R₁₀₃ and R₁₀₄ in General Formula (4) are respectively the same groups as R₁₀₃ and R₁₀₄ in General Formula (3).

6. The method for producing a 1,3-diol according to claim 5, wherein, in General Formulas (3) and (4), R₁₀₃ is a methyl group, an ethyl group, an n-propyl group, an isopropyl group, or an n-pentyl group, and R₁₀₄ is hydrogen.

7. A method for producing 1,3-butanediol, the method comprising:
a step of culturing a microorganism containing the following genes in the presence of a pyruvic acid-supplying compound selected from pyruvic acid and saccharides to produce 1,3-butanediol,
(a) first gene encoding an enzyme that catalyzes an aldol reaction between pyruvic acid and a carbonyl compound,
(b) second gene encoding an enzyme that catalyzes a decarboxylation reaction of an α-keto acid, and
(c) third gene encoding an enzyme that catalyzes a reduction reaction of an aldehyde.

8. A method for producing a 2,4-dihydroxycarboxylic acid, the method comprising:
a step of culturing a microorganism containing the following genes in the presence of a pyruvic acid-supplying compound selected from pyruvic acid and saccharides, and a carbonyl compound represented by General Formula (1) to produce a 2,4-dihydroxycarboxylic acid represented by General Formula (5),
(a) first gene encoding an enzyme that catalyzes an aldol reaction between pyruvic acid and a carbonyl compound, and
(d) fourth gene encoding an enzyme that catalyzes a reduction reaction of an α-keto acid,
General Formula (1): R₁₀₁C(O)R₁₀₂
where R₁₀₁ and R₁₀₂ are each independently hydrogen or an alkyl group having 1 to 5 carbon atoms, and
General Formula (5): R₁₀₁C(R₁₀₂)(OH)CH₂CH(OH)COOH
where R₁₀₁ and R₁₀₂ in General Formula (5) are respectively the same groups as R₁₀₁ and R₁₀₂ in General Formula (1).

9. The method for producing a 2,4-dihydroxycarboxylic acid according to claim 8, wherein R₁₀₁ and R₁₀₂ are hydrogen in General Formulas (1) and (5).

10. A method for producing a 2-amino-4-hydroxycarboxylic acid, the method comprising:
a step of reacting an α-keto acid represented by General Formula (2), a nitrogen source, and a reducing agent in the presence of glutamate/phenylalanine/leucine/valine dehydrogenase to produce a 2-amino-4-hydroxycarboxylic acid represented by General Formula (6),
General Formula (2): R₁₀₁C(R₁₀₂)(OH)CH₂COCOOH
where R₁₀₁ and R₁₀₂ are each independently hydrogen or an alkyl group having 1 to 5 carbon atoms, and
General Formula (6): R₁₀₁C(R₁₀₂)(OH)CH₂CH(NH₂)COOH
where R₁₀₁ and R₁₀₂ in General Formula (6) are respectively the same groups as R₁₀₁ and R₁₀₂ in General Formula (2).

11. A method for producing an α-keto acid, the method comprising:
a step of reacting a 2-amino-4-hydroxycarboxylic acid represented by General Formula (6) and an oxidizing agent in the presence of glutamate/phenylalanine/leucine/valine dehydrogenase to produce an α-keto acid represented by General Formula (2),
General Formula (6): R₁₀₁C(R₁₀₂)(OH)CH₂CH(NH₂)COOH
where R₁₀₁ and R₁₀₂ are each independently hydrogen or an alkyl group having 1 to 5 carbon atoms, and
General Formula (2): R₁₀₁C(R₁₀₂)(OH)CH₂COCOOH
where R₁₀₁ and R₁₀₂ in General Formula (2) are respectively the same groups as R₁₀₁ and R₁₀₂ in General Formula (6).

12. A method for producing a 2-amino-4-hydroxycarboxylic acid, the method comprising:
a step of culturing a microorganism containing the following genes in the presence of a pyruvic acid-supplying compound selected from pyruvic acid and saccharides, and a carbonyl compound represented by General Formula (1) to produce a 2-amino-4-hydroxycarboxylic acid represented by General Formula (6),
(a) first gene encoding an enzyme that catalyzes an aldol reaction between pyruvic acid and a carbonyl compound, and
(e) fifth gene encoding an enzyme that catalyzes a reductive amination reaction of an α-keto acid,
General Formula (1): R₁₀₁C(O)R₁₀₂
where R₁₀₁ and R₁₀₂ are each independently hydrogen or an alkyl group having 1 to 5 carbon atoms, and
General Formula (6): R₁₀₁C(R₁₀₂)(OH)CH₂CH(NH₂)COOH
where R₁₀₁ and R₁₀₂ in General Formula (6) are respectively the same groups as R₁₀₁ and R₁₀₂ in General Formula (1).

13. A method for producing at least one of homoserine, threonine, and 2-aminobutyric acid, the method comprising:
a step of culturing a microorganism containing the following genes in the presence of a pyruvic acid-supplying compound selected from pyruvic acid and saccharides, and formaldehyde to produce at least one of homoserine, threonine, and 2-aminobutyric acid,
(a) first gene encoding an enzyme that catalyzes an aldol reaction between pyruvic acid and a carbonyl compound, and
(e) fifth gene encoding an enzyme that catalyzes a reductive amination reaction of an α-keto acid.

14. A method for producing a 2-amino-4-methylthiocarboxylic acid, the method comprising:
a step of culturing a microorganism containing the following genes in the presence of a pyruvic acid-supplying compound selected from pyruvic acid and saccharides, methyl mercaptan, and a carbonyl compound represented by General Formula (1) to produce a 2-amino-4-methylthiocarboxylic acid represented by General Formula (7),
(a) first gene encoding an enzyme that catalyzes an aldol reaction between pyruvic acid and a carbonyl compound,
(e) fifth gene encoding an enzyme that catalyzes a reductive amination reaction of an α-keto acid, and
(f) sixth gene encoding an enzyme that catalyzes a reaction of substituting a phosphoric acid group contained in 2-amino-4-phosphonooxycarboxylic acid with a methylmercapto group contained in methyl mercaptan,
General Formula (1): R₁₀₁C(O)R₁₀₂
where R₁₀₁ and R₁₀₂ are each independently hydrogen or an alkyl group having 1 to 5 carbon atoms, and
General Formula (7): R₁₀₁C(R₁₀₂)(SCH₃)CH₂CH(NH₂)COOH
where R₁₀₁ and R₁₀₂ in General Formula (7) are respectively the same groups as R₁₀₁ and R₁₀₂ in General Formula (1).

15. The method for producing a 2-amino-4-methylthiocarboxylic acid according to claim 14, wherein R₁₀₁ and R₁₀₂ are hydrogen in General Formulas (1) and (7).

16. The method according to any one of claims 1 to 9 and 12 to 15, wherein the microorganism is an aerobic bacterium.

17. The method according to claim 16, wherein the aerobic bacterium is a coryneform bacterium.

18. A coryneform bacterium comprising:
a first gene selected from the group consisting of the following genes,
(1-1) gene encoding a protein that consists of an amino acid sequence set forth in SEQ ID NO: 2 and catalyzes an aldol reaction between pyruvic acid and a carbonyl compound,
(1-2) gene encoding a protein that consists of an amino acid sequence in which one or a plurality of amino acids is deleted, substituted, or added in SEQ ID NO: 2, and has an activity of catalyzing an aldol reaction between pyruvic acid and a carbonyl compound,
(1-3) gene encoding a protein that consists of an amino acid sequence set forth in SEQ ID NO: 4 and catalyzes an aldol reaction between pyruvic acid and a carbonyl compound,
(1-4) gene encoding a protein that consists of an amino acid sequence in which one or a plurality of amino acids is deleted, substituted, or added in SEQ ID NO: 4, and has an activity of catalyzing an aldol reaction between pyruvic acid and a carbonyl compound,
(1-5) gene encoding a protein that consists of an amino acid sequence set forth in SEQ ID NO: 6 and catalyzes an aldol reaction between pyruvic acid and a carbonyl compound, and
(1-6) gene encoding a protein that consists of an amino acid sequence in which one or a plurality of amino acids is deleted, substituted, or added in SEQ ID NO: 6, and has an activity of catalyzing an aldol reaction between pyruvic acid and a carbonyl compound.

19. The coryneform bacterium according to claim 18, further comprising:
a second gene selected from the group consisting of the following genes,
(2-1) gene encoding a protein that consists of an amino acid sequence set forth in SEQ ID NO: 24 and catalyzes a decarboxylation reaction of an α-keto acid, and
(2-2) gene encoding a protein that consists of an amino acid sequence in which one or a plurality of amino acids is deleted, substituted, or added in SEQ ID NO: 24, and has an activity of catalyzing a decarboxylation reaction of an α-keto acid; and
a third gene selected from the group consisting of the following genes,
(3-1) gene encoding a protein that consists of an amino acid sequence set forth in SEQ ID NO: 26 and catalyzes a reduction reaction of an aldehyde, and
(3-2) gene encoding a protein that consists of an amino acid sequence in which one or a plurality of amino acids is deleted, substituted, or added in SEQ ID NO: 26, and has an activity of catalyzing a reduction reaction of an aldehyde.

20. The coryneform bacterium according to claim 18, further comprising:
a fourth gene selected from the group consisting of the following genes,
(4-1) gene encoding a protein that consists of an amino acid sequence set forth in SEQ ID NO: 28 and catalyzes a reduction reaction of an α-keto acid, and
(4-2) gene encoding a protein that consists of an amino acid sequence in which one or a plurality of amino acids is deleted, substituted, or added in SEQ ID NO: 28, and has an activity of catalyzing a reduction reaction of an α-keto acid.

21. The coryneform bacterium according to claim 18, further comprising:
a fifth gene selected from the group consisting of the following genes,
(5-1) gene encoding a protein that consists of an amino acid sequence set forth in SEQ ID NO: 30 and catalyzes a reductive amination reaction of an α-keto acid,
(5-2) gene encoding a protein that consists of an amino acid sequence in which one or a plurality of amino acids is deleted, substituted, or added in SEQ ID NO: 30, and has an activity of catalyzing a reductive amination reaction of an α-keto acid,
(5-3) gene encoding a protein that consists of an amino acid sequence set forth in SEQ ID NO: 32 and catalyzes a reductive amination reaction of an α-keto acid,
(5-4) gene encoding a protein that consists of an amino acid sequence in which one or a plurality of amino acids is deleted, substituted, or added in SEQ ID NO: 32, and has an activity of catalyzing a reductive amination reaction of an α-keto acid,
(5-5) gene encoding a protein that consists of an amino acid sequence set forth in SEQ ID NO: 34 and catalyzes a reductive amination reaction of an α-keto acid, and
(5-6) gene encoding a protein that consists of an amino acid sequence in which one or a plurality of amino acids is deleted, substituted, or added in SEQ ID NO: 34, and has an activity of catalyzing a reductive amination reaction of an α-keto acid.

22. The coryneform bacterium according to claim 18, further comprising:
a fifth gene selected from the group consisting of the following genes,
(5-1) gene encoding a protein that consists of an amino acid sequence set forth in SEQ ID NO: 30 and catalyzes a reductive amination reaction of an α-keto acid,
(5-2) gene encoding a protein that consists of an amino acid sequence in which one or a plurality of amino acids is deleted, substituted, or added in SEQ ID NO: 30, and has an activity of catalyzing a reductive amination reaction of an α-keto acid,
(5-3) gene encoding a protein that consists of an amino acid sequence set forth in SEQ ID NO: 32 and catalyzes a reductive amination reaction of an α-keto acid,
(5-4) gene encoding a protein that consists of an amino acid sequence in which one or a plurality of amino acids is deleted, substituted, or added in SEQ ID NO: 32, and has an activity of catalyzing a reductive amination reaction of an α-keto acid,
(5-5) gene encoding a protein that consists of an amino acid sequence set forth in SEQ ID NO: 34 and catalyzes a reductive amination reaction of an α-keto acid, and
(5-6) gene encoding a protein that consists of an amino acid sequence in which one or a plurality of amino acids is deleted, substituted, or added in SEQ ID NO: 34, and has an activity of catalyzing a reductive amination reaction of an α-keto acid; and
a sixth gene selected from the group consisting of the following genes,
(6-1) gene encoding a protein that consists of an amino acid sequence set forth in SEQ ID NO: 36 and catalyzes a reaction of substituting a phosphoric acid group contained in 2-amino-4-phosphonooxycarboxylic acid with a methylmercapto group contained in methyl mercaptan, and
(6-2) gene encoding a protein that consists of an amino acid sequence in which one or a plurality of amino acids is deleted, substituted, or added in SEQ ID NO: 36, and has an activity of catalyzing a reaction of substituting a phosphoric acid group contained in 2-amino-4-phosphonooxycarboxylic acid with a methylmercapto group contained in methyl mercaptan.
